# EUROPEAN PATENT SPECIFICATION

(11) **EP 2 698 062 B1**
(45) Date of publication and mention of the grant of the patent: **24.06.2015**
(21) Application number: 13191894.8
(22) Date of filing: 20.12.2007
(51) Int. Cl.: A01N 43/58, A61K 31/50, A61K 31/495, C07D 237/32, C07D 403/10, C07D 403/12, C07D 403/14

(54) **Inhibitors of poly(adp-ribose)polymerase**
Hemmer von Poly(ADP-ribose)-Polymerase
Inhibiteurs de la poly(adp-ribose)polymérase

(30) Priority: 28.12.2006 US 882317 P
(43) Date of publication of application: 19.02.2014
(62) Divisional of application: 07869628.3
(73) Proprietor: Abbvie Inc., North Chicago, IL 60064 (US)
(72) Inventor: Gandhi, Viraj B., Gurnee, IL Illinois 60031 (US); Giranda, Vincent L., Gurnee, IL Illinois 60031 (US); Gong, Jianchun, Deerfield, IL Illinois 60015 (US); Penning, Thomas D., Elmhurst, IL Illinois 60126 (US); Zhu, Gui-dong, Gurnee, IL Illinois 60031 (US)
(74) Representative: Modiano, Micaela Nadia

(56) References cited:
- US-A1- 2005 085 476
- US-A1- 2006 149 059

## Description

### FIELD OF THE INVENTION

This invention relates to inhibitors of poly(ADP-ribose)polymerase, ways to make them and methods of treating patients using them.

### BACKGROUND OF THE INVENTION

Poly(ADP-ribose)polymerase (PARP) is essential for facilitating DNA repair, controlling RNA transcription, mediating cell death and regulating immune response. This activity makes PARP inhibitors targets for a number of disorders. PARP inhibitors have shown utility for treating diseases such as ischemia reperfusion injury, inflammatory disease, retroviral infections, ischemia reperfusion injury, myocardial infarction, stroke and other neural trauma, organ transplantation, reperfusion of the eye, kidney, gut and skeletal muscle, arthritis, gout, inflammatory bowel disease, CNS inflammation such as MS and allergic encephalitis, sepsis, septic shock, hemmorhagic shock, pulmonary fibrosis, and uveitis, diabetes and Parkinsons disease, liver toxicity following acetominophen overdose, cardiac and kidney toxicities from doxorubicin and platinum-based antineoplastic agents and skin damage secondary to sulfur mustards. PARP inhibitors have also been shown to potentiate radiation and chemotherapy by increasing cell death of cancer cells, limiting tumor growth, decreasing metastasis, and prolonging the survival of tumor-bearing animals.
US 2002/0183325 A1 describes phtalazinone derivatives as PARP inhibitors. US 2004/0023968 A1 describes phtalazinone derivatives as PARP inhibitors. US 2005/0085476 A1 describes fused pyridazine derivatives as PARP inhibitors. US 2005/0059663 A1 describes phtalazinone derivatives as PARP inhibitors. US 2006/0063767 A1 describes phtalazinone derivatives as PARP inhibitors. US 2006/0142293 A1 describes phtalazinone derivatives as PARP inhibitors. US 2006/0149059 A1 describes phtalazinone derivatives as PARP inhibitors. US 2007/0093489 A1 describes phtalazinone derivatives as PARP inhibitors.
There is therefore a need in the therapeutic arts for PARP inhibitors. Such compounds can be used to treat subjects suffering from cancer, and can further expand the range of treatment options available for such subjects.

### SUMMARY OF THE INVENTION

One embodiment of this invention, therefore, pertains to compounds that inhibit the activity of poly(ADP-ribose) polymerase and have formula I 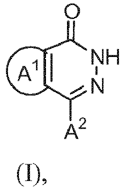 and pharmaceutically acceptable salts thereof, wherein
A¹ is R¹ or R², wherein A¹ is unsubstituted or substituted with one or two OH, CN, C₁-alkyl, C₂-alkyl, C₃-alkyl, C₄-alkyl, C₅-alkyl, cycloalkane, OR^{A} or NR^{A}R^{A};
R^{A} is H or alkyl;
R¹ is cycloalkane or cycloalkene each of which is unfused or fused with R^{1A};
R^{1A} is benzene, heteroarene, cycloalkane, cycloalkene, heterocycloalkane or heterocycloalkene;
R² is heterocycloalkane or heterocycloalkene; each of which is unfused or fused with R^{2A};
R^{2A} is benzene, heteroarene, cycloalkane, cycloalkene, heterocycloalkane or heterocycloalkene;
A² is OR⁴, NHR⁴, N(R⁴)₂, SR⁴, S(O)R⁴, SO₂R⁴ or R⁵;
wherein each R⁴ is C₁-alkyl, C₂-alkyl or C₃-alkyl; each of which is substituted with R¹⁰;
R⁵ is C₁-alkyl, C₂-alkyl, C₃-alkyl, C₄-alkyl or C₅-alkyl; each of which is substituted with R¹⁰, and further unsubstituted or substituted with one or two or three of independently selected OR¹⁰, NHR¹⁰, N(R¹⁰)₂, SR¹⁰, S(O)R¹⁰, SO₂R¹⁰ or CF₃;
wherein each R¹⁰ is R^{10A}, R^{10B} or R^{10C}; each of which must be attached at a carbon atom;
R^{10A} is phenyl which is unfused or fused with benzene, heteroarene, cycloalkane, cycloalkene, heterocycloalkane or heterocycloalkene; each of which are unfused or fused with benzene, heteroarene, cycloalkane, cycloalkene, heterocycloalkane or heterocycloalkene;
R^{10B} is 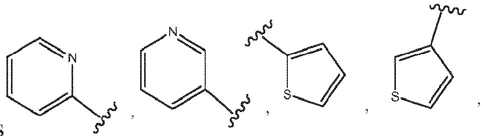 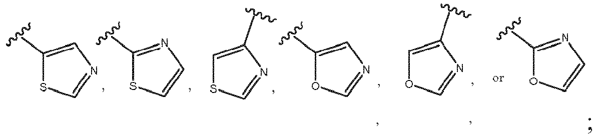 each of which is unfused or fused with benzene, heteroarene, cycloalkane, cycloalkene, heterocycloalkane or heterocycloalkene; each of which are unfused or fused with benzene, heteroarene, cycloalkane, cycloalkene, heterocycloalkane or heterocycloalkene;
R^{10C} is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene;
wherein each R¹⁰ is independently unsubstituted or substituted with one or two or three of independently selected, R¹¹, OR¹¹, SR¹¹, S(O)R¹¹, SO₂R¹¹, NH₂, NHR¹¹, N(R¹¹)₂, C(O)R¹¹, C(O)OR¹¹, C(O)NH₂, C(O)NHR¹¹, C(O)N(R¹¹)₂, NHC(O)R¹¹, NR¹¹C(O)R¹¹, NHSO₂R¹¹, NR¹¹SO₂R¹¹, NHC(O)OR¹¹, NR¹¹C(O)OR¹¹, NHSO₂NH₂, NHSO₂NHR¹¹, NHSO₂N(R¹¹)₂, SO₂NH₂ SO₂NHR¹¹, SO₂N(R¹¹)₂, NHC(O)NH₂, NHC(O)NHR¹¹, NHC(O)N(R¹¹)₂, NR¹¹C(O)N(R¹¹)₂, NO₂, OH, (O), C(O)H, C(O)OH, CN, CF₃, OCF₃, CF₂CF₃, F, Cl, Br or I;
wherein each R¹¹ is R¹², R¹³, R¹⁴ or R¹⁵;
R¹² is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R¹³ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R¹⁴ is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R¹⁵ is alkyl, alkenyl or alkynyl; each of which is unsubstituted or substituted with one or two of independently selected R¹⁶, OR¹⁶, SR¹⁶, S(O)₂R¹⁶, C(O)OH, NH₂,NHR¹⁶ N(R¹⁶)₂, C(O)R¹⁶, C(O)NH₂, C(O)NHR¹⁶, C(O)N(R¹⁶)₂, NHC(O)R¹⁶, NR¹⁶C(O)R¹⁶, NHC(O)OR¹⁶, NR¹⁶C(O)OR¹⁶, OH, F, Cl, Br or I;
wherein each R¹⁶ is R¹⁷ or R^{17A};
R¹⁷ is alkyl, alkenyl or alkynyl; each of which is unsubstituted or substituted with one or two of independently selected R¹⁸, C(O)OH, NH₂, NHR¹⁸ or N(R¹⁸)₂, C(O)R¹⁸, C(O)NH₂, C(O)NHR¹⁸, C(O)N(R¹⁸)₂, NHC(O)R¹⁸, NR¹⁸C(O)R¹⁸, F, Cl, Br or I;
R^{17A} is phenyl, heteroaryl, cycloalkyl, cycloalkenyl, heterocycloalkyl or heterocycloalkenyl, each of which is unfused or fused with benzene, heteroarene, cycloalkane, cycloalkene, heterocycloalkane or heterocycloalkene;
wherein each R¹⁸ is phenyl, heteroaryl, cycloalkyl, cycloalkenyl, heterocycloalkyl or heterocycloalkenyl;
wherein each of the moieties represented R¹², R¹³, R¹⁴, R^{17A}, and R¹⁸ are independently unsubstituted or substituted with one or two or three or four of independently selected R¹⁹, OR¹⁹, SR¹⁹, S(O)R¹⁹, SO₂R¹⁹, C(O)R¹⁹, CO(O)R¹⁹, OC(O)R¹⁹, OC(O)OR¹⁹, NH₂, NHR¹⁹,N(R¹⁹)₂, NHC(O)R¹⁹, NR¹⁹C(O)R¹⁹, NHS(O)₂R¹⁹, NR¹⁹S(O)₂R¹⁹, NHC(O)OR¹⁹, NR¹⁹C(O)OR¹⁹, NHC(O)NH₂, NHC(O)NHR¹⁹, NHC(O)N(R¹⁹)₂, NR¹⁹C(O)NHR¹⁹, NR¹⁹C(O)N(R¹⁹)₂, C(O)NH₂, C(O)NHR¹⁹, C(O)N(R¹⁹)₂, C(O)NHOH, C(O)NHOR¹⁹, C(O)NHSO₂R¹⁹, C(O)NR¹⁹SO₂R¹⁹, SO₂NH₂, SO₂NHR¹⁹, SO₂N(R¹⁹)₂, C(O)H, C(O)OH, C(N)NH₂, C(N)NHR¹⁹, C(N)N(R¹⁹)₂, CNOH, CNOCH₃ OH, (O), CN, N₃, NO₂, CF₃, CF₂CF₃, OCF₃, OCF₂CF₃, F, Cl, Br or I;
wherein each R¹⁹ is R²⁰, R²¹, R²² or R²³;
R²⁰ is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R²¹ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R²² is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R²³ is alkyl, alkenyl or alkynyl; each of which is unsubstituted or substituted with one or two of independently selected R²⁴, OR²⁴, SR²⁴, S(O)₂R²⁴, C(O)OH, NH₂, NHR²⁴ N(R²⁴)₂, C(O)R²⁴, C(O)NH₂, C(O)NHR²⁴, C(O)N(R²⁴)₂, NHC(O)R²⁴, NR²⁴C(O)R²⁴, NHC(O)OR²⁴, NR²⁴C(O)OR²⁴, NHS(O)₂R²⁴, NR²⁴S(O)₂R²⁴, OH, F, Cl, Br or I;
wherein each R²⁴ is R^{24A} or R^{24B};
R^{24A} is phenyl, heteroaryl, cycloalkyl, cycloalkenyl, heterocycloalkyl or heterocycloalkenyl each of which is unfused or fused with benzene, heteroarene, cycloalkane, cycloalkene, heterocycloalkane or heterocycloalkene;
R^{24B} is alkyl, alkenyl or alkynyl each of which is unsubstituted or substituted with one or two of independently selected R²⁵, OR²⁵, SR²⁵, S(O)₂R²⁵, C(O)OH, NH₂, NHR²⁵ N(R²⁵)₂, C(O)R²⁵, C(O)NH₂, C(O)NHR²⁵, C(O)N(R²⁵)₂, NHC(O)R²⁵, NR²⁵C(O)R²⁵, NHC(O)OR²⁵, NR²⁵C(O)OR²⁵, OH, F, Cl, Br or I;
wherein each R²⁵ is alkyl, phenyl, heteroaryl, cycloalkyl, cycloalkenyl, heterocycloalkyl or heterocycloalkenyl ; each of which is unsubstituted or substituted with NH₂, NH(CH₃), N(CH₃)₂, OH or OCH₃;
wherein each of the moieties represented by R²⁰, R²¹, R²², and R^{24A} are independently unsubstituted or substituted with one or two of independently selected R²⁶, OR²⁶, alkenyl, alkynyl, phenyl, OH, (O), C(O)OH, CN, CF₃, OCF₃, CF₂CF₃, F, Cl, Br or I; and
R²⁶ is alkyl.

Still another embodiment comprises pharmaceutical compositions comprising a compound having formula I and an excipient.

Still another embodiment comprises methods of inhibiting PARP in a mammal comprising administering thereto a therapeutically acceptable amount of a compound having formula I.

Still another embodiment comprises methods of treating cancer in a mammal comprising administering thereto a therapeutically acceptable amount of a compound having formula I 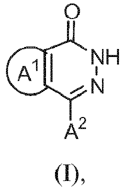 or a salt thereof, wherein
A¹ is R¹ or R², wherein A¹ is unsubstituted or substituted with one or two OH, CN, C₁-alkyl, C₂-alkyl, C₃-alkyl, C₄-alkyl, C₅-alkyl, cycloalkane, OR^{A} or NR^{A}R^{A};
R^{A} is H or alkyl;
R¹ is cycloalkane or cycloalkene each of which is unfused or fused with R^{1A};
R^{1A} is benzene, heteroarene, cycloalkane, cycloalkene, heterocycloalkane or heterocycloalkene;
R² is heterocycloalkane or heterocycloalkene; each of which is unfused or fused with R^{2A};
R^{2A} is benzene, heteroarene, cycloalkane, cycloalkene, heterocycloalkane or heterocycloalkene;
A² is OR⁴, NHR⁴, N(R⁴)₂, SR⁴, S(O)R⁴, SO₂R⁴ or R⁵;
wherein each R⁴ is C₁-alkyl, C₂-alkyl or C₃-alkyl; each of which is substituted with R¹⁰;
R⁵ is C₁-alkyl, C₂-alkyl, C₃-alkyl, C₄-alkyl or C₅-alkyl; each of which is substituted with R¹⁰, and further unsubstituted or substituted with one or two or three of independently selected OR¹⁰, NHR¹⁰, N(R¹⁰)₂, SR¹⁰, S(O)R¹⁰, SO₂R¹⁰ or CF₃;
wherein each R¹⁰ is R^{10A}, R^{10B} or R^{10C}; each of which must be attached at a carbon atom;
R^{10A} is phenyl which is unfused or fused with benzene, heteroarene, cycloalkane, cycloalkene, heterocycloalkane or heterocycloalkene; each of which are unfused or fused with benzene, heteroarene, cycloalkane, cycloalkene, heterocycloalkane or heterocycloalkene;
R^{10B} is 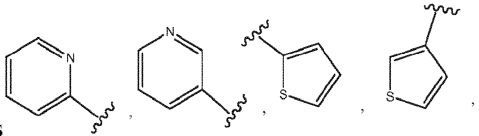 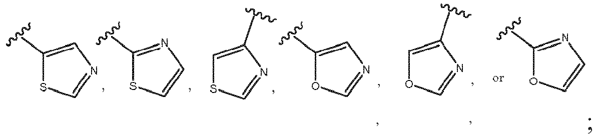 each of which is unfused or fused with benzene, heteroarene, cycloalkane, cycloalkene, heterocycloalkane or heterocycloalkene; each of which are unfused or fused with benzene, heteroarene, cycloalkane, cycloalkene, heterocycloalkane or heterocycloalkene;
R^{10C} is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene;
wherein each R¹⁰ is independently unsubstituted or substituted with one or two or three of independently selected, R¹¹, OR¹¹, SR¹¹, S(O)R¹¹, SO₂R¹¹, NH₂, NHR¹¹, N(R¹¹)₂, C(O)R¹¹, C(O)OR¹¹, C(O)NH₂, C(O)NHR¹¹, C(O)N(R¹¹)₂, NHC(O)R¹¹, NR¹¹C(O)R¹¹, NHSO₂R¹¹, NR¹¹SO₂R¹¹, NHC(O)OR¹¹, NR¹¹C(O)OR¹¹, NHSO₂NH₂, NHSO₂NHR¹¹, NHSO₂N(R¹¹)₂, SO₂NH₂, SO₂NHR¹¹, SO₂N(R¹¹)₂, NHC(O)NH₂, NHC(O)NHR¹¹, NHC(O)N(R¹¹)₂, NR¹¹C(O)N(R¹¹)₂, NO₂, OH, (O), C(O)H, C(O)OH, CN, CF₃, OCF₃, CF₂CF₃, F, Cl, Br or I;
wherein each R¹¹ is R¹², R¹³, R¹⁴ or R¹⁵;
R¹² is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R¹³ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R¹⁴ is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R¹⁵ is alkyl, alkenyl or alkynyl; each of which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NH₂, C(O)NHR¹⁶, C(O)N(R¹⁶)₂, NHC(O)R¹⁶, NR¹⁶C(O)R¹⁶, NHC(O)OR¹⁶, NR¹⁶C(O)OR¹⁶, OH, F, Cl, Br or I;
wherein each R¹⁶ is R¹⁷ or R^{17A};
R¹⁷ is alkyl, alkenyl or alkynyl; each of which is unsubstituted or substituted with one or two of independently selected R¹⁸, C(O)OH, NH₂, NHR¹⁸ or N(R¹⁸)₂, C(O)R¹⁸, C(O)NH₂, C(O)NHR¹⁸, C(O)N(R¹⁸)₂, NHC(O)R¹⁸, NR¹⁸C(O)R¹⁸, F, Cl, Br or I;
R^{17A} is phenyl, heteroaryl, cycloalkyl, cycloalkenyl, heterocycloalkyl or heterocycloalkenyl, each of which is unfused or fused with benzene, heteroarene, cycloalkane, cycloalkene, heterocycloalkane or heterocycloalkene;
wherein each R¹⁸ is phenyl, heteroaryl, cycloalkyl, cycloalkenyl, heterocycloalkyl or heterocycloalkenyl;
wherein each of the moieties represented by R¹², R¹³, R¹⁴, R^{17A}, and R¹⁸ are independently unsubstituted or substituted with one or two or three or four of independently selected R¹⁹, OR¹⁹, SR¹⁹, S(O)R¹⁹, SO₂R¹⁹, C(O)R¹⁹, CO(O)R¹⁹, OC(O)R¹⁹, OC(O)OR¹⁹, NH₂, NHR¹⁹, N(R¹⁹)₂, NHC(O)R¹⁹, NR¹⁹C(O)R¹⁹, NHS(O)₂R¹⁹, NR¹⁹S(O)₂R¹⁹, NHC(O)OR¹⁹, NR¹⁹C(O)OR¹⁹, NHC(O)NH₂, NHC(O)NHR¹⁹, NHC(O)N(R¹⁹)₂, NR¹⁹C(O)NHR¹⁹, NR¹⁹C(O)N(R¹⁹)₂, C(O)NH₂, C(O)NHR¹⁹, C(O)N(R¹⁹)₂, C(O)NHOH, C(O)NHOR¹⁹, C(O)NHSO₂R¹⁹, C(O)NR¹⁹SO₂R¹⁹, SO₂NH₂, SO₂NHR¹⁹, SO₂N(R¹⁹)₂, C(O)H, C(O)OH, C(N)NH₂, C(N)NHR¹⁹, C(N)N(R¹⁹)₂, CNOH, CNOCH₃, OH, (O), CN, N₃, NO₂, CF₃, CF₂CF₃, OCF₃, OCF₂CF₃, F, Cl, Br or I;
wherein each R¹⁹ is R²⁰, R²¹, R²² or R²³;
R²⁰ is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R²¹ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R²² is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R²³ is alkyl, alkenyl or alkynyl; each of which is unsubstituted or substituted with one or two of independently selected R²⁴, OR²⁴, SR²⁴, S(O)₂R²⁴, C(O)OH, NH₂, NHR²⁴ N(R²⁴)₂, C(O)R²⁴, C(O)NH₂, C(O)NHR²⁴, C(O)N(R²⁴)₂, NHC(O)R²⁴, NR²⁴C(O)R²⁴, NHC(O)OR²⁴, NR²⁴C(O)OR²⁴, NHS(O)₂R²⁴, NR²⁴S(O)₂R²⁴, OH, F, Cl, Br or I;
wherein each R²⁴ is ^{24A} or R^{24B};
R^{24A} is phenyl, heteroaryl, cycloalkyl, cycloalkenyl, heterocycloalkyl or heterocycloalkenyl each of which is unfused or fused with benzene, heteroarene, cycloalkane, cycloalkene, heterocycloalkane or heterocycloalkene;
R^{24B} is alkyl, alkenyl or alkynyl each of which is unsubstituted or substituted with one or two of independently selected R²⁵, OR²⁵, SR²⁵, S(O)₂R²⁵, C(O)OH, NH₂, NHR²⁵ N(R²⁵)₂, C(O)R²⁵, C(O)NH₂, C(O)NHR²⁵, C(O)N(R²⁵)₂, NHC(O)R²⁵, NR²⁵C(O)R²⁵, NHC(O)OR²⁵, NR²⁵C(O)OR²⁵, OH, F, Cl, Br or I;
wherein each R²⁵ is alkyl, phenyl, heteroaryl, cycloalkyl, cycloalkenyl, heterocycloalkyl or heterocycloalkenyl ; each of which is unsubstituted or substituted with NH₂, NH(CH₃), N(CH₃)₂, OH or OCH₃;
wherein each of the moieties represented by R²⁰, R²¹, R²², and R^{24A} are independently unsubstituted or substituted with one or two of independently selected R²⁶, OR²⁶, alkenyl, alkynyl, phenyl, OH, (O), C(O)OH, CN, CF₃, OCF₃, CF₂CF₃, F, Cl, Br or I; and
R²⁶ is alkyl.

Still another embodiment comprises methods for decreasing tumor volume in a mammal comprising administering thereto a therapeutically acceptable amount of a compound having formula I

Still another embodiment comprises the use of a compound of Formula I for the preparation of a medicament for the treatment of cancer.

Still another embodiment comprises a method of treating leukemia, colon cancer, glioblastomas, lymphomas, melanomas, carcinomas of the breast or cervical carcinomas in a mammal comprising administering thereto a therapeutically acceptable amount of a compound having formula I.

Still another embodiment comprises the use of a compound of Formula I for the preparation of a medicament for the treatment of leukemia, colon cancer, glioblastomas, lymphomas, melanomas, carcinomas of the breast or cervical carcinomas.

Still another embodiment comprises methods for potentiation of cytotoxic cancer therapy in a mammal comprising administering thereto a therapeutically acceptable amount of a compound having formula I.

Still another embodiment comprises methods for potentiation of radiation therapy in a mammal comprising administering thereto a therapeutically acceptable amount of a compound having formula I.

Still another embodiment comprises methods of treating ischemia reperfusion injury associated with myocardial infarction, stroke, neural trauma or organ transplantation in a mammal comprising administering thereto a therapeutically acceptable amount of a compound having formula 1.

Still another embodiment comprises methods of treating reperfusion of the eye, kidney, gut or skeletal muscle in a mammal comprising administering thereto a therapeutically acceptable amount of a compound having formula I.

Still another embodiment comprises methods of treatingarthritis, gout, inflammatory bowel disease, CNS inflammation, multiple sclerosis, allergic encephalitis, sepsis, septic shock, hemmorhagic shock, pulmonary fibrosis or uveitis in a mammal comprising administering thereto a therapeutically acceptable amount of a compound having formula I.

Still another embodiment comprises a method of treating rheumatoid arthritis or septic shock in a mammal comprising administering thereto a therapeutically acceptable amount of a compound having formula I.

Still another embodiment comprises methods of treating diabetes or Parkinsons disease in a mammal comprising administering thereto a therapeutically acceptable amount of a compound having formula I.

Still another embodiment comprises methods of treating hypoglycemia in a mammal comprising administering thereto a therapeutically acceptable amount of a compound having formula I.

Still another embodiment comprises methods of treating retroviral infection in a mammal comprising administering thereto a therapeutically acceptable amount of a compound having formula I.

Still another embodiment comprises methods of treating liver toxicity following acctominophcn overdose in a mammal comprising administering thereto a therapeutically acceptable amount of a compound having formula I.

Still another embodiment comprises a method of treating cardiac or kidney toxicities from doxorubicin or platinum based antineoplastic agents in a mammal comprising administering thereto a therapeutically acceptable amount of a compound having formula I.

Still another embodiment comprises methods of treating skin damage secondary to sulfur mustards in a mammal comprising administering thereto a therapeutically acceptable amount of a compound having formula I.

Still another embodiment comprises the compounds
2-fluoro-5-((4-oxo-3,4,5,6,7,8-hexahydrophthalazin-1-yl)methyl)benzoic acid; 4-(3-amino-4-fluorobenzyl)-5,6,7,8-tetrahydrophthalazin-1(2H)-one;
4-((2-fluoro-5-((4-oxo-3,4,5,6,7,8-hexahydrophthalazin-1-yl)methyl)phenyl)amino)-4-oxobutanoic acid;
1-(2-fluoro-5-((4-oxo-3,4,5,6,7,8-hexahydrophthalazin-1-yl)methyl)phenyl)pyrrolidine-2,5-dione;
4-(3-(1,4-diazepan-1-ylcarbonyl)-4-fluorobenzyl)-5,6,7,8-tetrahydrophthalazin-1(2H)-one;
4-(3-(aminomethyl)-4-fluorobenzyl)-5,6,7,8-tetrahydrophthalazin-1(2H)-one;
4-(3-((dimethylamino)methyl)-4-fluorobenzyl)-5,6,7,8-tetrahydrophthalazin-1(2H)-one;
4-(4-fluoro-3-((isopropylamino)methyl)benzyl)-5,6,7,8-tetrahydrophthalazin-1(2H)-one;
4-(3-((cyclohexylamino)methyl)-4-fluorobenzyl)-5,6,7,8-tetrahydrophthalazin-1(2H)-one;
4-(4-fluoro-3-((tetrahydro-2H-pyran-4-ylamino)methyl)benzyl)-5,6,7,8-tetrahydrophthalazin-1(2H)-one;
4-(4-fluoro-3-((methyl((1-methylpyrrolidin-3-yl)methyl)amino)methyl)benzyl)-5,6,7,8-tetrahydrophthalazin-1 (2H)-one;
4-(4-fluoro-3-((methyl(((2R)-1-methylpyrrolidin-2-yl)methyl)amino)methyl)benzyl)-5,6,7,8-tetrahydrophthalazin-1 (2H)-one;
4-(3-((cyclopropylamino)methyl)benzyl)-5,6,7,8-tetrahydrophthalazin-1(2H)-one;
4-(3-((isopropylamino)methyl)benzyl)-5,6,7,8-tetrahydrophthalazin-1(2H)-one;
4-(3-(morpholin-4-ylmethyl)benzyl)-5,6,7,8-tetrahydrophthalazin-1(2H)-one;
4-(3-(pyrrolidin-1-ylmethyl)benzyl)-5,6,7,8-tetrahydrophthalazin-1(2H)-one;
4-(3-((cyclohexylamino)methyl)benzyl)-5,6,7,8-tetrahydrophthalazin-1(2H)-one;
4-(3-((methylamino)methyl)benzyl)-5,6,7,8-tetrahydrophthalazin-1(2H)-one;
4-(3-((ethylamino)methyl)benzyl)-5,6,7,8-tetrahydrophthalazin-1(2H)-one;
4-(3-((4-methylpiperidin-1-yl)methyl)benzyl)-5,6,7,8-tetrahydrophthalazin-1(2H)-one;
4-(3-(((2-(4-(trifluoromethyl)phenyl)ethyl)amino)methyl)benzyl)-5,6,7,8-tetrahydrophthalazin-1(2H)-one;
4-(3-((cyclohexyl(methyl)amino)methyl)benzyl)-5,6,7,8-tetrahydrophthalazin-1(2H)-one;
4-(3-((2-ethylpyrrolidin-1-yl)methyl)benzyl)-5,6,7,8-tetrahydrophthalazin-1(2H)-one;
4-(4-((cyclopropylamino)methyl)benzyl)-5,6,7,8-tetrahydrophthalazin-1(2H)-one;
4-(4-((isopropylamino)methyl)benzyl)-5,6,7,8-tetrahydrophthalazin-1(2H)-one;
4-(4-(morpholin-4-ylmethyl)benzyl)-5,6,7,8-tetrahydrophthalazin-1(2H)-one;
4-(4-(pyrrolidin-1-ylmethyl)benzyl)-5,6,7,8-tetrahydrophthalazin-1(2H)-one;
4-(4-((cyclohexylamino)methyl)benzyl)-5,6,7,8-tetrahydrophthalazin-1(2H)-one;
4-(4-((4-phenylpiperidin-1-yl)methyl)benzyl)-5,6,7,8-tetrahydrophthalazin-1(2H)-one;
4-(4-((methylamino)methyl)benzyl)-5,6,7,8-tetrahydrophthalazin-1(2H)-one;
4-(4-((ethylamino)methyl)benzyl)-5,6,7,8-tetrahydrophthalazin-1(2H)-one;
4-(4-((4-methylpiperidin-1-yl)methyl)benzyl)-5,6,7,8-tetrahydrophthalazin-1(2H)-one;
4-(4-(((2-(3-(trifluoromethyl)phenyl)ethyl)amino)methyl)benzyl)-5,6,7,8-tetrahydrophthalazin-1(2H)-one;
4-(4-((cyclohexyl(methyl)amino)methyl)benzyl)-5,6,7,8-tetrahydrophthalazin-1(2H)-one;
4-(4-((2-methylpyrrolidin-1-yl)methyl)benzyl)-5,6,7,8-tetrahydrophthalazin-1(2H)-one;
4-(4-((4-methyl-1,4-diazepan-1-yl)methyl)benzyl)-5,6,7,8-tetrahydrophthalazin-1(2H)-one;
4-(3-((4-methyl-1,4-diazepan-1-yl)methyl)benzyl)-5,6,7,8-tetrahydrophthalazin-1(2H)-one;
4-(4-fluoro-3-pyrimidin-2-ylbenzyl)-5,6,7,8-tetrahydrophthalazin-1(2H)-one;
4-(4-fluoro-3-pyridin-3-ylbenzyl)-5,6,7,8-tetrahydrophthalazin-1(2H)-one;
4-(4-fluoro-3-pyridin-4-ylbenzyl)-5,6,7,8-tetrahydrophthalazin-1(2H)-one;
N,N-diethyl-2'-fluoro-5'-((4-oxo-3,4,5,6,7,8-hexahydrophthalazin-1-yl)methyl)-1,1'-biphenyl-2-carboxamide;
N-(2-fluoro-5-((4-oxo-3,4,5,6,7,8-hexahydrophthalazin-1-yl)methyl)phenyl)-3-piperidin-1-ylpropanamide;
N-(2-fluoro-5-((4-oxo-3,4,5,6,7,8-hexahydrophthalazin-1-yl)methyl)phenyl)-3-(4-methylpiperazin-1-yl)propanamide;
2-amino-N-(2-fluoro-5-((4-oxo-3,4,5,6,7,8-hexahydrophthalazin-1-yl)methyl)phenyl)acetamide;
3-cyclohexyl-N-(2-fluoro-5-((4-oxo-3,4,5,6,7,8-hexahydrophthalazin-1-yl)methyl)phenyl)propanamide;
N-(2-fluoro-5-((4-oxo-3,4,5,6,7,8-hexahydrophthalazin-1-yl)methyl)phenyl)piperidine-3-carboxamide;
4-(4-fluoro-3-(2-oxopyrrolidin-1-yl)benzyl)-5,6,7,8-tetrahydrophthalazin-1(2H)-one;
N-(2-fluoro-5-((4-oxo-3,4,5,6,7,8-hexahydrophthalazin-1-yl)methyl)phenyl)azetidine-3-carboxamide;
N-(2-(isopropylamino)ethyl)-3-((4-oxo-3,4,5,6,7,8-hexahydrophthalazin-1-yl)methyl)benzamide;
N-(2-fluoro-5-((4-oxo-3,4,5,6,7,8-hexahydrophthalazin-1-yl)methyl)phenyl)-2-morpholin-4-ylacetamide;
N-(2-morpholin-4-ylethyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-(2-pyrrolidin-1-ylethyl)benzamide;
4-(3-((2-methylpyrrolidin-1-yl)carbonyl)benzyl)-5,6,7,8-tetrahydrophthalazin-1(2H)-one;
N-azepan-1-yl-3-((4-oxo-3,4,5,6,7,8-hexahydrophthalazin-1-yl)methyl)benzamide;
4-(3-(piperazin-1-ylcarbonyl)benzyl)-5,6,7,8-tetrahydrophthalazin-1(2H)-one;
N-azetidin-3-yl-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-piperidin-3-ylbenzamide;
N-(4-(dimethylamino)phenyl)-3-((4-oxo-3,4,5,6,7,8-hexahydrophthalazin-1-yl)methyl)benzamide;
N-(2-(4-methylpiperazin-1-yl)ethyl)-3-((4-oxo-3,4,5,6,7,8-hexahydrophthalazin-1-yl)methyl)benzamide;
4-(3-((4-(isoxazol-5-ylcarbonyl)piperazin-1-yl)carbonyl)benzyl)-5,6,7,8-tetrahydrophthalazin-1 (2H)-one;
4-(3-((4-phenylpiperidin-1-yl)carbonyl)benzyl)-5,6,7,8-tetrahydrophthalazin-1(2H)-one;
3-((4-oxo-3,4,5,6,7,8-hexahydrophthalazin-1-yl)methyl)-N-(piperidin-2-ylmethyl)benzamide;
3-((4-oxo-3,4,5,6,7,8-hexahydrophthalazin-1-yl)methyl)-N-(piperidin-4-ylmethyl)benzamide;
3-((4-oxo-3,4,5,6,7,8-hexahydrophthalazin-1-yl)methyl)-N-(2-piperidin-1-ylethyl)benzamide;
N-(1-methylazetidin-3-yl)-3-((4-oxo-3,4,5,6,7,8-hexahydrophthalazin-1-yl)methyl)benzamide;
methyl 4-((4-oxo-3,4,5,6,7,8-hexahydrophthalazin-1-yl)methyl)pyridine-2-carboxylate;
N-methyl-4-((4-oxo-3,4,5,6,7,8-hexahydrophthalazin-1-yl)methyl)pyridine-2-carboxamide;
4-((2-(methylthio)pyrimidin-4-yl)methyl)-5,6,7,8-tetrahydrophthalazin-1(2H)-one;
4-((2-(methylsulfonyl)pyrimidin-4-yl)methyl)-5,6,7,8-tetrahydrophthalazin-1(2H)-one;
4-((2-(methylsulfinyl)pyrimidin-4-yl)methyl)-5,6,7,8-tetrahydrophthalazin-1(2*H*)-one;
4-((3-bromopyridin-4-yl)methyl)-5,6,7,8-tetrahydrophthalazin-1(2H)-one;
4-((6-bromopyridin-3-yl)methyl)-5,6,7,8-tetrahydrophthalazin-1(2H)-one;
4-((2-bromopyridin-3-yl)methyl)-5,6,7,8-tetrahydrophthalazin-1(2H)-one;
methyl 6-((4-oxo-3,4,5,6,7,8-hexahydrophthalazin-1-yl)methyl)pyridine-2-carboxylate;
N-ethyl-4-((4-oxo-3,4,5,6,7,8-hexahydrophthalazin-1-yl)methyl)pyridine-2-carboxamide;
N-isopropyl-4-((4-oxo-3,4,5,6,7,8-hexahydrophthalazin-1-yl)methyl)pyridine-2-carboxamide;
N-cyclohexyl-4-((4-oxo-3,4,5,6,7,8-hexahydrophthalazin-1-yl)methyl)pyridine-2-carboxamide;
N-((1-methylpiperidin-2-yl)methyl)-3-((4-oxo-3,4,5,6,7,8-hexahydrophthalazin-1-yl)methyl)benzamide;
N-((1-methylpiperidin-4-yl)methyl)-3-((4-oxo-3,4,5,6,7,8-hexahydrophthalazin-1-yl)methyl)benzamide;
N-methyl-6-((4-oxo-3,4,5,6,7,8-hexahydrophthalazin-1-yl)methyl)pyridine-2-carboxamide;
N-ethyl-6-((4-oxo-3,4,5,6,7,8-hexahydrophthalazin-1-yl)methyl)pyridine-2-carboxamide;
N-isopropyl-6-((4-oxo-3,4,5,6,7,8-hexahydrophthalazin-1-yl)methyl)pyridine-2-carboxamide;
N-cyclopropyl-6-((4-oxo-3,4,5,6,7,8-hexahydrophthalazin-1-yl)methyl)pyridine-2-carboxamide;
N-cyclohexyl-6-((4-oxo-3,4,5,6,7,8-hexahydrophthalazin-1-yl)methyl)pyridine-2-carboxamide;
methyl 3-((4-oxo-3,4,5,6,7,8-hexahydrophthalazin-1-yl)methyl)pyridine-2-carboxylate;
methyl 5-((4-oxo-3,4,5,6,7,8-hexahydrophthalazin-1-yl)methyl)pyridine-2-carboxylate;
4-((5-bromothien-2-yl)methyl)-5,6,7,8-tetrahydrophthalazin-1(2H)-one;
4-((3-bromothien-2-yl)methyl)-5,6,7,8-tetrahydrophthalazin-1(2H)-one;
4-(3-aminobenzyl)-5,6,7,8-tetrahydrophthalazin-1(2H)-one;
4-(3-bromobenzyl)-5,6,7,8-tetrahydrophthalazin-1(2H)-one;
4-(thien-2-ylmethyl)-5,6,7,8-tetrahydrophthalazin-1(2H)-one;
methyl 5-((4-oxo-3,4,5,6,7,8-hexahydrophthalazin-1-yl)methyl)thiophene-2-carboxylate;
N-methyl-5-((4-oxo-3,4,5,6,7,8-hexahydrophthalazin-1-yl)methyl)pyridine-2-carboxamide;
N-ethyl-5-((4-oxo-3,4,5,6,7,8-hexahydrophthalazin-1-yl)methyl)pyridine-2-carboxamide;
N-methyl-3-((4-oxo-3,4,5,6,7,8-hexahydrophthalazin-1-yl)methyl)pyridine-2-carboxamide;
N-ethyl-3-((4-oxo-3,4,5,6,7,8-hexahydrophthalazin-1-yl)methyl)pyridine-2-carboxamide;
N,N-dimethyl-N'-(3-((4-oxo-3,4,5,6,7,8-hexahydrophthalazin-1-yl)methyl)phenyl)sulfamide;
N-(3-((4-oxo-3,4,5,6,7,8-hexahydrophthalazin-1-yl)methyl)phenyl)-3-piperidin-1-ylpropanamide;
4-chloro-N-(3-((4-oxo-3,4,5,6,7,8-hexahydrophthalazin-1-yl)methyl)phenyl)butanamide;
4-(3-(2-oxopyrrolidin-1-yl)benzyl)-5,6,7,8-tetrahydrophthalazin-1(2H)-one;
4-((2-(2-oxoazetidin-1-yl)pyridin-4-yl)methyl)-5,6,7,8-tetrahydrophthalazin-1(2H)-one;
4-((2-(2-oxopyrrolidin-1-yl)pyridin-4-yl)methyl)-5,6,7,8-tetrahydrophthalazin-1(2H)-one;
4-((2-bromopyridin-4-yl)methyl)-5,6,7,8-tetrahydrophthalazin-1(2H)-one;
4-((6-(2-oxopyrrolidin-1-yl)pyridin-3-yl)methyl)-5,6,7,8-tetrahydrophthalazin-1(2H)-one;
4-((6-(2-oxoazetidin-1-yl)pyridin-3-yl)methyl)-5,6,7,8-tetrahydrophthalazin-1(2H)-one;
N-(5-((4-oxo-3,4,5,6,7,8-hexahydrophthalazin-1-yl)methyl)pyridin-2-yl)benzamide;
N-(5-((4-oxo-3,4,5,6,7,8-hexahydrophthalazin-1-yl)methyl)pyridin-2-yl)isonicotinamide;
N-(5-((4-oxo-3,4,5,6,7,8-hexahydrophthalazin-1-yl)methyl)pyridin-2-yl)nicotinamide;
4-((5'-((4-oxo-3,4,5,6,7,8-hexahydrophthalazin-1-yl)methyl)-2,2'-bipyridin-5-yl)methyl)-5,6,7,8-tetrahydrophthalazin-1(2H)-one;
N-methyl-5-((4-oxo-3,4,5,6,7,8-hexahydrophthalazin-1-yl)methyl)thiophene-2-carboxamide;
N¹-(3-((4-oxo-3,4,5,6,7,8-hexahydrophthalazin-1-yl)methyl)phenyl)glycinamide;
N-(3-((4-oxo-3,4,5,6,7,8-hexahydrophthalazin-1-yl)methyl)phenyl)azetidine-2-carboxamide;
N-(3-((4-oxo-3,4,5,6,7,8-hexahydrophthalazin-1-yl)methyl)phenyl)azetidine-3-carboxamide;
N-(3-((4-oxo-3,4,5,6,7,8-hexahydrophthalazin-1-yl)methyl)phenyl)methanesulfonamide;
N-(3-((4-oxo-3,4,5,6,7,8-hexahydrophthalazin-1-yl)methyl)phenyl)propane-2-sulfonamide;
N-(3-((4-oxo-3,4,5,6,7,8-hexahydrophthalazin-1-yl)methyl)phenyl)benzenesulfonamide;
N-(3-((4-oxo-3,4,5,6,7,8-hexahydrophthalazin-1-yl)methyl)phenyl)pyridine-3-sulfonamide;
N-(3-((4-oxo-3,4,5,6,7,8-hexahydrophthalazin-1-yl)methyl)phenyl)furan-2-sulfonamide;
1-methyl-N-(3-((4-oxo-3,4,5,6,7,8-hexahydrophthalazin-1-yl)methyl)phenyl)-1H-imidazole-4-sulfonamide;
N-(3-((4-oxo-3,4,5,6,7,8-hexahydrophthalazin-1-yl)methyl)phenyl)thiophene-2-sulfonamide;
4-cyano-N-(3-((4-oxo-3,4,5,6,7,8-hexahydrophthalazin-1-yl)methyl)phenyl)benzenesulfonamide;
N-(3-((4-oxo-3,4,5,6,7,8-hexahydrophthalazin-1-yl)methyl)phenyl)naphthalene-1-sulfonamide;
4-((6-bromopyridin-2-yl)methyl)-5,6,7,8-tetrahydrophthalazin-1(2H)-one;
4-((6-(2-oxopyrrolidin-1-yl)pyridin-2-yl)methyl)-5,6,7,8-tetrahydrophthalazin-1(2H)-one;
N-(6-((4-oxo-3,4,5,6,7,8-hexahydrophthalazin-1-yl)methyl)pyridin-2-yl)benzamide;
4-((3'-((isopropylamino)methyl)-1,1'-biphenyl-3-yl)methyl)-5,6,7,8-tetrahydrophthalazin-1(2H)-one;
4-((3'-((cyclopentylamino)methyl)-1,1'-biphenyl-3-yl)methyl)-5,6,7,8-tetrahydrophthalazin-1(2H)-one;
4-((3'-((2-methylpyrrolidin-1-yl)methyl)-1,1'-biphenyl-3-yl)methyl)-5,6,7,8-tetrahydrophthalazin-1 (2H)-one;
4-((3'-((cyclopropylamino)methyl)-1,1'-biphenyl-3-yl)methyl)-5,6,7,8-tetrahydrophthalazin-1(2H)-one;
4-((3'-((cyclobutylamino)methyl)-1,1'-biphenyl-3-yl)methyl)-5,6,7,8-tetrahydrophthalazin-1(2H)-one;
4-((2-bromo-1-oxidopyridin-4-yl)methyl)-5,6,7,8-tetrahydrophthalazin-1(2H)-one;
4-((1-oxido-2-(2-oxopyrrolidin-1-yl)pyridin-4-yl)methyl)-5,6,7,8-tetrahydrophthalazin-1(2H)-one;
methyl 5-((4-oxo-3,4,5,6,7,8-hexahydrophthalazin-1-yl)methyl)thiophene-3-carboxylate;
4-(3-((4-(2-(2-(2-aminoethoxy)ethoxy)ethyl)-1,4-diazepan-1-yl)carbonyl)-4-fluorobenzyl)phthalazin-1 (2H)-one;
1-methyl-N-(3-((4-oxo-3,4,5,6,7,8-hexahydrophthalazin-1-yl)methyl)phenyl)cyclopropanecarboxamide;
2-methyl-N-(3-((4-oxo-3,4,5,6,7,8-hexahydrophthalazin-1-yl)mcthyl)phcnyl)cyclopropanccarboxamidc;
3-ethoxy-N-(3-((4-oxo-3,4,5,6,7,8-hexahydrophthalazin-1-yl)methyl)phenyl)propanamide;
5-oxo-N-(3-((4-oxo-3,4,5,6,7,8-hexahydrophthalazin-1-yl)methyl)phenyl)-L-prolinamide;
5-oxo-N-(3-((4-oxo-3,4,5,6,7,8-hexahydrophthalazin-1-yl)methyl)phenyl)-D-prolinamide;
N¹-(3-((4-oxo-3,4,5,6,7,8-hexahydrophthalazin-1-yl)methyl)phenyl)cyclopropane-1,1-dicarboxamide;
2-(benzyloxy)-N-(3-((4-oxo-3,4,5,6,7,8-hexahydrophthalazin-1-yl)methyl)phenyl)acetamide;
N-(3-((4-oxo-3,4,5,6,7,8-hexahydrophthalazin-1-yl)methyl)phenyl)-3-phenylpropanamide;
3-(2,5-dimethoxyphenyl)-N-(3-((4-oxo-3,4,5,6,7,8-hexahydrophthalazin-1-yl)methyl)phenyl)propanamide;
N-(3-((4-oxo-3,4,5,6,7,8-hexahydrophthalazin-1-yl)methyl)phenyl)-1-phenylcyclopropanecarboxamide;
(2S)-N-(3-((4-oxo-3,4,5,6,7,8-hexahydrophthalazin-1-yl)methyl)phenyl)-2-phenylbutanamide;
N-(3-((4-oxo-3,4,5,6,7,8-hexahydrophthalazin-1-yl)methyl)phenyl)-4-phenylbutanamide;
2-(3-methylphenoxy)-N-(3-((4-oxo-3,4,5,6,7,8-hexahydrophthalazin-1-yl)methyl)phenyl)acetamide;
2-(2-methylphenoxy)-N-(3-((4-oxo-3,4,5,6,7,8-hexahydrophthalazin-1-yl)methyl)phenyl)acetamide;
2-(4-methylphenoxy)-N-(3-((4-oxo-3,4,5,6,7,8-hexahydrophthalazin-1-yl)methyl)phenyl)acetamide;
(2R)-2-methoxy-N-(3-((4-oxo-3,4,5,6,7,8-hexahydrophthalazin-1-yl)methyl)phenyl)-2-phenylacetamide;
(2S)-2-methoxy-N-(3-((4-oxo-3,4,5,6,7,8-hexahydrophthalazin-1-yl)methyl)phenyl)-2-phenylacetamide;
N-(3-((4-oxo-3,4,5,6,7,8-hexahydrophthalazin-1-yl)methyl)phenyl)-3-phenoxypropanamide;
N-(3-((4-oxo-3,4,5,6,7,8-hexahydrophthalazin-1-yl)methyl)phenyl)-4-thien-2-ylbutanamide;
1-acetyl-N-(3-((4-oxo-3,4,5,6,7,8-hexahydrophthalazin-1-yl)methyl)phenyl)piperidine-4-carboxamide;
2-(3,5-difluorophenyl)-N-(3-((4-oxo-3,4,5,6,7,8-hexahydrophthalazin-1-yl)methyl)phenyl)acetamide;
N²-acetyl-N¹-(3-((4-oxo-3,4,5,6,7,8-hexahydrophthalazin-1-yl)methyl)phenyl)-L-leucinamide;
N¹-(3-((4-oxo-3,4,5,6,7,8-hexahydrophthalazin-1-yl)methyl)phenyl)-N²,N²-dipropyl-L-alaninamide;
4-oxo-N-(3-((4-oxo-3,4,5,6,7,8-hexahydrophthalazin-1-yl)methyl)phenyl)-4-phenylbutanamide;
N-(2-oxo-2-(3-((4-oxo-3,4,5,6,7,8-hexahydrophthalazin-1-yl)methyl)phenylamino)ethyl)benzamide;
3-(3-methoxyphenyl)-N-(3-((4-oxo-3,4,5,6,7,8-hexahydrophthalazin-1-yl)methyl)phenyl)propanamide;
3-(4-methoxyphenyl)-N-(3-((4-oxo-3,4,5,6,7,8-hexahydrophthalazin-1-yl)methyl)phenyl)propanamide;
2-(3,4-dimethylphenoxy)-N-(3-((4-oxo-3,4,5,6,7,8-hexahydrophthalazin-1-yl)methyl)phenyl)acetamide;
(2R)-2-hydroxy-N-(3-((4-oxo-3,4,5,6,7,8-hexahydrophthalazin-1-yl)methyl)phenyl)-4-phenylbutanamide;
N-(3-((4-oxo-3,4,5,6,7,8-hexahydrophthalazin-1-yl)methyl)phenyl)-4-phenoxybutanamide;
4-oxo-N-(3-((4-oxo-3,4,5,6,7,8-hexahydrophthalazin-1-yl)methyl)phenyl)-4-thien-2-ylbutanamide;
2-((4-methylpyrimidin-2-yl)thio)-N-(3-((4-oxo-3,4,5,6,7,8-hexahydrophthalazin-1-yl)methyl)phenyl)acetamide;
3-(2-chlorophenyl)-N-(3-((4-oxo-3,4,5,6,7,8-hexahydrophthalazin-1-yl)methyl)phenyl)propanamide;
3-(4-chlorophenyl)-N-(3-((4-oxo-3,4,5,6,7,8-hexahydrophthalazin-1-yl)methyl)phenyl)propanamide;
3-methyl-N-(3-((4-oxo-3,4,5,6,7,8-hexahydrophthalazin-1-yl)methyl)phenyl)-2-phenylpentanamide;
2-(4-chloro-2-methylphenoxy)-N-(3-((4-oxo-3,4,5,6,7,8-hexahydrophthalazin-1-yl)methyl)phenyl)acetamide;
N-(3-((4-oxo-3,4,5,6,7,8-hexahydrophthalazin-1-yl)methyl)phenyl)-N'-phenylpentanediamide;
4-(4-methoxyphenyl)-4-oxo-N-(3-((4-oxo-3,4,5,6,7,8-hexahydrophthalazin-1-yl)methyl)phenyl)butanamide;
N-(3-((4-oxo-3,4,5,6,7,8-hexahydrophthalazin-1-yl)methyl)phenyl)-2,2-diphenylacetamide;
N-(3-((4-oxo-3,4,5,6,7,8-hexahydrophthalazin-1-yl)methyl)phenyl)-3-(phenylsulfonyl)propanamide;
N-(3-((4-oxo-3,4,5,6,7,8-hexahydrophthalazin-1-yl)methyl)phenyl)-2-(3-phenoxyphenyl)acetamide;
4-ethyl-N-(3-((4-oxo-3,4,5,6,7,8-hexahydrophthalazin-1-yl)methyl)phenyl)benzamide;
3-fluoro-2-methyl-N-(3-((4-oxo-3,4,5,6,7,8-hexahydrophthalazin-1-yl)methyl)phenyl)benzamide;
5-fluoro-2-methyl-N-(3-((4-oxo-3,4,5,6,7,8-hexahydrophthalazin-1-yl)methyl)phenyl)benzamide;
3-fluoro-4-methyl-N-(3-((4-oxo-3,4,5,6,7,8-hexahydrophthalazin-1-yl)methyl)phenyl)benzamide;
2,3-difluoro-N-(3-((4-oxo-3,4,5,6,7,8-hexahydrophthalazin-1-yl)methyl)phenyl)benzamide;
2,4-difluoro-N-(3-((4-oxo-3,4,5,6,7,8-hexahydrophthalazin-1-yl)methyl)phenyl)benzamide;
2,5-difluoro-N-(3-((4-oxo-3,4,5,6,7,8-hexahydrophthalazin-1-yl)methyl)phenyl)benzamide;
3,5-difluoro-N-(3-((4-oxo-3,4,5,6,7,8-hexahydrophthalazin-1-yl)methyl)phenyl)benzamide;
N-(3-((4-oxo-3,4,5,6,7,8-hexahydrophthalazin-1-yl)methyl)phenyl)-4-propylbenzamide;
4-isopropyl-N-(3-((4-oxo-3,4,5,6,7,8-hexahydrophthalazin-1-yl)methyl)phenyl)benzamide;
2-ethoxy-N-(3-((4-oxo-3,4,5,6,7,8-hexahydrophthalazin-1-yl)methyl)phenyl)benzamide;
4-isopropoxy-N-(3-((4-oxo-3,4,5,6,7,8-hexahydrophthalazin-1-yl)methyl)phenyl)benzamide;
4-(diethylamino)-N-(3-((4-oxo-3,4,5,6,7,8-hexahydrophthalazin-1-yl)methyl)phenyl)benzamide;
4-butoxy-N-(3-((4-oxo-3,4,5,6,7,8-hexahydrophthalazin-1-yl)methyl)phenyl)benzamide;
2-fluoro-N-(3-((4-oxo-3,4,5,6,7,8-hexahydrophthalazin-1-yl)methyl)phenyl)-5-(trifluoromethyl)benzamide;
2-chloro-N-(3-((4-oxo-3,4,5,6,7,8-hexahydrophthalazin-1-yl)methyl)phenyl)-5-(trifluoromethyl)benzamide;
N-(3-((4-oxo-3,4,5,6,7,8-hexahydrophthalazin-1-yl)methyl)phenyl)-2-furamide; N-(3-((4-oxo-3,4,5,6,7,8-hexahydrophthalazin-1-yl)methyl)phenyl)-3-furamide;
2,5-dimethyl-N-(3-((4-oxo-3,4,5,6,7,8-hexahydrophthalazin-1-yl)methyl)phenyl)-3-furamide;
N-(3-((4-oxo-3,4,5,6,7,8-hexahydrophthalazin-1-yl)methyl)phenyl)thiophene-2-carboxamide;
N-(3-((4-oxo-3,4,5,6,7,8-hexahydrophthalazin-1-yl)methyl)phenyl)thiophene-3-carboxamide;
3-methyl-N-(3-((4-oxo-3,4,5,6,7,8-hexahydrophthalazin-1-yl)methyl)phenyl)thiophene-2-carboxamide;
5-methyl-N-(3-((4-oxo-3,4,5,6,7,8-hexahydrophthalazin-1-yl)methyl)phenyl)thiophene-2-carboxamide;
N-(3-((4-oxo-3,4,5,6,7,8-hexahydrophthalazin-1-yl)methyl)phenyl)-1H-pyrrole-2-carboxamide;
1-methyl-N-(3-((4-oxo-3,4,5,6,7,8-hexahydrophthalazin-1-yl)methyl)phenyl)-1H-pyrrole-2-carboxamide;
2,5-dimethyl-N-(3-((4-oxo-3,4,5,6,7,8-hexahydrophthalazin-1-yl)methyl)phenyl)-1H-pyrrole-3-carboxamide;
1,2,5-trimethyl-N-(3-((4-oxo-3,4,5,6,7,8-hexahydrophthalazin-1-yl)methyl)phenyl)-1H-pyrrole-3-carboxamide;
N-(3-((4-oxo-3,4,5,6,7,8-hexahydrophthalazin-1-yl)methyl)phenyl)-1,3-thiazole-2-carboxamide;
N-(3-((4-oxo-3,4,5,6,7,8-hexahydrophthalazin-1-yl)methyl)phenyl)-1,3-thiazole-4-carboxamide;
N-(3-((4-oxo-3,4,5,6,7,8-hexahydrophthalazin-1-yl)methyl)phenyl)-1,3-thiazole-5-carboxamide;
N-(3-((4-oxo-3,4,5,6,7,8-hexahydrophthalazin-1-yl)methyl)phenyl)isoxazole-5-carboxamide;
3,5-dimethyl-N-(3-((4-oxo-3,4,5,6,7,8-hexahydrophthalazin-1-yl)methyl)phenyl)isoxazole-4-carboxamide;
N-(3-((4-oxo-3,4,5,6,7,8-hexahydrophthalazin-1-yl)methyl)phenyl)nicotinamide;
N-(3-((4-oxo-3,4,5,6,7,8-hexahydrophthalazin-1-yl)methyl)phenyl)isonicotinamide;
3-hydroxy-N-(3-((4-oxo-3,4,5,6,7,8-hexahydrophthalazin-1-yl)methyl)phenyl)pyridine-2-carboxamide;
2-hydroxy-N-(3-((4-oxo-3,4,5,6,7,8-hexahydrophthalazin-1-yl)methyl)phenyl)nicotinamide;
6-hydroxy-N-(3-((4-oxo-3,4,5,6,7,8-hexahydrophthalazin-1-yl)methyl)phenyl)nicotinamide;
N-(3-((4-oxo-3,4,5,6,7,8-hexahydrophthalazin-1-yl)methyl)phenyl)-2-pyridin-2-ylacetamide;
N-(3-((4-oxo-3,4,5,6,7,8-hexahydrophthalazin-1-yl)methyl)phenyl)-2-pyridin-3-ylacetamide;
5-methyl-N-(3-((4-oxo-3,4,5,6,7,8-hexahydrophthalazin-1-yl)methyl)phenyl)pyrazine-2-carboxamide;
N-(3-((4-oxo-3,4,5,6,7,8-hexahydrophthalazin-1-yl)methyl)phenyl)-1H-indole-3-carboxamide;
5-methyl-N-(3-((4-oxo-3,4,5,6,7,8-hexahydrophthalazin-1-yl)methyl)phenyl)-1-phenyl-1H-pyrazole-4-carboxamide;
6-chloro-N-(3-((4-oxo-3,4,5,6,7,8-hexahydrophthalazin-1-yl)methyl)phenyl)-2H-chromene-3-carboxamide;
N³,N³-dimethyl-N¹-(3-((4-oxo-3,4,5,6,7,8-hexahydrophthalazin-1-yl)methyl)phenyl)-beta-alaninamide;
4-(2-(3-bromophenyl)ethyl)-5,6,7,8-tetrahydrophthalazin-1(2H)-one;
4-(2-(3-bromo-4-fluorophenyl)ethyl)-5,6,7,8-tetrahydrophthalazin-1(2H)-one;
4-(2,2,2-trifluoro-1-phenylethyl)-5,6,7,8-tetrahydrophthalazin-1(2H)-one;
2-hydroxy-4-methyl-N-(3-((4-oxo-3,4,5,6,7,8-hexahydrophthalazin-1-yl)methyl)phenyl)benzamide;
4-acetyl-N-(3-((4-oxo-3,4,5,6,7,8-hexahydrophthalazin-1-yl)methyl)phenyl)benzamide;
3-methoxy-4-methyl-N-(3-((4-oxo-3,4,5,6,7,8-hexahydrophthalazin-1-yl)methyl)phenyl)benzamide;
4-ethoxy-N-(3-((4-oxo-3,4,5,6,7,8-hexahydrophthalazin-1-yl)methyl)phenyl)benzamide;
3-fluoro-4-methoxy-N-(3-((4-oxo-3,4,5,6,7,8-hexahydrophthalazin-1-yl)methyl)phenyl)benzamide;
N-(3-((4-oxo-3,4,5,6,7,8-hexahydrophthalazin-1-yl)methyl)phenyl)-1-naphthamide;
N-(3-((4-oxo-3,4,5,6,7,8-hexahydrophthalazin-1-yl)methyl)phenyl)-2-naphthamide;
5-chloro-2-hydroxy-N-(3-((4-oxo-3,4,5,6,7,8-hexahydrophthalazin-1-yl)methyl)phenyl)benzamide;
4-tert-butyl-N-(3-((4-oxo-3,4,5,6,7,8-hexahydrophthalazin-1-yl)methyl)phenyl)benzamide;
4-(acetylamino)-N-(3-((4-oxo-3,4,5,6,7,8-hexahydrophthalazin-1-yl)methyl)phenyl)benzamide;
N-(3-((4-oxo-3,4,5,6,7,8-hexahydrophthalazin-1-yl)methyl)phenyl)-4-propoxybenzamide;
1-hydroxy-N-(3-((4-oxo-3,4,5,6,7,8-hexahydrophthalazin-1-yl)methyl)phenyl)-2-naphthamide;
2-chloro-5-(methylthio)-N-(3-((4-oxo-3,4,5,6,7,8-hexahydrophthalazin-1-yl)methyl)phenyl)benzamide;
3,4-diethoxy-N-(3-((4-oxo-3,4,5,6,7,8-hexahydrophthalazin-1-yl)methyl)phenyl)benzamide;
2-benzyl-N-(3-((4-oxo-3,4,5,6,7,8-hexahydrophthalazin-1-yl)methyl)phenyl)benzamide;
2-anilino-N-(3-((4-oxo-3,4,5,6,7,8-hexahydrophthalazin-1-yl)methyl)phenyl)benzamide;
2-benzoyl-N-(3-((4-oxo-3,4,5,6,7,8-hexahydrophthalazin-1-yl)methyl)phenyl)benzamide;
N-(3-((4-oxo-3,4,5,6,7,R-hexahydrophthalazin-1-yl)methyl)phenyl)-2-(2-phenylethyl)benzamide;
5-bromo-2-chloro-N-(3-((4-oxo-3,4,5,6,7,8-hexahydrophthalazin-1-yl)methyl)phenyl)benzamide;
2-(4-methylbenzoyl)-N-(3-((4-oxo-3,4,5,6,7,8-hexahydrophthalazin-1-yl)methyl)phenyl)benzamide;
2-iodo-N-(3-((4-oxo-3,4,5,6,7,8-hexahydrophthalazin-1-yl)methyl)phenyl)benzamide;
3-iodo-N-(3-((4-oxo-3,4,5,6,7,8-hexahydrophthalazin-1-yl)methyl)phenyl)benzamide;
4-iodo-N-(3-((4-oxo-3,4,5,6,7,8-hexahydrophthalazin-1-yl)methyl)phenyl)benzamide;
N-(2'-fluoro-5'-((4-oxo-3,4,5,6,7,8-hexahydrophthalazin-1-yl)methyl)-1,1'-biphenyl-3-yl)acetamide;
4-((6-fluoro-3'-(methylsulfonyl)-1,1'-biphenyl-3-yl)methyl)-5,6,7,8-tetrahydrophthalazin-1(2H)-one;
4-((6-fluoro-3'-(pyrrolidin-1-ylcarbonyl)-1,1'-biphenyl-3-yl)methyl)-5,6,7,8-tetrahydrophthalazin-1 (2H)-one;
4-((6-fluoro-4'-(pyrrolidin-1-ylcarbonyl)-1,1'-biphenyl-3-yl)methyl)-5,6,7,8-tetrahydrophthalazin-1(2H)-one;
2'-fluoro-5'-((4-oxo-3,4,5,6,7,8-hexahydrophthalazin-1-yl)methyl)-1,1'-biphenyl-3-carboxamide;
2'-fluoro-N,N-dimethyl-5'-((4-oxo-3,4,5,6,7,8-hexahydrophthalazin-1-yl)methyl)-1,1'-biphenyl-4-carboxamide;
4-(3,3,3-trifluoro-2-phenylpropyl)-5,6,7,8-tetrahydrophthalazin-1(2H)-one; 4-(2-phenylethyl)-5,6,7,8-tetrahydrophthalazin-1(2H)-one;
4-(2-(3-bromophenyl)propyl)-5,6,7,8-tetrahydrophthalazin-1(2H)-one;
tert-butyl 2-((4-oxo-3,4,5,6,7,8-hexahydrophthalazin-1-yl)methyl)piperazine-1-carboxylate;
4-benzyl 1-tert-butyl 2-((4-oxo-3,4,5,6,7,8-hexahydrophthalazin-1-yl)methyl)piperazine-1,4-dicarboxylate;
4-(2-(3-nitrophenyl)ethyl)-5,6,7,8-tetrahydrophthalazin-1(2H)-one;
4-(2-(3-aminophenyl)ethyl)-5,6,7,8-tetrahydrophthalazin-1(2H)-one;
4-(piperazin-2-ylmethyl)-5,6,7,8-tetrahydrophthalazin-1(2H)-one;
4-(2-(3-(2-oxopyrrolidin-1-yl)phenyl)ethyl)-5,6,7,8-tetrahydrophthalazin-1(2H)-one;
N-(3-(2-(4-oxo-3,4,5,6,7,8-hexahydrophthalazin-1-yl)ethyl)phenyl)-2-phenoxyacetamide;
4-(2-(6-fluoro-3'-(morpholin-4-ylcarbonyl)-1,1'-biphenyl-3-yl)ethyl)-5,6,7,8-tetrahydrophthalazin-1 (2H)-one;
methyl 3-(2-(4-oxo-3,4,5,6,7,8-hexahydrophthalazin-1-yl)ethyl)benzoate;
methyl 3-(1-methyl-2-(4-oxo-3,4,5,6,7,8-hexahydrophthalazin-1-yl)ethyl)benzoate;
4-(2-(6-fluoro-4'-(morpholin-4-ylcarbonyl)-1,1'-biphenyl-3-yl)ethyl)-5,6,7,8-tetrahydrophthalazin-1 (2H)-one;
4-(2-(6-fluoro-2'-(pyrrolidin-1-ylcarbonyl)-1,1'-biphenyl-3-yl)ethyl)-5,6,7,8-tetrahydrophthalazin-1(2H)-one;
4-(2-(6-fluoro-3'-(pyrrolidin-1-ylcarbonyl)-1,1'-biphenyl-3-yl)ethyl)-5,6,7,8-tetrahydrophthalazin-1(2H)-one;
N-cyclopropyl-2'-fluoro-5'-(2-(4-oxo-3,4,5,6,7,8-hexahydrophthalazin-1-yl)ethyl)-1,1'-biphenyl-3-carboxamide;
N-(2-(dimethylamino)ethyl)-2'-fluoro-5'-(2-(4-oxo-3,4,5,6,7,8-hexahydrophthalazin-1-yl)ethyl)-1,1'-biphenyl-3-carboxamide;
2'-fluoro-5'-(2-(4-oxo-3,4,5,6,7,8-hexahydrophthalazin-1-yl)ethyl)-1,1'-biphenyl-3-carboxamide;
N-(2'-fluoro-5'-(2-(4-oxo-3,4,5,6,7,8-hexahydrophthalazin-1-yl)ethyl)-1,1'-biphenyl-3-yl)methanesulfonamide;
N-(2'-fluoro-5'-(2-(4-oxo-3,4,5,6,7,8-hexahydrophthalazin-1-yl)ethyl)-1,1'-biphenyl-3-yl)acetamide;
4-(2-(6-fluoro-3'-(morpholin-4-ylcarbonyl)-1,1'-biphenyl-3-yl)propyl)-5,6,7,8-tetrahydrophthalazin-1 (2H)-one;
4-(2-(6-fluoro-3'-(pyrrolidin-1-ylcarbonyl)-1,1'-biphenyl-3-yl)propyl)-5,6,7,8-tetrahydrophthalazin-1 (2H)-one;
N-cyclopropyl-2'-fluoro-5'-(1-methyl-2-(4-oxo-3,4,5,6,7,8-hexahydrophthalazin-1-yl)ethyl)-1,1 '-biphenyl-3-carboxamide;
4-(3-amino-4-chlorobenzyl)-5,6,7,8-tetrahydrophthalazin-1(2H)-one;
4-(3-amino-4-methoxybenzyl)-5,6,7,8-tetrahydrophthalazin-1(2H)-one;
4-(3-amino-4-hydroxybenzyl)-5,6,7,8-tetrahydrophthalazin-1(2H)-one;
4-(3-amino-4-methylbenzyl)-5,6,7,8-tetrahydrophthalazin-1(2H)-one;
N-(2-(dimethylamino)ethyl)-3'-(1-methyl-2-(4-oxo-3,4,5,6,7,8-hexahydrophthalazin-1-yl)ethyl)-1,1'-biphenyl-3-carboxamide;
3'-(1-methyl-2-(4-oxo-3,4,5,6,7,8-hexahydrophthalazin-1-yl)ethyl)-1,1'-biphenyl-3-carboxamide;
N-(3'-(1-methyl-2-(4-oxo-3,4,5,6,7,8-hexahydrophthalazin-1-yl)ethyl)-1,1'-biphenyl-3-yl)acetamide;
3'-(1-(4-oxo-3,4,5,6,7,8-hexahydrophthalazin-1-yl)ethyl)1,1'-biphenyl-3-carboxamide;
N-(3'-(1-(4-oxo-3,4,5,6,7,8-hexahydrophthalazin-1-yl)ethyl)-1,1'-biphenyl-3-yl)acetamide;
N-(2-(dimethylamino)ethyl)-3'-(1-(4-oxo-3,4,5,6,7,8-hexahydrophthalazin-1-yl)ethyl)-1,1'-biphenyl-3-carboxamide;
3-(1-methyl-2-(4-oxo-3,4,5,6,7,8-hexahydrophthalazin-1-yl)ethyl)benzoic acid;
N-(2-fluoro-5-((4-oxo-3,4,5,6,7,8-hexahydrophthalazin-1-yl)methyl)phenyl)-4-(4-methoxyphenyl)-4-oxobutanamide;
1-(2-fluoro-5-((4-oxo-3,4,5,6,7,8-hexahydrophthalazin-1-yl)methyl)phenyl)-3,4-dimethyl-1 H-pyrrole-2,5-dione;
3-(2-fluoro-5-((4-oxo-3,4,5,6,7,8-hexahydrophthalazin-1-yl)methyl)phenyl)-3-azabicyclo(3.1.0)hexane-2,4-dione;
4-((4-(phenoxyacetyl)piperazin-2-yl)methyl)-5,6,7,8-tetrahydrophthalazin-1(2H)-one;
4-(2-(3-bromo-4-fluorophenyl)propyl)-5,6,7,8-tetrahydrophthalazin-1(2H)-one;
4-oxo-N-(3-(2-(4-oxo-3,4,5,6,7,8-hexahydrophthalazin-1-yl)ethyl)phenyl)-4-phenylbutanamide;
2'-fluoro-5'-(1-methyl-2-(4-oxo-3,4,5,6,7,8-hexahydrophthalazin-1-yl)ethyl)-1,1'-biphenyl-3-carboxamide;
N-(2'-fluoro-5'-(1-methyl-2-(4-oxo-3,4,5,6,7,8-hexahydrophthalazin-1-yl)ethyl)-1,1'-biphenyl-3-yl)acetamide;
N-((2'-fluoro-5'-(1-methyl-2-(4-oxo-3,4,5,6,7,8-hexahydrophthalazin-1-yl)ethyl)-1,1'-biphenyl-3-yl)methyl)methanesulfonamide;
2-(2-fluoro-5-((4-oxo-3,4,5,6,7,8-hexahydrophthalazin-1-yl)methyl)phenyl)hexahydro-1H-isoindolc-1,3(2H)-dionc;
1-(2-fluoro-5-((4-oxo-3,4,5,6,7,8-hexahydrophthalazin-1-yl)methyl)phenyl)-3,3-dimethylpyrrolidine-2,5-dione;
4-(4-fluoro-3-(2-methyl-5-oxopyrrolidin-1-yl)benzyl)-5,6,7,8-tetrahydrophthalazin-1(2H)-one;
4-(4-fluoro-3-(2-oxo-1,3-oxazolidin-3-yl)benzyl)-5,6,7,8-tetrahydrophthalazin-1(2H)-one;
4-(4-fluoro-3-(2-oxoazepan-1-yl)benzyl)-5,6,7,8-tetrahydrophthalazin-1(2H)-one;
1-(2-fluoro-5-((4-oxo-3,4,5,6,7,8-hexahydrophthalazin-1-yl)methyl)phenyl)piperidine-2,6-dione;
4-(4-fluoro-3-(2-oxoimidazolidin-1-yl)benzyl)-5,6,7,8-tetrahydrophthalazin-1(2H)-one;
4-(3-(1,1-dioxidoisothiazolidin-2-yl)-4-fluorobenzyl)-5,6,7,8-tetrahydrophthalazin-1(2H)-one;
4-(4-fluoro-3-(2-oxoazetidin-1-yl)benzyl)-5,6,7,8-tetrahydrophthalazin-1(2H)-one;
4-(4-fluoro-3-(2-oxopiperidin-1-yl)benzyl)-5,6,7,8-tetrahydrophthalazin-1(2H)-one;
N-(3-furylmethyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-(thien-2-ylmethyl)benzamide;
3-((4-oxo-3,4,5,6,7,8-hexahydrophthalazin-1-yl)methyl)-N-(thien-3-ylmethyl)benzamide;
3-((4-oxo-3,4,5,6,7,8-hexahydrophthalazin-1-yl)methyl)-N-(pyridin-3-ylmethyl)benzamide;
3-((4-oxo-3,4,5,6,7,8-hexahydrophthalazin-1-yl)methyl)-N-(pyridin-4-ylmethyl)benzamide;
N-(2-(dimethylamino)ethyl)-3-((4-oxo-3,4,5,6,7,8-hexahydrophthalazin-1-yl)methyl)benzamide;
N-(3-(dimethylamino)propyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-(3-pyrrolidin-1-ylpropyl)benzamide;
3-((4-oxo-3,4,5,6,7,8-hexahydrophthalazin-1-yl)methyl)-N-(3-piperidin-1-ylpropyl)benzamide;
N-(3-morpholin-4-ylpropyl)-3-((4-oxo-3,4,5,6,7,8-hexahydrophthalazin-1-yl)methyl)benzamide;
N-(2-(1H-indol-3-yl)ethyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-1,3-thiazol-2-ylbenzamide;
benzyl 2-oxo-2-(3-(2-(4-oxo-3,4,5,6,7,8-hexahydrophthalazin-1-yl)ethyl)phenylamino)ethylcarbamate;
4-oxo-N-(3-(2-(4-oxo-3,4,5,6,7,8-hexahydrophthalazin-1-yl)ethyl)phenyl)-4-(4-phenoxyphenyl)butanamide;
benzyl 3-(((3-(2-(4-oxo-3,4,5,6,7,8-hexahydrophthalazin-1-yl)ethyl)phenyl)amino)carbonyl)piperidine-1-carboxylate;
2-(4-methylphenoxy)-N-(3-(2-(4-oxo-3,4,5,6,7,8-hexahydrophthalazin-1-yl)ethyl)phenyl)acetamide;
2-(4-methoxyphenoxy)-N-(3-(2-(4-oxo-3,4,5,6,7,8-hexahydrophthalazin-1-yl)ethyl)phenyl)acetamide;
4-(4-fluoro-3-(3-methyl-2-oxoimidazolidin-1-yl)benzyl)-5,6,7,8-tetrahydrophthalazin-1(2H)-one;
4-(4-fluoro-3-(2-oxotetrahydropyrimidin-1(2H)-yl)benzyl)-5,6,7,8-tetrahydrophthalazin-1(2H)-one;
4-(3-(3-tert-butyl-2-oxoimidazolidin-1-yl)-4-fluorobenzyl)-5,6,7,8-tetrahydrophthalazin-1(2H)-one;
4-(4-fluoro-3-((1S,4R)-3-oxo-2-azabicyclo(2.2.1)hept-2-yl)benzyl)-5,6,7,8-tetrahydrophthalazin-1 (2H)-one;
N-(2-ethylphenyl)-3-((4-oxo-3,4,5,6,7,8-hexahydrophthalazin-1-yl)methyl)benzamide;
N-(3-ethylphenyl)-3-((4-oxo-3,4,5,6,7,8-hexahydrophthalazin-1-yl)methyl)benzamide;
N-(4-ethylphenyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-(2-propylphenyl)benzamide;
N-(2-isopropylphenyl)-3-((4-oxo-3,4,5,6,7,8-hexahydrophthalazin-1-yl)methyl)benzamide;
N-(4-isopropylphenyl)-3-((4-oxo-3,4,5,6,7,8-hexahydrophthalazin-1-yl)methyl)benzamide;
N-(3-tert-butylphenyl)-3-((4-oxo-3,4,5,6,7,8-hexahydrophthalazin-1-yl)methyl)benzamide;
N-(4-tert-butylphenyl)-3-((4-oxo-3,4,5,6,7,8-hexahydrophthalazin-1-yl)methyl)benzamide;
N-1,1'-biphenyl-4-yl-3-((4-oxo-3,4,5,6,7,8-hexahydrophthalazin-1-yl)methyl)benzamide;
N-(2-fluoro-4-methylphenyl)-3-((4-oxo-3,4,5,6,7,8-hexahydrophthalazin-1-yl)methyl)benzamide;
N-(3-fluoro-4-methylphenyl)-3-((4-oxo-3,4,5,6,7,8-hexahydrophthalazin-1-yl)methyl)benzamide;
N-(4-fluoro-2-methylphenyl)-3-((4-oxo-3,4,5,6,7,8-hexahydrophthalazin-1-yl)methyl)benzamide;
N-(4-fluoro-3-methylphenyl)-3-((4-oxo-3,4,5,6,7,8-hexahydrophthalazin-1-yl)methyl)benzamide;
N-(3-chloro-4-methylphenyl)-3-((4-oxo-3,4,5,6,7,8-hexahydrophthalazin-1-yl)methyl)benzamide;
N-(4-chloro-3-methylphenyl)-3-((4-oxo-3,4,5,6,7,8-hexahydrophthalazin-1-yl)methyl)benzamide;
N-(3-bromo-4-methylphenyl)-3-((4-oxo-3,4,5,6,7,8-hexahydrophthalazin-1-yl)methyl)benzamide;
N-(4-bromo-3-methylphenyl)-3-((4-oxo-3,4,5,6,7,8-hexahydrophthalazin-1-yl)methyl)benzamide;
N-(3-fluoro-4-methoxyphenyl)-3-((4-oxo-3,4,5,6,7,8-hexahydrophthalazin-1-yl)methyl)benzamide;
N-(3-methoxy-5-(trifluoromethyl)phenyl)-3-((4-oxo-3,4,5,6,7,8-hexahydrophthalazin-1-yl)methyl)benzamide;
N-(2-hydroxy-6-methylphenyl)-3-((4-oxo-3,4,5,6,7,8-hexahydrophthalazin-1-yl)methyl)benzamide;
N-(3-hydroxy-2-methylphenyl)-3-((4-oxo-3,4,5,6,7,8-hexahydrophthalazin-1-yl)methyl)benzamide;
N-(3-hydroxy-4-methylphenyl)-3-((4-oxo-3,4,5,6,7,8-hexahydrophthalazin-1-yl)methyl)benzamide;
N-(2-methoxy-5-methylphenyl)-3-((4-oxo-3,4,5,6,7,8-hexahydrophthalazin-1-yl)methyl)benzamide;
N-(3-methoxy-4-methylphenyl)-3-((4-oxo-3,4,5,6,7,8-hexahydrophthalazin-1-yl)methyl)benzamide;
N-(3-hydroxy-4-methoxyphenyl)-3-((4-oxo-3,4,5,6,7,8-hexahydrophthalazin-1-yl)methyl)benzamide;
N-(2-ethoxyphenyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-(4-propoxyphenyl)benzamide;
N-(5-tert-butyl-2-methoxyphenyl)-3-((4-oxo-3,4,5,6,7,8-hexahydrophthalazin-1-yl)mcthyl)bcnzamidc;
N-(5-(acetylamino)-2-methoxyphenyl)-3-((4-oxo-3,4,5,6,7,8-hexahydrophthalazin-1-yl)methyl)benzamide;
N-2,3-dihydro-1,4-benzodioxin-6-yl-3-((4-oxo-3,4,5,6,7,8-hexahydrophthalazin-1-yl)methyl)benzamide;
N-(5-chloro-2,4-dimethoxyphenyl)-3-((4-oxo-3,4,5,6,7,8-hexahydrophthalazin-1-yl)methyl)benzamide;
N-(3-(methylthio)phenyl)-3-((4-oxo-3,4,5,6,7,8-hexahydrophthalazin-1-yl)methyl)benzamide;
N-(4-(methylthio)phenyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-(4-piperidin-1-ylphenyl)benzamide;
N-(4-morpholin-4-ylphenyl)-3-((4-oxo-3,4,5,6,7,8-hexahydrophthalazin-1-yl)methyl)benzamide;
N-(2-anilinophenyl)-3-((4-oxo-3,4,5,6,7,8-hexahydrophthalazin-1-yl)methyl)benzamide;
N-(4-((4-methoxyphenyl)amino)phenyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-quinolin-6-ylbenzamide;
N-(5-hydroxy-1-naphthyl)-3-((4-oxo-3,4,5,6,7,8-hexahydrophthalazin-1-yl)methyl)benzamide;
N-1H-indazol-6-yl-3-((4-oxo-3,4,5,6,7,8-hexahydrophthalazin-1-yl)methyl)benzamide; 8-(4-fluorobenzyl)pyrido(2,3-d)pyridazin-5(6H)-one;
8-(3-chloro-4-fluorobenzyl)pyrido(2,3-d)pyridazin-5(6H)-one;
(3aR)-8-((4-oxo-3,4,5,6,7,8-hexahydrophthalazin-1-yl)methyl)-2,3,3a,4-tetrahydro-1H-pyrrolo(2,1-c)(1,4)benzoxazin-1-one;
N-(2-fluoro-5-((4-oxo-3,4,5,6,7,8-hexahydrophthalazin-1-yl)methyl)phenyl)-N-methylmethanesulfonamide;
N-(2-fluoro-5-((4-oxo-3,4,5,6,7,8-hexahydrophthalazin-1-yl)methyl)phenyl)-2-hydroxy-2-methylpropanamide;
(3aS)-8-((4-oxo-3,4,5,6,7,8-hexahydrophthalazin-1-yl)methyl)-2,3,3a,4-tetrahydro-1H-pyrrolo(2,1-c)(1,4)benzoxazin-1-one;
3-((4-oxo-3,4,5,6,7,8-hexahydrophthalazin-1-yl)methyl)-N-(2-phenylethyl)benzamide;
N-(2-(2-methylphenyl)ethyl)-3-((4-oxo-3,4,5,6,7,8-hexahydrophthalazin-1-yl)methyl)benzamide;
N-(2-(3-methylphenyl)ethyl)-3-((4-oxo-3,4,5,6,7,8-hexahydrophthalazin-1-yl)methyl)benzamide;
N-(2-(4-methylphenyl)ethyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-(2-pyridin-2-ylethyl)benzamide;
3-((4-oxo-3,4,5,6,7,8-hexahydrophthalazin-1-yl)methyl)-N-(2-pyridin-3-ylethyl)benzamide;
3-((4-oxo-3,4,5,6,7,8-hexahydrophthalazin-1-yl)methyl)-N-(2-pyridin-4-ylethyl)benzamide;
N-(2-(2-methoxyphenyl)ethyl)-3-((4-oxo-3,4,5,6,7,8-hexahydrophthalazin-1-yl)methyl)benzamide;
N-(2-(3-methoxyphenyl)ethyl)-3-((4-oxo-3,4,5,6,7,8-hexahydrophthalazin-1-yl)methyl)benzamide;
N-(2-(4-methoxyphenyl)ethyl)-3-((4-oxo-3,4,5,6,7,8-hexahydrophthalazin-1-yl)methyl)benzamide;
N-(2-(2-fluorophenyl)ethyl)-3-((4-oxo-3,4,5,6,7,8-hexahydrophthalazin-1-yl)methyl)benzamide;
N-(2-(3-fluorophenyl)ethyl)-3-((4-oxo-3,4,5,6,7,8-hexahydrophthalazin-1-yl)methyl)benzamide;
N-(2-(4-fluorophenyl)ethyl)-3-((4-oxo-3,4,5,6,7,8-hexahydrophthalazin-1-yl)methyl)benzamide;
N-(2-(2-chlorophenyl)ethyl)-3-((4-oxo-3,4,5,6,7,8-hexahydrophthalazin-1-yl)methyl)benzamide;
N-(2-(3-chlorophenyl)ethyl)-3-((4-oxo-3,4,5,6,7,8-hexahydrophthalazin-1-yl)methyl)benzamide;
N-(2-(4-chlorophenyl)ethyl)-3-((4-oxo-3,4,5,6,7,8-hexahydrophthalazin-1-yl)methyl)benzamide;
N-(2-(3-bromophenyl)ethyl)-3-((4-oxo-3,4,5,6,7,8-hexahydrophthalazin-1-yl)methyl)benzamide;
N-(2-(4-bromophenyl)ethyl)-3-((4-oxo-3,4,5,6,7,8-hexahydrophthalazin-1-yl)mcthyl)bcnzamidc;
N-(2-(1,1'-biphenyl-4-yl)ethyl)-3-((4-oxo-3,4,5,6,7,8-hexahydrophthalazin-1-yl)methyl)benzamide;
3-((4-oxo-3,4,5,6,7,8-hexahydrophthalazin-1-yl)methyl)-N-(2-(3-(trifluoromethyl)phenyl)ethyl)benzamide;
3-((4-oxo-3,4,5,6,7,8-hexahydrophthalazin-1-yl)methyl)-N-(2-(4-(trifluoromethyl)phenyl)ethyl)benzamide;
3-((4-oxo-3,4,5,6,7,8-hexahydrophthalazin-1-yl)methyl)-N-(2-(4-phenoxyphenyl)ethyl)benzamide;
N-(2-(3,4-dimethylphenyl)ethyl)-3-((4-oxo-3,4,5,6,7,8-hexahydrophthalazin-1-yl)methyl)benzamide;
N-(2-(2,4-dimethylphenyl)ethyl)-3-((4-oxo-3,4,5,6,7,8-hexahydrophthalazin-1-yl)methyl)benzamide;
N-(2-(2,5-dimethylphenyl)ethyl)-3-((4-oxo-3,4,5,6,7,8-hexahydrophthalazin-1-yl)methyl)benzamide;
N-(2-(3-ethoxy-4-methoxyphenyl)ethyl)-3-((4-oxo-3,4,5,6,7,8-hexahydrophthalazin-1-yl)methyl)benzamide;
N-(2-(4-ethoxy-3-methoxyphenyl)ethyl)-3-((4-oxo-3,4,5,6,7,8-hexahydrophthalazin-1-yl)methyl)benzamide;
N-(2-(2,3-dimethoxyphenyl)ethyl)-3-((4-oxo-3,4,5,6,7,8-hexahydrophthalazin-1-yl)methyl)benzamide;
N-(2-(2,4-dimethoxyphenyl)ethyl)-3-((4-oxo-3,4,5,6,7,8-hexahydrophthalazin-1-yl)methyl)benzamide;
N-(2-(2,5-dimethoxyphenyl)ethyl)-3-((4-oxo-3,4,5,6,7,8-hexahydrophthalazin-1-yl)methyl)benzamide;
N-(2-(3,4-dimethoxyphenyl)ethyl)-3-((4-oxo-3,4,5,6,7,8-hexahydrophthalazin-1-yl)methyl)benzamide;
N-(2-(3,5-dimethoxyphenyl)ethyl)-3-((4-oxo-3,4,5,6,7,8-hexahydrophthalazin-1-yl)methyl)benzamide;
N-(2-(1,3-benzodioxol-5-yl)ethyl)-3-((4-oxo-3,4,5,6,7,8-hexahydrophthalazin-1-yl)methyl)benzamide;
N-(2-(2,3-dichlorophenyl)ethyl)-3-((4-oxo-3,4,5,6,7,8-hexahydrophthalazin-1-yl)mcthyl)bcnzamidc;
N-(2-(3,4-dichlorophenyl)ethyl)-3-((4-oxo-3,4,5,6,7,8-hexahydrophthalazin-1-yl)methyl)benzamide;
N-(2-(2,6-dichlorophenyl)ethyl)-3-((4-oxo-3,4,5,6,7,8-hexahydrophthalazin-1-yl)methyl)benzamide;
(3aS,4R,7S,7aR)-5-(2-fluoro-5-((4-oxo-3,4,5,6,7,8-hexahydrophthalazin-1-yl)methyl)phenyl)-2,2-dimethyltetrahydro-4,7-methano(1,3)dioxolo(4,5-c)pyridin-6(3aH)-one;
4-(1-(3-bromo-4-fluorophenyl)ethyl)-5,6,7,8-tetrahydrophthalazin-1(2H)-one;
4-(1-(4-fluoro-3-(2-oxopyrrolidin-1-yl)phenyl)ethyl)-5,6,7,8-tetrahydrophthalazin-1(2H)-one;
8-(4-fluorobenzyl)-2,3,4,6-tetrahydropyrido(2,3-d)pyridazin-5(1H)-one;
8-(3-bromo-4-fluorobenzyl)pyrido(2,3-d)pyridazin-5(6H)-one;
N-(2-(dimethylamino)ethyl)-N-ethyl-3-((4-oxo-3,4,5,6,7,8-hexahydrophthalazin-1-yl)methyl)benzamide;
N-(2-(diethylamino)ethyl)-N-methyl-3-((4-oxo-3,4,5,6,7,8-hexahydrophthalazin-1-yl)methyl)benzamide;
N-benzyl-N-ethyl-3-((4-oxo-3,4,5,6,7,8-hexahydrophthalazin-1-yl)methyl)benzamide;
N-benzyl-N-isopropyl-3-((4-oxo-3,4,5,6,7,8-hexahydrophthalazin-1-yl)methyl)benzamide;
N-benzyl-N-butyl-3-((4-oxo-3,4,5,6,7,8-hexahydrophthalazin-1-yl)methyl)benzamide;
N,N-dibenzyl-3-((4-oxo-3,4,5,6,7,8-hexahydrophthalazin-1-yl)methyl)benzamide;
N-benzyl-N-(2-hydroxyethyl)-3-((4-oxo-3,4,5,6,7,8-hexahydrophthalazin-1-yl)methyl)benzamide;
N-methyl-3-((4-oxo-3,4,5,6,7,8-hexahydrophthalazin-1-yl)methyl)-N-(2-pyridin-2-ylethyl)benzamide;
N-(2-(3,4-dimethoxyphenyl)ethyl)-N-methyl-3-((4-oxo-3,4,5,6,7,8-hexahydrophthalazin-1-yl)methyl)benzamide;
4-(3-((4-hydroxypiperidin-1-yl)carbonyl)benzyl)-5,6,7,8-tetrahydrophthalazin-1(2H)-one;
1-(3-((4-oxo-3,4,5,6,7,8-hexahydrophthalazin-1-yl)methyl)benzoyl)piperidine-3-carboxamide;
1-(3-((4-oxo-3,4,5,6,7,8-hexahydrophthalazin-1-yl)methyl)benzoyl)piperidine-4-carboxamide;
4-(3-((4-(2-oxo-2,3-dihydro-1H-benzimidazol-1-yl)piperidin-1-yl)carbonyl)benzyl)-5,6,7,8-tetrahydrophthalazin-1 (2H)-one;
4-(3-((4-methylpiperazin-1-yl)carbonyl)benzyl)-5,6,7,8-tetrahydrophthalazin-1(2H)-one;
4-(3-((4-ethylpiperazin-1-yl)carbonyl)benzyl)-5,6,7,8-tetrahydrophthalazin-1(2H)-one;
4-(3-((4-oxo-3,4,5,6,7,8-hexahydrophthalazin-1-yl)methyl)benzoyl)piperazine-1-carbaldehyde;
4-(3-((4-acetylpiperazin-1-yl)carbonyl)benzyl)-5,6,7,8-tetrahydrophthalazin-1(2H)-one;
4-(3-((4-(2-hydroxyethyl)piperazin-1-yl)carbonyl)benzyl)-5,6,7,8-tetrahydrophthalazin-1(2H)-one;
4-(3-((4-phenylpiperazin-1-yl)carbonyl)benzyl)-5,6,7,8-tetrahydrophthalazin-1(2H)-one;
4-(3-((4-pyridin-2-ylpiperazin-1-yl)carbonyl)benzyl)-5,6,7,8-tetrahydrophthalazin-1(2H)-one;
4-(3-((4-pyrimidin-2-ylpiperazin-1-yl)carbonyl)benzyl)-5,6,7,8-tetrahydrophthalazin-1(2H)-one;
4-(3-((4-(2-(2-hydroxyethoxy)ethyl)piperazin-1-yl)carbonyl)benzyl)-5,6,7,8-tetrahydrophthalazin-1 (2H)-one;
4-(3-((4-(2-fluorophenyl)piperazin-1-yl)carbonyl)benzyl)-5,6,7,8-tetrahydrophthalazin-1(2H)-one;
4-(3-((4-(4-fluorophenyl)piperazin-1-yl)carbonyl)benzyl)-5,6,7,8-tetrahydrophthalazin-1(2H)-one;
4-(3-((4-(2-chlorophenyl)piperazin-1-yl)carbonyl)benzyl)-5,6,7,8-tetrahydrophthalazin-1(2H)-one;
4-(3-((4-methyl-1,4-diazepan-1-yl)carbonyl)benzyl)-5,6,7,8-tetrahydrophthalazin-1(2H)-one;
4-(3-(1,1-dioxido-1,2-thiazinan-2-yl)-4-fluorobenzyl)-5,6,7,8-tetrahydrophthalazin-1(2H)-one;
8-(4-fluoro-3-(2-oxoazetidin-1-yl)benzyl)pyrido(2,3-d)pyridazin-5(6H)-one;
8-(3-chloro-4-fluorobenzyl)-2,3,4,6-tetrahydropyrido(2,3-d)pyridazin-5(1H)-one;
4-(1-(4-fluoro-3-(2-oxoazetidin-1-yl)phenyl)ethyl)-5,6,7,8-tetrahydrophthalazin-1(2H)-one;
1-(3-((4-oxo-3,4,5,6,7,8-hexahydrophthalazin-1-yl)methyl)phenyl)pyrrolidine-2,5-dione;
N-(2-fluoro-5-((4-oxo-3,4,5,6,7,8-hexahydrophthalazin-1-yl)methyl)phenyl)-2-(2-oxopyrrolidin-1-yl)acetamide;
N-(2-fluoro-5-((4-oxo-3,4,5,6,7,8-hexahydrophthalazin-1-yl)methyl)phenyl)-5-methyl-1-phenyl-1 H-pyrazole-4-carboxamide;
N-(2-fluoro-5-((4-oxo-3,4,5,6,7,8-hexahydrophthalazin-1-yl)methyl)phenyl)-5-oxohexanamide;
N-(2-fluoro-5-((4-oxo-3,4,5,6,7,8-hexahydrophthalazin-1-yl)methyl)phenyl)-3-methoxypropanamide;
N-(2-fluoro-5-((4-oxo-3,4,5,6,7,8-hexahydrophthalazin-1-yl)methyl)phenyl)-N'-phenylpentanediamide;
benzyl 2-(dimethylamino)-5-((4-oxo-3,4,5,6,7,8-hexahydrophthalazin-1-yl)methyl)phenylcarbamate;
8-(4-fluoro-3-(2-oxoazetidin-1-yl)benzyl)-2,3,4,6-tetrahydropyrido(2,3-d)pyridazin-5(1H)-one;
4-(3-bromo-4-fluorophenyl)-5,6,7,8-tetrahydrophthalazin-1(2H)-one;
4-(4-fluoro-3-(2-oxoazetidin-1-yl)phenyl)-5,6,7,8-tetrahydrophthalazin-1(2H)-one;
2-fluoro-5-((5-oxo-5,6-dihydropyrido(2,3-d)pyridazin-8-yl)methyl)benzamide;
8-(3-amino-4-fluorobenzyl)pyrido(2,3-d)pyridazin-5(6H)-one;
8-(4-fluoro-3-(2-oxopyrrolidin-1-yl)benzyl)-2,3,4,6-tetrahydropyrido(2,3-d)pyridazin-5(1H)-one;
methyl 2-fluoro-5-((5-oxo-1,2,3,4,5,6-hexahydropyrido(2,3-d)pyridazin-8-yl)methyl)benzoate;
8-(3-amino-4-fluorobenzyl)-2,3,4,6-tetrahydropyrido(2,3-d)pyridazin-5(1H)-one;
2-fluoro-5-((5-oxo-1,2,3,4,5,6-hexahydropyrido(2,3-d)pyridazin-8-yl)methyl)benzoic acid;
N-ethyl-2-fluoro-5-((5-oxo-1,2,3,4,5,6-hexahydropyrido(2,3-d)pyridazin-8-yl)methyl)benzamide;
N-cyclobutyl-2-fluoro-5-((5-oxo-1,2,3,4,5,6-hexahydropyrido(2,3-d)pyridazin-8-yl)methyl)benzamide;
2-fluoro-5-((5-oxo-1,2,3,4,5,6-hexahydropyrido(2,3-d)pyridazin-8-yl)methyl)-N-(2-pyrrolidin-1-ylethyl)benzamide;
8-(4-fluoro-3-((4-(morpholin-4-ylcarbonyl)piperazin-1-yl)carbonyl)benzyl)-2,3,4,6-tetrahydropyrido(2,3-d)pyridazin-5(1H)-one;
N-(2-fluoro-5-((5-oxo-1,2,3,4,5,6-hexahydropyrido(2,3-d)pyridazin-8-yl)methyl)phenyl)-N'-phenylpentanediamide;
1-(2-fluoro-5-((5-oxo-1,2,3,4,5,6-hexahydropyrido(2,3-d)pyridazin-8-yl)methyl)phenyl)pyrrolidine-2,5-dione;
N-(2-fluoro-5-((5-oxo-1,2,3,4,5,6-hexahydropyrido(2,3-d)pyridazin-8-yl)methyl)phenyl)-3-methoxypropanamide;
N-(2-fluoro-5-((5-oxo-1,2,3,4,5,6-hexahydropyrido(2,3-d)pyridazin-8-yl)methyl)phenyl)-5-oxohexanamide;
N-(2-fluoro-5-((5-oxo-1,2,3,4,5,6-hexahydropyrido(2,3-d)pyridazin-8-yl)methyl)phenyl)-3-phenoxypropanamide;
N-(2-fluoro-5-((5-oxo-1,2,3,4,5,6-hexahydropyrido(2,3-d)pyridazin-8-yl)methyl)phenyl)-4-oxo-4-phenylbutanamide;
2-(4-(benzyloxy)phenoxy)-N-(2-fluoro-5-((5-oxo-1,2,3,4,5,6-hexahydropyrido(2,3-d)pyridazin-8-yl)methyl)phenyl)acetamide;
N-(2-fluoro-5-((5-oxo-1,2,3,4,5,6-hexahydropyrido(2,3-d)pyridazin-8-yl)methyl)phenyl)-2-(4-methoxyphenoxy)acetamide;
N-cyclopropyl-2-fluoro-5-((5-oxo-1,2,3,4,5,6-hexahydropyrido(2,3-d)pyridazin-8-yl)methyl)benzamide;
8-(3-((4-(2-ethoxyethyl)piperazin-1-yl)carbonyl)-4-fluorobenzyl)-2,3,4,6-tetrahydropyrido(2,3-d)pyridazin-5(1H)-one;
2-fluoro-5-((5-oxo-1,2,3,4,5,6-hexahydropyrido(2,3-d)pyridazin-8-yl)methyl)-N-(2-piperidin-1-ylethyl)benzamide;
2-fluoro-5-((5-oxo-1,2,3,4,5,6-hexahydropyrido(3,2-d)pyridazin-8-yl)methyl)-N-(2-oxo-2-(piperidin-1-yl)ethyl)benzamide;
4-(4-fluoro-3-((4-pyrimidin-2-ylpiperazin-1-yl)carbonyl)benzyl)-5,6,7,8-tetrahydrophthalazin-1(2H)-one; and
4-(4-fluoro-3-(2-oxopyrrolidin-1-yl)phenyl)-5,6,7,8-tetrahydrophthalazin-1(2H)-one;
and therapeutically salts, prodrugs, esters, amides, salts of prodrugs, salts of esters and salts of amides thereof.

### DETAILED DESCRIPTION OF THE INVENTION

Variable moieties of compounds herein are represented by identifiers (capital letters with numerical and/or alphabetical superscripts) and may be specifically embodied.

It is meant to be understood that proper valences arc maintained for all combinations herein, that monovalent moieties having more than one atom are attached through their left ends.

It is also meant to be understood that a specific embodiment of a variable moiety may be the same or different as another specific embodiment having the same identifier.
Abbreviations which have been used in the descriptions of the schemes and the examples that follow are:
BOC is Di-*tert*-butyl dicarbonate
C-18 is dimethyl-octadecylsilane
DCI for chemical ionization for direct introduction,
DME for 1,2-dimethoxyethane,
DMSO for dimethylsulfoxide,
ESI for electrospray ionization,
HATU for O-(7-azabenzotriazol-1-yl)-N,N,N',N'-tetramethyluronium hexafluorophosphate,
HPLC for high performance liquid chromatography,
MS for mass spectrometry,
TFA for trifluoroacetic acid,
As used in reference to ¹H NMR, the symbol "δ" refers to a ¹H NMR chemical shift.
As used in reference to ¹H NMR, the abbreviation "br" refers to a broad ¹H NMR signal.
As used in reference to ¹H NMR, the abbreviation "d" refers to a doublet ¹H NMR peak.
As used in reference to ¹H NMR, the abbreviation "dd" refers to a doublet of doublets ¹H NMR peak.
As used in reference to ¹H NMR, the abbreviation "m" refers to a multiplet ¹H NMR peak. As used in reference to ¹H NMR, the abbreviation "q" refers to a quartet ¹H NMR peak.
As used in reference to ¹H NMR, the abbreviation "s" refers to a singlet ¹H NMR peak.
As used in reference to ¹H NMR, the abbreviation "t" refers to a triplet ¹H NMR peak.

The term "alkenyl," as used herein, means monovalent, straight or branched chain hydrocarbon moieties having one or more than one carbon-carbon double bonds, such as C₂-alkenyl, C₃-alkenyl, C₄-alkenyl, C₅-alkenyl, C₆-alkenyl and the like.

The term "alkyl," as used herein, means monovalent, saturated, straight or branched chain hydrocarbon moieties, such as C₁-alkyl, C₂-alkyl, C₃-alkyl, C₄-alkyl, C₅-alkyl, C₆-alkyl and the like.

The term "alkynyl," as used herein, means monovalent, straight or branched chain hydrocarbon moieties having one or more than one carbon-carbon triple bonds, such as C₂-alkynyl, C₃-alkynyl, C₄-alkynyl, C₅-alkynyl, C₆-alkynyl and the like.

The term "cycloalkane," as used herein, means saturated cyclic or bicyclic hydrocarbon moieties, such as C₄-cycloalkane, C₅-cycloalkane, C₆-cycloalkane, C₇-cycloalkane, C₈-cycloalkane, C₉-cycloalkane, C₁₀-cycloalkane, C₁₁-cycloalkane, C₁₂-cycloalkane and the like.

The term "cycloalkyl," as used herein, means monovalent, saturated cyclic and bicyclic hydrocarbon moieties, such as C₃-cycloalkyl, C₄-cycloalkyl, C₅-cycloalkyl, C₆-cycloalkyl, C₇-cycloalkyl, C₈-cycloalkyl, C₉-cycloalkyl, C₁₀-cycloalkyl, C₁₁-cycloalkyl, C₁₂-cycloalkyl and the like.

The term "cycloalkene," as used herein, means cyclic and bicyclic hydrocarbon moieties having one or more than one carbon-carbon double bonds, such as C₅-cycloalkene, C₆-cycloalkene, C₇-cycloalkene, C₈-cycloalkene, C₉-cycloalkene, C₁₀-cycloalkene, C₁₁-cycloalkene, C₁₂-cycloalkene and the like.

The term "cycloalkenyl," as used herein, means monovalent, cyclic hydrocarbon moieties having one or more than one carbon-carbon double bonds, such as C₄-cycloalkenyl, C₅-cycloalkenyl, C₆-cycloalkenyl, C₇-cycloalkenyl, C₈-cycloalkenyl, C₉-cycloalkenyl, C₁₀-cycloalkenyl, C₁₁-cycloalkenyl, C₁₂-cycloalkenyl and the like.

The term "heteroarene," as used herein, means furan, imidazole, isothiazole, isoxazole, 1,2,3-oxadiazole, 1,2,5-oxadiazole, 1,3,4-oxadiazole, oxazole, pyrazine, pyrazole, pyridazine, pyridine, pyrimidine, pyrrole, thiazole, 1,3,4-thiadiazole, thiophene, triazine and 1,2,3-triazole.

The term "heteroaryl," as used herein, means furanyl, imidazolyl, isothiazolyl, isoxazolyl, 1,2,3-oxadiazoyl, 1,2,5-oxadiazolyl, 1,3,4-oxadiazolyl, oxazolyl, pyrazinyl, pyrazolyl, pyridazinyl, pyridinyl, pyrimidinyl, pyrrolyl, tetrazolyl, thiazolyl, 1,2,3-thiadiazoyl, 1,2,5-thiadiazolyl, 1,3,4-thiadiazolyl, thiophenyl, triazinyl and 1,2,3-triazolyl.

The term "heterocycloalkane," as used herein, means cycloalkane having one or two or three CH₂ moieties replaced with independently selected O, S, S(O), SO₂ or NH and one or two CH moieties unreplaced or replaced with N and also means cycloalkane having one or two or three CH₂ moieties unreplaced or replaced with independently selected O, S, S(O), SO₂ or NH and one or two CH moieties replaced with N.

The term "heterocycloalkene," as used herein, means cycloalkene having one or two or three CH₂ moieties replaced with independently selected O, S, S(O), SO₂ or NH and one or two CH moieties unreplaced or replaced with N and also means cycloalkene having one or two or three CH₂ moieties unreplaced or replaced with independently selected O, S, S(O), SO₂ or NH and one or two CH moieties replaced with N.

The term "heterocycloalkyl," as used herein, means cycloalkyl having one or two or three CH₂ moieties replaced with independently selected O, S, S(O), SO₂ or NH and one or two CH moieties unreplaced or replaced with N and also means cycloalkyl having one or two or three CH₂ moieties unreplaced or replaced with independently selected O, S, S(O), SO₂ or NH and one or two CH moieties replaced with N.

The term "heterocycloalkenyl," as used herein, means cycloalkenyl having one or two or three CH₂ moieties replaced with independently selected O, S, S(O), SO₂ or NH and one or two CH moieties unreplaced or replaced with N and also means cycloalkenyl having one or two or three CH₂ moieties unreplaced or replaced with independently selected O, S, S(O), SO₂ or NH and one or two CH moieties replaced with N.

The term "cyclic moiety," as used herein, means benzene, cycloalkane, cycloalkyl, cycloalkene, cycloalkenyl, hctcroarcnc, heteroaryl, heterocycloalkane, heterocycloalkyl, heterocycloalkene, heterocycloalkenyl and phenyl.

Compounds of this invention may contain asymmetrically substituted carbon atoms in the R or S configuration, wherein the terms "R" and "S" are as defined in Pure Appl. Chem. (1976) 45, 13-10. Compounds having asymmetrically substituted carbon atoms with equal amounts of R and S configurations are racemic at those atoms. Atoms having excess of one configuration over the other are assigned the configuration in excess, preferably an excess of about 85%-90%, more preferably an excess of about 95%-99%, and still more preferably an excess greater than about 99%. Accordingly, this invention is meant to embrace racemic mixtures, relative and absolute diastereoisomers and the compounds thereof.

Compounds of this invention may also contain carbon-carbon double bonds or carbon-nitrogen double bonds in the Z or E configuration, in which the term "Z" represents the larger two substituents on the same side of a carbon-carbon or carbon-nitrogen double bond and the term "E" represents the larger two substituents on opposite sides of a carbon-carbon or carbon-nitrogen double bond. The compounds of this invention may also exist as a mixture of "Z" and "E" isomers.

Compounds of this invention containing NH, C(O)H, C(O)OH, C(O)NH₂, OH or SH moieties may have attached thereto prodrug-forming moieties. The prodrug-forming moieties are removed by metabolic processes and release the compounds having the freed NH, C(O)H, C(O)OH, C(O)NH₂, OH or SH in vivo. Prodrugs are useful for adjusting such pharmacokinetic properties of the compounds as solubility and/or hydrophobicity, absorption in the gastrointestinal tract, bioavailability, tissue penetration, and rate of clearance.

Metabolites of compounds having Formula I, produced by in vitro or in vivo metabolic processes, may also have utility for treating diseases caused or exacerbated by unregulated or overexpressed poly(ADP-ribose)polymerase.

Certain precursor compounds of compounds having Formula I may be metabolized in vitro or in vivo to form compounds having Formula I and may thereby also have utility for treating diseases caused or exacerbated by unregulared or overexpressed poly(ADP-ribose)polymerase.

Compounds having Formula I may exist as acid addition salts, basic addition salts or zwitterions. Salts of compounds having Formula I are prepared during their isolation or following their purification. Acid addition salts are those derived from the reaction of a compound having Formula I with acid. Accordingly, salts including the acetate, adipate, alginate, bicarbonate, citrate, aspartate, benzoate, benzenesulfonate (besylate), bisulfate, butyrate, camphorate, camphorsufonate, digluconate, formate, fumarate, glycerophosphate, glutamate, hemisulfate, heptanoate, hexanoate, hydrochloride, hydrobromide, hydroiodide, lactobionate, lactate, maleate, mesitylenesulfonate, methanesulfonate, naphthylenesulfonate, nicotinate, oxalate, pamoate, pectinate, persulfate, phosphate, picrate, propionate, succinate, tartrate, thiocyanate, trichloroacetic, trifluoroacetic, para-toluenesulfonate and undecanoate salts of the compounds having Formula I are meant to be embraced by this invention. Basic addition salts of compounds are those derived from the reaction of the compounds having Formula I with the bicarbonate, carbonate, hydroxide, or phosphate of cations such as lithium, sodium, potassium, calcium and magnesium.

Compounds having Formula I may be administered, for example, bucally, ophthalmically, orally, osmotically, parenterally (intramuscularly, intraperintoneally intrasternally, intravenously, subcutaneously), rectally, topically, transdermally and vaginally.

Therapeutically effective amounts of a compound having Formula I depend on recipient of treatment, disease treated and severity thereof, composition comprising it, time of administration, route of administration, duration of treatment, potency, rate of clearance and whether or not another drug is co-administered. The amount of a compound having Formula I used to make a composition to be administered daily to a patient in a single dose or in divided doses is from about 0.001 to about 200 mg/kg body weight. Single dose compositions contain these amounts or a combination of submultiples thereof.

Compounds having Formula I may be administered with or without an excipient. Excipients include, for example, encapsulators and additives such as absorption accelerators, antioxidants, binders, buffers, coating agents, coloring agents, diluents, disintegrating agents, emulsifiers, extenders, fillers, flavoring agents, humectants, lubricants, perfumes, preservatives, propellants, releasing agents, sterilizing agents, sweeteners, solubilizers, wetting agents and mixtures thereof.

Compounds having Formula I may be radiolabeled with a radioactive isotope such as carbon (i.e. ¹³C), hydrogen (i.e. ³H), nitrogen (i.e. ¹⁵N), phosphorus (i.e. ³²P), sulfur (i.e. ³⁵S), iodide (i.e. ¹²⁵I) and the like. Radioactive isotopes may be incorporated into the compounds having Formula I by reacting the same and a radioactive derivitizing agent or by incorporating a radiolabeled intermediate into their syntheses. The radiolabeled compounds of Formula I are useful for both prognostic and diagnostic applications and for in vivo and in vitro imaging.

Compounds having Formula I may be incorporated into devices such as, but not limited to, arterio-venous grafts, billiary stents, by-pass grafts, catheters, central nervous system shunts, coronary stents, drug delivery balloons, peripheral stents and ureteural stents, each of which may be used in areas such as, but not limited to, the vasculature for introduction of a compound having Formula I into selected tissues or organs in the body. One measure of the effectivness of compounds having Formula I is reduction or elimination of device-associated thrombi and complications associated therewith.

Compounds having Formula I can used as a radiosensitizers which enhance the efficacy of radiotherapy. Examples of radiotherapy include, but are not limited to, external beam radiotherapy, teletherapy, brachtherapy and sealed and unsealed source radiotherapy.

Excipients for preparation of compositions comprising a compound having Formula I to be administered orally include, for example, agar, alginic acid, aluminum hydroxide, benzyl alcohol, benzyl benzoate, 1,3-butylene glycol, carbomers, castor oil, cellulose, cellulose acetate, cocoa butter, corn starch, corn oil, cottonseed oil, cross-povidone, diglycerides, ethanol, ethyl cellulose, ethyl laureate, ethyl olcatc, fatty acid esters, gelatin, germ oil, glucose, glycerol, groundnut oil, hydroxypropylmethyl celluose, isopropanol, isotonic saline, lactose, magnesium hydroxide, magnesium stearate, malt, mannitol, monoglycerides, olive oil, peanut oil, potassium phosphate salts, potato starch, povidone, propylene glycol, Ringer's solution, safflower oil, sesame oil, sodium carboxymethyl cellulose, sodium phosphate salts, sodium lauryl sulfate, sodium sorbitol, soybean oil, stearic acids, stearyl fumarate, sucrose, surfactants, talc, tragacanth, tetrahydrofurfuryl alcohol, triglycerides, water and mixtures thereof. Excipients for preparation of compositions comprising a compound having Formula I to be administered ophthalmically or orally include, for example, 1,3-butylene glycol, castor oil, corn oil, cottonseed oil, ethanol, fatty acid esters of sorbitan, germ oil, groundnut oil, glycerol, isopropanol, olive oil, polyethylene glycols, propylene glycol, sesame oil, water and mixtures thereof. Excipients for preparation of compositions comprising a compound having Formula I to be administered osmotically include, for example, chlorofluoro-hydrocarbons, ethanol, water and mixtures thereof. Excipients for preparation of compositions comprising a compound having Formula I to be administered parenterally include, for example, 1,3-butanediol, castor oil, corn oil, cottonseed oil, dextrose, germ oil, groundnut oil, liposomes, oleic acid, olive oil, peanut oil, Ringer's solution, safflower oil, sesame oil, soybean oil, U.S.P. or isotonic sodium chloride solution, water and mixtures thereof. Excipients for preparation of compositions comprising a compound having Formula I to be administered rectally or vaginally include, for example, cocoa butter, polyethylene glycol, wax and mixtures thereof.

Compounds having formula I are also expected to be useful when used with alkylating agents, angiogenesis inhibitors, antibodies, antimetabolites, antimitotics, antiproliferatives, aurora kinase inhibitors, Bcr-Abl kinase inhibitors, biologic response modifiers, cyclin-dependent kinase inhibitors, cell cycle inhibitors, cyclooxygenase-2 inhibitors, leukemia viral oncogene homolog (ErbB2) receptor inhibitors, growth factor inhibitors, heat shock protein (HSP)-90 inhibitors, histone deacetylase (HDAC) inhibitors inhibitors, hormonal therapies, immunologicals, intercalating antibiotics, kinase inhibitors, mammalian target of rapomycin inhibitors, mitogen-activated extracellular signal-regulated kinase inhibitors, non-steroidal anti-inflammatory drugs (NSAID's), platinum chemotherapeutics, polo-like kinase inhibitors, proteasome inhibitors, purine analogs, pyrimidine analogs, receptor tyrosine kinase inhibitors, retinoids/deltoids plant alkaloids, topoisomerase inhibitors and the like.

Alkylating agents include altretamine, AMD-473, AP-5280, apaziquone, bendamustine, brostallicin, busulfan, carboquone, carmustine (BCNU), chlorambucil, Cloretazine™ (VNP 40101M), cyclophosphamide, decarbazine, estramustine, fotemustine, glufosfamide, ifosfamide, KW-2170, lomustine (CCNU), mafosfamide, melphalan, mitobronitol, mitolactol, nimustine, nitrogen mustard N-oxide, ranimustine, temozolomide, thiotepa, treosulfan, trofosfamide and the like.

Angiogenesis inhibitors include endothelial-specific receptor tyrosine kinase (Tie-2) inhibitors, epidermal growth factor receptor (EGFR) inhibitors, insulin growth factor-2 receptor (IGFR-2) inhibitors, matrix metalloproteinase-2 (MMP-2) inhibitors, matrix metalloproteinase-9 (MMP-9) inhibitors, platelet-derived growth factor receptor (PDGFR) inhibitors, thrombospondin analogs vascular endothelial growth factor receptor tyrosine kinase (VEGFR) inhibitors and the like.

Aurora kinase inhibitors include AZD-1152, MLN-8054, VX-680 and the like.

Bcr-Abl kinase inhibitors include DASATINIB^{®} (BMS-354825), GLEEVEC^{®} (imatinib) and the like.

CDK inhibitors include AZD-5438, BMI-1040, BMS-032, BMS-387, CVT-2584, flavopyridol, GPC-286199, MCS-5A, PD0332991, PHA-690509, seliciclib (CYC-202, R-roscovitine), ZK-304709 and the like.

COX-2 inhibitors include ABT-963, ARCOXIA^{®} (etoricoxib), BEXTRA^{®} (valdecoxib), BMS347070, CELEBREX™ (celecoxib), COX-189 (lumiracoxib), CT-3, DERAMAXX^{®} (deracoxib), JTE-522, 4-methyl-2-(3,4-dimethylphenyl)-1-(4-sulfamoylphenyl-1H-pyrrole), MK-663 (etoricoxib), NS-398, parecoxib, RS-57067, SC-58125, SD-8381, SVT-2016, S-2474, T-614, VIOXX^{®} (rofecoxib) and the like.

EGFR inhibitors include ABX-EGF, anti-EGFr immunoliposomes, EGF-vaccine, EMD-7200, ERBITUX^{®} (cetuximab), HR3, IgA antibodies, IRESSA^{®} (gefitinib), TARCEVA^{®} (erlotinib or OSI-774), TP-38, EGFR fusion protein, TYKERB^{®} (lapatinib) and the like.

ErbB2 receptor inhibitors include CP-724-714, CI-1033 (canertinib), Herceptin^{®} (trastuzumab), TYKERB^{®} (lapatinib), OMNITARG^{®} (2C4, petuzumab), TAK-165, GW-572016 (ionafarnib), GW-282974, EKB-569, PI-166, dHER2 (HER2 vaccine), APC-8024 (HER-2 vaccine), anti-HER/2neu bispecific antibody, B7.her2IgG3, AS HER2 trifunctional bispecfic antibodies, mAB AR-209, mAB 2B-1 and the like.

Histone deacetylase inhibitors include depsipeptide, LAQ-824, MS-275, trapoxin, suberoylanilide hydroxamic acid (SAHA), TSA, valproic acid and the like.

HSP-90 inhibitors include 17-AAG-nab, 17-AAG, CNF-101, CNF-1010, CNF-2024, 17-DMAG, geldanamycin, IPI-504, KOS-953, MYCOGRAB^{®}, NCS-683664, PU24FC1, PU-3, radicicol, SNX-2112, STA-9090 VER49009 and the like.

MEK inhibitors include ARRY-142886, ARRY-438162 PD-325901, PD-98059 and the like.

mTOR inhibitors include AP-23573, CCI-779, everolimus, RAD-001, rapamycin, temsirolimus and the like.

Non-steroidal anti-inflammatory drugs include AMIGESIC^{®} (salsalate), DOLOBID^{®} (diflunisal), MOTRIN^{®} (ibuprofen), ORUDIS^{®} (ketoprofen), RELAFEN^{®} (nabumetone), FELDENE^{®} (piroxicam) ibuprofin cream, ALEVE^{®} and NAPROSYN^{®} (naproxen), VOLTAREN^{®} (diclofenac), INDOCIN^{®} (indomethacin), CLINORIL^{®} (sulindac), TOLECTIN^{®} (tolmetin), LODINE^{®} (etodolac), TORADOL^{®} (ketorolac), DAYPRO^{®} (oxaprozin) and the like.

PDGFR inhibitors include C-451, CP-673, CP-868596 and the like.

Platinum chemotherapeutics include cisplatin, ELOXATIN^{®} (oxaliplatin) eptaplatin, lobaplatin, nedaplatin, PARAPLATIN^{®} (carboplatin), satraplatin and the like.

Polo-like kinase inhibitors include BI-2536 and the like.

Thrombospondin analogs include ABT-510, ABT-567, ABT-898, TSP-1 and the like.

VEGFR inhibitors include AVASTIN^{®} (bevacizumab), ABT-869, AEE-788, ANGIOZYME™, axitinib (AG-13736), AZD-2171, CP-547,632, IM-862, Macugen (pegaptamib), NEXAVAR^{®} (sorafenib, BAY43-9006), pazopanib (GW-786034), (PTK-787, ZK-222584), SUTENT^{®} (sunitinib, SU-11248), VEGF trap, vatalanib, ZACTIMA™ (vandetanib, ZD-6474) and the like.

Antimetabolites include ALIMTA^{®} (premetrexed disodium, LY231514, MTA), 5-azacitidine, XELODA^{®} (capecitabine), carmofur, LEUSTAT^{®} (cladribine), clofarabine, cytarabine, cytarabine ocfosfate, cytosine arabinoside, decitabine, deferoxamine, doxifluridine, eflornithine, EICAR, enocitabine, ethnylcytidine, fludarabine, hydroxyurea, 5-fluorouracil (5-FU) alone or in combination with leucovorin, GEMZAR^{®} (gemcitabine), hydroxyurea, ALKERAN^{®}(melphalan), mercaptopurine, 6-mercaptopurine riboside, methotrexate, mycophenolic acid, nelarabine, nolatrexed, ocfosate, pelitrexol, pentostatin, raltitrexed, Ribavirin, triapine, trimetrexate, S-1, tiazofurin, tegafur, TS-1, vidarabine, UFT and the like.

Antibiotics include intercalating antibiotics aclarubicin, actinomycin D, amrubicin, annamycin, adriamycin, BLENOXANE^{®} (bleomycin), daunorubicin, CAELYX^{®} or MYOCET^{®} (doxorubicin), elsamitrucin, epirbucin, glarbuicin, ZAVEDOS^{®} (idarubicin), mitomycin C, nemorubicin, neocarzinostatin, peplomycin, pirarubicin, rebeccamycin, stimalamer, streptozocin, VALSTAR^{®} (valrubicin), zinostatin and the like.

Topoisomerase inhibitors include aclarubicin, 9-aminocamptothecin, amonafide, amsacrine, becatecarin, belotecan, BN-80915, CAMPTOSAR^{®} (irinotecan hydrochloride), camptothecin, CARDIOXANE^{®} (dexrazoxine), diflomotecan, edotecarin, ELLENCE^{®} or PHARMORUBICIN^{®} (epirubicin), etoposide, exatecan, 10-hydroxycamptothecin, gimatecan, lurtotecan, mitoxantrone, orathecin, pirarbucin, pixantrone, rubitecan, sobuzoxane, SN-38, tafluposide, topotecan and the like.

Antibodies include AVASTIN^{®} (bevacizumab), CD40-specific antibodies, chTNT-1/B, denosumab, ERBITUX^{®} (cetuximab), HUMAX-CD4^{®} (zanolimumab), IGF1R-specific antibodies, lintuzumab, PANOREX^{®} (edrecolomab), RENCAREX^{®} (WX G250), RITUXAN^{®} (rituximab), ticilimumab, trastuzimab and and the like.

Hormonal therapies include ARIMIDEX^{®} (anastrozole), AROMASIN^{®} (exemestane), arzoxifene, CASODEX^{®} (bicalutamide), CETROTIDE^{®} (cetrorelix), degarelix, deslorelin, DESOPAN^{®} (trilostane), dexamethasone, DROGENIL^{®}, (flutamide), EVISTA^{®} (raloxifene), fadrozole, FARESTON^{®} (toremifene), FASLODEX^{®} (fulvestrant),FEMARA^{®}, (letrozole), formestane, glucocorticoids, HECTOROL^{®} or RENAGEL^{®} (doxercalciferol), lasofoxifene, leuprolide acetate, MEGACE^{®} (megesterol), MIFEPREX^{®} (mifepristone), NILANDRON™ (nilutamide), NOLVADEX^{®} (tamoxifen citrate), PLENAXIS™ (abarelix), predisone, PROPECIA^{®} (finasteride), rilostane, SUPREFACT^{®} (buserelin), TRELSTAR^{®} (luteinizing hormone releasing hormone (LHRH)), vantas, VETORYL^{®}, (trilostane or modrastane), ZOLADEX^{®} (fosrelin, goserelin) and the like.

Deltoids and retinoids include seocalcitol (EB1089, CB1093), lexacalcitrol (KH1060), fenretinide, PANRETIN^{®} (aliretinoin), ATRAGEN^{®} (liposomal tretinoin), TARGRETIN^{®} (bexarotene), LGD-1550 and the like.

Plant alkaloids include, but are not limited to, vincristine, vinblastine, vindesine, vinorelbine and the like.

Proteasome inhibitors include VELCADE^{®} (bortezomib), MG132, NPI-0052, PR-171 and the like.

Examples of immunologicals include interferons and other immune-enhancing agents. Interferons include interferon alpha, interferon alpha-2a, interferon alpha-2b, interferon beta, interferon gamma-1a ACTIMMUNE^{®} (interferon gamma-1b), or interferon gamma-n1, combinations thereof and the like. Other agents include ALFAFERONE^{®}, BAM-002, BEROMUN^{®} (tasonermin), BEXXAR^{®} (tositumomab), CamPath^{®} (alemtuzumab), CTLA4 (cytotoxic lymphocyte antigen 4), decarbazine, denileukin, epratuzumab, GRANOCYTE^{®} (lenograstim), lentinan, leukocyte alpha interferon, imiquimod, MDX-010, melanoma vaccine, mitumomab, molgramostim, MYLOTARG™ (gemtuzumab ozogamicin), NEUPOGEN^{®} (filgrastim), OncoVAC-CL, OvaRex^{®} (oregovomab), pemtumomab (Y-muHMFG1), PROVENGE^{®}, sargaramostim, sizofilan, teceleukin, TheraCys^{®}, ubenimex, VIRULIZIN^{®}, Z-100, WF-10, PROLEUKIN^{®} (aldesleukin), ZADAXIN^{®} (thymalfasin), ZENAPAX^{®} (daclizumab), ZEVALIN^{®} (90Y-Ibritumomab tiuxetan) and the like.

Biological response modifiers are agents that modify defense mechanisms of living organisms or biological responses, such as survival, growth, or differentiation of tissue cells to direct them to have anti-tumor activity and include include krestin, lentinan, sizofiran, picibanil PF-3512676 (CpG-8954), ubenimex and the like.

Pyrimidine analogs include cytarabine (ara C), cytosine arabinoside, doxifluridine, FLUDARA^{®} (fludarabine), 5-FU (5-fluorouracil), floxuridine, GEMZAR^{®} (gemcitabine), TOMUDEX^{®} (ratitrexed), TROXATYL™ (triacetyluridine troxacitabine) and the like.

Purine analogs include LANVIS^{®} (thioguanine) and PURI-NETHOL^{®} (mercaptopurine).

Antimitotic agents include batabulin, epothilone D (KOS-862), N-(2-((4-hydroxyphenyl)amino)pyridin-3-yl)-4-methoxybenzenesulfonamide, ixabepilone (BMS 247550), paclitaxel, TAXOTERE^{®} (docetaxel), PNU100940 (109881), patupilone, XRP-9881, vinflunine, ZK-EPO and the like.

Compounds of the present invention are also intended to be used as a radiosensitizer that enhances the efficacy of radiotherapy. Examples of radiotherapy include, but are not limited to, external beam radiotherapy, tclcthcrapy, brachthcrapy and scaled and unscaled source radiotherapy.

Additionally, compounds having formula I may be combined with other chemptherapeutic agents such as ABRAXANE™ (ABI-007), ABT-100 (farnesyl transferase inhibitor), ADVEXIN^{®}, ALTOCOR^{®} or MEVACOR^{®} (lovastatin), AMPLIGEN^{®} (poly I:poly C12U, a synthetic RNA), APTOSYN™ (exisulind), AREDIA^{®} (pamidronic acid), arglabin, L-asparaginase, atamestane (1-methyl-3,17-dione-androsta-1,4-diene), AVAGE^{®} (tazarotne), AVE-8062, BEC2 (mitumomab), cachectin or cachexin (tumor necrosis factor), canvaxin (vaccine), CeaVac™ (cancer vaccine), CELEUK^{®} (celmoleukin), CEPLENE^{®} (histamine dihydrochloride), CERVARIX™ (human papillomavirus vaccine), CHOP^{®} (C: CYTOXAN^{®} (cyclophosphamide); H: ADRIAMYCIN^{®} (hydroxydoxorubicin); O: Vincristine (ONCOVIN^{®}); P: prednisone), CyPat™, combrestatin A4P, DAB(389)EGF or TransMID-107R™ (diphtheria toxins), dacarbazine, dactinomycin, 5,6-dimethylxanthenone-4-acetic acid (DMXAA), eniluracil, EVIZON™ (squalamine lactate), DIMERICINE^{®} (T4N5 liposome lotion), discodermolide, DX-8951f (exatecan mesylate), enzastaurin, EP0906, GARDASIL^{®} (quadrivalent human papillomavirus (Types 6, 11, 16, 18) recombinant vaccine), gastrimmune, genasense, GMK (ganglioside conjugate vaccine), GVAX^{®} (prostate cancer vaccine), halofuginone, histerelin, hydroxycarbamide, ibandronic acid, IGN-101, IL-13-PE38, IL-13-PE38QQR (cintredekin besudotox), IL-13-pseudomonas exotoxin, interferon-α, interferon-γ, JUNOVAN™ or MEPACT™ (mifamurtide), lonafarnib, 5,10-methylenetetrahydrofolate, miltefosine (hexadecylphosphocholine), NEOVASTAT^{®}(AE-941), NEUTREXIN^{®} (trimetrexate glucuronate), NIPENT^{®} (pentostatin), ONCONASE^{®} (a ribonuclease enzyme), ONCOPHAGE^{®} (melanoma vaccine treatment), OncoVAX (IL-2 Vaccine), ORATHECIN™ (rubitecan), OSIDEM^{®} (antibody-based cell drug), OvaRex^{®} MAb ( murine monoclonal antibody), paditaxel, PANDIMEX™ (aglycone saponins from ginseng comprising 20(S)protopanaxadiol (aPPD) and 20(S)protopanaxatriol (aPPT)), panitumumab, PANVAC^{®}-VF (investigational cancer vaccine), pegaspargase, PEG Interferon A, phenoxodiol, procarbazine, rebimastat, REMOVAB^{®} (catumaxomab), REVLIMID^{®} (lenalidomide), RSR13 (efaproxiral), SOMATULINE^{®} LA (lanreotide), SORIATANE^{®} (acitretin), staurosporine (Streptomyces staurospores), talabostat (PT100), TARGRETIN^{®} (bexarotene), Taxoprexin^{®} (DHA-paclitaxel), TELCYTA™ (TLK286), tcmilifcnc, TEMODAR^{®} (tcmozolomidc), tesmilifene, thalidomide, THERATOPE^{®} (STn-KLH), thymitaq (2-amino-3,4-dihydro-6-methyl-4-oxo-5-(4-pyridylthio)quinazoline dihydrochloride), TNFerade™ (adenovector: DNA carrier containing the gene for tumor necrosis factor-α), TRACLEER^{®} or ZAVESCA^{®} (bosentan), tretinoin (Retin-A), tetrandrine, TRISENOX^{®} (arsenic trioxide), VIRULIZIN^{®}, ukrain (derivative of alkaloids from the greater celandine plant), vitaxin (anti-alphavbeta3 antibody), XCYTRIN^{®} (motexafin gadolinium), XINLAY™ (atrasentan), XYOTAX™ (paclitaxel poliglumex), YONDELIS™ (trabectedin), ZD-6126, ZINECARD^{®} (dexrazoxane), zometa (zolendronic acid), zorubicin and the like.

In one embodiment, compounds having Formula I are used in a method of treating cancer in a mammal comprising administering thereto a therapeutically acceptable amount of a compound of claim 1 in combination with a chemotherapeutic agent selected from temozolomide, dacarbazine, cyclophosphamide, carmustine, melphalan, lomustine, carboplatin, cisplatin, 5-FU +/- leucovorin, gemcitabine, methotrexate, bleomycin, irinotecan, camptothecin, or topotecan.

It is expected that compounds having formula I would also inhibit growth of cells derived from a pediatric cancer or neoplasm including embryonal rhabdomyosarcoma, pediatric acute lymphoblastic leukemia, pediatric acute myelogenous leukemia, pediatric alveolar rhabdomyosarcoma, pediatric anaplastic ependymoma, pediatric anaplastic large cell lymphoma, pediatric anaplastic medulloblastoma, pediatric atypical teratoid/rhabdoid tumor of the central nervous syatem, pediatric biphenotypic acute leukemia, pediatric Burkitts lymphoma, pediatric cancers of Ewing's family of tumors such as primitive neuroectodermal rumors, pediatric diffuse anaplastic Wilm's tumor, pediatric favorable histology Wilm's tumor, pediatric glioblastoma, pediatric medulloblastoma, pediatric neuroblastoma, pediatric neuroblastoma-derived myelocytomatosis, pediatric pre-B-cell cancers (such as leukemia), pediatric psteosarcoma, pediatric rhabdoid kidney tumor, pediatric rhabdomyosarcoma, and pediatric T-cell cancers such as lymphoma and skin cancer and the like (commonly-owned United States Application Ser No. 10/988,338), Cancer Res., 2000, 60, 6101-10); and autoimmune disorders include, acquired immunodeficiency disease syndrome, autoimmune lymphoprolifcrativc syndrome, hemolytic anemia, inflammatory diseases, thrombocytopcnia and the like (Current Allergy and Asthma Reports 2003, 3:378-384; Br. J. Haematol. 2000 Sep; 110(3): 584-90; Blood 2000 Feb 15;95(4):1283-92; and New England Journal of Medicine 2004 Sep; 351(14): 1409-1418).

### PARP Enzyme Inhihition Assay

Nicotinamide[2,5',8-3H]adenine dinucleotide and strepavidin SPA beads were purchased from Amersham Biosiences. Recombinant Human Poly(ADP-Ribose) Polymerase (PARP), purified from E.coli and 6-Biotin-17-NAD⁺, were purchase from Trevigen. NAD⁺, histone, aminobenzamide, 3-amino benzamide and Calf Thymus DNA (dcDNA) were purchased from Sigma. Stem loop oligonucleotide containing MCAT sequence was obtained from Qiagen. The oligos were dissoloved to 1 mM in annealing buffer containing 10 mM Tris HCl pH 7.5, 1 mM EDTA, and 50 mM NaCl, incubated for 5 minutes at 95°C, and annealed at 45°C for 45 minutes. Histone H1 (95% electrophoretically pure) was purchased from Roche. Biotinylated histone H1 was prepared by treating the protein with Sulfo-NHS-LC-Biotin from Pierce. The biotinylation reaction was conducted by slowly and intermittently adding 3 equivalents of 10mM Sulfo-NHS-LC-Biotin to 100µM Histone H1 in phosphate-buffered saline, pH 7.5, at 4°C with gentle vortexing over 1 minute followed by subsequent 4°C incubation for 1 hour. Streptavidin coated (FlashPlate Plus) microplates were purchased from Perkin Elmer.

PARP1 assay was conducted in PARP assay buffer containing 50 mM Tris pH 8.0, 1mM DTT, 4 mM MgCl₂. PARP reactions contained 1.5 µM [³H]-NAD¹ (1.6uCi/mmol), 200 nM biotinylated histone H1, 200 nM slDNA, and 1 nM PARP enzyme. Auto reactions utilizing SPA bead-based detection were carried out in 100 µL volumes in white 96 well plates. Reactions were initiated by adding 50 µl of 2X NAD⁺ substrate mixture to 50 µL of 2X enzyme mixture containing PARP and DNA. These reactions were terminated by the addition of 150 µL of 1.5 mM benzamide (~1000-fold over its IC50). 170 µL of the stopped reaction mixtures were transferred to streptavidin Flash Plates, incubated for 1 hour, and counted using a TopCount microplate scintillation counter. The EC₅₀ₛ for exemplified compounds of this invention are provided in Table 1.

### Cellular PARP assay:

C41 cells were treated with a compound of this invention for 30 minutes in 96 well plate. PARP was then activated by damaging DNA with 1 mM H₂O₂ for 10 minutes. The cells were then washed with ice-cold PBS once and fixed with pre-chilled methanol:acetone (7:3) at -20°C for 10 minutes. After air-drying, the plates were rehydrated with PBS and blocked 5% non-fat dry milk in PBS-TWEEN20® (Sigma, St. Louis, MO) (0.05%) (blocking solution) for 30 minutes at room temperature. The cells were incubated with anti-PAR antibody 10H (1:50) in Blocking solution at 37°C for 60 minutes followed by washing with PBS- TWEEN20® 5 times, and incubation with goat anti-mouse fluorescein 5(6)-isothiocyanate-coupled antibody (1:50) and 1 µg/ml 4',6-diamidino-2-phenylindole (DAPI) in blocking solution at 37°C for 60 minutes. After washing with PBS- TWEEN20® 5 times, the analysis was performed using an FMAX FLUORESCENCE MICROPLATE READER® (Molecular Devices, Sunnyvalle, CA), set at the excitation wavelength of 490 nm and emission wavelength of 528 nm fluorescein 5(6)-isothiocyanate (FITC) or the excitation wavelength of 355 nm and emission wavelength of 460 nm (DAPI). The PARP activity (FITC signal) was normalized with cell numbers (DAPI).

The cellular assay measures the formation of poly ADP-ribose by PARP within cells and demonstrates that compounds of this invention penetrate cell membranes and inhibit PARP in intact cells. Due to variability in the cellular assay, 2-(1-propylpiperidin-4-yl)-1*H-*benzimidazole-4-carboxamide was run as a comparator in each assay and data reported as the ratio of test compound EC₅₀ relative to the EC₅₀ of 2-(1-propylpiperidin-4-yl)-1*H-*benzimidazole-4-carboxamide obtained in that particular assay. The mean EC₅₀ of 2-(1-propylpiperidin-4-yl)-1*H*-benzimidazole-4-carboxamide for all assays carried out was 0.0032 µM (n=270) and generally ranged from 0.001 to 0.013 µM. (ratio EC₅₀ = EC₅₀ test compound / EC50 comparator compound). The EC₅₀ data (nM) are shown in TABLE 1.

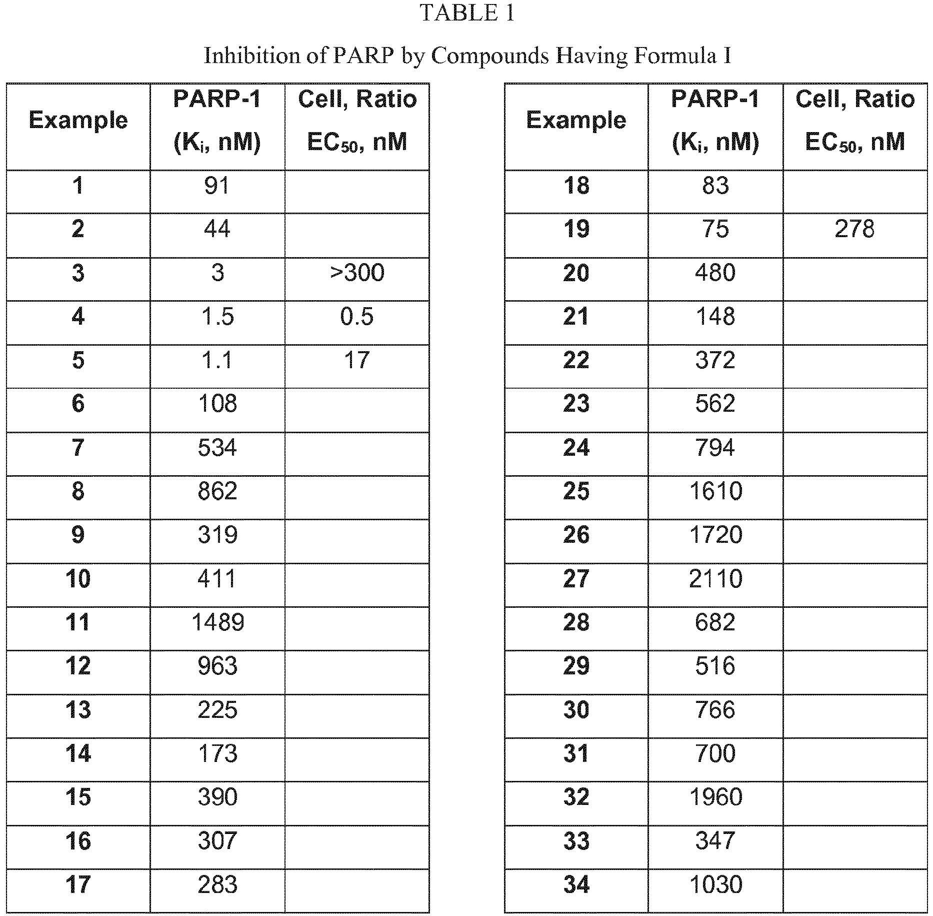

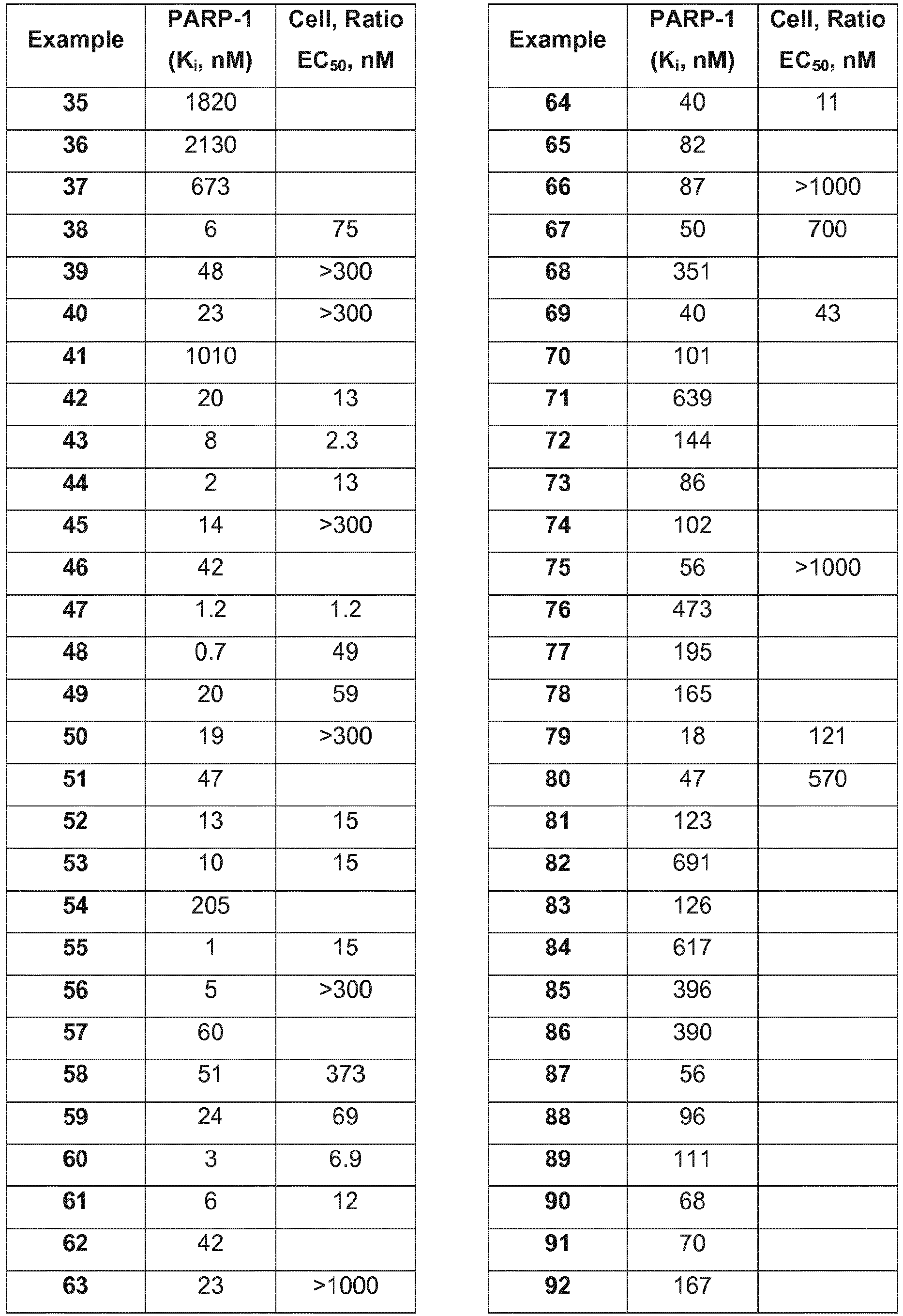

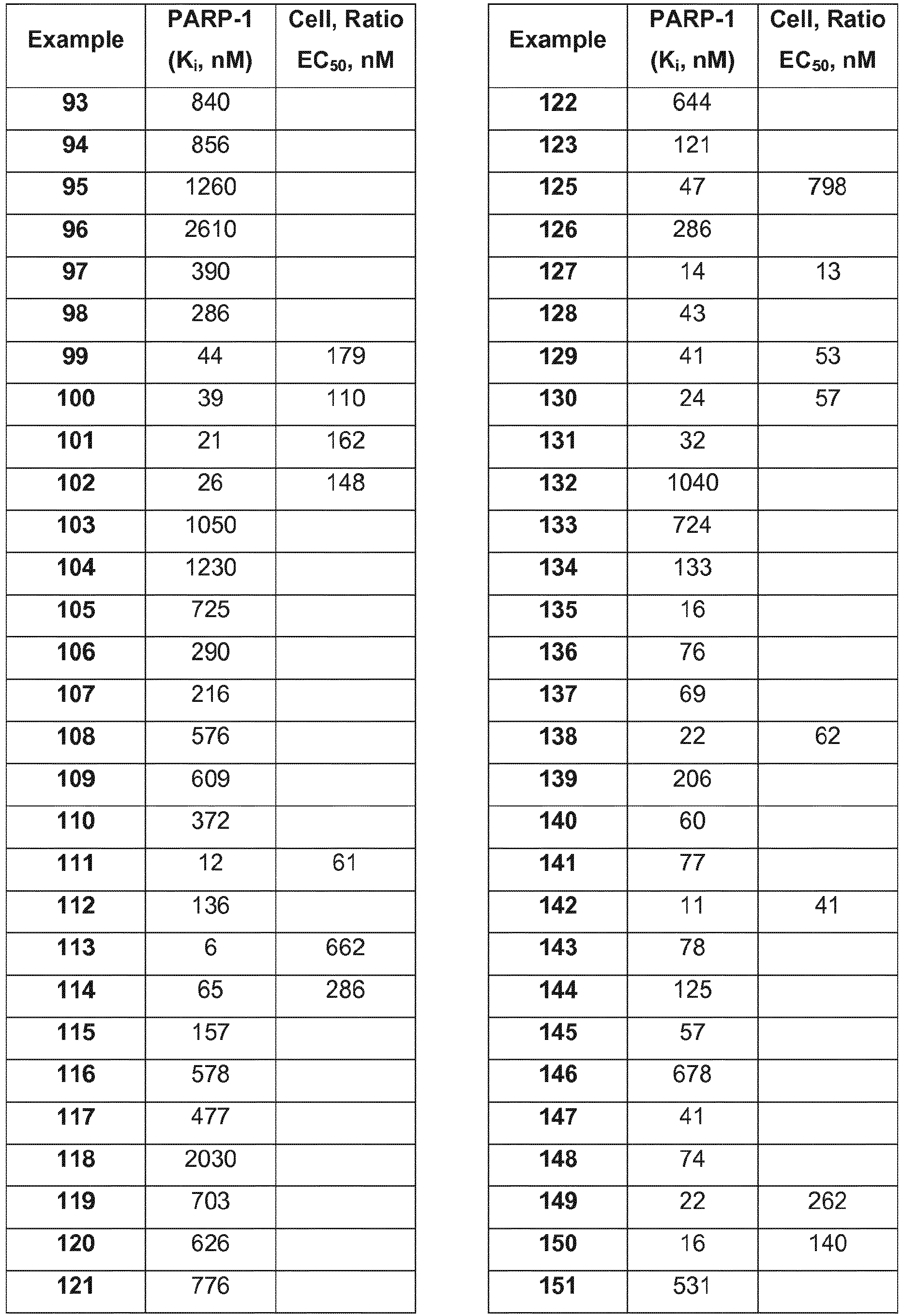

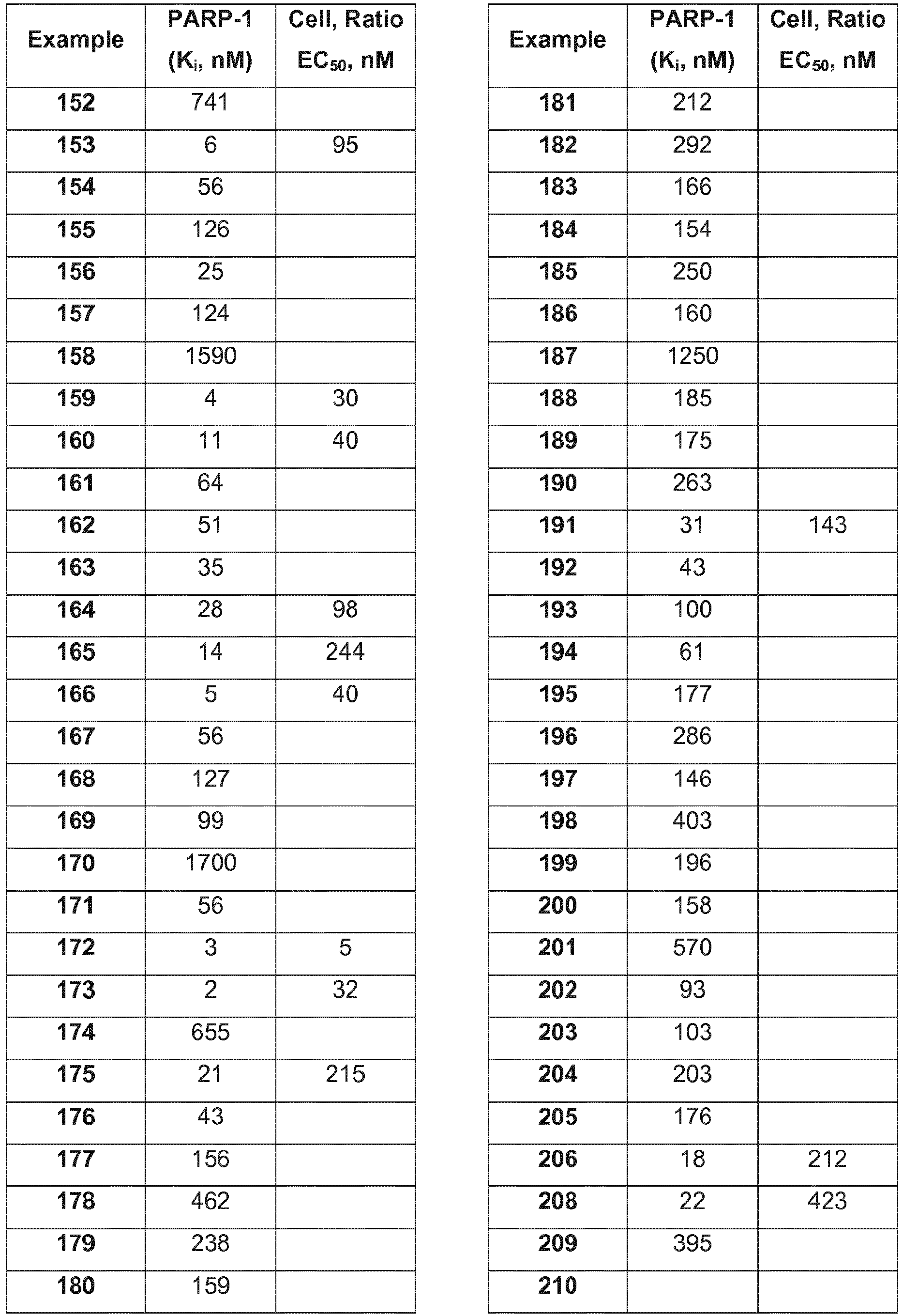

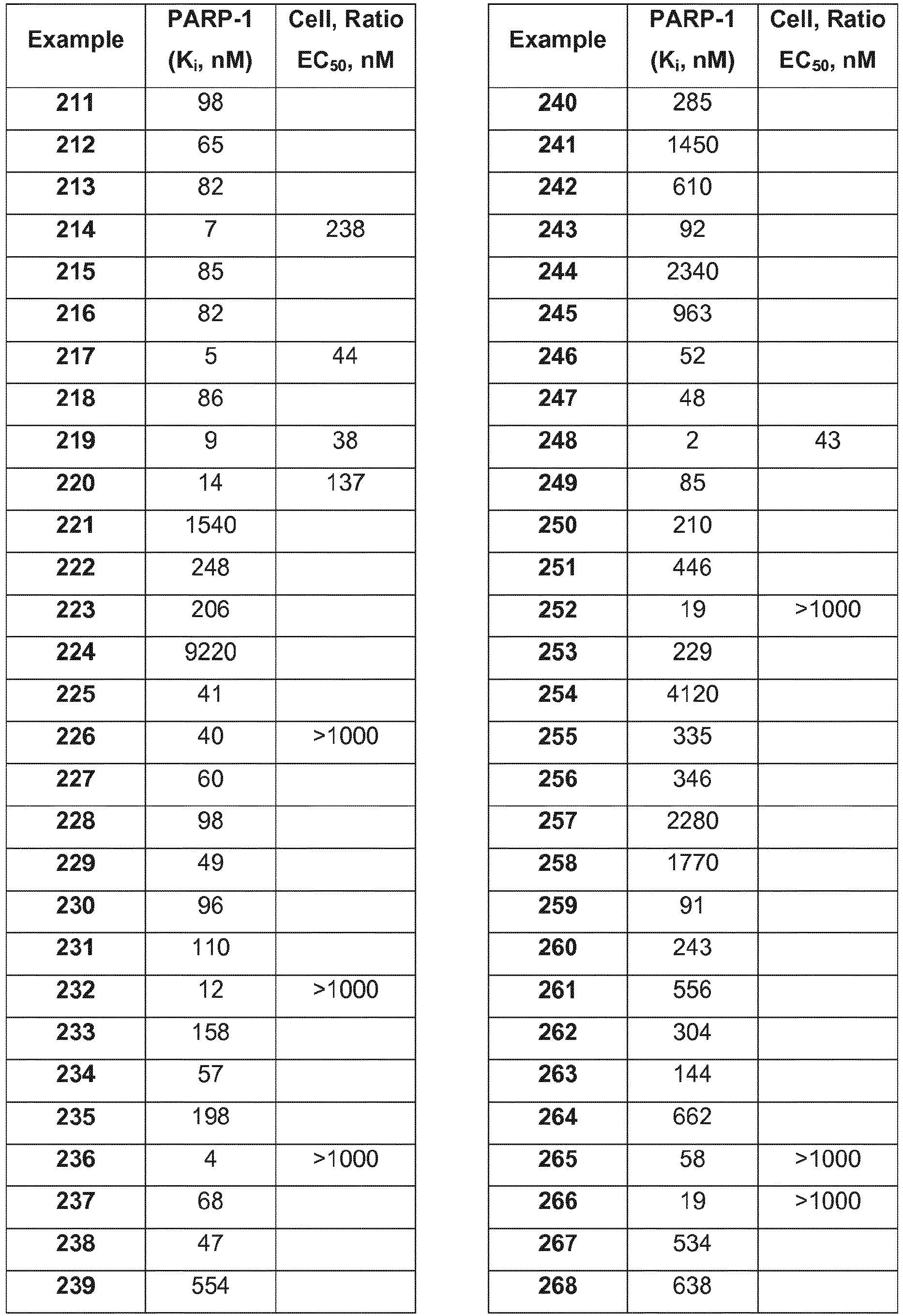

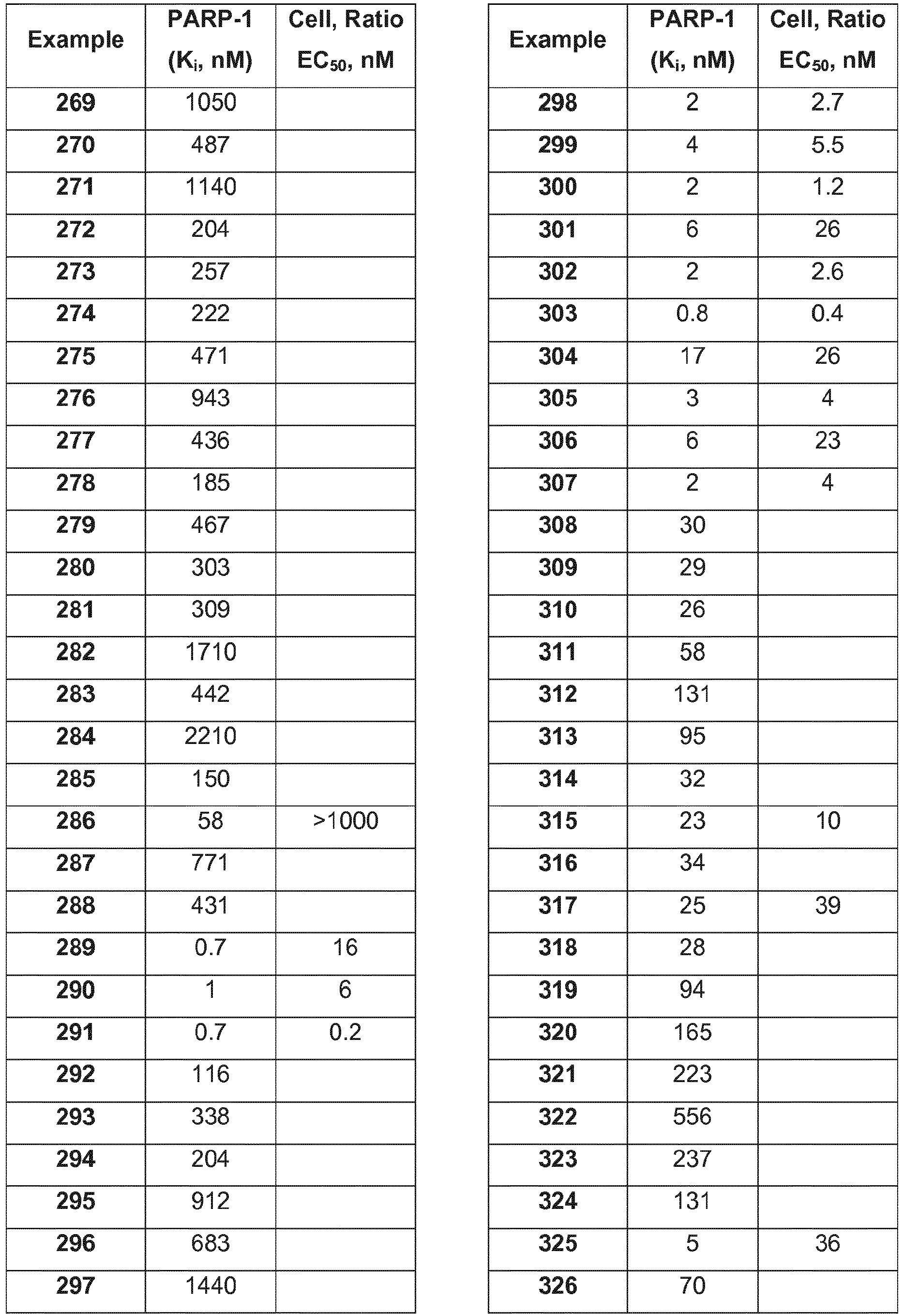

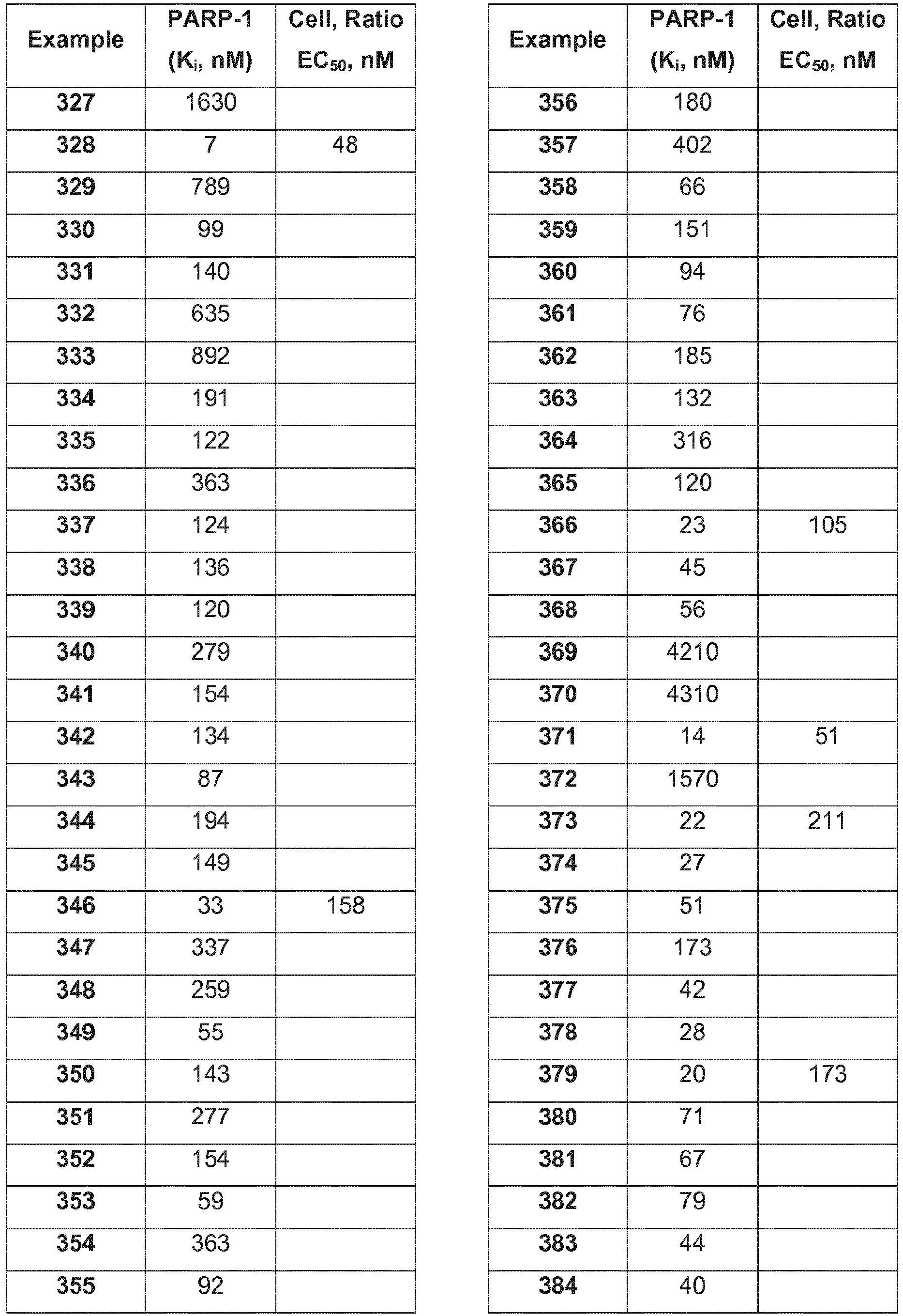

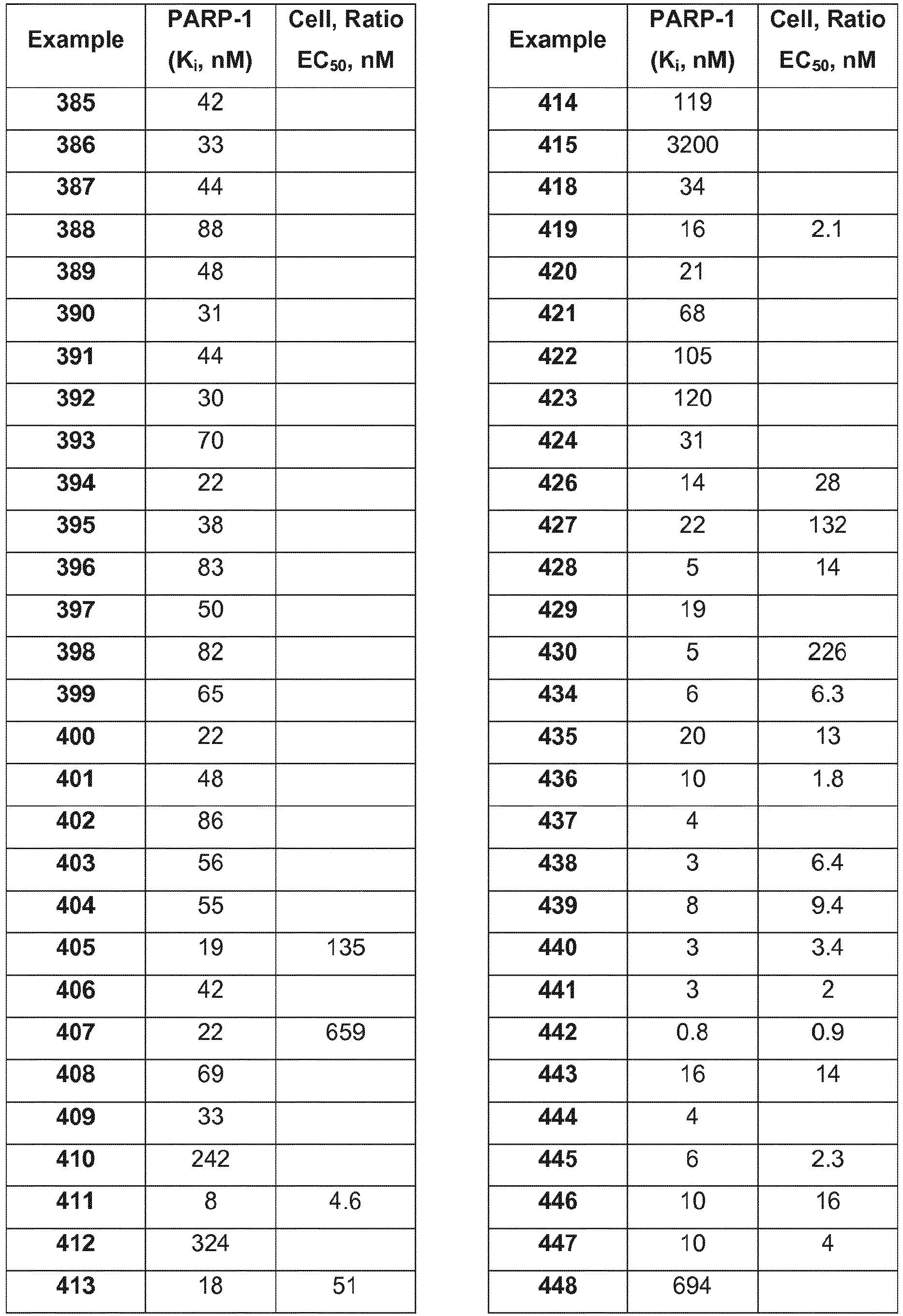

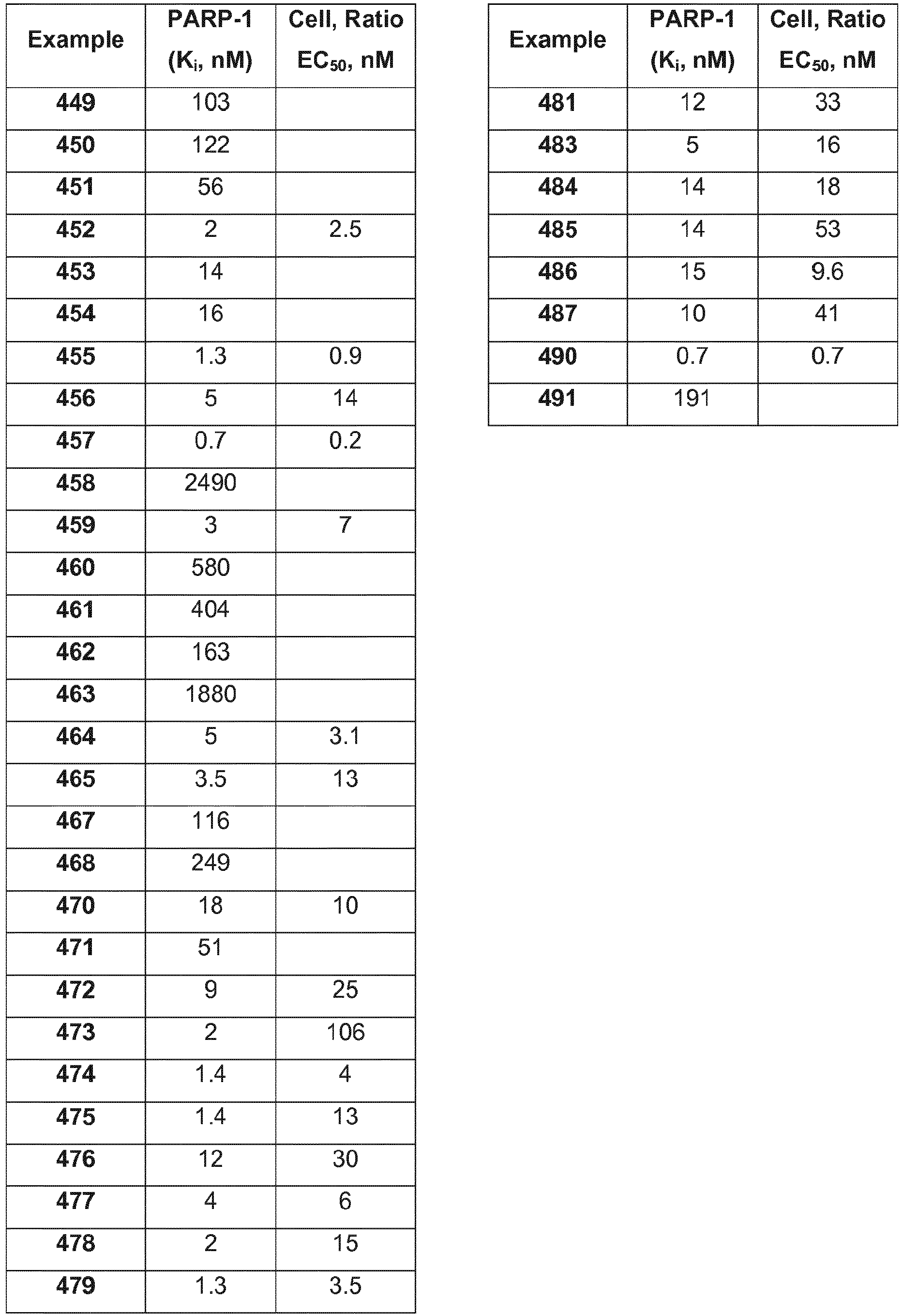

Selected compounds of Formula I wherein A¹ is R¹, wherein R¹ is unsubstituted cyclohexane which is unfused, and A² is R⁵, R⁵ is C₁-alkyl were run in the PARP Enzyme Inhihition Assay and the PARP Cellular Assay described above. Compounds outside of Formula I wherein at the position of A² is instead a bond also were run in the PARP Enzyme Inhihition Assay and the PARP Cellular Assay described above. The results of the assays are described in TABLE 2, below:

**TABLE 2**

| **A² is R⁵, wherein R⁵ is C₁ -alkyl** | **PARP -1 (Ki, nM)** | **Cell Ratio EC₅₀** | **No A²** | **PARP -1 (Ki, nM)** | **Cell Ratio EC₅₀** |
|---|---|---|---|---|---|
| 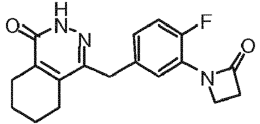 | 6 | 16.4 | 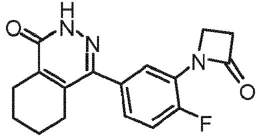 | 291 | |
| 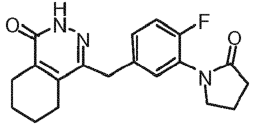 | 1.2 | 0.85 | 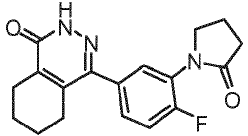 | 191 | |

Selected compounds of Formula I wherein A¹ is R¹, wherein R¹ is unsubstituted cyclohexane which is unfused, and A² is R⁵, R⁵ is C₁-alkyl wherein R⁵ is substituted with R¹⁰, wherein R¹⁰ is phenyl, either with a para-subsituted F, as shown in Formula (Is): 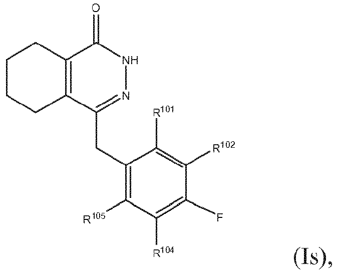 or without a para-subsituted F were run in the PARP Enzyme Inhihition Assay and the PARP Cellular Assay described above.

| **Hydrogen analogs** | **PARP-1 (Kᵢ, nM)** | **Cell Ratio EC₅₀** | **Fluoro Analogs** | **PARP-1 (Kᵢ, nM)** | **Cell Ratio EC₅₀** |
|---|---|---|---|---|---|
| 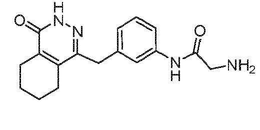 | 12 | 22.5 | 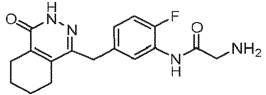 | 2.9 | 5.8 |
| 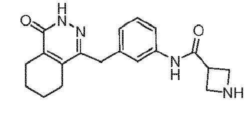 | 6.3 | 246 | 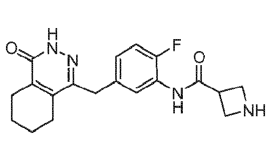 | 0.7 | 34.4 |
| 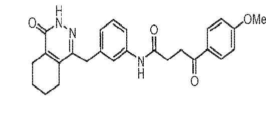 | 1.9 | 20.9 | 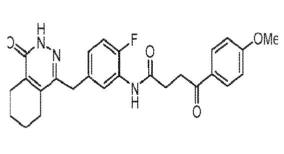 | 0.7 | 2.6 |
| 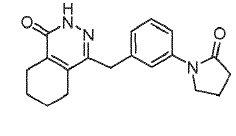 | 39.6 | 40.9 | 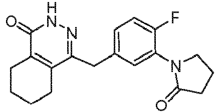 | 1.2 | 0.85 |
| 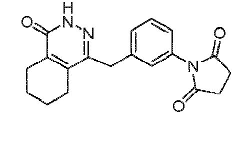 | 2 | 1.7 | 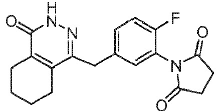 | 1.1 | 0.19 |
| 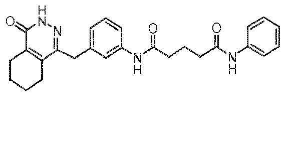 | 3.2 | 3.2 | 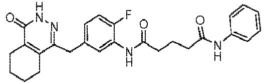 | 0.6 | 0.34 |
| 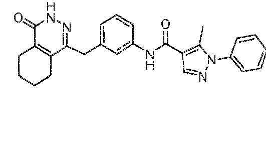 | 9 | 14.1 | 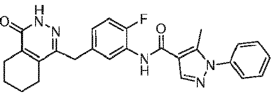 | 15.8 | 17.6 |
| 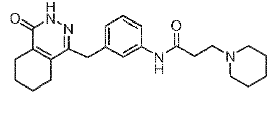 | 280 | | 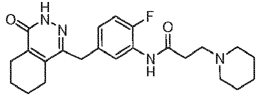 | 18.4 | 5.6 |
| 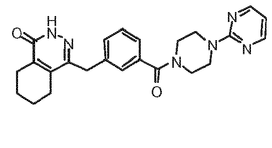 | 0.8 | 0.93 | 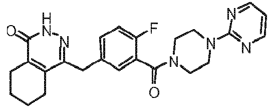 | 0.7 | 0.3 |

As PARP inhibitors, the compounds of this invention have numerous therapeutic applications related to ischemia reperfusion injury, inflammatory diseases, degenerative diseases, protection from adverse effects of cytotoxic compounds, and potentiation of cytotoxic cancer therapy. In particular, compounds of this invention potentiate radiation and chemotherapy by increasing cell death of cancer cells, limiting tumor growth, decreasing metastasis, and prolonging the survival of tumor-bearing mammals. Compounds having fomula I can treat leukemia, colon cancer, glioblastomas, lymphomas, melanomas, carcinomas of the breast, and cervical carcinomas.

Other therapeutic applications include retroviral infection, arthritis, gout, inflammatory bowel disease, CNS inflammation, multiple sclerosis, allergic encephalitis, sepsis, septic shock, hemmorhagic shock, pulmonary fibrosis, uveitis, diabetes, Parkinsons disease, myocardial infarction, stroke, other neural trauma, organ transplantation, reperfusion of the eye, reperfusion of the kidney, reperfusion of the gut, reperfusion of skeletal muscle, liver toxicity following acetominophen overdose, cardiac and kidney toxicities from doxorubicin and platinum based antineoplastic agents, and skin damage secondary to sulfur mustards. (G. Chen et al. Cancer Chemo. Pharmacol. 22 (1988), 303; C. Thiemermann et al., Proc. Natl. Acad. Sci. USA 94 (1997), 679-683 D. Weltin et al. Int. J. Immunopharmacol. 17 (1995), 265- 271; H. Kröger et al. Inflammation 20 (1996), 203-215; W. Ehrlich et al. Rheumatol. Int. 15 (1995), 171-172; C. Szabo et al., Proc. Natl. Acad. Sci. USA 95 (1998), 3867-3872; S. Cuzzocrea et al. Eur. J. Pharmacol. 342 (1998), 67-76; V. Burkhart et al., Nature Medicine (1999), 5314-19).

### Compounds of Formula I

In one embodiment of formula I 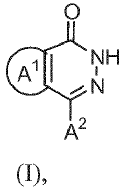 or a salt thereof, wherein
A¹ is R¹ or R², wherein A¹ is unsubstituted or substituted with one or two OH, CN, C₁-alkyl, C₂-alkyl, C₃-alkyl, C₄-alkyl, C₅-alkyl, cycloalkane, OR^{A} or NR^{A}R^{A};
R^{A} is H or alkyl;
R¹ is cycloalkane or cycloalkene each of which is unfused or fused with R^{1A};
R^{1A} is benzene, heteroarene, cycloalkane, cycloalkene, heterocycloalkane or heterocycloalkene;
R² is heterocycloalkane or heterocycloalkene; each of which is unfused or fused with R^{2A};
R^{2A} is benzene, heteroarene, cycloalkane, cycloalkene, heterocycloalkane or heterocycloalkene;
A² is OR⁴, NHR⁴, N(R⁴)₂, SR⁴, S(O)R⁴, SO₂R⁴ or R⁵;
wherein each R⁴ is C₁-alkyl, C₂-alkyl or C₃-alkyl; each of which is substituted with R¹⁰;
R⁵ is C₁-alkyl, C₂-alkyl, C₃-alkyl, C₄-alkyl or C₅-alkyl; each of which is substituted with R¹⁰, and further unsubstituted or substituted with one or two or three of independently selected OR¹⁰, NHR¹⁰, N(R¹⁰)₂, SR¹⁰, S(O)R¹⁰, SO₂R¹⁰ or CF₃;
wherein each R¹⁰ is R^{10A}, R^{10B} or R^{10C}; each of which must be attached at a carbon atom;
R^{10A} is phenyl which is unfused or fused with benzene, heteroarene, cycloalkane, cycloalkcnc, hctcrocycloalkanc or hctcrocycloalkcnc; each of which arc unfused or fused with benzene, heteroarene, cycloalkane, cycloalkene, heterocycloalkane or heterocycloalkene; 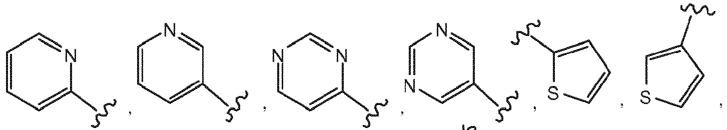
R^{10B} is 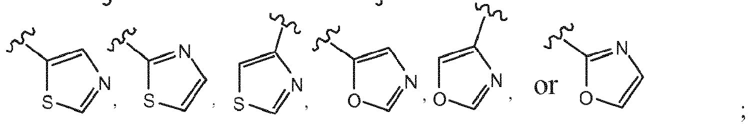 each of which is unfused or fused with benzene, heteroarene, cycloalkane, cycloalkene, heterocycloalkane or heterocycloalkene; each of which are unfused or fused with benzene, heteroarene, cycloalkane, cycloalkene, heterocycloalkane or heterocycloalkene;
R^{10C} is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene;
wherein each R¹⁰ is independently unsubstituted or substituted with one or two or three of independently selected R¹¹, OR¹¹, SR¹¹, S(O)R¹¹, SO₂R¹¹, NH₂, NHR¹¹, N(R¹¹)₂, C(O)R¹¹, C(O)OR¹¹, C(O)NH₂, C(O)NHR¹¹, C(O)N(R¹¹)₂, NHC(O)R¹¹, NR¹¹C(O)R¹¹, NHSO₂R¹¹, NR¹¹SO₂R¹¹, NHC(O)OR¹¹, NR¹¹C(O)OR¹¹, NHSO₂NH₂, NHSO₂NHR¹¹, NHSO₂N(R¹¹)₂, SO₂NH₂, SO₂NHR¹¹, SO_{2N}(R¹¹)₂, NHC(O)NH₂, NHC(O)NHR¹¹, NHC(O)N(R¹¹)₂, NR¹¹C(O)N(R¹¹)₂, NO₂, OH, (O), C(O)H, C(O)OH, CN, CF₃, OCF₃, CF₂CF₃, F, Cl, Br or I;
wherein each R¹¹ is R¹², R¹³, R¹⁴ or R¹⁵;
R¹² is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R¹³ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R¹⁴ is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R¹⁵ is alkyl, alkenyl or alkynyl; each of which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NH₂, C(O)NHR¹⁶, C(O)N(R¹⁶)₂, NHC(O)R¹⁶, NR¹⁶C(O)R¹⁶, NHC(O)OR¹⁶, NR¹⁶C(O)OR¹⁶, OH, F, Cl, Br or I;
wherein each R¹⁶ is R¹⁷ or R^{17A};
R¹⁷ is alkyl, alkenyl or alkynyl; each of which is unsubstituted or substituted with one or two of independently selected R¹⁸, C(O)OH, NH₂, NHR¹⁸ or N(R¹⁸)₂, C(O)R¹⁸, C(O)NH₂, C(O)NHR¹⁸, C(O)N(R¹⁸)₂, NHC(O)R¹⁸, NR¹⁸C(O)R¹⁸, F, Cl, Br or I;
R^{17A} is phenyl, heteroaryl, cycloalkyl, cycloalkenyl, heterocycloalkyl or heterocycloalkenyl, each of which is unfused or fused with benzene, heteroarene, cycloalkane, cycloalkene, heterocycloalkane or heterocycloalkene;
wherein each R¹⁸ is phenyl, heteroaryl, cycloalkyl, cycloalkenyl, heterocycloalkyl or heterocycloalkenyl;
wherein each of the moieties represented by R¹², R¹³, R¹⁴, R^{17A}, and R¹⁸ are independently unsubstituted or substituted with one or two or three or four of independently selected R¹⁹, OR¹⁹, SR¹⁹, S(O)R¹⁹, SO₂R¹⁹, C(O)R¹⁹, CO(O)R¹⁹, OC(O)R¹⁹, OC(O)OR¹⁹, NH₂, NHR¹⁹, N(R¹⁹)₂, NHC(O)R¹⁹, NR¹⁹C(O)R¹⁹, NHS(O)₂R¹⁹, NR¹⁹S(O)₂R¹⁹, NHC(O)OR¹⁹, NR¹⁹C(O)OR¹⁹, NHC(O)NH₂, NHC(O)NHR¹⁹, NHC(O)N(R¹⁹)₂, NR¹⁹C(O)NHR¹⁹, NR¹⁹C(O)N(R¹⁹)₂, C(O)NH₂, C(O)NHR¹⁹, C(O)N(R¹⁹)₂, C(O)NHOH, C(O)NHOR¹⁹, C(O)NHSO₂R¹⁹, C(O)NR¹⁹SO₂R¹⁹, SO₂NH₂, SO₂NHR¹⁹, SO₂N(R¹⁹)₂, C(O)H, C(O)OH, C(N)NH₂, C(N)NHR¹⁹, C(N)N(R¹⁹)₂, CNOH, CNOCH₃, OH, (O), CN, N₃, NO₂, CF₃, CF₂CF₃, OCF₃, OCF₂CF₃, F, Cl, Br or I;
wherein each R¹⁹ is R²⁰, R²¹, R²² or R²³;
R²⁰ is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R²¹ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R²² is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
R²³ is alkyl, alkenyl or alkynyl; each of which is unsubstituted or substituted with one or two of independently selected R²⁴, OR²⁴, SR²⁴, S(O)₂R²⁴, C(O)OH, NH₂, NHR²⁴ N(R²⁴)₂, C(O)R²⁴, C(O)NH₂, C(O)NHR²⁴, C(O)N(R²⁴)₂, NHC(O)R²⁴, NR²⁴C(O)R²⁴, NHC(O)OR²⁴, NR²⁴C(O)OR²⁴ , NHS(O)₂R²⁴, NR²⁴S(O)₂R²⁴, OH, F, Cl, Br or I;
wherein each R²⁴ is R^{24A} or R^{24B};
R^{24A} is phenyl, heteroaryl, cycloalkyl, cycloalkenyl, heterocycloalkyl or heterocycloalkenyl each of which is unfused or fused with benzene, heteroarene, cycloalkane, cycloalkene, heterocycloalkane or heterocycloalkene;
R^{24B} is alkyl, alkenyl or alkynyl each of which is unsubstituted or substituted with one or two of independently selected R²⁵, OR²⁵, SR²⁵, S(O)₂R²⁵, C(O)OH, NH₂, NHR²⁵ N(R²⁵)₂, C(O)R²⁵, C(O)NH₂, C(O)NHR²⁵, C(O)N(R²⁵)₂, NHC(O)R²⁵, NR²⁵C(O)R²⁵, NHC(O)OR²⁵, NR²⁵C(O)OR²⁵, OH, F, Cl, Br or I;
wherein each R²⁵ is alkyl, phenyl, heteroaryl, cycloalkyl, cycloalkenyl, heterocycloalkyl or heterocycloalkenyl ; each of which is unsubstituted or substituted with NH₂, NH(CH₃), N(CH₃)₂, OH or OCH₃;
wherein each of the moieties represented by R²⁰, R²¹, R²², and R^{24A} are independently unsubstituted or substituted with one or two of independently selected R²⁶, OR²⁶, alkenyl, alkynyl, phenyl, OH, (O), C(O)OH, CN, CF₃, OCF₃, CF₂CF₃, F, Cl, Br or I; and
R²⁶ is alkyl.

### Embodiments of Formula I

Selected subclasses of compounds of interest that fall within the scope of the compounds of formula I are shown in the various embodiments described below, wherein A¹, R¹, R^{A}, R^{1A}, R² , R^{2A} A², R⁴, R⁵, R¹⁰, R^{10A}, R^{10B}, R^{10C}, R¹¹, R¹², R¹³, R¹⁴, R¹⁵, R¹⁶, R¹⁷, R^{17A}, R¹⁸, R¹⁹, R²⁰, R²¹, R²², R²³, R^{24A}, R^{24B}, R²⁵ and R²⁶ can be as defined for the compounds of Formula I and as defined in the various embodiments described throughout this specification.

### Embodiments of A¹

In one embodiment of formula I, A¹ is R¹ or R², wherein R¹ is an unfused cycloalkane and R² is an unfused heterocycloalkane, wherein A¹ is unsubstituted or is substituted with one or two OH, CN, C₁-alkyl, C₂-alkyl, C₃-alkyl, C₄-alkyl, C₅-alkyl, cycloalkane, OR^{A} or NR^{A}R^{A}; wherein R^{A} is H or alkyl. In another embodiment of formula I, A¹ is R¹ or R², wherein R¹ is cyclohexane and R² is piperidinyl, wherein A¹ is unsubstituted or is substituted with one or two C₁-alkyl, C₂-alkyl or C₃-alkyl. In another embodiment of formula I, A¹ is R¹ or R², wherein R¹ is unsubstituted cyclohexane and R² is unsubstituted piperidinyl. In another embodiment of formula I, A¹ is R¹, and R¹ is unsubstituted cyclohexane, as shown in formula (Ia):

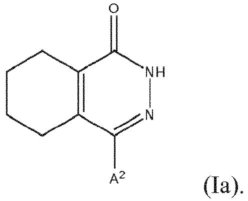

### Embodiments of A²

In one embodiment of formula I, A² is OR⁴, NHR⁴, N(R⁴)₂, SR⁴, S(O)R⁴, SO₂R⁴ or R⁵; wherein each R⁴ is C₁-alkyl, C₂-alkyl or C₃-alkyl; each of which is substituted with R¹⁰ as described in Formula I; and R⁵ is C₁-alkyl, C₂-alkyl or C₃-alkyl wherein R⁵ is substituted as described in formula I. In another embodiment of formula I, A² is R⁵, and R⁵ is C₁-alkyl, C₂-alkyl or C₃-alkyl wherein R⁵ is substituted as described in formula I. In another embodiment of formula I, A² is R⁵, wherein R⁵ is C₁-alkyl, which is substituted with R¹⁰, and further unsubstituted or substituted with one or two or three of independently selected NHR¹⁰, N(R¹⁰)₂, SR¹⁰, S(O)R¹⁰, SO₂R¹⁰ or CF₃, wherein R¹⁰ is as described in formula I. In another embodiment of formula I, A² is R⁵, wherein R⁵ is C₁-alkyl, substituted with R¹⁰ as described in Formula I and further unsubstituted as shown in formula (Ib): 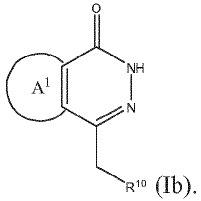 In another embodiment of formula I, A² is R⁵, wherein R⁵ is C₂-alkyl, substituted with R¹⁰ as described in Formula I and further unsubstituted as shown in formulas (Ic) and (Id): 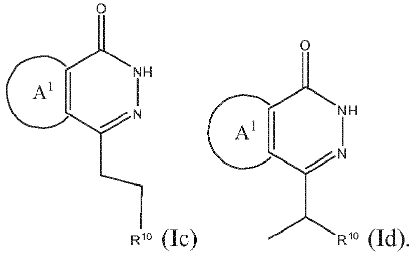 In another embodiment of formula I, A² is R⁵, wherein R⁵ is C₃-alkyl, substituted with R¹⁰ as described in Formula I and further unsubstituted. In another embodiment of formula I, A² is R⁵, wherein R⁵ is C₁-alkyl or C₂-alkyl; each of which are substituted with R¹⁰ as described in Formula I and further substituted with CF₃.

### Embodiments of R¹⁰

In one embodiment of formula I, R¹⁰ R^{10A}, R^{10B} or R^{10C}, wherein R^{10A} is phenyl which is unfused or fused with heterocycloalkane, which is fused heterocycloalkane, R^{10B} is 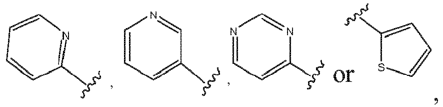 and R^{10C} is heterocycloalkyl, which is unfused, wherein R¹⁰ is optionally substituted as described in Formula I. In another embodiment of formula I, R¹⁰ is R^{10A}, R^{10B} or R^{10C}, wherein R^{10A} is phenyl which is unfused or fused with heterocycloalkane, which is fused heterocycloalkane, R^{10B} is 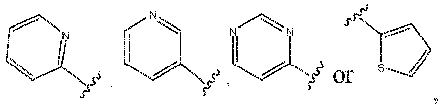 and R^{10C} is heterocycloalkyl, which is unfused; wherein R¹⁰ is substituted with F and further unsubstituted or substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br, wherein R¹¹ is as described in Formula I. In another embodiment of formula I, R¹⁰ is R^{10A}, R^{10B} or R^{10C}, wherein R^{10A} is phenyl which is unfused or fused with heterocycloalkane, which is fused heterocycloalkane, R^{10B} is 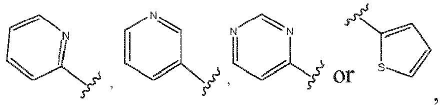 and R^{10C} is heterocycloalkyl, which is unfused; wherein R¹⁰ is substituted with F and further unsubstituted or substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein each R¹¹ is R¹², R¹³, R¹⁴ or R¹⁵; wherein R¹² is phenyl which is unfused or fused with benzene, heteroarene, heterocycloalkane or heterocycloalkene; R¹³ is heteroaryl, which is unfused; R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene; and R¹⁵ is alkyl which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I, wherein R¹⁶ is as described in Formula I. In another embodiment of formula I, R¹⁰ is R^{10A}, R^{10B} or R^{10C}, wherein R^{10A} is phenyl which is unfused or fused with heterocycloalkane, which is fused heterocycloalkane,
R^{10B} is 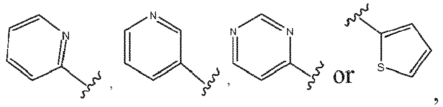 and R^{10C} is heterocycloalkyl, which is unfused; wherein R¹⁰ is substituted with F and further unsubstituted or substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein each R¹¹ is R¹², R¹³, R¹⁴ or R¹⁵; wherein R¹² is phenyl which is unfused or fused with benzene, heteroarene, heterocycloalkane or heterocycloalkene; R¹³ is heteroaryl, which is unfused;
R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene; and R¹⁵ is alkyl which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl which is unsubstituted or substituted with R¹⁸; R^{17A} is phenyl, heteroaryl, cycloalkyl, heterocycloalkyl or heterocycloalkenyl, each of which is unfused or fused with benzene or heterocycloalkane; and R¹⁸ is phenyl or heterocycloalkyl, which is unfused; wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, -N(R¹⁹)₂, NHC(O)R¹⁹, -NHS(O)₂R¹⁹, C(O)NH₂, C(O)NHR¹⁹, C(O)N(R¹⁹)₂, C(O)H, OH, (O), CN, CF₃, F, Cl, Br or I, wherein R¹⁹ is as described in Formula I. In another embodiment of formula I, R¹⁰ is R^{10A}, R^{10B} or R^{10C}, wherein R^{10A} is phenyl which is unfused or fused with heterocycloalkane, which is fused heterocycloalkane, R^{10B} is 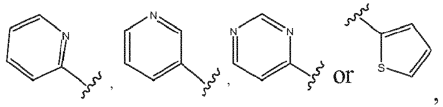 and R^{10C} is heterocycloalkyl, which is unfused; wherein R¹⁰ is substituted with F and further unsubstituted or substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹,NHSO₂N(R¹¹)₂, NO₂, OH, (O), C(O)OH, F, Cl or Br; wherein each R¹¹ is R¹², R¹³, R¹⁴ or R¹⁵; wherein R¹² is phenyl which is unfused or fused with benzene, heteroarene, heterocycloalkane or heterocycloalkene; R¹³ is heteroaryl, which is unfused;
R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene; and R¹⁵ is alkyl which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl which is unsubstituted or substituted with R¹⁸; R^{17A} is phenyl, heteroaryl, cycloalkyl, heterocycloalkyl or heterocycloalkenyl, each of which is unfused or fused with benzene or heterocycloalkane; R¹⁸ is phenyl or heterocycloalkyl, which is unfused; wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R R²¹, R²² or R²³; R²⁰ is phenyl, which is unfused; R21 is heteroaryl, which is unfused; R²² is cycloalkyl or heterocycloalkyl; each of which is unfused or fused with benzene; and R²³ is alkyl which is unsubstituted or substituted with R²⁴, OR²⁴, NHR²⁴N(R²⁴)₂, NHS(O)₂R²⁴ or OH, wherein R²⁴ is as described in Formula I. In another embodiment of formula I, R¹⁰ R^{10A}, R^{10B} or R^{10C}, wherein R^{10A} is phenyl which is unfused or fused with heterocycloalkane, which is fused heterocycloalkane, R^{10B} is 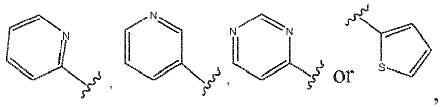 and R^{10C} is heterocycloalkyl, which is unfused; wherein R¹⁰ is substituted with F and further unsubstituted or substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein each R¹¹ is R¹², R¹³, R¹⁴ or R¹⁵; wherein R¹² is phenyl which is unfused or fused with benzene, heteroarene, heterocycloalkane or heterocycloalkene; R¹³ is heteroaryl, which is unfused;
R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene; and R¹⁵ is alkyl which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl which is unsubstituted or substituted with R¹⁸; R^{17A} is phenyl, heteroaryl, cycloalkyl, heterocycloalkyl or heterocycloalkenyl, each of which is unfused or fused with benzene or heterocycloalkane; R¹⁸ is phenyl or heterocycloalkyl, which is unfused; wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R²⁰, R²¹, R²² or R²³; R²⁰ is phenyl, which is unfused; R²¹ is heteroaryl, which is unfused; R²² is cycloalkyl or heterocycloalkyl; each of which is unfused or fused with benzene; and R²³ is alkyl which is unsubstituted or substituted with R²⁴, OR²⁴, NHR²⁴ N(R²⁴)₂, NHS(O)₂R²⁴ or OH; wherein each R²⁴ is R^{24A} or R^{24B}; R^{24A} is phenyl, cycloalkyl, heterocycloalkyl or heterocycloalkenyl, which is unfused or fused with heterocycloalkane; R^{24B} is alkyl, which is unsubstituted or substituted with OR²⁵, OH, F, Cl, Br or I; R²⁵ is alkyl, which is unsubstituted or substituted with NH₂; wherein the moieties represented by R²⁰, R²¹, R²², and R^{24A} are independently unsubstituted or substituted with one or two of independently selected R²⁶, OR²⁶ (O), F, Cl, Br or I; and R²⁶ is alkyl. In another embodiment of formula I, R¹⁰ is R^{10A}, R^{10B} or R^{10C}, wherein R^{10A} is 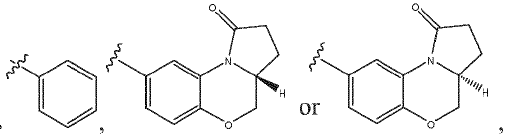 R^{10B} is 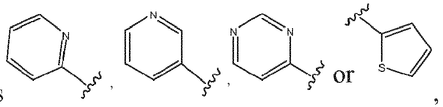 and R^{10C} is 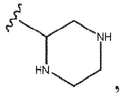 wherein R¹⁰ is optionally substituted as described in Formula I. In another embodiment of formula I, R¹⁰ is R^{10A}, wherein R^{10A} is phenyl which is unfused, wherein R¹⁰ is optionally substituted as described in Formula I. In another embodiment of formula I, R¹⁰ is R^{10A}, wherein R^{10A} is phenyl which is unfused, wherein R¹⁰ is substituted with F and further unsubstituted or substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein each R¹¹ is R¹², R¹³, R¹⁴ or R¹⁵; wherein R¹² is phenyl which is unfused or fused with benzene, heteroarene, heterocycloalkane or heterocycloalkene; R¹³ is heteroaryl, which is unfused;
R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene; and R¹⁵ is alkyl which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl which is unsubstituted or substituted with R¹⁸; R^{17A} is phenyl, heteroaryl, cycloalkyl, heterocycloalkyl or heterocycloalkenyl, each of which is unfused or fused with benzene or heterocycloalkane; R¹⁸ is phenyl or heterocycloalkyl, which is unfused; wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R²⁰, R²¹, R²² or R²³; R²⁰ is phenyl, which is unfused; R²¹ is heteroaryl, which is unfused; R²² is cycloalkyl or heterocycloalkyl; each of which is unfused or fused with benzene; and R²³ is alkyl which is unsubstituted or substituted with R²⁴, OR²⁴, NHR²⁴ N(R²⁴)₂, NHS(O)₂R²⁴ or OH; wherein each R²⁴ is R^{24A} or R^{24B}; R^{24A} is phenyl, cycloalkyl, heterocycloalkyl or heterocycloalkenyl, which is unfused or fused with heterocycloalkane; R^{24B} is alkyl, which is unsubstituted or substituted with OR²⁵, OH, F, Cl, Br or I; R²⁵ is alkyl, which is unsubstituted or substituted with NH₂; wherein the moieties represented by R²⁰, R²¹, R²², and R^{24A} are independently unsubstituted or substituted with one or two of independently selected R²⁶, OR²⁶ (O), F, Cl, Br or I; and R²⁶ is alkyl. In another embodiment of formula I, R¹⁰ is R^{10A}, wherein R^{10A} is phenyl which is unfused, wherein R¹⁰ is substituted with F and further substituted with NHC(O)R¹¹ wherein R¹¹ is R¹⁵, wherein R¹⁶ is optionally substituted as described in Formula I. In another embodiment of formula I, R¹⁰ is R^{10A}, wherein R^{10A} is phenyl which is unfused, wherein R¹⁰ is substituted with F and further substituted with R¹¹, wherein R¹¹ is R¹² or R¹⁴, wherein R¹⁴ is heterocycloalkyl which is unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I, wherein R¹⁹ is as described in Formula I. In another embodiment of formula I, R¹⁰ is R^{10A}, wherein R^{10A} is phenyl which is unfused, wherein R¹⁰ is substituted with F and further substituted with R¹¹, wherein R¹¹ is phenyl, pyrrolidinyl, azabicylclo(3.1.0)hexanyl, hexahydro-1H-isoindolyl, oxazolidinyl, azepanyl, piperidinyl, imidazolidinyl, thiazolidinyl, thiazinyl, azetidinyl, tctrahydropyrimidinyl, or azabicylo(2.2.1)hept-2-yl; each of which are independently unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, OF₃, F, Cl, Br or I wherein R¹⁹ is as described in Formula I. In another embodiment of formula I, R¹⁰ is R^{10A}, wherein R^{10A} is phenyl which is unfused, wherein R¹⁰ is substituted with F and further substituted with R¹¹, wherein R¹¹ is phenyl, pyrrolidinyl, azabicylclo(3.1.0)hexanyl, hexahydro-1H-isoindolyl, oxazolidinyl, azepanyl, piperidinyl, imidazolidinyl, thiazolidinyl, thiazinyl, azetidinyl, tetrahydropyrimidinyl, or azabicylo(2.2.1)hept-2-yl; each of which are independently substituted with one or two (O). In another embodiment of formula I, R¹⁰ is R^{10A}, wherein R^{10A} is phenyl which is unfused, wherein R¹⁰ is substituted with F and further substituted with R¹¹, wherein R¹¹ is R¹⁴ wherein R¹⁴ is heterocycloalkyl which is unsubstituted or substituted with one or two (O). In another embodiment of formula I, R¹⁰ is R^{10A}, wherein R^{10A} is phenyl which is unfused, wherein R¹⁰ is substituted with F and further substituted with R¹¹, wherein R¹¹ is R¹⁴, wherein R¹⁴ is pyrrolidinyl which is substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R19)₂, C(O)H, OH, (O), CN, CF₃, F, Cl, Br or I wherein R¹⁹ is as described in Formula I and wherein R¹⁴ is substituted with at least one (O). In another embodiment of formula I, R¹⁰ is R^{10A}, wherein R^{10A} is phenyl which is unfused, wherein R¹⁰ is substituted with F and further substituted with R¹¹, wherein R¹¹ is R¹⁴ wherein R¹⁴ is pyrrolidinyl which is substituted with one or two (O).

### Embodiments of Multiple Substituents

The following are additional embodiments of the compounds of Formula I. Unless otherwise specified, substituents are as described in Formula I.

In one embodiment of Formula I, R¹ is cycloalkane, which is unfused; R² is heterocycloalkane, which is unfused, and A² is R⁵.

### Embodiments where A¹ is Cyclohexane, A² is R⁵

In one embodiment of Formula I, A¹ is R¹, wherein R¹ is unsubstituted cyclohexane which is unfused, and A² is R⁵, which is as described in Formula I. In another embodiment of Formula I, A¹ is R¹, wherein R¹ is unsubstituted cyclohexane which is unfused, and A² is R⁵, R⁵ is C₁-alkyl, C₂-alkyl or C₃-alkyl wherein R⁵ is substituted as described in Formula I. In another embodiment of Formula I, A¹ is R¹, wherein R¹ is unsubstituted cyclohexane which is unfused, and A² is R⁵, R⁵ is C₁-alkyl, C₂-alkyl or C₃-alkyl wherein R¹⁰ is R^{10A}, wherein is R^{10A} is phenyl which is unfused and substituted with F, and further substituted with NHC(O)R¹¹, wherein R¹¹ is R¹⁵. In another embodiment of Formula 1, A¹ is R¹, wherein R¹ is unsubstituted cyclohexane which is unfused, and A² is R⁵, R⁵ is C₁-alkyl, C₂-alkyl or C₃-alkyl wherein R¹⁰ is R^{10A}, wherein is R^{10A} is phenyl which is unfused and substituted with F, and further substituted with NHC(O)R¹¹, wherein R¹¹ is R¹⁵wherein R¹⁵ is alkyl, which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl, which is unsubstituted or substituted with one or two of independently selected R¹⁸; R^{17A} is phenyl, heteroaryl, cycloalkyl, heterocycloalkyl or heterocycloalkenyl, each of which is unfused or fused with benzene or heterocycloalkane; wherein each R¹⁸ is phenyl or heterocycloalkyl; wherein each of the moieties represented by R^{17A} and R¹⁸ are independently unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R19)₂, C(O)H, OH, (O), CN, CF₃, F, Cl, Br or I; wherein each R¹⁹ is R²⁰, R²¹, R²² or R²³_{;} R²⁰ is phenyl which is unfused; R²¹ is heteroaryl which is unfused; R²² is cycloalkyl or heterocycloalkyl; each of which are unfused or fused with benzene; R²³ is alkyl, which is unsubstituted or substituted with one or two of independently selected R²⁴, OR²⁴, NHR²⁴ N(R²⁴)₂, NHS(O)₂R²⁴ or OH; wherein each R²⁴ is R^{24A} or R^{24B}; R^{24A} is unsubstituted phenyl, cycloalkyl, heterocycloalkyl or heterocycloalkenyl each of which is unfused or fused with heterocycloalkane; R^{24B} is alkyl, which is unsubstituted or substituted with one or two of independently selected OR²⁵ or OH; wherein each R²⁵ is alkyl unsubstituted or substituted with NH₂; wherein each R²⁰ is unsubstituted or substituted with one or two of independently selected R²⁶, OR²⁶, (O), F, Cl, Br or I; and R²⁶ is alkyl. In another embodiment of Formula I, A¹ is R¹, wherein R¹ is unsubstituted cyclohexane which is unfused, and A² is R⁵, R⁵ is C₁-alkyl, C₂-alkyl or C₃-alkyl wherein R¹⁰ is substituted with F, and further substituted with R¹⁴ wherein each R¹⁰ is independently unsubstituted or substituted with one or two or three of independently selected R¹¹, OR¹¹, SR¹¹, S(O)R¹¹,SO₂R¹¹, NH₂, NHR¹¹, N(R¹¹)₂, C(O)R¹¹, C(O)OR¹¹, C(O)NH₂, C(O)NHR¹¹, C(O)N(R¹¹)₂, NHC(O)R¹¹, NR¹¹C(O)R¹¹, NHSO₂R¹¹, NR¹¹SO₂R¹¹, NHC(O)OR¹¹, NR¹¹COOR¹¹, NHSO₂NH₂, NHSO₂NHR¹¹, NHSO₂N(R¹¹)₂, SO₂NH₂, SO₂NHR¹¹, SO₂N(R¹¹)₂, NHC(O)NH₂, NHC(O)NHR¹¹, NHC(O)N(R¹¹)₂, NR¹¹C(O)N(R¹¹)₂, NO₂, OH, (O), C(O)H, C(O)OH, CN, CF₃, OCF₃, CF₂CF₃, F, Cl, Br or I; wherein R¹⁴ is pyrrolidinyl, azetidinyl, pyrrolyl, 1,3-oxazolidinyl, azepanyl, piperidinyl, imidazolidinyl, tetrahydropyrimidin(2H)-yl, azabicyclo(2.2.1)heptyl or 1,6-dihydropyridazyl; each of which unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene; and wherein the moiety represented by R¹⁴ is substituted with one or two (O) substituents. In another embodiment of Formula I, A¹ is R¹, wherein R¹ is unsubstituted cyclohexane which is unfused, and A² is R⁵, R⁵ is C₁-alkyl, C₂-alkyl or C₃-alkyl wherein R⁵ is substituted with R¹⁰, and further unsubstituted or substituted with one or two or three of independently selected NHR¹⁰, N(R¹⁰)₂, SR¹⁰, S(O)R¹⁰, SO₂R¹⁰ or CF₃, wherein R¹⁰ is as described in formula I. In another embodiment of Formula I, A¹ is R¹, wherein R¹ is unsubstituted cyclohexane which is unfused, and A² is R⁵, R⁵ is C₁-alkyl, C₂-alkyl or C₃-alkyl wherein R⁵ is substituted with R¹⁰, and further unsubstituted or substituted with one CF₃, wherein R¹⁰ is as described in formula I. In another embodiment of Formula I, A¹ is R¹, wherein R¹ is unsubstituted cyclohexane which is unfused, and A² is R⁵ selected from the following Formulas (Ie), (If), (Ig), (Ih), (Ii) or (Ij): 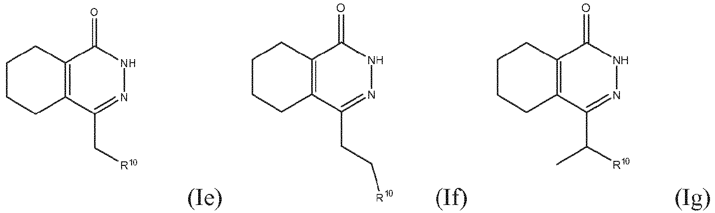 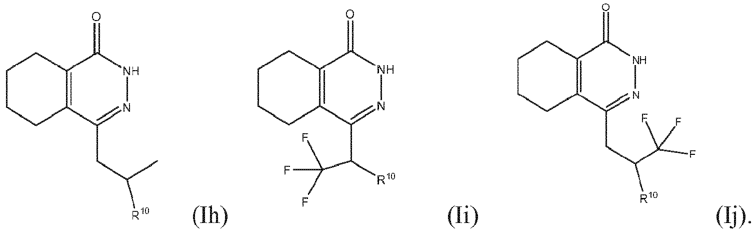 In one embodiment of Formula(Ii), R¹⁰ is R^{10A}, R^{10B} or R^{10C}; each of which must be attached at a carbon atom; R^{10A} is phenyl which is unfused or fused with heterocycloalkane, which is fused with heterocycloalkane; R^{10B} is 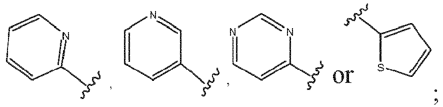 R^{10C} is heterocycloalkyl, which is unfused; wherein R¹⁰ is substituted with C(O)R¹¹, C(O)NHR¹¹, C(O)N(R¹¹)₂ or NHC(O)R¹¹, and is further unsubstituted or substituted with one or two or three of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl, Br or I; wherein each R¹¹ is R¹², R¹³, R¹⁴ or R¹⁵; R¹² is phenyl which is unfused or fused with benzene, heteroarene, heterocycloalkane or heterocycloalkene; R¹³ is heteroaryl, which is unfused; R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene; R¹⁵ is alkyl, which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹¹ or R^{17A}; R¹⁷ is alkyl, which is unsubstituted or substituted with R¹⁸; R^{17A} is phenyl, heteroaryl, cycloalkyl, heterocycloalkyl or heterocycloalkenyl, each of which is unfused or fused with benzene or heterocycloalkane; R¹⁸ is phenyl or heterocycloalkyl, which is unfused; wherein the moieties represented by R¹², R¹³, R¹⁴, R^{17A}, and R¹⁸ are independently unsubstituted or substituted with one or two independently selected R¹⁹, OR¹⁹, SR¹⁹ SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R²⁰, R²¹, R²² or R²³; R²⁰ is phenyl, which is unfused; R²¹ is heteroaryl, which is unfused; R²² is cycloalkyl,or heterocycloalkyl each of which is unfused or fused with benzene; R²³ is alkyl which is unsubstituted or substituted with one or two of independently selected R²⁴, OR²⁴, NHR²⁴ N(R²⁴)₂, NHS(O)₂R²⁴, OH, F, Cl, Br or I; wherein each R²⁴ is R^{24A} or R^{24B}; R^{24A} is phenyl, cycloalkyl, heterocycloalkyl or heterocycloalkenyl; each of which is unfused or fused with heterocycloalkane; R^{24B} is alkyl which is unsubstituted or substituted with OR²⁵, OH, F, Cl, Br or I; R²⁵ is alkyl each of which is unsubstituted or substituted with NH₂; wherein the moieties represented by R²⁰, R²¹, R²², and R^{24A} are independently unsubstituted or substituted with one or two of independently selected R²⁶, OR²⁶, (O), F, Cl, Br or I; and R²⁶ is alkyl. In another embodiment of Formula I, A¹ is R¹, wherein R¹ is unsubstituted cyclohexane which is unfused, and A² is R⁵, R⁵ is C₁-alkyl wherein R⁵ is substituted with R¹⁰, wherein R¹⁰ is as described in formula I, as described in Formula (Ie) 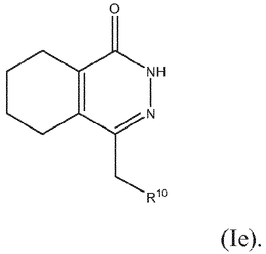 In another embodiment of Formula I, A¹ is R¹, wherein R¹ is unsubstituted cyclohexane which is unfused, and A² is R⁵, R⁵ is unbranched C₂-alkyl wherein R⁵ is substituted with R¹⁰, wherein R¹⁰ is as described in formula I, as described in Formula (If)

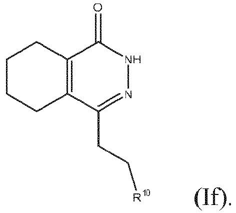

### Embodiments of Formula (Ie)

In one embodiment of Formula (Ie), R¹⁰ is R^{10A}, R^{10B} or R^{10C}, wherein R^{10A} is phenyl which is unfused or fused with heterocycloalkane, which is fused heterocycloalkane, R^{10B} is 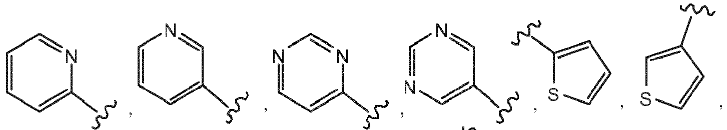 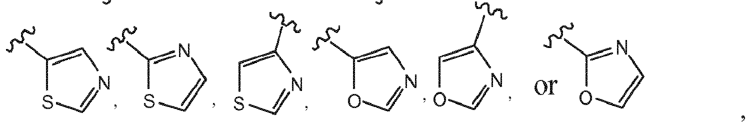 and R10^{C} is heterocycloalkyl, which is unfused; wherein R¹⁰ is substituted as described in formula I. In another embodiment of Formula (Ie), R¹⁰ is R^{10A}, R^{10B} or R^{10C}, wherein R^{10A} is phenyl which is unfused or fused with heterocycloalkane, which is fused heterocycloalkane, R^{10B} is 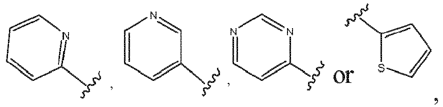 and R^{10C} is heterocycloalkyl, which is unfused; wherein R¹⁰ is substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein R¹¹ is as described in Formula I. In another embodiment of Formula (Ie), R¹⁰ is R^{10A}, R^{10B} or R^{10C}, wherein R^{10A} is phenyl which is unfused or fused with heterocycloalkane, which is fused heterocycloalkane, R^{10B} is 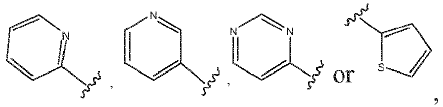 and R^{10C} is heterocycloalkyl, which is unfused; wherein R¹⁰ is substituted with F and further unsubstituted or substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein R¹¹ is as described in Formula I. In another embodiment of Formula (Ie), R¹⁰ is R^{10A}, R^{10B} or R^{10C}, wherein R^{10A} is phenyl which is unfused or fused with heterocycloalkane, which is fused heterocycloalkane, R^{10B} is 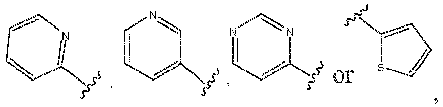 and R^{10C} is heterocycloalkyl, which is unfused; wherein R¹⁰ is substituted with F and further unsubstituted or substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹_{,} C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein R¹¹ is R¹², R¹³, R¹⁴ or R¹⁵; R¹² is phenyl which is unfused or fused with benzene, heteroarene, heterocycloalkane or heterocycloalkene; R¹³ is heteroaryl, which is unfused; R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene; R¹⁵ is alkyl which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl which is unsubstituted or substituted with R¹⁸; R^{17A} is phenyl, heteroaryl, cycloalkyl, heterocycloalkyl or heterocycloalkenyl, each of which is unfused or fused with benzene or heterocycloalkane; R¹⁸ is phenyl or heterocycloalkyl, which is unfused; wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R19)₂, C(O)H, OH, (O), CN, CF₃, F, Cl, Br or I; wherein each R¹⁹ is R²⁰, R²¹, R²² or R²³; R²⁰ is phenyl, which is unfused; R²¹ is heteroaryl, which is unfused; R²² is cycloalkyl or heterocycloalkyl; each of which is unfused or fused with benzene; R²³ is alkyl which is unsubstituted or substituted with R²⁴, OR²⁴, NHR²⁴ N(R²⁴)₂, NHS(O)₂R²⁴ or OH; wherein each R²⁴ is R^{24A} or R^{21B}. R^{24A} is phenyl, cycloalkyl, heterocycloalkyl or heterocycloalkenyl, which is unfused or fused with heterocycloalkane; R^{24B} is alkyl, which is unsubstituted or substituted with OR²⁵, OH, F, Cl, Br or I; R²⁵ is alkyl, which is unsubstituted or substituted with NH₂; wherein the moieties represented by R²⁰, R²¹, R²², and R^{24A} are independently unsubstituted or substituted with one or two of independently selected R²⁶, OR²⁶ (O), F, Cl, Br or I; and R²⁶ is alkyl. In another embodiment of Formula (Ie), R¹⁰ is R^{10A}, R^{10B} or R^{10C}, wherein R^{10A} is phenyl which is unfused, 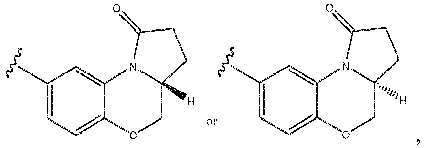 R^{10B} is 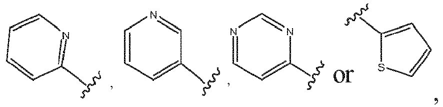 and R^{10C} is 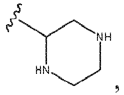 wherein R¹⁰ is optionally substituted as described in Formula I. In another embodiment of Formula (Ie), R¹⁰ is R^{10A}, R^{10B} or R^{10C} as described in Formulas (Ik), (Il), (Im), (In), (Io) or (Ip) 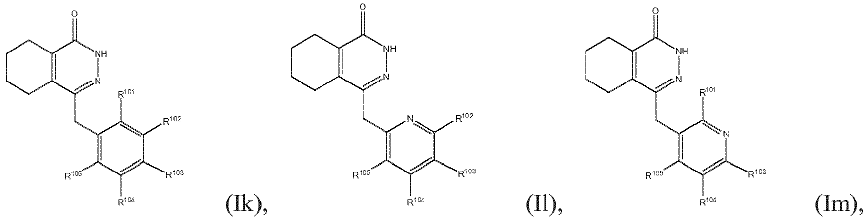 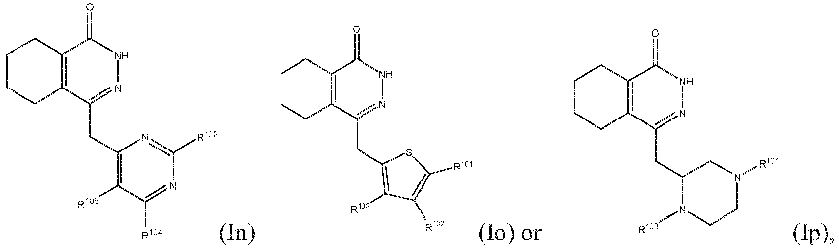 wherein R¹⁰¹, R¹⁰², R¹⁰³, R¹⁰⁴, and R¹⁰⁵, are independently selected from R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹,NR¹¹SO₂R¹¹, NHC(O)OR¹¹, NHSO₂N(R¹¹)₂, NO₂, OH, (O), C(O)OH, F, Cl or Br; wherein R¹¹ is as described in Formula I.
In another embodiment of Formula (Ie), R¹⁰ is R^{10A} or R^{10B}, as described in Formulas (Ik), (Il), (Im), (In), (Io) or (Ip). In another embodiment of Formula (Ie), R¹⁰ is phenyl, as shown in Formula (Ik): 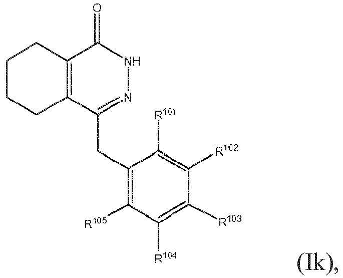 wherein R¹⁰¹, R¹⁰², R¹⁰³, R¹⁰⁴, and R¹⁰⁵, are independently selected from H, R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein R¹¹ is as described in Formula I. In another embodiment of Formula (Ik), at least one of R¹⁰¹, R¹⁰², R¹⁰³, R¹⁰⁴, and R¹⁰⁵ are F, and at least one is R¹¹, wherein R¹¹ is phenyl, pyrrolyl, azabicylclo(3.1.0)hexanyl, hexahydro-1H-isoindolyl, 1,3-oxazolidinyl, azepanyl, piperidinyl, imidazolidinyl, thiazolidinyl, thiazinyl, azetidinyl, 1,6-dihydropyridazyl, tetrahydropyrimidin(2H)-yl or azabicylo(2.2.1)hept-2-yl; each of which are independently unsubstituted or substituted with one or two or three of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R²⁰, R²¹, R²² or R²³; R²⁰ is phenyl, which is unfused; R²¹ is heteroaryl, which is unfused; R²² is cycloalkyl,or heterocycloalkyl each of which is unfused or fused with benzene; R²³ is alkyl which is unsubstituted or substituted with one or two of independently selected R²⁴, OR²⁴, NHR²⁴ N(R²⁴)₂, NHS(O)₂R¹⁹, OH, F, Cl, Br or I; wherein each R²⁴ is R^{24A} or R²⁴B; R^{24A} is phenyl, cycloalkyl, heterocycloalkyl or heterocycloalkenyl; each of which is unfused or fused with heterocycloalkane; R^{24B} is alkyl which is unsubstituted or substituted with OR²⁵, OH, F, Cl, Br or I; R²⁵ is alkyl each of which is unsubstituted or substituted with NH₂; wherein the moieties represented by R²⁰, R²¹, R²², and R^{24A} are independently unsubstituted or substituted with one or two of independently selected R²⁶, OR²⁶, (O), F, Cl, Br or I; and R²⁶ is alkyl. In another embodiment of Formula (Ik), R¹⁰¹, R¹⁰⁴, and R¹⁰⁵ are H, and R¹⁰² is R¹¹, wherein R¹¹ is selected from pyrrolidinyl, oxazolyl, imidazolidinyl, isothiazolidinyl, piperidinyl, and azepanyl, wherein R¹⁰² is substituted with one or two (O) substituents. In another embodiment of Formula (Ik), R¹⁰¹, R¹⁰⁴, and R¹⁰⁵ are H, and R¹⁰² is R¹¹, wherein R¹¹ is pyrrolidinyl.

### Further Embodiments of Formula (Ik)

In one embodiment of Formula (Ik), R¹⁰² is NHC(O)R¹¹, as described in Formula (Iq): 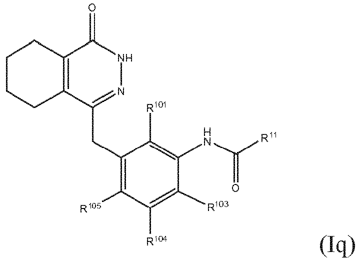 wherein R¹⁰¹, R¹⁰³, R¹⁰⁴, and R¹⁰⁵ are independently selected from H, R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein R¹¹ is as described in Formula I. In one embodiment of Formula (Iq), R¹¹ is R¹⁵, wherein R¹⁶ is optionally substituted as described in Formula I and R¹⁰¹, R¹⁰³, R¹⁰⁴, and R¹⁰⁵ are as described in Formula (Iq). In another embodiment of Formula (Iq), R¹⁰³, is F, and R¹⁰¹, R¹⁰⁴ and R¹⁰⁵ are independently selected from H, R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; and R¹¹ is R¹⁵, wherein R¹⁶ is optionally substituted as described in Formula I. In another embodiment of Formula (Iq), one of R¹⁰¹, R¹⁰³, R¹⁰⁴ and R¹⁰⁵ is F, R¹¹ is R¹⁵, wherein R¹⁶ is optionally substituted as described in Formula I. In another embodiment of Formula (Iq), R¹⁰¹, R¹⁰⁴ and R¹⁰⁵ is F. In another embodiment of Formula (Iq), R¹⁰³, is F. In another embodiment of Formula (Iq), one of R¹⁰¹, R¹⁰³, R¹⁰⁴ and R¹⁰⁵ is F, R¹¹ is R¹⁵, wherein R¹⁶ is alkyl, which is unsubstituted or substituted with one or two of independently selected R¹⁶, OR¹⁶, SR¹⁶, S(O)₂R¹⁶, C(O)OH, NH₂, NHR¹⁶ N(R¹⁶)_{2,} C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl, which is unsubstituted or substituted with one or two of independently selected R¹⁸; R^{17A} is phenyl, heteroaryl, cycloalkyl, heterocycloalkyl or heterocycloalkenyl, each of which is unfused or fused with benzene or heterocycloalkane; wherein each R¹⁸ is phenyl or heterocycloalkyl; wherein each of the moieties represented by R^{17A} and R¹⁸ are independently unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R²⁰, R²¹, R²² or R²³; R²⁰ is phenyl which is unfused; R²¹ is heteroaryl which is unfused; R²² is cycloalkyl or heterocycloalkyl; each of which are unfused or fused with benzene; R²³ is alkyl, which is unsubstituted or substituted with one or two of independently selected R²⁴, OR²⁴, NHR²⁴ N(R²⁴)₂, NHS(O)₂R²⁴ or OH; wherein each R²⁴ is R^{24A} or R^{24BA}; R^{24A} is unsubsituted phenyl, cycloalkyl, heterocycloalkyl or heterocycloalkenyl each of which is unfused or fused with heterocycloalkane; R^{24B} is alkyl, which is unsubstituted or substituted with one or two of independently selected OR²⁵ or OH; wherein each R²⁵ is alkyl unsubstituted or substituted with NH₂; wherein each R²⁰ is unsubstituted or substituted with one or two of independently selected R²⁶, OR²⁶, (O), F, Cl, Br or I; and R²⁶ is alkyl. In another embodiment of Formula (Iq), R¹⁰³, is F, and R¹⁰¹, R¹⁰⁴ and R¹⁰⁵ are each H, R¹¹ is R¹⁵, wherein R¹⁶ is optionally substituted as described in Formula I. In another embodiment of Formula (Iq), R¹¹ is R¹² or R¹⁴ wherein R¹⁴ is heterocycloalkyl which is unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R19)₂, C(O)H, OH, (O), CN, CF₃, F, Cl, Br or I; wherein R¹⁹ is as described in Formula I. In another embodiment of Formula (Iq), R¹¹ is selected from phenyl, pyrrolidinyl, azabicylclo(3.1.0)hexanyl, hexahydro-1H-isoindolyl, oxazolidinyl, azepanyl, piperidinyl, imidazolidinyl, thiazolidinyl, thiazinyl, azetidinyl, tetrahydropyrimidinyl or azabicylo(2.2.1)hept-2-yl; each of which are independently unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, C(O)NH₂, C(O)NHR¹⁹, C(O)N(R¹⁹)₂, C(O)H, OH, (O), CN, CF₃, F, Cl, Br or I; wherein R¹⁹ is as described in Formula I.

In one embodiment of Formula (Ik), R¹⁰² is R¹¹, wherein R¹¹ is pyrrolidinyl as described in Formula (Ir): 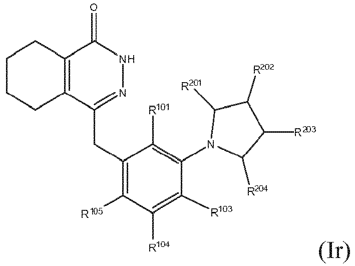 wherein R¹⁰¹, R¹⁰³, R¹⁰⁴, and R¹⁰⁵, are independently selected from H, R¹¹, OR¹¹, SR¹¹, S(O)R¹¹,SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein R¹¹ is as described in Formula I, and R²⁰¹, R²⁰², R²⁰³, and R²⁰⁴ are independently H, R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein R¹⁹ is as described in Formula I. In one embodiment of Formula (Ir), R¹⁰³ is F, and R¹⁰¹, R¹⁰⁴, and R¹⁰⁵ are H, wherein R²⁰¹, R²⁰², R²⁰³, and R²⁰⁴ are independently H, R¹⁹, OR¹⁹, SR¹⁹, SO₂R¹⁹, C(O)R¹⁹, CO(O)R¹⁹, NHR¹⁹, N(R¹⁹)2, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein R¹⁹ is as described in Formula I. In one embodiment of Formula (Ir), one or two of R²⁰¹, R²⁰², R²⁰³, and R²⁰⁴ is (O). In another embodiment of Formula (Ir), two of R²⁰¹, R²⁰², R²⁰³, and R²⁰⁴ are (O). In another embodiment of Formula (Ir), R²⁰¹ and R²⁰⁴ are (O) and R²⁰², and R²⁰³ are H, as described in Formula (Ir₁): 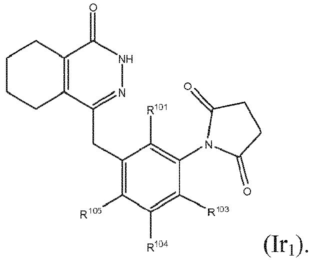 In one embodiment of Formula (Ir₁), R¹⁰³ is F and R¹⁰¹, R¹⁰⁴, and R¹⁰⁵, are independently selected from H, R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)2, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein R¹¹ is as described in Formula I.

In one embodiment of Formula (Ik), R¹⁰³ is F, as described in Formula (Is): 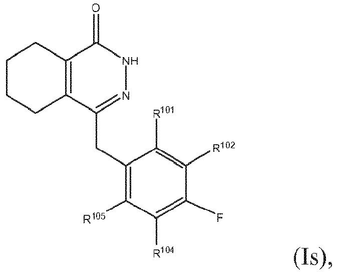 wherein R¹⁰¹, R¹⁰², R¹⁰⁴, and R¹⁰⁵, are independently selected from H, R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein R¹¹ is as described in Formula I. In another embodiment of Formula (Is), R¹⁰¹, R¹⁰², R¹⁰⁴, and R¹⁰⁵, are independently selected from H, R¹¹, OR¹¹, SR¹¹, S(O)R¹¹, SO₂R¹¹, NH₂, -N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein each R¹¹ is R¹², R¹³, R¹⁴ or R¹⁵; wherein R¹² is phenyl which is unfused or fused with benzene, heteroarene, heterocycloalkane or heterocycloalkene; R¹³ is heteroaryl, which is unfused;
R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene; and R¹⁵ is alkyl which is unsubstituted or substituted with one or two of independently selected R¹⁶,16 OR¹⁶, SR¹⁶, S(O)ZR'6, 16) NH₂, HR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl which is unsubstituted or substituted with R¹⁸; R^{17A} is phenyl, heteroaryl, cycloalkyl, heterocycloalkyl or heterocycloalkenyl, each of which is unfused or fused with benzene or heterocycloalkane; R¹⁸ is phenyl or heterocycloalkyl, which is unfused; wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, -N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R²⁰, R²¹, R²² or R²³; R²⁰ is phenyl, which is unfused; R²¹ is heteroaryl, which is unfused; R²² is cycloalkyl or heterocycloalkyl; each of which is unfused or fused with benzene; and R²³ is alkyl which is unsubstituted or substituted with R²⁴, OR²⁴, NHR²⁴ N(R²⁴)₂, NHS(O)₂R²⁴ or OH; wherein each R²⁴ is R^{24A} or R^{24B}; R^{24A} is phenyl, cycloalkyl, heterocycloalkyl or heterocycloalkenyl, which is unfused or fused with heterocycloalkane; R^{24B} is alkyl, which is unsubstituted or substituted with OR²⁵, OH, F, Cl, Br or I; R²⁵ is alkyl, which is unsubstituted or substituted with NH₂; wherein the moieties represented by R²⁰, R²¹, R²², and R^{24A} are independently unsubstituted or substituted with one or two of independently selected R²⁶, OR²⁶ (O), F, Cl, Br or I; and R²⁶ is alkyl. In another embodiment of Formula (Is), R¹¹ is selected from phenyl, pyrrolidinyl, azabicylclo(3.1.0)hexanyl, hexahydro-1H-isoindolyl, oxazolidinyl, azepanyl, piperidinyl, imidazolidinyl, thiazolidinyl, thiazinyl, azetidinyl, tetrahydropyrimidinyl or azabicylo(2.2.1)hept-2-yl; each of which are independently unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein R¹⁹ is as described in Formula I. In another embodiment of Formula (Is), R¹⁰² is R¹¹, wherein R¹¹ is selected from pyrrolidinyl, oxazolyl, imidazolidinyl, isothiazolidinyl, piperidinyl, piperazinyl and azepanyl, wherein R¹⁰² is substituted with one or two (O) substituents. In another embodiment of Formula (Is), R¹⁰² is R¹¹, wherein R¹¹ is selected from pyrrolidinyl substituted with one or two (O) substituents. In another embodiment of Formula (Is), R¹⁰¹, R¹⁰⁴ and R¹⁰⁵ are H, and R¹⁰² is selected from R¹¹, OR¹¹, NHC(O)R¹¹, or C(O)NHR¹¹; wherein R¹¹ is as described in Formula I. In another embodiment of Formula (Is), wherein R¹⁰¹, R¹⁰⁴ and R¹⁰⁵ are H, and R¹⁰² is selected from R¹¹, OR¹¹, NHC(O)R¹¹, or C(O)NHR¹¹; wherein R¹¹ is phenyl, pyrrolidinyl, azabicylclo(3.1.0)hexanyl, hexahydro-1H-isoindolyl, oxazolidinyl, azepanyl, piperidinyl, imidazolidinyl, thiazolidinyl, thiazinyl, azetidinyl, tetrahydropyrimidinyl, or azabicylo(2.2.1)hept-2-yl; each of which are independently unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I wherein R¹⁹ is as described in Formula I. In another embodiment of Formula (Is), wherein R¹⁰¹, R¹⁰⁴ and R¹⁰⁵ are H, and R¹⁰² is selected from R¹¹, OR¹¹, NHC(O)R¹¹, or C(O)NHR¹¹; wherein R¹¹ is phenyl, pyrrolidinyl, azabicylclo(3.1.0)hexanyl, hexahydro-1 H-isoindolyl, oxazolidinyl, azepanyl, piperidinyl, imidazolidinyl, thiazolidinyl, thiazinyl, azetidinyl, tetrahydropyrimidinyl, or azabicylo(2.2.1)hept-2-yl; each of which are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I wherein R¹⁹ is as described in Formula I. In another embodiment of Formula (Is), wherein R¹⁰¹, R¹⁰⁴ and R¹⁰⁵ are H, and R¹⁰² is selected from R¹¹, OR¹¹, NHC(O)R¹¹, or C(O)NHR¹¹; wherein R¹¹ is R¹⁵ and R¹⁵ is alkyl which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl which is unsubstituted or substituted with R¹⁸; R^{17A} is phenyl, heteroaryl, cycloalkyl, heterocycloalkyl or heterocycloalkenyl each of which is unfused or fused with benzene or heterocycloalkane; R¹⁸ is phenyl or heterocycloalkyl, which is unfused; wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R²⁰, R²¹, R²² or R²³; R²⁰ is phenyl, which is unfused; R²¹ is heteroaryl, which is unfused; R²² is cycloalkyl or heterocycloalkyl; each of which is unfused or fused with benzene; and R²³ is alkyl which is unsubstituted or substituted with R²⁴, OR²⁴, NHR²⁴ N(R²⁴)₂, NHS(O)₂R²⁴ or OH; wherein each R²⁴ is R^{24A} or R^{24B}; R^{24A} is phenyl, cycloalkyl, heterocycloalkyl or heterocycloalkenyl, which is unfused or fused with heterocycloalkane; R^{24B} is alkyl, which is unsubstituted or substituted with OR²⁵, OH, F, Cl, Br or I; R²⁵ is alkyl, which is unsubstituted or substituted with NH₂; wherein the moieties represented by R²⁰, R²¹, R²², and R^{24A} are independently unsubstituted or substituted with one or two of independently selected R²⁶, OR²⁶ (O), F, Cl, Br or I; and R²⁶ is alkyl.
In one embodiment, the compound of Formula (Is) is selected from:
2-fluoro-5-((4-oxo-3,4,5,6,7,8-hexahydrophthalazin-1-yl)methyl)benzoic acid;
4-(3-amino-4-fluorobenzyl)-5,6,7,8-tetrahydrophthalazin-1(2H)-one;
4-((2-fluoro-5-((4-oxo-3,4,5,6,7,8-hexahydrophthalazin-1-yl)methyl)phenyl)amino)-4-oxobutanoic acid;
1-(2-fluoro-5-((4-oxo-3,4,5,6,7,8-hexahydrophthalazin-1-yl)methyl)phenyl)pyrrolidine-2,5-dione;
4-(3-(1,4-diazepan-1-ylcarbonyl)-4-fluorobenzyl)-5,6,7,8-tetrahydrophthalazin-1(2H)-one;
4-(3-(aminomethyl)-4-fluorobenzyl)-5,6,7,8-tetrahydrophthalazin-1(2H)-one;
4-(3-((dimethylamino)methyl)-4-fluorobenzyl)-5,6,7,8-tetrahydrophthalazin-1(2H)-one;
4-(4-fluoro-3-((isopropylamino)methyl)benzyl)-5,6,7,8-tetrahydrophthalazin-1(2H)-one;
4-(3-((cyclohexylamino)methyl)-4-fluorobenzyl)-5,6,7,8-tetrahydrophthalazin-1(2H)-one;
4-(4-fluoro-3-((tetrahydro-2H-pyran-4-ylamino)methyl)benzyl)-5,6,7,8-tetrahydrophthalazin-1(2H)-one;
4-(4-fluoro-3-((methyl((1-methylpyrrolidin-3-yl)methyl)amino)methyl)benzyl)-5,6,7,8-tetrahydrophthalazin-1(2H)-one;
4-(4-fluoro-3-((methyl(((2R)-1-methylpyrrolidin-2-yl)methyl)amino)methyl)benzyl)-5,6,7,8-tetrahydrophthalazin-1(2H)-one;
4-(4-fluoro-3-pyrimidin-2-ylbenzyl)-5,6,7,8-tetrahydrophthalazin-(2H)-one;
4-(4-fluoro-3-pyridin-3-ylbenzyl)-5,6,7,8-tetrahydrophthalazin-1(2H)-one;
4-(4-fluoro-3-pyridin-4-ylbenzyl)-5,6,7,8-tetrahydrophthalazin-1(2H)-one;
N,N-diethyl-2'-fluoro-5'-((4-oxo-3,4,5,6,7,8-hexahydrophthalazin-1-yl)methyl)-1,1'-biphenyl-2-carboxamide;
N-(2-fluoro-5-((4-oxo-3,4,5,6,7,8-hexahydrophthalazin-1-yl)methyl)phenyl)-3-piperidin-1-ylpropanamide;
N-(2-fluoro-5-((4-oxo-3,4,5,6,7,8-hexahydrophthalazin-1-yl)methyl)phenyl)-3-(4-methylpiperazin-1-yl)propanamide;
2-amino-N-(2-fluoro-5-((4-oxo-3,4,5,6,7,8-hexahydrophthalazin-1-yl)methyl)phenyl)acetamide;
3-cyclohexyl-N-(2-fluoro-5-((4-oxo-3,4,5,6,7,8-hexahydrophthalazin-1-yl)methyl)phenyl)propanamide;
N-(2-fluoro-5-((4-oxo-3,4,5,6,7,8-hexahydrophthalazin-1-yl)methyl)phenyl)piperidine-3-carboxamide;
4-(4-fluoro-3-(2-oxopyrrolidin-1-yl)benzyl)-5,6,7,8-tetrahydrophthalazin-1(2H)-one;
N-(2-fluoro-5-((4-oxo-3,4,5,6,7,8-hexahydrophthalazin-1-yl)methyl)phenyl)azetidine-3-carboxamide;
N-(2-fluoro-5-((4-oxo-3,4,5,6,7,8-hexahydrophthalazin-1-yl)methyl)phenyl)-2-morpholin-4-ylacetamide;
N-(2'-fluoro-5'-((4-oxo-3,4,5,6,7,8-hexahydrophthalazin-1-yl)methyl)-1,1'-biphenyl-3-yl)acetamide;
4-((6-fluoro-3'-(methylsulfonyl)-1,1'-biphenyl-3-yl)methyl)-5,6,7,8-tetrahydrophthalazin-1(2H)-one;
4-((6-fluoro-3'-(pyrrolidin-1-ylcarbonyl)-1,1'-biphenyl-3-yl)methyl)-5,6,7,8-tetrahydrophthalazin-1 (2H)-one;
4-((6-fluoro-4'-(pyrrolidin-1-ylcarbonyl)-1,1'-biphenyl-3-yl)methyl)-5,6,7,8-tetrahydrophthalazin-1 (2H)-one;
N,N-diethyl-2'-fluoro-5'-((4-oxo-3,4,5,6,7,8-hexahydrophthalazin-1-yl)methyl)-1,1'-biphenyl-3-carboxamide;
2'-fluoro-N,N-dimethyl-5'-((4-oxo-3,4,5,6,7,8-hexahydrophthalazin-1-yl)methyl)-1,1'-biphenyl-4-carboxamide;
N-(2-fluoro-5-((4-oxo-3,4,5,6,7,8-hexahydrophthalazin-l-yl)methyl)phenyl)-4-(4-mcthoxyphcnyl)-4-oxobutanamidc;
1-(2-fluoro-5-((4-oxo-3,4,5,6,7,8-hexahydrophthalazin-1-yl)methyl)phenyl)-3,4-dimethyl-1 H-pyrrole-2,5-dione;
3-(2-fluoro-5-((4-oxo-3,4,5,6,7,8-hexahydrophthalazin-1-yl)methyl)phenyl)-3-azabicyclo(3.1.0)hexane-2,4-dione;
2-(2-fluoro-5-((4-oxo-3,4,5,6,7,8-hexahydrophthalazin-1-yl)methyl)phenyl)hexahydro-1H-isoindole-1,3(2H)-dione;
1-(2-fluoro-5-((4-oxo-3,4,5,6,7,8-hexahydrophthalazin-1-yl)methyl)phenyl)-3,3-dimethylpyrrolidine-2,5-dione;
4-(4-fluoro-3-(2-methyl-5-oxopyrrolidin-1-yl)benzyl)-5,6,7,8-tetrahydrophthalazin-1(2H)-one;
4-(4-fluoro-3-(2-oxo-1,3-oxazolidin-3-yl)benzyl)-5,6,7,8-tetrahydrophthalazin-1(2H)-one;
4-(4-fluoro-3-(2-oxoazepan-1-yl)benzyl)-5,6,7,8-tetrahydrophthalazin-1(2H)-one;
1-(2-fluoro-5-((4-oxo-3,4,5,6,7,8-hexahydrophthalazin-1-yl)methyl)phenyl)piperidine-2,6-dione;
4-(4-fluoro-3-(2-oxoimidazolidin-1-yl)benzyl)-5,6,7,8-tetrahydrophthalazin-1 (2H)-one;
4-(3-(1,1-dioxidoisothiazolidin-2-yl)-4-fluorobenzyl)-5,6,7,8-tetrahydrophthalazin-1(2H)-one;
4-(4-fluoro-3-(2-oxoazetidin-1-yl)benzyl)-5,6,7,8-tetrahydrophthalazin-1(2H)-one;
4-(4-fluoro-3-(2-oxopiperidin-1-yl)benzyl)-5,6,7,8-tetrahydrophthalazin-1(2H)-one;
4-(4-fluoro-3-(3-methyl-2-oxoimidazolidin-1-yl)benzyl)-5,6,7,8-tetrahydrophthalazin-1(2H)-one;
4-(4-fluoro-3-(2-oxotetrahydropyrimidin-1(2H)-yl)benzyl)-5,6,7,8-tetrahydrophthalazin-1(2H)-one;
4-(3-(3-tert-butyl-2-oxoimidazolidin-1-yl)-4-fluorobenzyl)-5,6,7,8-tetrahydrophthalazin-1(2H)-one;
4-(4-fluoro-3-((1S,4R)-3-oxo-2-azabicyclo(2.2.1)hept-2-yl)benzyl)-5,6,7,8-tetrahydrophthalazin-1(2H)-one;
N-(2-fluoro-5-((4-oxo-3,4,5,6,7,8-hexahydrophthalazin-1-yl)methyl)phenyl)-N-methylmethanesulfonamide;
N-(2-fluoro-5-((4-oxo-3,4,5,6,7,8-hexahydrophthalazin-1-yl)methyl)phenyl)-2-hydroxy-2-methylpropanamide;
(3aS,4R,7S,7aR)-5-(2-fluoro-5-((4-oxo-3,4,5,6,7,8-hexahydrophthalazin-1-yl)methyl)phenyl)-2,2-dimethyltetrahydro-4,7-methano(1,3)dioxolo(4,5-c)pyridin-6(3aH)-one;
4-(3-(1,1-dioxido-1,2-thiazinan-2-yl)-4-fluorobenzyl)-5,6,7,8-tetrahydrophthalazin-1(2H)-one;
N-(2-fluoro-5-((4-oxo-3,4,5,6,7,8-hexahydrophthalazin-1-yl)methyl)phenyl)-2-(2-oxopyrrolidin-1-yl)acetamide;
N-(2-fluoro-5-((4-oxo-3,4,5,6,7,8-hexahydrophthalazin-1-yl)methyl)phenyl)-5-methyl-1-phenyl-1 H-pyrazole-4-carboxamide;
N-(2-fluoro-5-((4-oxo-3,4,5,6,7,8-hexahydrophthalazin-l-yl)methyl)phenyl)-5-oxohexanamide;
N-(2-fluoro-5-((4-oxo-3,4,5,6,7,8-hexahydrophthalazin-1-yl)methyl)phenyl)-3-methoxypropanamide;
N-(2-fluoro-5-((4-oxo-3,4,5,6,7,8-hexahydrophthalazin-1-yl)methyl)phenyl)-N'-phenylpentanediamide;
4-(4-fluoro-3-((4-pyrimidin-2-ylpiperazin-1-yl)carbonyl)benzyl)-5,6,7,8-tetrahydrophthalazin-1(2H)-one; or
4-(4-fluoro-3-(2-oxopyrrolidin-1-yl)phenyl)-5,6,7,8-tetrahydrophthalazin-1(2H)-one.

In another embodiment, the compound of Formula (Is) is selected from
4-((2-fluoro-5-((4-oxo-3,4,5,6,7,8-hexahydrophthalazin-1-yl)methyl)phenyl)amino)-4-oxobutanoic acid;
1-(2-fluoro-5-((4-oxo-3,4,5,6,7,8-hexahydrophthalazin-1-yl)methyl)phenyl)pyrrolidine-2,5-dione;
4-(4-fluoro-3-(2-oxopyrrolidin-1-yl)benzyl)-5,6,7,8-tetrahydrophthalazin-1(2H)-one;
N-(2-fluoro-5-((4-oxo-3,4,5,6,7,8-hexahydrophthalazin-1-yl)methyl)phenyl)-3-(4-methylpiperazin-1-yl)propanamide;
3-(2-fluoro-5-((4-oxo-3,4,5,6,7,8-hexahydrophthalazin-1-yl)methyl)phenyl)-3-azabicyclo(3.1.0)hexane-2,4-dione;
1-(2-fluoro-5-((4-oxo-3,4,5,6,7,8-hexahydrophthalazin-1-yl)methyl)phenyl)-3,3-dimethylpyrrolidine-2,5-dione;
4-(4-fluoro-3-(2-oxo-1,3-oxazolidin-3-yl)benzyl)-5,6,7,8-tetrahydrophthalazin-1 (2H)-one;
4-(4-fluoro-3-(3-methyl-2-oxoimidazolidin-1-yl)benzyl)-5,6,7,8-tetrahydrophthalazin-1(2H)-one;
4-(4-fluoro-3-(2-oxoazepan-1-yl)benzyl)-5,6,7,8-tetrahydrophthalazin-1(2H)-one;
1-(2-fluoro-5-((4-oxo-3,4,5,6,7,8-hexahydrophthalazin-1-yl)methyl)phenyl)piperidine-2,6-dione;
4-(3-(1,1-dioxidoisothiazolidin-2-yl)-4-fluorobenzyl)-5,6,7,8-tetrahydrophthalazin-1(2H)-one;
4-(4-fluoro-3-(2-oxopiperidin-1-yl)benzyl)-5,6,7,8-tetrahydrophthalazin-1(2H)-one; or
4-(4-fluoro-3-((4-pyrimidin-2-ylpiperazin-1-yl)carbonyl)benzyl)-5,6,7,8-tetrahydrophthalazin-1 (2H)-one.

In one embodiment of Formula (Ik), R¹⁰² is C(O)R¹¹, as described in Formula (It): 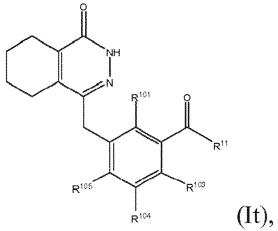 wherein R¹¹ is as described in Formula I. In one embodiment of Formula (It), R¹⁰¹, R¹⁰³, R¹⁰⁴ and R¹⁰⁵ are H. In another embodiment of Formula (It), R¹⁰³ is F and R¹⁰¹, R¹⁰⁴ and R¹⁰⁵ are H. In another embodiment of Formula (It), R¹¹ is R¹⁵ and R¹⁵ is alkyl which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl which is unsubstituted or substituted with R¹⁸; R^{17A} is phenyl, heteroaryl, cycloalkyl, heterocycloalkyl or heterocycloalkenyl, each of which is unfused or fused with benzene or heterocycloalkane: R¹⁸ is phenyl or heterocycloalkyl, which is unfused; wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R²⁰, R²¹, R²² or R²³; R²⁰ is phenyl, which is unfused; R²¹ is heteroaryl, which is unfused; R²² is cycloalkyl or heterocycloalkyl; each of which is unfused or fused with benzene; and R²³ is alkyl which is unsubstituted or substituted with R²⁴, OR²⁴, NHR²⁴ N(R²⁴)₂, NHS(O)₂R²⁴ or OH; wherein each R²⁴ is R^{24A} or R^{24B}; R^{24A} is phenyl, cycloalkyl, heterocycloalkyl or heterocycloalkenyl, which is unfused or fused with heterocycloalkane; R^{24B} is alkyl, which is unsubstituted or substituted with OR²⁵, OH, F, Cl, Br or I; R²⁵ is alkyl, which is unsubstituted or substituted with NH₂; wherein the moieties represented by R²⁰, R²¹, R²² , and R^{24A} are independently unsubstituted or substituted with one or two of independently selected R²⁶, OR²⁶ (O), F, Cl, Br or I; and R²⁶ is alkyl. In another embodiment of Formula (It), R¹¹ is phenyl, pyrrolidinyl, azabicylclo(3.1.0)hexanyl, hexahydro-1H-isoindolyl, oxazolidinyl, azepanyl, piperidinyl, imidazolidinyl, thiazolidinyl, thiazinyl, azetidinyl, tetrahydropyrimidinyl, or azabicylo(2.2.1)hept-2-yl; each of which are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I wherein R¹⁹ is as described in Formula I.

In one embodiment of Formula (Ik), R¹⁰² is C(O)NHR¹¹, as described in Formula (Iu): 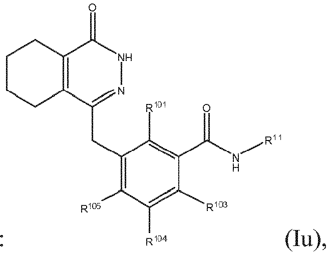 wherein R¹¹ is as described in Formula I. In one embodiment of Formula (Iu), R¹⁰¹, R¹⁰³, R¹⁰⁴ and R¹⁰⁵ are H. In another embodiment of Formula (Iu), R¹⁰³ is F and R¹⁰¹, R¹⁰⁴ and R¹⁰⁵ are H. In another embodiment of Formula (Iu), R¹¹ is R¹⁵ and R¹⁵ is alkyl which is unsubstituted or substituted with one or two of independently selected R¹⁶, OR¹⁶, SR¹⁶, S(O)₂R¹⁶, C(O)OH, NH₂, NHR¹⁶N(R¹⁶)₂, C(O)R¹⁶, C(O)NHR¹⁶, NHC(O)R¹⁶, NHC(O)OR¹⁶, OH, F, Cl, Br or I; wherein each R¹⁶ is R¹⁷ or R^{17A}; R¹⁷ is alkyl which is unsubstituted or substituted with R¹⁸; R^{17A} is phenyl, heteroaryl, cycloalkyl, heterocycloalkyl or heterocycloalkenyl, each of which is unfused or fused with benzene or heterocycloalkane; R¹⁸ is phenyl or heterocycloalkyl, which is unfused; wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I; wherein each R¹⁹ is R²⁰, R²¹, R²² or R²³; R²⁰ is phenyl, which is unfused; R²¹ is heteroaryl, which is unfused; R²² is cycloalkyl or heterocycloalkyl; each of which is unfused or fused with benzene; and R²³ is alkyl which is unsubstituted or substituted with R²⁴, OR²⁴, NHR²⁴ N(R²⁴)₂, NHS(O)₂R²⁴ or OH; wherein each R²⁴ is R^{24A} or R^{24B}; R^{24A} is phenyl, cycloalkyl, heterocycloalkyl or heterocycloalkenyl, which is unfused or fused with heterocycloalkane; R^{24B} is alkyl, which is unsubstituted or substituted with OR²⁵, OH, F, Cl, Br or I; R²⁵ is alkyl, which is unsubstituted or substituted with NH₂; wherein the moieties represented by R²⁰, R²¹, R²², and R^{24A} are independently unsubstituted or substituted with one or two of independently selected R²⁶, OR²⁶ (O), F, Cl, Br or I; and R²⁶ is alkyl. In another embodiment of Formula (Iu), R¹¹ is phenyl, pyrrolidinyl, azabicylclo(3.1.0)hexanyl, hexahydro-1H-isoindolyl, oxazolidinyl, azepanyl, piperidinyl, imidazolidinyl, thiazolidinyl, thiazinyl, azetidinyl, tetrahydropyrimidinyl, or azabicylo(2.2.1)hept-2-yl; each of which are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I wherein R¹⁹ is as described in Formula I.

In one embodiment of Formula (Ik), R¹⁰² is phenyl which is unsubstituted or substituted with one or two or three or four of independently selected R¹⁹, OR¹⁹, SR¹⁹, S(O)R¹⁹, SO₂R¹⁹, C(O)R¹⁹, CO(O)R¹⁹, OC(O)R¹⁹, OC(O)OR¹⁹, NH₂, NHR¹⁹, N(R¹⁹)₂, NHC(O)R¹⁹, NR¹⁹C(O)R¹⁹, NHS(O)₂R¹⁹, NR¹⁹S(O)₂R¹⁹, NHC(O)OR¹⁹, NR¹⁹C(O)OR¹⁹, NHC(O)NH₂, NHC(O)NHR¹⁹, NHC(O)N(R¹⁹)₂, NR¹⁹C(O)NHR¹⁹, NR¹⁹C(O)N(R¹⁹)₂, C(O)NH₂, C(O)NHR¹⁹, C(O)N(R¹⁹)₂, C(O)NHOH, C(O)NHOR¹⁹, C(O)NHSO₂R¹⁹, C(O)NR¹⁹SO₂R¹⁹, SO₂NH₂, SO₂NHR¹⁹, SO₂N(R¹⁹)₂, C(O)H, C(O)OH, C(N)NH₂, C(N)NHR¹⁹, C(N)N(R¹⁹)₂, CNOH, CNOCH₃, OH, (O), CN, N₃, NO₂, CF₃, CF₂CF₃, OCF₃, OCF₂CF₃, F, Cl, Br or I; wherein R¹⁹ is as described in Formula I.

In one embodiment of Formula (Ik), R¹⁰² is heterocycloalkyl which is unsubstituted or substituted with one or two or three or four of independently selected R¹⁹, OR¹⁹, SR¹⁹, S(O)R¹⁹, SO₂R¹⁹, C(O)R¹⁹, CO(O)R¹⁹, OC(O)R¹⁹, OC(O)OR¹⁹, NH₂, NHR¹⁹, N(R¹⁹)₂, NHC(O)R¹⁹, NR¹⁹C(O)R¹⁹, NHS(O)₂R¹⁹, NR¹⁹S(O)₂R¹⁹, NHC(O)OR¹⁹, NR¹⁹C(O)OR¹⁹, NHC(O)NH₂, NHC(O)NHR¹⁹, NHC(O)N(R¹⁹)₂, NR¹⁹C(O)NHR¹⁹, NR¹⁹C(O)N(R¹⁹)₂, C(O)NH₂, C(O)NHR¹⁹, C(O)N(R¹⁹)₂, C(O)NHOH, C(O)NHOR¹⁹, C(O)NHSO₂R¹⁹, C(O)NR¹⁹SO₂R¹⁹, SO₂NH₂, SO₂NHR¹⁹, SO₂N(R¹⁹)₂, C(O)H, C(O)OH, C(N)NH₂, C(N)NHR¹⁹, C(N)N(R¹⁹)₂, CNOH, CNOCH₃, OH, (O), CN, N₃, NO₂, CF₃, CF₂CF₃, OCF₃, OCF₂CF₃, F, Cl, Br or I; wherein R¹⁹ is as described in Formula I.

### Embodiments where A¹ is Piperidine, A² is R⁵

In one embodiment of Formula (I) A¹ is R², wherein R² is unsubstituted piperidine which is unfused, and A² is R⁵, which is as described in Formula I. In another embodiment of Formula I, A¹ is R², wherein R² is unsubstituted piperidine which is unfused, and A² is R⁵, R⁵ is C₁-alkyl, C₂-alkyl or C₃-alkyl wherein R⁵ is substituted with R¹⁰, and further unsubstituted or substituted with one or two or three of independently selected NHR¹⁰, N(R¹⁰)₂, SR¹⁰, S(O)R¹⁰, SO₂R¹⁰ or CF₃, wherein R¹⁰ is as described in formula I. In another embodiment of Formula I, A¹ is R², wherein R² is unsubstituted piperidine which is unfused, and A² is R⁵, R⁵ is C₁-alkyl, C₂-alkyl or C₃-alkyl wherein R⁵ is substituted with R¹⁰, and further unsubstituted or substituted with one CF₃, wherein R¹⁰ is as described in formula I. In another embodiment of Formula I, A¹ is R², wherein R² is unsubstituted piperidine which is unfused, A² is C₁-alkyl, and R¹⁰ is phenyl, as shown in Formula (Iv): 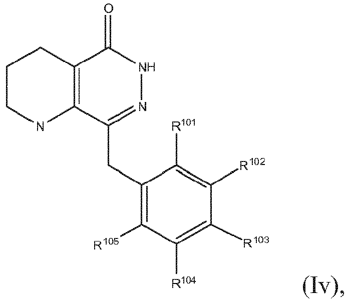 wherein R¹⁰¹, R¹⁰², R¹⁰³, R¹⁰⁴, and R¹⁰⁵, are independently selected from H, R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)2, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein each R¹¹ is R¹², R¹³, R¹⁴ or R¹⁵_{;} R¹² is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene; R¹³ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene; R¹⁴ is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene; R¹⁵ is alkyl, alkenyl or alkynyl; each of which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NH₂, C(O)NHR¹⁶, C(O)N(R¹⁶)₂, NHC(O)R¹⁶, NR¹⁶C(O)R¹⁶, NHC(O)OR¹⁶, NR¹⁶C(O)OR¹⁶, OH, F, Cl, Br or I; wherein each R¹⁶ is R¹⁷ or R^{17A}; R¹⁷ is alkyl, alkenyl or alkynyl; each of which is unsubstituted or substituted with one or two of independently selected R¹⁸, C(O)OH, NH₂, NHR¹⁸ or N(R¹⁸)₂, C(O)R¹⁸, C(O)NH₂, C(O)NHR¹⁸, C(O)N(R¹⁸)₂, NHC(O)R¹⁸, NR¹⁸C(O)R¹⁸, F, Cl, Br or I; R^{17A} is phenyl, heteroaryl, cycloalkyl, cycloalkenyl, heterocycloalkyl or heterocycloalkenyl, each of which is unfused or fused with benzene, heteroarene, cycloalkane, cycloalkene, heterocycloalkane or heterocycloalkene; wherein each R¹⁸ is phenyl, heteroaryl, cycloalkyl, cycloalkenyl, heterocycloalkyl or heterocycloalkenyl; wherein each of the moieties represented by R¹², R¹³, R¹⁴, R^{17A}, and R¹⁸ are independently unsubstituted or substituted with one or two or three or four of independently selected R¹⁹, OR¹⁹, SR¹⁹, S(O)R¹⁹, SO₂R¹⁹, C(O)R¹⁹, CO(O)R¹⁹, OC(O)R¹⁹, OC(O)OR¹⁹, NH₂, NHR¹⁹, N(R¹⁹)₂, NHC(O)R¹⁹, NR¹⁹C(O)R¹⁹, NHS(O)₂R¹⁹, NR¹⁹S(O)₂R¹⁹, NHC(O)OR¹⁹, NR¹⁹C(O)OR¹⁹, NHC(O)NH₂, NHC(O)NHR¹⁹, NHC(O)N(R¹⁹)₂, NR¹⁹C(O)NHR¹⁹, NR¹⁹C(O)N(R¹⁹)₂, C(O)NH₂, C(O)NHR¹⁹, C(O)N(R¹⁹)₂, C(O)NHOH, C(O)NHOR¹⁹, C(O)NHSO₂R¹⁹, C(O)NR¹⁹SO₂R¹⁹, SO₂NH₂, SO₂NHR¹⁹, SO₂N(R¹⁹)₂, C(O)H, C(O)OH, C(N)NH₂, C(N)NHR¹⁹, C(N)N(R¹⁹)₂, CNOH, CNOCH₃, OH, (O), CN, N₃, NO₂, CF₃, CF₂CF₃, OCF₃, OCF₂CF₃, F, Cl, Br or I; wherein each R¹⁹ is R²⁰, R²¹, R²² or R²³; R²⁰ is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene; R²¹ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene; R²² is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene; R²³ is alkyl, alkenyl or alkynyl; each of which is unsubstituted or substituted with one or two of independently selected R²⁴, OR²⁴, SR²⁴, S(O)₂R²⁴, C(O)OH, NH₂,NHR²⁴N(R²⁴)₂, C(O)R²⁴, C(O)NH₂, C(O)NHR²⁴, C(O)N(R²⁴)₂, NHC(O)R²⁴, NR²⁴C(O)R²⁴, NHC(O)OR²⁴, NR²⁴C(O)OR²⁴, NHS(O)₂R²⁴, NR²⁴S(O)2R²⁴, OH, F, Cl, Br or I; wherein each R²⁴ is R^{24A} or R^{24B}; R^{24A} is phenyl, heteroaryl, cycloalkyl, cycloalkenyl, heterocycloalkyl or heterocycloalkenyl each of which is unfused or fused with benzene, heteroarene, cycloalkane, cycloalkene, heterocycloalkane or heterocycloalkene; R^{24B} is alkyl, alkenyl or alkynyl each of which is unsubstituted or substituted with one or two of independently selected R²⁵, OR²⁵, SR²⁵, S(O)₂R²⁵, C(O)OH, NH₂, NHR²⁵ N(R²⁵)₂, C(O)R²⁵, C(O)NH₂, C(O)NHR²⁵, C(O)N(R²⁵)₂, NHC(O)R²⁵, NR²⁵C(O)R²⁵, NHC(O)OR²⁵, NR²⁵C(O)OR²⁵, OH, F, Cl, Br or I; wherein each R²⁵ is alkyl, phenyl, heteroaryl, cycloalkyl, cycloalkenyl, heterocycloalkyl or heterocycloalkenyl ; each of which is unsubstituted or substituted with NH₂, NH(CH₃), N(CH₃)₂, OH or OCH₃; wherein each of the moieties represented by R²⁰, R²¹, R²², and R^{24A} are independently unsubstituted or substituted with one or two of independently selected R²⁶, OR²⁶, alkenyl, alkynyl, phenyl, OH, (O), C(O)OH, CN, CF₃, OCF₃, CF₂CF₃, F, Cl, Br or I; and R²⁶ is alkyl. In another embodiment, the compound of Formula (Iv) is selected from
8-(4-fluorobenzyl)-2,3,4,6-tetrahydropyrido(2,3-d)pyridazin-5(1H)-one; 8-(4-fluoro-3-(2-oxoazetidin-1-yl)benzyl)-2,3,4,6-tetrahydropyrido(2,3-d)pyridazin-5(1H)-one;
8-(3-chloro-4-fluorobenzyl)-2,3,4,6-tetrahydropyrido(2,3-d)pyridazin-5(1H)-one
8-(4-fluoro-3-(2-oxopyrrolidin-1-yl)benzyl)-2,3,4,6-tetrahydropyrido(2,3-d)pyridazin-5(1H)-one;
methyl 2-fluoro-5-((5-oxo-1,2,3,4,5,6-hexahydropyrido(2,3-d)pyridazin-8-yl)methyl)benzoate;
8-(3-amino-4-fluorobenzyl)- 2,3,4,6-tetrahydropyrido(2,3-d)pyridazin-5(1H)-one;
2-fluoro-5-((5-oxo-1,2,3,4,5,6-hexahydropyrido(2,3-d)pyridazin-8-yl)methyl)benzoic acid;
N-ethyl-2-fluoro-5-((5-oxo-1,2,3,4,5,6-hexahydropyrido(2,3-d)pyridazin-8-yl)methyl)benzamide;
N-cyclobutyl-2-fluoro-5-((5-oxo-1,2,3,4,5,6-hexahydropyrido(2,3-d)pyridazin-8-yl)methyl)benzamide;
2-fluoro-5-((5-oxo-1,2,3,4,5,6-hexahydropyrido(2,3-d)pyridazin-8-yl)methyl)-N-(2-pyrrolidin-1-ylethyl)benzamide;
8-(4-fluoro-3-((4-(morpholin-4-ylcarbonyl)piperazin-1-yl)carbonyl)benzyl)-2,3,4,6-tetrahydropyrido(2,3-d)pyridazin-5(1H)-one;
N-(2-fluoro-5-((5-oxo-1,2,3,4,5,6-hexahydropyrido(2,3-d)pyridazin-8-yl)methyl)phenyl)-N'-phenylpentanediamide;
1-(2-fluoro-5-((5-oxo-1,2,3,4,5,6-hexahydropyrido(2,3-d)pyridazin-8-yl)methyl)phenyl)pyrrolidine-2,5-dione;
N-(2-fluoro-5-((5-oxo-1,2,3,4,5,6-hexahydropyrido(2,3-d)pyridazin-8-yl)methyl)phenyl)-3-methoxypropanamide;
N-(2-fluoro-5-((5-oxo-1,2,3,4,5,6-hexahydropyrido(2,3-d)pyridazin-8-yl)methyl)phenyl)-5-oxohexanamide;
N-(2-fluoro-5-((5-oxo-1,2,3,4,5,6-hexahydropyrido(2,3-d)pyridazin-8-yl)methyl)phenyl)-3-phenoxypropanamide;
N-(2-fluoro-5-((5-oxo-1,2,3,4,5,6-hexahydropyrido(2,3-d)pyridazin-8-yl)methyl)phenyl)-4-oxo-4-phenylbutanamide;
2-(4-(benzyloxy)phenoxy)-N-(2-fluoro-5-((5-oxo-1,2,3,4,5,6-hexahydropyrido(2,3-d)pyridazin-8-yl)methyl)phenyl)acetamide;
N-(2-fluoro-5-((5-oxo-1,2,3,4,5,6-hexahydropyrido(2,3-d)pyridazin-8-yl)methyl)phenyl)-2-(4-methoxyphenoxy)acetamide;
N-cyclopropyl-2-fluoro-5-((5-oxo-1,2,3,4,5,6-hexahydropyrido(2,3-d)pyridazin-8-yl)methyl)benzamide;
8-(3-((4-(2-ethoxyethyl)piperazin-1-yl)carbonyl)-4-fluorobenzyl)-2,3,4,6-tetrahydropyrido(2,3-d)pyridazin-5(1H)-one; or
2-fluoro-5-((5-oxo-1,2,3,4,5,6-hexahydropyrido(2,3-d)pyridazin-8-yl)methyl)-N-(2-piperidin-1-ylethyl)benzamide.

### Schemes

The starting materials used herein are commercially available or may be prepared by routine methods well known to those of ordinary skill in the art. The compounds of the present invention may be prepared using the methods illustrated in the general synthetic schemes and experimental procedures detailed below. The general synthetic schemes are presented for purposes of illustration and are not intended to be limiting.

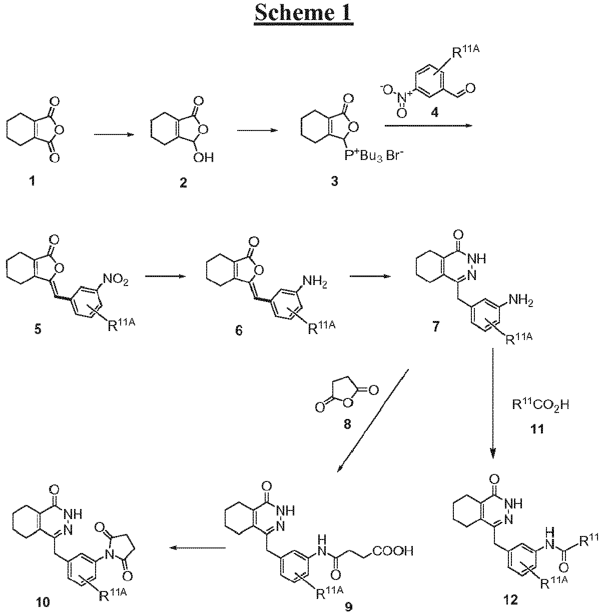

As shown in Scheme 1, the bicyclic anhydride **(1)** can be reduced to the alcohol **(2)** using a reducing agent such as but not limited to sodium borohydride. The reaction is typically conducted in a solvent such as but not limited to tetrahydrofuran at below room temperature to reflux. Conversion of **(2)** to the phosphonium salt **(3)** may be carried out by reacting the former with a trialkyl phosphine such as but not limited to tri-n-butyl phosphine in the presence of hydrobromic acid. The reaction is typically conducted in a solvent such as but not limited to acetic acid at reflux. Reaction of **(3)** with a nitrobenzaldehyde of Formula **(4),** wherein R^{11A} is a substituent on R¹⁰ as described herein, in the presence of a base such as but not limited to triethylamine will provide a lactone of Formula **(5).** The reaction is typically conducted in a solvent such as but not limited to dichloromethane at room temperature. Reduction of the nitro group of a compound of Formula **(5)** with a reducing agent such as but not limited to iron powder and NH₄Cl will provide the corresponding aniline of Formula **(6).** The reaction is typically conducted in a solvent such as but not limited to ethanol at reflux. Reaction of the aniline of Formula **(6)** with hydrazine will provide a tetrahydrophthalazinone of Formula **(7).** The reaction is typically conducted in a solvent such as but not limited to ethanol at an elevated temperature. Reaction of a compound of Formula **(7)** with either an anhydride of Formula **(8)** or with an acid of Formula **(11)** under standard peptide coupling conditions known to those skilled in the art and widely available in the literature will provide compounds of Formula **(9)** and **(12),** respectively. An acid of Formula (9) may be further modified to an imide of Formula **(10)** using standard peptide coupling conditions including the use of 1,1'-carbonyldiimidazole (CDI) as the coupling agent.

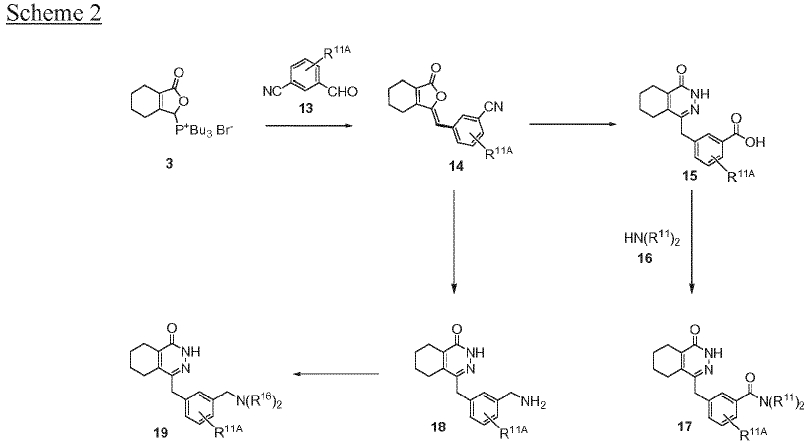

Alternatively, as shown in Scheme 2, the phosphonium salt **(3)** can be reacted with a cyanobenzaldehyde of Formula **(13)** to provide a lactone of Formula **(14).** The reaction is typically conducted under basic conditions in a solvent such as but not limited to dichloromethane at room temperature. Hydrolysis of the nitrile of Formula **(14)** to the corresponding acid, followed by addition of hydrazine will provide the tetrahydrophthalazinone of Formula **(15).** The hydrolysis step is typically conducted with an aqueous base such as but not limited to sodium hydroxide at elevated temperatures. The second step is also conducted under aqueous conditions at elevated temperatures. Coupling the acid of Formula **(15)** with an amine of Formula **(16),** wherein each R¹¹ is as described in Formula I herein or is H or is a heterocyclic amine R¹⁴, under standard peptide coupling conditions known to those skilled in the art and widely available in the literature, will provide an amide of Formula **(17).** Alternatively, a compound of Formula **(14)** can be converted to a tetrahydrophthalazinone using hydrazine as previously described, followed by reduction to the primary amine of Formula **(18)** under standard Raney-nickel reduction conditions. Treatment of compounds of Formula **(18)** under standard reductive amination conditions with an aldehyde R¹⁶CHO or ketone R¹⁶C(O)R¹⁶ and then optionally with a second aldehyde R¹⁶CHO or ketone R¹⁶C(O)R¹⁶, will provide secondary or tertiary amines of Formula **(19)** (wherein each R¹⁶ can be H or as defined in Formula (I)).

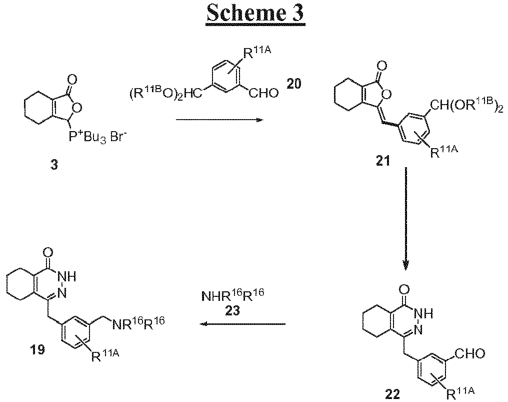

In a manner similar to the procedure described in Scheme 1, the phosphonium salt **(3)** can be reacted with a benzaldehyde of Formula **(20),** wherein R^{11B} is alkyl such as but not limited to ethyl and R^{11A} is as previously defined in Scheme 1. Reaction of a compound of Formula **(21)** with hydrazine as described in Scheme 1, followed by hydrolysis using an aqueous acid such as but not limited to sulfuric acid will provide a compound of Formula **(22).** The reaction is typically performed at elevated temperatures in a solvent such as but not limited tot ethanol. Reaction of a compound of Formula **(22)** with an amine of Formula **(23)** under reductive amination conditions known to those skilled in the art and widely available in the literature will provide a tetrahydrophthalazinone of Formula **(19).**

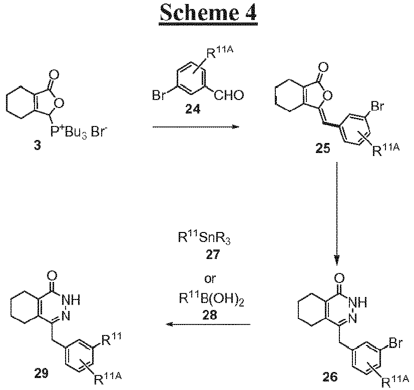

As shown in Scheme 4, the phosphonium salt **(3)** can be reacted with a bromobenzaldehyde of Formula **(24)** to provide a compound of Formula **(25)** using the conditions described in Scheme 1. Reaction of a compound of Formula **(25)** with hydrazine as described in Scheme 1 will provide a tetrahydrophthalazinone of Formula **(26),** which can be coupled with stannane of Formula **(27)** or a borate of Formula **(28)** to provide a compound of Formula **(29)** wherein R¹¹ is a substituted or unsubstituted phenyl or heteroaryl. Coupling conditions include those known by those skilled in the art and widely available in the literature for Suzuki and Stille type couplings.

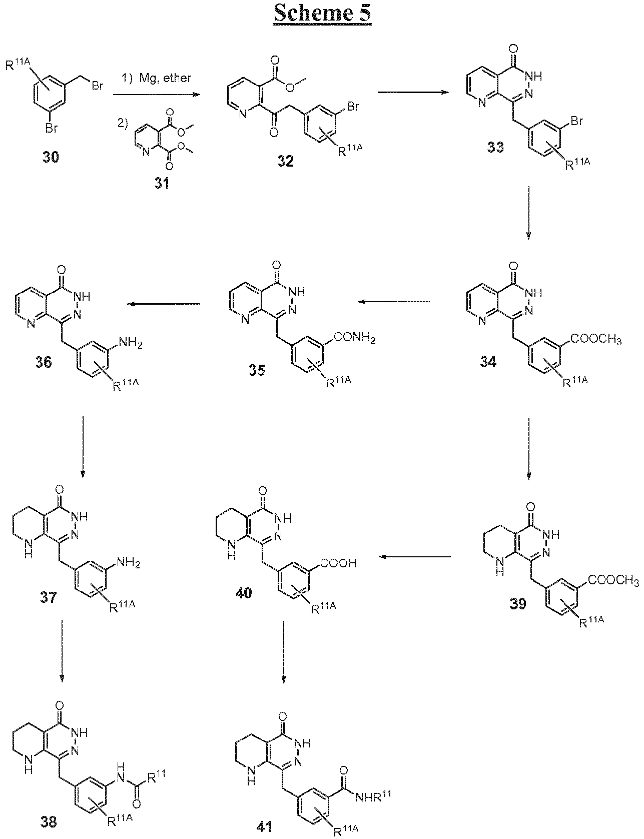

A benzylic bromide of Formula **(30)** wherein R¹¹ is as described herein, can be converted to a Grignard reagent and then added to a diester **(31)** to give a keto-ester of Formula **(32)** as shown in Scheme 5. The addition of the Grignard reagent is typically performed at cold temperatures, before warming up the reaction to room temperature. The reaction is typically performed in a solvent such as but not limited to tetrahydrofuran, ether and the like, or mixtures thereof. The Grignard reagent may be purchased commercially or prepared from Mg using standard conditions available in the literature. The addition of hydrazine to a compound of Formula **(32)** under conditions described in Scheme 1 at room temperature will provide a phthalazinone of Formula **(33).** The bromide can be converted to an ester of Formula **(34)** under palladium catalyzed carboxylation conditions. The transformation typically requires the use of a palladium catalyst and a base, such as but not limited to triethylamine, in addition to carbon monoxide and methanol. Typical palladium catalysts include [1,1'-bis(diphenylphosphino)ferrocene]dichloropalladium(II) dichloromethane and the like. The reaction is typically conducted at elevated temperatures and may require the use of a solvent such as but not limited to N,N-dimethylformamide. The ester of Formula **(34)** can be converted to a primary amide of Formula **(35)** using ammonia, followed by a Hoffman rearrangement with bromine and aqueous potassium hydroxide to provide an aniline of Formula **(36).** The first step typically requires an elevated temperature, and the second step typically requires a decreased temperature for the additions, followed by heating. The pyridine ring can be reduced under catalytic conditions, such as but not limited to the use of hydrogen gas and platinum on carbon to provide a compound of Formula **(37).** Amide formation using either an acid chloride of Formula R¹¹C(O)Cl or an acid of Formula R¹¹C(O)OH under standard peptide coupling conditions known to those skilled in the art and widely available in the literature will provide compounds of Formula **(38).** Alternatively, an ester of Formula **(34)** can be reduced to a compound of Formula **(39)** using the conditions described above, followed by hydrolysis to provide an acid of Formula **(40).** Typical hydrolysis conditions include but are not limited to using an aqueous base such as lithium hydroxide at elevated temperatures. Amide formation using a primary or secondary amine of Formula NH₂R¹¹ or NH(R¹¹)₂ employing standard peptide coupling conditions known to those skilled in the art and widely available in the literature, will provide an amide of Formula **(41).**

The following examples are presented to provide what is believed to be the most useful and readily understood description of procedures and conceptual aspects of this invention. The exemplified compounds were named using ACD/ChemSketch Version 5.06 (05 June 2001, Advanced Chemistry Development Inc. ,Toronto, Ontario), except for Examples 160, 320 and 487, which were named using ChemDraw® Ver. 9.0.5 (CambridgeSoft, Cambridge, MA). Intermediates were named using IUPAC standards.

### EXAMPLE 1

### 2-fluoro-5-((4-oxo-3,4,5,6,7,8-hexahydrophthalazin-1-yl)methyl)benzoic acid EXAMPLE 1A

### 3-hydroxy-4,5,6,7-tetrahydro-2-benzofuran-1(3H)-one

To a solution of 1-cyclohexene-1,2-dicarboxylic anhydride (25.2 g) in tetrahydrofuran (125 mL) at 0°C was added sodium borohydride (1.51 g). The mixture was warmed to ambient temperature for 30 minutes, heated at reflux for 5 hours, cooled, treated with 1N hydrochloric acid and concentrated. The concentrate was partitioned between ethyl acetate and brine, and the organic layer was washed with brine and water and concentrated. The concentrate was purified by flash chromatography with 50% ethyl acetate in hexane.

### EXAMPLE 1B

### tributyl(3-oxo-1,3,4,5,6,7-hexahydro-2-benzofuran-1-yl)phosphonium bromide

A solution of EXAMPLE 1 A (3 g) in acetic acid (10 mL) at ambient temperature was treated with tri-n-butyl phosphine (4.81 mL) and 33% hydrobromic acid in acetic acid (3.34 mL), heated at reflux for 21 hours, cooled and concentrated. The concentrate was purified by flash chromatography on silica gel with 10% methanol in dichloromethane.

### EXAMPLE 1C

### 2-fluoro-5-((3-oxo-4,5,6,7-tetrahydroisobenzofuran-1(3H)-ylidene)methyl)benzonitrile

To a solution of EXAMPLE 1B (3.05 g) in dichloromethane (30 mL) was added 2-fluoro-5-formylbenzonitrile (1.08 g) and triethylamine (1.02 mL). The mixture was stirred at ambient temperature for 16 hours and concentrated. The concentrate was partitioned between ethyl acetate and brine. The organic layer was washed with brine and concentrated. The concentrate was purified by flash chromatography on silica gel with 50% ethyl acetate in hexane.

### EXAMPLE 1D

### 2-fluoro-5-((4-oxo-3,4,5,6,7,8-hexahydrophthalazin-1-yl)methyl)benzoic acid

To a suspension of EXAMPLE 1C (1.46 g) in water (15 mL) was added 50% sodium hydroxide. The mixture was heated at 90°C for 1 hour. After cooling to 70°C, hydrazine monohydrate (0.54 mL) was added, and the solution was stirred at 70°C for 17 hours. The solution was cooled to ambient temperature and brought to pH 4 with 6N hydrochloric acid. The precipitate was filtered, washed with water and dried. ¹H NMR (DMSO-d₆) δ1.55-1.69 (m, 4H), 2.31-2.42 (m, 4H), 3.93 (s, 2H), 7.24 (dd, J=10.8, 8.5 Hz, 1H), 7.40-7.48 (m, 1H), 7.68 (dd, J=6.9, 2.2 Hz, 1H), 12.61 (s, 1H), 13.22 (brs, 1H).

### EXAMPLE 2

### 4-(3-amino-4-fluorobenzyl)-5,6,7,8-tetrahydrophthalazin-1(2H)-one

### EXAMPLE 2A

### 3-(4-fluoro-3-nitrobenzylidene)-4,5,6,7-tetrahydroisobenzofuran-1(3H)-one

This example was prepared as described in EXAMPLE 1C by substituting 4-fluoro-3-nitrobenzaldehyde for 2-fluoro-5-formylbenzonitrile.

### EXAMPLE 2B

### 3-(3-amino-4-fluorobenzylidene)-4,5,6,7-tetrahydroisobenzofuran-1(3H)-one

A solution of EXAMPLE 2A (2.25 g) and ammonium chloride (0.83 g) in ethanol (35 mL) and water (25 mL) at 70°C was treated with iron powder (4.35 g), stirred for 3 hours and filtered through diatomaceous earth (CELITE^{®}, World Minerals, Santa Barbara, CA) with hot ethanol. The filtrate was concentrated, and the concentrate was stirred with water for 30 minutes and filtered. The solid was washed with water and dried.

### EXAMPLE 2C

### 4-(3-amino-4-fluorobenzyl)-5,6,7,8-tetrahydrophthalazin-1(2H)-one

To a solution of EXAMPLE 2B (1.42 g) in ethanol (10 mL) was added hydrazine monohydrate (0.27 mL). The mixture stirred at reflux for 1 hour, cooled to 0°C, and filtered. The solid was washed with water and dried. ¹H NMR (CD₃OD) δ 1.63-1.75 (m, 4H), 2.36-2.45 (m, 2H), 2.46-2.53 (m, 2H), 3.84 (s, 2H), 6.42-6.49 (m, 1H), 6.64 (dd, J=8.6, 2.2 Hz, 1H), 6.86 (dd, J=11.2, 8.1 Hz, 1H).

### EXAMPLE 3

### 4-((2-fluoro-5-((4-oxo-3,4,5,6,7,8-hexahydrophthalazin-1-yl)methyl)phenyl)amino)-4-oxobutanoic acid

To a solution of EXAMPLE 2 (872 mg) in acetonitrile was added succinic anhydride (370 mg). The mixture was heated at reflux for 17 hours, cooled and concentrated. The concentrate was purified by HPLC (Zorbax® C-18 ODS packing material [Agilent Technologies, Santa Clara, CA], 0-100% acetonitrile/water with 0.1% trifluoroacetic acid). ¹H NMR (DMSO-d₆) 81.53-1.66 (m, 4H), 2.30-2.43 (m, 4H), 2.55-2.67 (m, 2H), 3.26-3.31 (m, 2H), 3.85 (s, 2H), 6.85-6.99 (m, 1H), 7.15 (dd, J=10.8, 8.5 Hz, 1H), 7.74 (d, J=6.4 Hz, 1H), 9.70 (brs, 1H), 12.09 (brs, 1H), 12.61 (s, 1H).

### EXAMPLE 4

### 1-(2-fluoro-5-((4-oxo-3,4,5,6,7,8-hexahydrophthalazin-1-yl)methyl)phenyl)pyrrolidine-2,5-dione

To EXAMPLE 3 (905 mg) in dichloromethane (30 mL) and N,N-dimethylformamide (6 mL) was added 1,1'-carbonyldiimidazole (785 mg). The mixture was stirred at ambient temperature for 3 hours and concentrated. The concentrate was purified by HPLC (Zorbax® C-18 ODS packing material [Agilent Technologies, Santa Clara, CA], 0-100% acetonitrile/water with 0.1% trifluoroacetic acid). ¹H NMR (DMSO-d₆): δ 1.57-1.69 (m, 4H), 2.32-2.42 (m, 4H), 2.78-2.89 (m, 4H), 3.93 (s, 2H), 7.09-7.13 (m, 1H), 7.32-7.33 (m, 1H), 7.34 (d, J=1.2 Hz, 1H), 12.62 (s, 1H).

### EXAMPLE 5

### 4-(3-(1,4-diazepan-1-ylcarbonyl)-4-fluorobenzyl)-5,6,7,8-tetrahydrophthalazin-1(2H)-one EXAMPLE 5A

### tert-butyl 4-(2-fluoro-5-((4-oxo-3,4,5,6,7,8-hexahydrophthalazin-1-yl)methyl)benzoyl)-1,4-diazepane-1-carboxylate

To EXAMPLE 1 (294 mg) in 1:1 N,N-dimethylformamide/pyridine (6 mL) was added 1,1'-carbonyldiimidazole (166 mg). The mixture was stirred at ambient temperature for 30 minutes, and tert-butyl 1-homopiperazine carboxylate (189 µL) was added. The mixture was stirred for 18 hours and concentrated. The concentrate was purified by flash chromatography on silica gel with 5% methanol in ethyl acetate.

### EXAMPLE 5B

### 4-(3-(1,4-diazepan-1-ylcarbonyl)-4-fluorobenzyl)-5,6,7,8-tetrahydrophthalazin-l(2H)-one

To a solution of EXAMPLE 5A (330 mg) in dichloromethane (8 mL) at 0°C was added trifluoroacetic acid (8 mL). The solution was warmed to ambient temperature, and acetonitrile was added. The mixture was concentrated. The concentrate was purified by HPLC (Zorbax® C-18 ODS packing material [Agilent Technologies, Santa Clara, CA], 0-100% acetonitrile/water with 0.1% trifluoroacetic acid). The product was dissolved in methanol/dichloromethane and treated with 1M hydrochloric acid in diethyl ether and filtered to give the title compound as the hydrochloride salt. ¹H NMR (CD₃OD) δ 1.70-1.76 (m, 4H), 2.02-2.11 (m, 2H), 2.52 (d, J=27.5 Hz, 4H), 3.32-3.36 (m, 2H), 3.40-3.46 (m, 2H), 3.51 (t, J=6.1 Hz, 2H), 3.95-4.01 (m, 2H), 4.06 (s, 2H), 7.19 (t, J=9.0 Hz, 1H), 7.29-7.34 (m, 1H), 7.36-7.41 (m, 1H).

### EXAMPLE 6

### 4-(3-(aminomethyl)-4-fluorobenzyl)-5,6,7,8-tetrahydrophthalazin-1(2H)-one

### EXAMPLE 6A

This example was prepared as described in EXAMPLE 2C by substituting EXAMPLE 1C for EXAMPLE 2B.

### EXAMPLE 6B

### 4-(3-(aminomethyl)-4-fluorobenzyl)-5,6,7,8-tetrahydrophthalazin-1(2H)-one

To a solution of EXAMPLE 6A (1.5 g) in 20% ammonia in methanol (150 mL) was added Raney nickel (15 g). The mixture was shaken under hydrogen (60 psi) at ambient temperature for 2 hours, filtered, and concentrated. The concentrate was purified by HPLC (Zorbax® C-18 ODS packing material [Agilent Technologies, Santa Clara, CA], 0-100% acetonitrile/water with 0.1% trifluoroacetic acid) to give the title compound as the trifluoroacetate salt. ¹H NMR (CD₃OD) δ1.55-1.65 (m, 4H), 2.33-2.41 (m, 4H), 3.90 (s, 2H), 4.04 (s, 2H), 7.21-7.25 (m, 1H), 7.27-7.29 (m, 1H), 7.31 (d, J=7.0 Hz, 1H), 8.20-8.27 (brs, 2H).

### EXAMPLE 7

### 4-(3-((dimethylamino)methyl)-4-fluorobenzyl)-5,6,7,8-tetrahydrophthalazin-1(2H)-one

To a solution of EXAMPLE 6 (75 mg) in methanol (8 mL) was added 37 wt% formaldehyde in water (39 µL) and triethylamine (36 µL). The solution was stirred at ambient temperature for 1 hour. Sodium cyanoborohydride (49 mg) and zinc chloride (35 mg) were added, and the mixture was stirred for 60 hours and was concentrated. The concentrate was dissolved in trifluoroacetic acid/methanol and purified by HPLC (Zorbax® C-18 ODS packing material [Agilent Technologies, Santa Clara, CA], 0-100% acetonitrile/water with 0.1% trifluoroacetic acid). The product was dissolved in methanol/dichloromethane and treated with 1M hydrochloric acid in diethyl ether to give the title compound as the hydrochloride salt. ¹H NMR (CD₃OD) δ1.68-1.8 (m, 4H), 2.50-2.60 (m, 4H), 2.88 (s, 6H), 4.10 (s, 2H), 4.39 (s, 2H), 7.22-7.27 (m, 1H), 7.40-7.44 (m, 1H), 7.46 (dd, J=6.9, 2.0 Hz, 1H).

### EXAMPLE 8

### 4-(4-fluoro-3-((isopropylamino)methyl)benzyl)-5,6,7,8-tetrahydrophthalazin-1(2H)-one

This example was prepared as the hydrochloride salt as described in EXAMPLE 7 by substituting acetone for formaldehyde. ¹H NMR (CD₃OD) δ1.39 (d, J=6.7 Hz, 6H), 1.68-1.77 (m, 4H), 2.43-2.59 (m, 4H), 3.41-3.50 (m, 1H), 4.05 (s, 2H), 4.24 (s, 2H), 7.18-7.24 (m, 1H), 7.35-7.38 (m, 1H), 7.38-7.42 (m, 1H).

### EXAMPLE 9

### 4-(3-((cyclohexylamino)methyl)-4-fluorobenzyl)-5,6,7,8-tetrahydrophthalazin-1(2H)-one

This example was prepared as the hydrochloride salt as described in EXAMPLE 7 by substituting cyclohexanone for formaldehyde. ¹H NMR (CD₃OD) δ 1.32-1.46 (m, 4H), 1.68-1.81 (m, 6H), 1.84-1.94 (m, 2H), 2.13-2.22 (m, 2H), 2.43-2.61 (m, 4H), 3.08-3.18 (m, 1H), 4.07 (s, 2H), 4.26 (s, 2H), 7.18-7.23 (m, 1H), 7.35-7.39 (m, 1H), 7.40-7.43 (m, 1H).

### EXAMPLE 10

### 4-(4-fluoro-3-((tetrahydro-2H-pyran-4-ylamino)methyl)benzyl)-5,6,7,8-tetrahydrophthalazin-1(2H)-one

This example was prepared as the hydrochloride salt as described in EXAMPLE 7 by substituting tetrahydro-4H-pyran-4-one for formaldehyde. ¹H NMR (CD₃OD) δ 1.66-1.76 (m, 6H), 2.04-2.14 (m, 2H), 2.40-2.57 (m, 4H), 3.40-3.51 (m, 3H), 4.03 (s, 2H), 4.05 (d, J=4.6 Hz, 2H), 4.29 (s, 2H), 7.18-7.25 (m, 1H), 7.36-7.39 (m, 1H), 7.40 (d, J=1.8 Hz, 1H).

### EXAMPLE 11

### 4-(4-fluoro-3-((methyl((1-methylpyrrolidin-3-yl)methyl)amino)methyl)benzyl)-5,6,7,8-tetrahydrophthalazin-1 (2H)-one

To a solution of EXAMPLE 6 (75 mg) in methanol (8 mL) was added 3-formyl-pyrrolidine-1-carboxylic acid tert-butyl ester (104 mg) and triethylamine (36 µL). The mixture was stirred at ambient temperature for 1 hour. Sodium cyanoborohydride (49 mg) and zinc chloride (35 mg) were added. The mixture was stirred for 60 hours and trifluoracetic acid was added and the mixture stirred for one hour and was concentrated. The concentrate was dissolved in water/acetonitrile and was purified by HPLC (Zorbax® C-18 ODS packing material [Agilent Technologies, Santa Clara, CA], 0-100% acetonitrile/water with 0.1% trifluoroacetic acid). The residue was treated as described above with with 37 wt% formaldehyde in water (39 µL), followed by treatment with 1M hydrochloric acid in diethyl ether to obtain the title compound as the HCl salt. ¹H NMR (CD₃OD) δ1.69-1.74 (m, 6H), 1.80-1.88 (m, 3H), 2.10-2.20 (m, 2H), 2.44-2.58 (m, 6H), 3.10 (dd, J=11.4, 7.5 Hz, 2H), 3.22-3.26 (m, 1H), 3.34-3.38 (m, 2H), 3.52-3.56 (m, 1H), 4.03 (s, 2H), 4.31 (s, 2H), 7.18-7.23 (m, 1H), 7.35-7.39 (m, 1H), 7.49 (dd, J=6.9, 2.0 Hz, 1H).

### EXAMPLE 12

### 4-(4-fluoro-3-((methyl(((2R)-1-methylpyrrolidin-2-yl)methyl)amino)methyl)benzyl)-5,6,7,8-tetrahydrophthalazin-1(2H)-one

This example was prepared as the hydrochloride salt as described in EXAMPLE 11 by substituting N-(tert-butoxycarbonyl)-D-prolinal for 3-formyl-pyrrolidine-1-carboxylic acid tert-butyl ester. ¹H NMR (CD₃OD) δ 1.74-1.83 (m, 4H), 1.95-2.07 (m, 1H), 2.10-2.28 (m, 2H), 2.52-2.70 (m, 5H), 2.91 (s, 3H), 3.04 (s, 3H), 3.21-3.29 (m, 1H), 3.63-3.69 (m, 1H), 3.73-3.81 (m, 1H), 3.90-4.00 (m, 1H), 4.05-4.13 (m, 1H), 4.20 (s, 2H), 4.50-4.62 (m, 2H), 7.27 (t, J=9.1 Hz, 1H), 7.44-7.49 (m, 1H), 7.64 (d, J=5.2 Hz, 1H).

### EXAMPLE 13

### 4-(3-((cyclopropylamino)methyl)benzyl)-5,6,7,8-tetrahydrophthalazin-1(2H)-one

### EXAMPLE 13A

### 3-(3-(diethoxymethyl)benzylidene)-4,5,6,7-tetrahydroisobenzofuran-1(3H)-one

This example was prepared as described in EXAMPLE 1C by substituting 3-(diethoxymethyl)benzaldehyde for 2-fluoro-5-formylbenzonitrile.

### EXAMPLE 13B

### 4-(3-(diethoxymethyl)benzyl)-5,6,7,8-tetrahydrophthalazin-1(2H)-one

This example was prepared as described in EXAMPLE 2C by substituting EXAMPLE 13A for EXAMPLE 2B.

### EXAMPLE 13C

### 3-((4-oxo-3,4,5,6,7,8-hexahydrophthalazin-1-yl)methyl)benzaldehyde

To a solution of EXAMPLE 13B (681 mg) in a 1:1 mixture of ethanol/water (20 mL) was added concentrated sulfuric acid (0.4 mL). The mixture was refluxed for 16 hours. The mixture was cooled and concentrated, and the concentrate was triturated with saturated sodium bicarbonate. The solid was filtered, washed with water and dried.

### EXAMPLE 13D

### 4-(3-((cyclopropylamino)methyl)benzyl)-5,6,7,8-tetrahydrophthalazin-1(2H)-one

A solution of EXAMPLE 13C (80 mg) and cyclopropylamine (51 mg) in methanol (8 mL) was stirred at ambient temperature for 1 hour. Sodium cyanoborohydride (57 mg) was added, and the solution was stirred for 18 hours and was concentrated. The concentrate was dissolved in methanol/trifluoroacetic acid and was purified by HPLC (Zorbax® C-18 ODS packing material [Agilent Technologies, Santa Clara, CA], 0-100% acetonitrile/water with 0.1% trifluoroacetic acid). The product was dissolved in methanol/dichloromethane and was treated with 1M hydrochloric acid in diethyl ether and concentrated to give the title compound as the hydrochloride salt. ¹H NMR (CD₃OD) δ 0.82-0.92 (m, 4H), 1.65-1.77 (m, 4H), 2.41-2.60 (m, 4H), 2.69-2.79 (m, 1H), 4.11 (s, 2H), 4.28 (s, 2H), 7.31 (d, J=6.7 Hz, 1H), 7.34-7.40 (m, 2H), 7.41-7.45 (m, 1H).

### EXAMPLE 14

### 4-(3-((isopropylamino)methyl)benzyl)-5,6,7,8-tetrahydrophthalazin-1(2H)-one

This example was prepared as the hydrochloride salt as described in EXAMPLE 13 by substituting isopropylamine for cyclopropylamine. ¹H NMR (CD₃OD) δ1.38 (d, J=6.7 Hz, 6H), 1.68-1.79 (m, 4H), 2.41-2.65 (m, 4H), 3.38-3.48 (m, 1H), 4.12 (s, 2H), 4.17 (s, 2H), 7.31 (d, J=7.1 Hz, 1H), 7.35-7.38 (m, 1H), 7.38-7.41 (m, 1H), 7.41-7.46 (m, 1H).

### EXAMPLE 15

### 4-(3-(morpholin-4-ylmethyl)benzyl)-5,6,7,8-tetrahydrophthalazin-1(2H)-one

This example was prepared as the hydrochloride salt as described in EXAMPLE 13 by substituting morpholine for cyclopropylamine. ¹H NMR (CD₃OD) δ1.67-1.78 (m, 4H), 2.39-2.57 (m, 4H), 3.13-3.24 (m, 2H), 3.32-3.39 (m, 2H), 3.71-3.80 (m, 2H), 4.03 (dd, J=13.3, 3.2 Hz, 2H), 4.08 (s, 2H), 4.34 (s, 2H), 7.37 (d, J=6.7 Hz, 1H), 7.39-7.42 (m, 1H), 7.41-7.44 (m, 1H), 7.44-7.48 (m, 1H).

### EXAMPLE 16

### 4-(3-(pyrrolidin-1-ylmethyl)benzyl)-5,6,7,8-tetrahydrophthalazin-1(2H)-one

This example was prepared as the hydrochloride salt as described in EXAMPLE 13 by substituting pyrrolidine for cyclopropylamine. ¹H NMR (CD₃OD) δ1.67-1.79 (m, 4H), 1.95-2.07 (m, 2H), 2.11-2.24 (m, 2H), 2.44-2.65 (m, 4H), 3.09-3.26 (m, 2H), 3.41-3.54 (m, 2H), 4.15 (s, 2H), 4.35 (s, 2H), 7.32-7.37 (m, 1H), 7.39-7.47 (m, 3H).

### EXAMPLE 17

### 4-(3-((cyclohexylamino)methyl)benzyl)-5,6,7,8-tetrahydrophthalazin-1(2H)-one

This example was prepared as the hydrochloride salt as described in EXAMPLE 13 by substituting cyclohexylamine for cyclopropylamine. ¹H NMR (CD₃OD) δ1.20-1.27 (m, 1H), 1.31-1.45 (m, 4H), 1.66-1.78 (m, 5H), 1.85-1.93 (m, 2H), 2.12-2.20 (m, 2H), 2.45-2.60 (m, 4H), 3.08 (dd, J=14.6, 7.6 Hz, 1H), 4.11 (s, 2H), 4.19 (s, 2H), 7.32 (d, J=7.0 Hz, 1H), 7.34-7.38 (m, 1H), 7.38-7.42 (m, 1H), 7.41-7.45 (m, 1H).

### EXAMPLE 18

### 4-(3-((methylamino)methyl)benzyl)-5,6,7,8-tetrahydrophthalazin-1(2H)-one

This example was prepared as the hydrochloride salt as described in EXAMPLE 13 by substituting 2M methylamine in methanol for cyclopropylamine. ¹H NMR (CD₃OD) δ1.69-1.78 (m, 4H), 2.45-2.59 (m, 4H), 2.70 (s, 3H), 4.13 (s, 2H), 4.16 (s, 2H), 7.30-7.33 (m, 1H), 7.33-7.37 (m, 1H), 7.37-7.40 (m, 1H), 7.43 (t, J=7.4 Hz, 1H).

### EXAMPLE 19

### 4-(3-((ethylamino)methyl)benzyl)-5,6,7,8-tetrahydrophthalazin-1(2H)-one

This example was prepared as the hydrochloride salt as described in EXAMPLE 13 by substituting 2M ethylamine in methanol for cyclopropylamine. ¹H NMR (CD₃OD) δ1.33 (t, J=7.2 Hz, 3H), 1.66-1.80 (m, 4H), 2.42-2.62 (m, 4H), 3.10 (q, J=7.4 Hz, 2H), 4.13 (s, 2H), 4.16 (s, 2H), 7.31 (d, J=7.1 Hz, 1H), 7.34-7.37 (m, 1H), 7.38-7.40 (m, 1H), 7.41-7.45 (m, 1H).

### EXAMPLE 20

### 4-(3-((4-methylpiperidin-1-yl)methyl)benzyl)-5,6,7,8-tetrahydrophthalazin-1(2H)-one

This example was prepared as the hydrochloride salt as described in EXAMPLE 13 by substituting 4-methylpiperidine for cyclopropylamine. ¹H NMR (CD₃OD) δ0.99 (d, J=6.4 Hz, 3H), 1.39-1.54 (m, 2H), 1.67-1.76 (m, 5H), 1.83-1.95 (m, 2H), 2.44-2.63 (m, 4H), 2.92-3.04 (m, 2H), 3.35-3.46 (m, 2H), 4.15 (s, 2H), 4.26 (s, 2H), 7.34-7.37 (m, 1H), 7.40-7.44 (m, 2H), 7.44-7.47 (m, 1H).

### EXAMPLE 21

### 4-(3-(((2-(4-(trifluoromethyl)phenyl)ethyl)amino)methyl)benzyl)-5,6,7,8-tetrahydrophthalazin-1(2H)-one

This example was prepared as the hydrochloride salt as described in EXAMPLE 13 by substituting 3-(trifluoromethyl)phenethylamine for cyclopropylamine. ¹H NMR (CD₃OD) δ1.64-1.72 (m, 4H), 2.40-2.57 (m, 4H), 3.06-3.14 (m, 2H), 3.27-3.29 (m, 2H), 4.07 (s, 2H), 4.22 (s, 2H), 7.33 (d, J=7.3 Hz, 1H), 7.35-7.38 (m, 1H), 7.38-7.42 (m, 1H), 7.44 (t, J=7.5 Hz, 1H), 7.55 (d, J=0.9 Hz, 1H), 7.55-7.58 (m, 1H), 7.59 (d, J=5.2 Hz, 1H), 7.60-7.62 (m, 1H).

### EXAMPLE 22

### 4-(3-((cyclohexyl(methyl)amino)methyl)benzyl)-5,6,7,8-tetrahydrophthalazin-1(2H)-one

This example was prepared as the hydrochloride salt as described in EXAMPLE 13 by substituting N-methyl cyclohexylamine for cyclopropylamine. ¹H NMR (CD₃OD) δ1.23-1.40 (m, 3H), 1.53-1.65 (m, 2H), 1.67-1.79 (m, 5H), 1.90-2.00 (m, 2H), 2.02-2.18 (m, 2H), 2.40-2.49 (m, 2H), 2.49-2.58 (m, 2H), 2.71 (s, 3H), 3.16-3.28 (m, 1H), 4.07 (s, 2H), 4.17 (d, J=12.9 Hz, 1H), 4.45 (d, J=13.2 Hz, 1H), 7.34-7.36 (m, 1H), 7.37-7.39 (m, 1H), 7.41 (s, 1H), 7.43-7.49 (m, 1H).

### EXAMPLE 23

### 4-(3-((2-ethylpyrrolidin-1-yl)methyl)benzyl)-5,6,7,8-tetrahydrophthalazin-1(2H)-one

This example was prepared as the hydrochloride salt as described in EXAMPLE 13 by substituting 2-ethylpyrrolidine for cyclopropylamine. ¹H NMR (CD₃OD) δ0.94 (t, J=7.5 Hz, 3H), 1.52-1.63 (m, 1H), 1.69-1.77 (m, 4H), 1.78-1.87 (m, 2H), 1.91-2.04 (m, 1H), 2.05-2.17 (m, 1H), 2.31-2.44 (m, 1H), 2.45-2.64 (m, 4H), 3.19-3.28 (m, 1H), 3.34-3.47 (m, 2H), 4.15 (s, 2H), 4.21 (d, J=12.9 Hz, 1H), 4.50 (d, J=13.2 Hz, 1H), 7.33-7.38 (m, 1H), 7.40-7.44 (m, 2H), 7.44-7.48 (m, 1H).

### EXAMPLE 24

### 4-(4-((cyclopropylamino)methyl)benzyl)-5,6,7,8-tetrahydrophthalazin-1(2H)-one

### EXAMPLE 24A

### 3-(4-(diethoxymethyl)benzylidene)-4,5,6,7-tetrahydroisobenzofuran-1(3H)-one

This example was prepared as described in EXAMPLE 1C by substituting 4-(diethoxymethyl)benzaldehyde for 2-fluoro-5-formylbenzonitrile.

### EXAMPLE 24B

### 4-(4-(diethoxymethyl)benzyl)-5,6,7,8-tetrahydrophthalazin-1(2H)-one

This example was prepared as described in EXAMPLE 2C by substituting EXAMPLE 24A for EXAMPLE 2B.

### EXAMPLE 24C

### 4-((4-oxo-3,4,5,6,7,8-hexahydrophthalazin-1-yl)methyl)benzaldehyde

This example was prepared as described in EXAMPLE 13C by substituting EXAMPLE 24B for EXAMPLE 13B.

### EXAMPLE 24D

### 4-(4-((cyclopropylamino)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C. ¹H NMR (CD₃OD) δ0.82-0.88 (m, 2H), 0.89-0.94 (m, 2H), 1.62-1.77 (m, 4H), 2.35-2.44 (m, 2H), 2.45-2.55 (m, 2H), 2.70-2.82 (m, 1H), 4.02 (s, 2H), 4.27 (s, 2H), 7.30 (d, J=8.3 Hz, 2H), 7.43 (d, J=8.0 Hz, 2H).

### EXAMPLE 25

### 4-(4-((isopropylamino)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and 2-propylamine for cyclopropylamine. ¹H NMR (CD₃OD) 61.38 (d, J=6.4 Hz, 6H), 1.65-1.72 (m, 4H), 2.37-2.45 (m, 2H), 2.46-2.52 (m, 2H), 3.39-3.50 (m, 1H), 4.01 (s, 2H), 4.17 (s, 2H), 7.31 (d, J=8.3 Hz, 2H), 7.44 (d, J=8.0 Hz, 2H).

### EXAMPLE 26

### 4-(4-(morpholin-4-ylmethyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and morpholine for cyclopropylamine. ¹H NMR (CD₃OD) 61.65-1.76 (m, 4H), 2.43-2.48 (m, 2H), 2.49-2.58 (m, 2H), 3.13-3.24 (m, 2H), 3.33-3.37 (m, 2H), 3.36-3.41 (m, 1H), 3.70-3.80 (m, 2H), 3.99-4.03 (m, 1H), 4.06 (s, 2H), 4.34 (s, 2H), 7.35 (d, J=8.0 Hz, 2H), 7.49 (d, J=8.0 Hz, 2H).

### EXAMPLE 27

### 4-(4-(pyrrolidin-1-ylmethyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and pyrrolidine for cyclopropylamine. ¹H NMR (CD₃OD) δ1.63-1.76 (m, 4H), 1.94-2.06 (m, 2H), 2.10-2.24 (m, 2H), 2.37-2.46 (m, 2H), 2.46-2.55 (m, 2H), 3.11-3.23 (m, 2H), 3.38-3.58 (m, 2H), 4.02 (s, 2H), 4.34 (s, 2H), 7.33 (d, J=7.7 Hz, 2H), 7.46 (d, J=8.0 Hz, 2H).

### EXAMPLE 28

### 4-(4-((cyclohexylamino)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and cyclohexylamine for cyclopropylamine. ¹H NMR (CD₃OD) δ1.21-1.29 (m, 1H), 1.33-1.43 (m, 4H), 1.64-1.75 (m, 5H), 1.86-1.93 (m, 2H), 2.13-2.21 (m, 2H), 2.37-2.44 (m, 2H), 2.49 (t, J=4.9 Hz, 2H), 3.05-3.16 (m, 1H), 4.01 (s, 2H), 4.18 (s, 2H), 7.30 (d, J=8.0 Hz, 2H), 7.43 (d, J=8.0 Hz, 2H).

### EXAMPLE 29

### 4-(4-((4-phenylpiperidin-1-yl)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and 4-phenylpiperidine for cyclopropylamine. ¹H NMR (CD₃OD) δ1.64-1.76 (m, 4H), 1.93-2.03 (m, 2H), 2.05-2.15 (m, 2H), 2.40-2.49 (m, 2H), 2.48-2.55 (m, 2H), 2.81-2.94 (m, 1H), 3.10-3.23 (m, 2H), 3.54-3.64 (m, 2H), 4.04 (s, 2H), 4.33 (s, 2H), 7.19-7.22 (m, 1H), 7.24 (d, J=7.1 Hz, 2H), 7.28-7.33 (m, 2H), 7.36 (d, J=8.0 Hz, 2H), 7.51 (d, J=7.7 Hz, 2H).

### EXAMPLE 30

### 4-(4-((methylamino)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and 2M methylamine in methanol for cyclopropylamine. ¹H NMR (CD₃OD) δ1.64-1.73 (m, 4H), 2.38-2.44 (m, 2H), 2.47-2.54 (m, 2H), 2.71 (s, 3H), 4.02 (s, 2H), 4.15 (s, 2H), 7.31 (d, J=8.3 Hz, 2H), 7.43 (d, J=8.0 Hz, 2H).

### EXAMPLE 31

### 4-(4-((ethylamino)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and 2M ethylamine in methanol for cyclopropylamine. ¹H NMR (CD₃OD) δ1.32 (t, J=7.4 Hz, 3H), 1.64-1.72 (m, 4H), 2.37-2.44 (m, 2H), 2.45-2.52 (m, 2H), 3.10 (q, J=7.4 Hz, 2H), 4.01 (s, 2H), 4.15 (s, 2H), 7.30 (d, J=8.3 Hz, 2H), 7.43 (d, J=8.0 Hz, 2H).

### EXAMPLE 32

### 4-(4-((4-methylpiperidin-1-yl)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and 4-methylpiperidine for cyclopropylamine. ¹H NMR (CD₃OD) δ0.99 (d, J=6.4 Hz, 3H), 1.33-1.48 (m, 2H), 1.66-1.75 (m, 5H), 1.85-1.95 (m, 2H), 2.40-2.48 (m, 2H), 2.48-2.57 (m, 2H), 2.90-3.05 (m, 2H), 3.44 (d, J=12.3 Hz, 2H), 4.04 (s, 2H), 4.25 (s, 2H), 7.33 (d, J=8.0 Hz, 2H), 7.46 (d, J=8.0 Hz, 2H).

### EXAMPLE 33

### 4-(4-(((2-(3-(trifluoromethyl)phenyl)ethyl)amino)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and 3-(trifluoromethyl)phenethylamine for cyclopropylamine. ¹H NMR (CD₃OD) δ1.62-1.7 (m, 4H), 2.38-2.44 (m, 2H), 2.46-2.52 (m, 2H), 3.06-3.14 (m, 2H), 3.27-3.35 (m, 2H), 4.02 (s, 2H), 4.22 (s, 2H), 7.31 (d, J=8.3 Hz, 2H), 7.45 (d, J=8.3 Hz, 2H), 7.53-7.57 (m, 2H), 7.57-7.62 (m, 2H).

### EXAMPLE 34

### 4-(4-((cyclohexyl(methyl)amino)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and N-methylcyclohexylamine for cyclopropylamine. ¹H NMR (CD₃OD) δ1.20-1.3 (m, 1H), 1.32-1.44 (m, 2H), 1.54-1.63 (m, 2H), 1.65-1.75 (m, 5H), 1.91-2.01 (m, 2H), 2.05-2.18 (m, 2H), 2.39-2.46 (m, 2H), 2.47-2.53 (m, 2H), 2.71 (s, 3H), 3.23-3.29 (m, 1H), 4.03 (s, 2H), 4.13 (d, J=13.2 Hz, 1H), 4.47 (d, J=13.2 Hz, 1H), 7.34 (d, J=8.3 Hz, 2H), 7.43-7.46 (m, 2H).

### EXAMPLE 35

### 4-(4-((2-methylpyrrolidin-1-yl)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and 2-methylpyrrolidine for cyclopropylamine. ¹H NMR (CD₃OD) δ1.39 (d, J=6.7 Hz, 3H), 1.66-1.78 (m, 5H), 1.93-2.02 (m, 1H), 2.03-2.13 (m, 1H), 2.29-2.38 (m, 1H), 2.39-2.45 (m, 2H), 2.47-2.53 (m, 2H), 3.15-3.27 (m, 1H), 3.34-3.40 (m, 1H), 3.51-3.62 (m, 1H), 4.02 (s, 2H), 4.09-4.17 (m, 1H), 4.43-4.55 (m, 1H), 7.33 (d, J=8.0 Hz, 2H), 7.46 (d, J=8.3 Hz, 2H).

### EXAMPLE 36

### 4-(4-((4-methyl-1,4-diazepan-1-yl)methyl)benzyl)-5,6,7,8-tetrahydrophthalazin-1(2H)-one

This example was prepared as the hydrochloride salt as described in EXAMPLE 13D by substituting EXAMPLE 24C for EXAMPLE 13C and 1-methylhomopiperazine for cyclopropylamine. ¹H NMR (CD₃OD) δ1.68-1.79 (m, 4H), 2.29-2.42 (m, 2H), 2.46-2.53 (m, 2H), 2.53-2.62 (m, 2H), 2.97 (s, 3H), 3.35-3.44 (m, 1H), 3.47-3.65 (m, 2H), 3.67-3.96 (m, 5H), 4.11 (s, 2H), 4.46 (s, 2H), 7.35 (d, J=8.0 Hz, 2H), 7.59 (d, J=8.0 Hz, 2H).

### EXAMPLE 37

### 4-(3-((4-methyl-1,4-diazepan-1-yl)methyl)benzyl)-5,6,7,8-tetrahydrophthalazin-1(2H)-one

This example was prepared as the hydrochloride salt as described in EXAMPLE 13D by substituting 1-methylhomopiperazine for cyclopropylamine. ¹H NMR (CD₃OD) δ1.68-1.81 (m, 4H), 2.29-2.41 (m, 2H), 2.44-2.53 (m, 2H), 2.55-2.64 (m, 2H), 2.98 (s, 3H), 3.33-3.40 (m, 1H), 3.40-3.56 (m, 2H), 3.58-3.72 (m, 1H), 3.73-3.97 (m, 4H), 4.15 (s, 2H), 4.46 (s, 2H), 7.37 (d, J=7.7 Hz, 1H), 7.46 (t, J=7.8 Hz, 1H), 7.51 (d, J=6.1 Hz, 2H).

### EXAMPLE 38

### 4-(4-fluoro-3-pyrimidin-2-ylbenzyl)-5,6,7,8-tetrahydrophthalazin-1(2H)-one

### EXAMPLE 38A

### 3-(3-bromo-4-fluorobenzylidene)-4,5,6,7-tetrahydroisobenzofuran-l(3H)-one

This example was prepared as described in EXAMPLE 1C by substituting 3-bromo-4-fluorobenzaldehyde for 2-fluoro-5-formylbenzonitrile.

### EXAMPLE 38B

### 4-(3-bromo-4-fluorobenzyl)-5,6,7,8-tetrahydrophthalazin-1(2H)-one

This example was prepared as described in EXAMPLE 2D by substituting EXAMPLE 38A for EXAMPLE 2B.

### EXAMPLE 38C

### 4-(4-fluoro-3-pyrimidin-2-ylbenzyl)-5,6,7,8-tetrahydrophthalazin-1(2H)-one

To EXAMPLE 38B (75 mg) in N,N-dimethylformamide (8 mL) was added 2-tributylstannylpyrimidine (81 mg), tris(dibenzylidineacetone)dipalladium(0) (20 mg), tri-o-tolylphosphine (20 mg) and triethylamine (92 µL). The mixture was stirred at 70°C for 17 hours. After cooling, the mixture was filtered, and the filtrate was concentrated. The concentrate was purified by HPLC (Zorbax® C-18 ODS packing material [Agilcnt Technologies, Santa Clara, CA], 0-100% acetonitrile/water with 0.1% trifluoroacetic acid). The product was dissolved in methanol/dichloromethane and treated with 1M hydrochloric acid in diethyl ether and concentrated to provide the title compound as the hydrochloride salt. ¹H NMR (CD₃OD) δ1.69-1.78 (m, 4H), 2.48-2.60 (m, 4H), 4.11 (s, 2H), 7.27 (dd, J=10.8, 8.5 Hz, 1H), 7.43-7.50 (m, 1H), 7.61 (t, J=5.1 Hz, 1H), 7.87 (dd, J=7.1, 2.4 Hz, 1H), 9.01 (d, J=5.1 Hz, 2H).

### EXAMPLE 39

### 4-(4-fluoro-3-pyridin-3-ylbenzyl)-5,6,7,8-tetrahydrophthalazin-1(2H)-one

To EXAMPLE 38B (75 mg), 3-pyridineboronic acid (54 mg) and dichlorobis(triphenylphosphine)palladium (II) (28 mg) in 7:3:2 1,2-dimethoxyethane/ water/ethanol (3 mL) was added 2M sodium carbonate (0.22 mL). The mixture was stirred in a CEM Explorer® microwave reactor (Matthews, NC) for 10 minutes at 150°C. After cooling, the mixture was filtered, and the filtrate was concentrated. The concentrate was purified by HPLC (Zorbax® C-18 ODS packing material [Agilent Technologies, Santa Clara, CA], 0-100% acetonitrile/water with 0.1% trifluoroacetic acid). The product was dissolved in methanol/dichloromethane and was treated with 1M hydrochloric acid in diethyl ether and concentrated to provide the title compound as the hydrochloride salt. ¹H NMR (CD₃OD) δ1.70-1.80 (m, 4H), 2.50-2.62 (m, 4H), 4.17 (s, 2H), 7.33 (dd, J=10.7, 8.5 Hz, 1H), 7.40-7.48 (m, 1H), 7.60 (dd, J=7.3, 1.8 Hz, 1H), 8.22 (dd, J=8.2, 5.8 Hz, 1H), 8.87 (d, J=8.2 Hz, 1H), 8.90 (d, J=5.5 Hz, 1H), 9.12 (s, 1H).

### EXAMPLE 40

### 4-(4-fluoro-3-pyridin-4-ylbenzyl)-5,6,7,8-tetrahydrophthalazin-1(2H)-one

This example was prepared as the hydrochloride salt as described in EXAMPLE 39 by substituting 4-pyridine boronic acid for 3-pyridine boronic acid. ¹H NMR (CD₃OD) δ1.68-1.84 (m, 4H), 2.46-2.64 (m, 4H), 4.15 (s, 2H), 7.35 (dd, J=11.0, 8.5 Hz, 1H), 7.48-7.53 (m, 1H), 7.69 (dd, J=7.2, 2.0 Hz, 1H), 8.32 (d, J=5.8 Hz, 2H), 8.91 (d, J=6.7 Hz, 2H).

### EXAMPLE 41

### N,N-diethyl-2'-fluoro-5'-((4-oxo-3,4,5,6,7,8-hexahydrophthalazin-1-yl)methyl)-1,1'-biphenyl-2-carboxamide

This example was prepared as the hydrochloride salt as described in EXAMPLE 39 by substituting (2-(N,N-diethylaminocarbonyl)phenyl)boronic acid for 3-pyridineboronic acid. ¹H NMR (CD₃OD) δ0.83 (t, J=7.2 Hz, 3H), 0.93 (t, J=7.2 Hz, 3H), 1.72-1.83 (m, 4H), 2.51-2.66 (m, 4H), 2.73-3.01 (m, 2H), 3.02-3.25 (m, 2H), 4.11 (s, 2H), 7.13-7.17 (m, 1H), 7.17-7.19 (m, 1H), 7.26-7.31 (m, 1H), 7.38-7.41 (m, 2H), 7.47-7.50 (m, 1H), 7.51-7.54 (m, 1H).

### EXAMPLE 42

### N-(2-fluoro-5-((4-oxo-3,4,5,6,7,8-hexahydrophthalazin-1-yl)methyl)phenyl)-3-piperidin-1-ylpropanamide

To 3-(1-piperidinyl)propionic acid (28 mg) in dichloromethane (3 mL) was added oxalyl chloride (24 µL) and a drop of N,N-dimethylformamide. The mixture was stirred at ambient temperature for 1 hour and concentrated. The concentrate was dissolved in dichloromethane (3 mL) and added to a solution of EXAMPLE 2C (50 mg) in tetrahydrofuran (3 mL). Triethylamine (31 µL) was also added. The mixture was stirred at ambient temperature for 16 hours and was concentrated. The concentrate was purified by HPLC (Zorbax® C-18 ODS packing material [Agilent Technologies, Santa Clara, CA], 0-100% acetonitrile/water with 0.1% trifluoroacetic acid). The product was dissolved in methanol/dichloromethane and treated with 1M hydrochloric acid in diethyl ether and was concentrated to provide the title compound as the hydrochloride salt. ¹H NMR (CD₃OD) δ1.50-1.59 (m, 1H), 1.68-1.75 (m, 4H), 1.76-1.87 (m, 3H), 1.92-2.01 (m, 2H), 2.41-2.57 (m, 4H), 2.94-2.98 (m, 2H), 2.98-3.03 (m, 2H), 3.45 (t, J=6.9 Hz, 2H), 3.57 (d, J=12.2 Hz, 2H), 3.99 (s, 2H), 6.99-7.05 (m, 1H), 7.11 (dd, J=10.4, 8.5 Hz, 1H), 7.81 (dd, J=7.3, 1.8 Hz, 1H).

### EXAMPLE 43

### N-(2-fluoro-5-((4-oxo-3,4,5,6,7,8-hexahydrophthalazin-1-yl)methyl)phenyl)-3-(4-methylpiperazin-1-yl)propanamide

This example was prepared as the hydrochloride salt as described in EXAMPLE 42 by substituting 3-(4-methylpiperazin-1-yl)propionic acid for 3-(1-piperidinyl)propionic acid. ¹H NMR (CD₃OD) δ 1.65-1.79 (m, 4H), 2.38-2.58 (m, 4H), 3.03 (s, 3H), 3.07 (t, J=6.7 Hz, 2H), 3.60-3.65 (m, 2H), 3.65-3.68 (m, 3H), 3.70-3.89 (m, 4H), 3.97-4.06 (m, 1H), 4.00 (s, 2H), 6.99-7.04 (m, 1H), 7.12 (dd, J=10.7, 8.5 Hz, 1H), 7.83 (dd, J=7.3, 1.8 Hz, 1H).

### EXAMPLE 44

### 2-amino-N-(2-fluoro-5-((4-oxo-3,4,5,6,7,8-hexahydrophthalazin-1-yl)methyl)phenyl)acetamide

A solution of EXAMPLE 2 (50 mg) and Boc-L-glycine N-hydroxysuccinimide ester (54 mg) in tetrahydrofuran (4 mL) was stirred at ambient temperature for 16 hours and was concentrated. To this solid in dichloromethane (2 mL) was added trifluoroacetic acid (1 mL) and the mixture stirred at ambient temperature for 1 hour and concentrated. The concentrate was purified by HPLC (Zorbax® C-18 ODS packing material [Agilent Technologies, Santa Clara, CA], 0-100% acetonitrile/water with 0.1% trifluoroacetic acid) to provide the title compound as the trifluoroacetate salt. ¹H NMR (CD₃OD) δ 1.65-1.74 (m, 4H), 2.38-2.55 (m, 4H), 3.89 (s, 2H), 3.96 (s, 2H), 7.00-7.06 (m, 1H), 7.09-7.16 (m, 1H), 7.87 (dd, J=7.4, 2.1 Hz, 1H).

### EXAMPLE 45

### 3-cyclohexyl-N-(2-fluoro-5-((4-oxo-3,4,5,6,7,8-hexahydrophthalazin-1-yl)methyl)phenyl)propanamide

This example was prepared as described in EXAMPLE 42 by substituting cyclohexanepropionic acid for 3-(1-piperidinyl)propionic acid. ¹H NMR (CD₃OD) δ 0.90-1.00 (m, 2H) 1.16-1.33 (m, 4H) 1.53-1.61 (m, 2H) 1.63-1.68 (m, 1H) 1.70-1.74 (m, 5H) 1.74-1.82 (m, 3H) 2.39-2.46 (m, 4H) 2.48-2.51 (m, 2H) 3.94 (s, 2H) 6.96-7.01 (m, 1H) 7.07 (dd, J=10.7, 8.5 Hz, 1H) 7.69 (dd, J=7.2, 1.7 Hz, 1H).

### EXAMPLE 46

### N-(2-fluoro-5-((4-oxo-3,4,5,6,7,8-hexahydrophthalazin-1-yl)methyl)phenyl)piperidine-3-carboxamide

This example was prepared as the trifluoroacetate salt as described in EXAMPLE 42 by substituting 1-(tert-butoxycarbonyl)-3-piperidine carboxylic acid for 3-(1-piperidinyl)propionic acid. ¹H NMR (CD₃OD) δ 1.66-1.76 (m, 4H), 1.79-1.87 (m, 1H), 1.89-2.03 (m, 2H), 2.12 (dd, J=9.3, 4.9 Hz, 1H), 2.38-2.56 (m, 4H), 2.96-3.04 (m, 1H), 3.08-3.15 (m, 1H), 3.17-3.25 (m, 2H), 3.33-3.35 (m, 1H), 3.95 (s, 2H), 6.99-7.06 (m, 1H), 7.07-7.15 (m, 1H), 7.71 (dd, J=7.5, 2.0 Hz, 1H).

### EXAMPLE 47

### 4-(4-fluoro-3-(2-oxopyrrolidin-1-yl)benzyl)-5,6,7,8-tetrahydrophthalazin-1(2H)-one

To a solution of EXAMPLE 2 (200 mg) in dichloromethane (5 mL) was added 4-chlorobutanoylchloride (103 mg) and triethylamine (0.12 mL). The solution was stirred at ambient temperature for 16 hours and was concentrated. The concentrate was dissolved in ethanol (2 mL) and added to a solution of 21 wt% sodium ethoxide in ethanol (0.47 mL). The mixture was stirred at ambient temperature for 16 hours, treated with 2M hydrochloric acid (1 mL) and concentrated. The concentrate was purified by HPLC (Zorbax® C-18 ODS packing material [Agilent Technologies, Santa Clara, CA], 0-100% acetonitrile/water with 0.1% trifluoroacetic acid). ¹H NMR (CD₃OD) δ 1.66-1.77 (m, 4H), 2.15-2.27 (m, 2H), 2.40-2.51 (m, 4H), 2.51-2.58 (m, 2H), 3.78-3.86 (m, 2H), 3.97 (s, 2H), 7.11-7.15 (m, 1H), 7.16-7.19 (m, 1H), 7.22-7.27 (m, 1H).

### EXAMPLE 48

### N-(2-fluoro-5-((4-oxo-3,4,5,6,7,8-hexahydrophthalazin-l-yl)methyl)phenyl)azetidine-3-carboxamide

This example was prepared as the trifluoroacetate salt as described in EXAMPLE 42 by substituting 1-(tert-butoxycarbonyl)-3-azetidine carboxylic acid for 3-(1-piperidinyl)propionic acid. ¹H NMR (CD₃OD) δ 1.64-1.78 (m, 4H), 2.40-2.49 (m, 2H), 2.47-2.55 (m, 2H), 3.81-3.93 (m, 1H), 3.96 (s, 2H), 4.20-4.33 (m, 4H), 6.99-7.06 (m, 1H), 7.07-7.15 (m, 1H), 7.87 (dd, J=7.3, 2.2 Hz, 1H).

### EXAMPLE 49

### N-(2-(isopropylamino)ethyl)-3-((4-oxo-3,4,5,6,7,8-hexahydrophthalazin-1-yl)methyl)benzamide

### EXAMPLE 49A

### methyl 3-((3-oxo-4,5,6,7-tetrahydroisobenzofuran-1(3H)-ylidene)methyl)benzoate

This example was prepared as described in EXAMPLE 1C by substituting methyl-3-formylbenzoate for 2-fluoro-5-formylbenzonitrile.

### EXAMPLE 49B

### 3-((3-oxo-4,5,6,7-tetrahydroisobenzofuran-1(3H)-ylidene)methyl)benzoic acid

EXAMPLE 49A (6.09 g) in 1:1 tetrahydrofuran/water (60 mL) at ambient temperature was treated with lithium hydroxide monohydrate (1.8 g) and stirred for 16 hours. The mixture was acidified with 2N hydrochloric acid and partitioned between ethyl acetate and brine. The organic layer was washed with water and concentrated, and the concentrate was purified by flash chromatography on silica gel with ethyl acetate.

### EXAMPLE 49C

### 3-((4-oxo-3,4,5,6,7,8-hexahydrophthalazin-1-yl)methyl)benzoic acid

This example was prepared as described in EXAMPLE 2C by substituting EXAMPLE 49B for EXAMPLE 2B.

### EXAMPLE 49D

### N-(2-(isopropylamino)ethyl)-3-((4-oxo-3,4,5,6,7,8-hexahydrophthalazin-1-yl)methyl)benzamide

To a solution of EXAMPLE 49C (75 mg) in N,N-dimethylformamide (3 mL) was added N-isopropylethylenediamine (27 mg), 1-(3-dimethylaminopropyl)-3-ethylcarbodiimide hydrochloride (50 mg), 1-hydroxybenzotriazole hydrate (35 mg) and triethylamine (0.11 mL). The mixture was stirred at ambient temperature for 16 hours and was partitioned between brine and water. The organics were washed with brine and concentrated. The concentrate was purified by HPLC (Zorbax® C-18 ODS packing material [Agilent Technologies, Santa Clara, CA], 0-100% acetonitrile/water with 0.1% trifluoroacetic acid) to provide the title compound as the trifluoroacetate salt. ¹H NMR (CD₃OD) δ 1.34 (d, J=6.7 Hz, 6H), 1.65-1.74 (m, 4H), 2.37-2.44 (m, 2H), 2.47-2.54 (m, 2H), 3.23 (t, J=5.9 Hz, 2H), 3.39-3.47 (m, 1H), 3.67 (t, J=5.9 Hz, 2H), 4.05 (s, 2H), 7.41-7.44 (m, 2H), 7.71-7.74 (m, 2H).

### EXAMPLE 50

### N-(2-fluoro-5-((4-oxo-3,4,5,6,7,8-hexahydrophthalazin-1-yl)methyl)phenyl)-2-morpholin-4-ylacetamide

This example was prepared as the trifluoroacetate salt as described in EXAMPLE 41 by substituting morpholin-4-yl-acetic acid for 3-(1-piperidinyl)propionic acid. ¹H NMR (CD₃OD) δ 1.65-1.73 (m, 4H), 2.38-2.46 (m, 2H), 2.46-2.52 (m, 2H), 3.34-3.52 (m, 4H), 3.90-4.03 (m, 6H), 4.19 (s, 2H), 7.03-7.09 (m, 1H), 7.10-7.17 (m, 1H), 7.85 (dd, J=7.3, 2.1 Hz, 1H).

### EXAMPLE 51

### N-(2-morpholin-4-ylethyl)-3-((4-oxo-3,4,5,6,7,8-hexahydrophthalazin-1-yl)methyl)benzamide

### EXAMPLE 51A

### 3-((4-oxo-3,4,5,6,7,8-hexahydrophthalazin-1-yl)methyl)benzoic acid

The title compound was prepared according to procedure for EXAMPLE 1 substituting 3-formylbenzonitrile for 2-fluoro-5-formylbenzonitrile in EXAMPLE 1C. MS (DCI/NH₃) m/z 285 (M+H)⁺.

### EXAMPLE 51B

### N-(2-morpholin-4-ylethyl)-3-((4-oxo-3,4,5,6,7,8-hexahydrophthalazin-1-yl)methyl)benzamide

To a solution of EXAMPLE 51A (75 mg, 0.26 mmol) in anhydrous dichloromethane (5 mL) was added 4-(2-aminoethyl)morpholine (68 mg, 0.52 mmol), benzotriazol-1-yl-oxytripyrrolidinophosphonium hexafluorophosphate (271 mg, 0.52 mmol), and N,N'-diisopropylethylamine (0.18 mmol, 1.04 mmol) under nitrogen. The reaction mixture was stirred at room temperature for 16 hours, and concentrated. The residue was separated by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 397 (M+H)⁺; ¹H NMR (500 MHz, CD₃OD): δ 1.64 - 1.75 (m, 4H), 2.37 - 2.45 (m, 2H), 2.45 - 2.55 (m, 2H), 3.15 - 3.27 (m, 2H), 3.40 (t, *J=*5.80 Hz, 2H), 3.63 - 3.71 (m, 2H), 3.74 - 3.81 (m, 4H), 4.02 - 4.13 (m, 4H), 7.42 - 7.46 (m, 2H), 7.71 - 7.75 (m, 2H).

### EXAMPLE 52

### 3-((4-oxo-3,4,5,6,7,8-hexahydrophthalazin-1-yl)methyl)-N-(2-pyrrolidin-1-ylethyl)benzamide

The title compound was prepared as a trifluoroacetic acid salt according to procedure for EXAMPLE 51 substituting 1-(2-aminoethyl)pyrrolidine for 4-(2-aminoethyl)morpholine. MS (DCI/NH₃) m/z 381 (M+H)⁺; ¹H NMR (500 MHz, CD₃OD): δ 1.65 - 1.74 (m, 4H), 1.97 - 2.08 (m, 2H), 2.13 - 2.22 (m, 2H), 2.39 - 2.45 (m, 2H), 2.46 - 2.54 (m, 2H), 3.10 - 3.20 (m, 2H), 3.42 (t, *J*=5.80 Hz, 2H), 3.73 (t, *J*=5.95 Hz, 2H), 3.75 - 3.82 (m, 2H), 4.05 (s, 2H), 7.42 - 7.46 (m, 2H), 7.70 - 7.74 (m, 2H).

### EXAMPLE 53

### 4-(3-((2-methylpyrrolidin-1-yl)carbonyl)benzyl)-5,6,7,8-tetrahydrophthalazin-1(2H)-one

The title compound was prepared as a trifluoroacetic acid salt according to procedure for EXAMPLE 51 substituting 2-methylpyrrolidine for 4-(2-aminoethyl)morpholine. MS (DCI/NH₃) m/z 352 (M+H)⁺; ¹H NMR (500 MHz, CD₃OD): δ 0.86 (d, *J*=6.41 Hz, 1H), 1.33 (d, *J*=6.41 Hz, 2H), 1.60 - 1.67 (m, 2H), 1.70 (d, *J*=2.75 Hz, 3H), 1.73 - 1.80 (m, 1H), 1.90 - 1.99 (m, 1H), 2.11 - 2.22 (m, 1H), 2.39 - 2.47 (m, 2H), 2.46 - 2.56 (m, 2H), 3.43 - 3.51 (m, 1H), 3.57 - 3.67 (m, 1H), 4.02 (s, 2H), 4.21 - 4.28 (m, 1H), 7.24 - 7.33 (m, 2H), 7.33 - 7.36 (m, 1H), 7.37 - 7.42 (m, 1H).

### EXAMPLE 54

### N-azepan-1-yl-3-((4-oxo-3,4,5,6,7,8-hexahydrophthalazin-1-yl)methyl)benzamide

The title compound was prepared as a trifluoroacetic acid salt according to procedure for EXAMPLE 51 substituting 1-aminohomopiperidine for 4-(2-aminoethyl)morpholine. MS (DCI/NH₃) m/z 381 (M+H)⁺; ¹H NMR (500 MHz, CD₃OD): δ 1.68 - 1.74 (m, 4H), 1.73-1.81 (m, 4H), 1.92 - 2.01 (m, 4H), 2.41 - 2.46 (m, 2H), 2.48 - 2.54 (m, 2H), 3.53 - 3.60 (m, 4H), 4.06 (s, 2H), 7.45 - 7.50 (m, 2H), 7.70 - 7.73 (m, 2H).

### EXAMPLE 55

### 4-(3-(piperazin-1-ylcarbonyl)benzyl)-5,6,7,8-tetrahydrophthalazin-1(2H)-one

### EXAMPLE 55A

### tert-butyl 4-(3-((4-oxo-3,4,5,6,7,8-hexahydrophthalazin-1-yl)methyl)benzoyl)piperazine-1-carboxylate

The title compound was prepared according to procedure for EXAMPLE 51 substituting tert-butyl 1-piperazine carboxylate for 4-(2-aminoethyl)morpholine. MS (DCI/NH₃) m/z 453 (M+H)⁺.

### EXAMPLE 55B

### 4-(3-(piperazin-1-ylcarbonyl)benzyl)-5,6,7,8-tetrahydrophthalazin-1(2H)-one

To a solution of EXAMPLE 55A (480 mg, 1.76 mmol) in methylene chloride (10 mL) was added trifluoroacetic acid (5 mL). The solution was stirred at room temperature for 1 hour, and was concentrated. The residue was purified by HPLC (Zorbax® C-18 ODS packing material [Agilent Technologies, Santa Clara, CA], 0.1 % trifluoroacetic acid/CH₃CN/H₂O) to provide the title compound as a trifluoroacetic acid salt. MS (DCI/NH₃) m/z 353 (M+H)⁺; ¹H NMR (500 MHz, CD₃OD): δ 1.68 - 1.74 (m, 4H), 2.43 - 2.48 (m, 2H), 2.48 - 2.54 (m, 2H), 3.19 - 3.29 (m, 3H), 3.67 - 3.97 (m, 5H), 4.04 (s, 2H), 7.30 - 7.33 (m, 1H), 7.33 - 7.36 (m, 1H), 7.36 - 7.39 (m, 1H), 7.44 (t, *J*=7.48 Hz, 1H).

### EXAMPLE 56

### N-azetidin-3-yl-3-((4-oxo-3,4,5,6,7,8-hexahydrophthalazin-1-yl)methyl)benzamide

The title compound was prepared as a trifluoroacetic acid salt according to procedure for EXAMPLE 55 substituting 3-amino-1-N-Boc-azetidine for tert-butyl 1-piperazine carboxylate. MS (DCI/NH₃) m/z 339 (M+H)⁺; ¹H NMR (500 MHz, CD₃OD): δ 1.63 - 1.76 (m, 4H), 2.37 - 2.45 (m, 2H), 2.46 - 2.55 (m, 2H), 4.05 (s, 2H), 4.28 - 4.37 (m, 4H), 4.76 - 4.82 (m, 1H), 7.41 - 7.45 (m, 2H), 7.69 - 7.74 (m, 2H).

### EXAMPLE 57

### 3-((4-oxo-3,4,5,6,7,8-hexahydrophthalazin-1-yl)methyl)-N-piperidin-3-ylbenzamide

The title compound was prepared as a trifluoroacetic acid salt according to procedure for EXAMPLE 55 substituting (+/-)-3-amino-1-N-Boc-piperidine for tert-butyl 1-piperazine carboxylate. MS (DCI/NH₃) m/z 367 (M+H)⁺; ¹H NMR (500 MHz, CD₃OD): δ 1.68 - 1.72 (m, 4H), 1.72 - 1.77 (m, 1H), 1.80 - 1.89 (m, 1H), 2.02 - 2.15 (m, 2H), 2.37 - 2.46 (m, 2H), 2.47 - 2.54 (m, 2H), 2.85 - 3.00 (m, 2H), 3.33 - 3.39 (m, 1H), 3.52 (dd, *J*=12.21, 4.27 Hz, 1H), 4.04 (s, 2H), 4.18 - 4.26 (m, 1H), 7.40 - 7.43 (m, 2H), 7.66 - 7.71 (m, 2H).

### EXAMPLE 58

### N-(4-(dimethylamino)phenyl)-3-((4-oxo-3,4,5,6,7,8-hexahydrophthalazin-1-yl)methyl)benzamide

The title compound was prepared as a trifluoroacetic acid salt according to procedure for EXAMPLE 51 substituting N,N-dimethyl-1,4-phenylenediamine for 4-(2-aminoethyl)morpholine. MS (DCI/NH₃) m/z 403 (M+H)⁺; ¹H NMR (500 MHz, CD₃OD): δ 1.67 - 1.73 (m, 4H), 2.42 - 2.48 (m, 2H), 2.47 - 2.55 (m, 2H), 3.28 (s, 6H), 4.08 (s, 2H), 7.43 - 7.45 (m, 1H), 7.45 - 7.49 (m, 1H), 7.55 (d, *J*=8.85 Hz, 2H), 7.77 - 7.80 (m, 1H), 7.79 - 7.82 (m, 1H), 7.89 - 7.93 (m, 2H).

### EXAMPLE 59

### N-(2-(4-methylpiperazin-1-yl)ethyl)-3-((4-oxo-3,4,5,6,7,8-hexahydrophthalazin-1-yl)methyl)benzamide

The title compound was prepared as a trifluoroacetic acid salt according to procedure for EXAMPLE 51 substituting 2-(4-methyl-piperazin-1-yl)-ethylamine for 4-(2-aminoethyl)morpholine. MS (DCI/NH₃) m/z 410 (M+H)⁺; ¹H NMR (500 MHz, CD₃OD): δ 1.67 - 1.73 (m, 4H), 2.41 - 2.46 (m, 2H), 2.48 - 2.54 (m, 2H), 2.82 (t, *J*=6.41 Hz, 2H), 2.87 (s, 3H), 2.89 - 3.09 (m, 3H), 3.17 - 3.26 (m, 2H), 3.33 - 3.41 (m, 1H), 3.57 (t, *J*=6.26 Hz, 2H), 4.04 (s, 2H), 4.72 - 4.83 (m, 2H), 7.39 - 7.44 (m, 2H), 7.63 - 7.66 (m, 1H), 7.66 - 7.69 (m, 1H).

### EXAMPLE 60

### 4-(3-((4-(isoxazol-5-ylcarbonyl)piperazin-1-yl)carbonyl)benzyl)-5,6,7,8-tetrahydrophthalazin-1 (2H)-one

A solution of isoxazole-5-carboxylic acid (32 mg, 0.28 mmol) in a mixture of anhydrous N,N-dimethylformamide (2 mL) and pyridine (2 mL) was treated with 1,1'-carbonyldiimidazole (48 mg, 0.30 mmol) at 40 °C for 2 hours. EXAMPLE 55 (50 mg, 0.14 mmol) was added and the reaction mixture was heated at 60 °C for 3 hours. After cooling, the reaction mixture was concentrated on a rotary evaporator and the residue was purified by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 448 (M+H)⁺; ¹H NMR (500 MHz, CD₃OD): δ 1.65 - 1.76 (m, 4H), 2.41 - 2.47 (m, 2H), 2.48 - 2.56 (m, 2H), 3.51 - 3.66 (m, 3H), 3.66 - 3.79 (m, 3H), 3.79 - 3.94 (m, 2H), 4.04 (s, 2H), 7.27 - 7.30 (m, 1H), 7.32 - 7.35 (m, 1H), 7.36 - 7.39 (m, 1H), 7.43 (t, *J*=7.48 Hz, 1H), 7.61 - 7.66 (m, 1H), 7.86 - 7.91 (m, 1H).

### EXAMPLE 61

### 4-(3-((4-phenylpiperidin-1-yl)carbonyl)benzyl)-5,6,7,8-tetrahydrophthalazin-1(2H)-one

The title compound was prepared as a trifluoroacetic acid salt according to procedure for EXAMPLE 51 substituting 4-phenylpiperidine for 4-(2-aminoethyl)morpholine. MS (DCI/NH₃) m/z 428 (M+H)⁺; ¹H NMR (500 MHz, CD₃OD): δ 1.54 - 1.65 (m, 1H), 1.64-1.71 (m, 4H), 1.72 - 1.82 (m, 2H), 1.91 - 1.99 (m, 1H), 2.40 - 2.45 (m, 2H), 2.49 - 2.51 (m, 2H), 2.80 - 2.89 (m, 1H), 2.89 - 2.98 (m, 1H), 3.15 - 3.26 (m, 1H), 3.71 - 3.82 (m, 1H), 4.04 (s, 2H), 4.72 - 4.81 (m, 1H), 7.16 - 7.20 (m, 1H), 7.22 - 7.26 (m, 3H), 7.27 - 7.30 (m, 2H), 7.30 - 7.32 (m, 1H), 7.34 (d, *J*=7.93 Hz, 1H), 7.42 (t, *J*=7.63 Hz, 1H).

### EXAMPLE 62

### 3-((4-oxo-3,4,5,6,7,8-hexahydrophthalazin-1-yl)methyl)-N-(piperidin-2-ylmethyl)benzamide

The title compound was prepared as a trifluoroacetic acid salt according to procedure for EXAMPLE 55 substituting tert-butyl 2-(aminomethyl)piperidine-1-carboxylate for tert-butyl 1-piperazine carboxylate. MS (DCI/NH₃) m/z 381 (M+H)⁺; ¹H NMR (500 MHz, CD₃OD): δ1.46 - 1.56 (m, 2H), 1.60 - 1.78 (m, 6H), 1.79 - 1.93 (m, 2H), 2.38 - 2.47 (m, 2H), 2.47 - 2.55 (m, 2H), 3.14 (dd, *J*=13.27, 4.42 Hz, 2H), 3.46 - 3.52 (m, 1H), 3.55 - 3.64 (m, 1H), 4.04 (s, 2H), 4.91 - 5.05 (m, 1H), 7.27 - 7.32 (m, 1H), 7.35 - 7.40 (m, 2H), 7.43 (t, *J*=7.48 Hz, 1H).

### EXAMPLE 63

### 3-((4-oxo-3,4,5,6,7,8-hexahydrophthalazin-1-yl)methyl)-N-(piperidin-4-ylmethyl)benzamide

The title compound was prepared as a trifluoroacetic acid salt according to procedure for EXAMPLE 55 substituting 4-aminomethyl-piperidine-1-carboxylic acid tert-butyl ester for tert-butyl 1-piperazine carboxylate. MS (DCI/NH₃) m/z 381 (M+H)⁺; ¹H NMR (500 MHz, CD₃OD): δ 1.40 - 1.52 (m, 2H), 1.64 - 1.74 (m, 4H), 1.91 - 2.03 (m, 3H), 2.37 - 2.47 (m, 2H), 2.47 - 2.55 (m, 2H), 2.93 - 3.02 (m, 2H), 3.32 - 3.36 (m, 2H), 3.37 - 3.44 (m, 2H), 4.04 (s, 2H), 7.38 - 7.43 (m, 2H), 7.65 - 7.69 (m, 2H).

### EXAMPLE 64

### 3-((4-oxo-3,4,5,6,7,8-hexahydrophthalazin-1-yl)methyl)-N-(2-piperidin-1-ylethyl)benzamide

The title compound was prepared as a trifluoroacetic acid salt according to procedure for EXAMPLE 51 substituting 2-(piperidin-1-yl)ethanamine for 4-(2-aminoethyl)morpholine. MS (DCI/NH₃) m/z 395 (M+H)⁺; ¹H NMR (500 MHz, CD₃OD): δ 1.50 - 1.58 (m, 1H), 1.67 - 1.73 (m, 4H), 1.76 - 1.88 (m, 3H), 1.97 (d, *J*=14.34 Hz, 2H), 2.38 - 2.47 (m, 2H), 2.46 - 2.54 (m, 2H), 2.93 - 3.04 (m, 2H), 3.32 - 3.37 (m, 2H), 3.68 (d, *J*=12.21 Hz, 2H), 3.74 (t, *J*=6.10 Hz, 2H), 4.05 (s, 2H), 7.42 - 7.45 (m, 2H), 7.70 - 7.74 (m, 2H).

### EXAMPLE 65

### N-(1-methylazetidin-3-yl)-3-((4-oxo-3,4,5,6,7,8-hexahydrophthalazin-1-yl)methyl)benzamide

To a solution of EXAMPLE 56 (25 mg, 0.07 mmol) in methanol (2 mL) was added formaldehyde (37% in water, 16 µL, 0.21 mmol) and triethylamine (10 µL, 0.07 mmol). The mixture was stirred at room temperature for 2 hours before sodium cyanoborohydride (13 mg, 0.21 mmol) and zinc chloride (10 mg) were added. The reaction mixture was stirred at room temperature for 16 hours, and concentrated. The residue was dissolved in 1:1 mixture of acetonitrile and water, and purified by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 353 (M+H)⁺; ¹H NMR (500 MHz, CD₃OD): δ 1.63 - 1.74 (m, 4H), 2.36 - 2.45 (m, 2H), 2.46 - 2.53 (m, 2H), 3.01 (d, *J*=17.70 Hz, 3H), 4.06 (d, *J*=10.68 Hz, 2H), 4.21 - 4.28 (m, 1H), 4.31 (dd, *J*=11.44, 8.70 Hz, 1H), 4.56 - 4.66 (m, 2H), 5.51 (s, 1H), 7.41 - 7.44 (m, 1H), 7.44 - 7.48 (m, 1H), 7.70 - 7.78 (m, 2H).

### EXAMPLE 66

### methyl 4-((4-oxo-3,4,5,6,7,8-hexahydrophthalazin-1-yl)methyl)pyridine-2-carboxylate

### EXAMPLE 66A

### 3-((2-bromopyridin-4-yl)methylene)-4,5,6,7-tetrahydroisobenzofuran-1(3H)-one

The title compound was prepared according to the procedure for EXAMPLE 1C, substituting 2-bromo-pyridine-4-carbaldehyde for 2-fluoro-5-formylbenzonitrile. MS (DCI/NH₃) m/z 307 (M+H)⁺.

### EXAMPLE 66B

### 4-((2-bromopyridin-4-yl)methyl)-5,6,7,8-tetrahydrophthalazin-1(2H)-one

The title compound was prepared according to the procedure for EXAMPLE 2C substituting EXAMPLE 66A for EXAMPLE 2B. MS (DCI/NH₃) m/z 321 (M+H)⁺.

### EXAMPLE 66C

### methyl 4-((4-oxo-3,4,5,6,7,8-hexahydrophthalazin-1-yl)methyl)pyridine-2-carboxylate

A mixture of EXAMPLE 66B (800 mg, 2.5 mmol), dichloro(1,1'-ferrocenylbis(diphenyl-phosphine))palladium(II) dichloromethane(125 mg, 0.15 mmol) and triethylamine (1 ml) in a mixture of methanol (40 ml) and N,N-dimethylformamide (16 ml) was heated at 110 °C in a pressure vessel under 30 psi of carbon monoxide for 16 hours. After cooling, the solid material was filtered off, and the filtrate was concentrated. The residual solid was washed with methanol, and dried to provide the title compound. MS (DCI/NH₃) m/z 300 (M+H)⁺.

### EXAMPLE 67

### N-methyl-4-((4-oxo-3,4,5,6,7,8-hexahydrophthalazin-1-yl)methyl)pyridine-2-carboxamide

A solution of EXAMPLE 66 (100 mg, 0.33 mmol) in methanol (5 ml) was treated with methylamine (2.0 N in methanol, 2 ml) at 50 °C for 24 hours, and concentrated. The residue was washed with methanol, and dried to provide the title compound. MS (DCI/NH₃) m/z 299 (M+H)⁺.

### EXAMPLE 68

### 4-((2-(methylthio)pyrimidin-4-yl)methyl)-5,6,7,8-tetrahydrophthalazin-1(2H)-one

### EXAMPLE 68A

### 3-((2-(methylthio)pyrimidin-4-yl)methylene)-4,5,6,7-tetrahydroisobenzofuran-1(3H)-one

The title compound was prepared according to the procedure for EXAMPLE 1C, substituting 2-methylthio-4-pyrimidine-carboxyaldehyde for 2-fluoro-5-formylbenzonitrile. MS (DCI/NH₃) m/z 275 (M+H)⁺.

### EXAMPLE 68B

### 4-((2-(methylthio)pyrimidin-4-yl)methyl)-5,6,7,8-tetrahydrophthalazin-1(2H)-one

The title compound was prepared according to the procedure for EXAMPLE 2C, substituting EXAMPLE 68A for EXAMPLE 2B. MS (DCI/NH₃) m/z 289 (M+H)⁺.

### EXAMPLE 69

### 4-((2-(methylsulfonyl)pyrimidin-4-yl)methyl)-5,6,7,8-tetrahydrophthalazin-1(2H)-one

To a suspension of EXAMPLE 68 (280 mg, 1 mmol) in methylene chloride (5 mL) was added m-chloroperoxybenzoic acid (256 mg, 1.5 mmol). The reaction mixture was stirred at room temperature for 4 hours, and concentrated. The residual solid was separated by flash chromatography on silica gel (80% ethyl acetate in hexane) to provide the title compound. MS (DCI/NH₃) m/z 321 (M+H)⁺; H NMR (300 MHz, CD₃OD): δ 1.61 - 1.89 (m, 4 H), 2.37 - 2.71 (m, 4 H), 3.32 (s, 3H), 4.29 (s, 2H), 7.65 (d, *J*=5.09 Hz, 1 H), 8.88 (d, *J*=5.43 Hz, 1 H).

### EXAMPLE 70

### 4-((2-(methylsulfinyl)pyrimidin-4-yl)methyl)-5,6,7,8-tetrahydrophthalazin-1(2H)-one

The title compound was isolated as a side product in EXAMPLE 69. MS (ESI) m/z 305 (M+H)⁺.

### EXAMPLE 71

### 4-((3-bromopyridin-4-yl)methyl)-5,6,7,8-tetrahydrophthalazin-1(2H)-one

### EXAMPLE 71A

### 3-((3-bromopyridin-4-yl)methylene)-4,5,6,7-tetrahydroisobenzofuran-1(3H)-one

The title compound was prepared according to the procedure for EXAMPLE 1C, substituting 3-bromoisonicotinaldehyde for 2-fluoro-5-formylbenzonitrile. MS (DCI/NH₃) m/z 306 (M+H)⁺.

### EXAMPLE 71B

### 4-((3-bromopyridin-4-yl)methyl)-5,6,7,8-tetrahydrophthalazin-1(2H)-one

The title compound was prepared according to the procedure for EXAMPLE 2C, substituting EXAMPLE 71A for EXAMPLE 2B. MS (DCI/NH₃) m/z 321 (M+H)⁺.

### EXAMPLE 72

### 4-((6-bromopyridin-3-yl)methyl)-5,6,7,8-tetrahydrophthalazin-1(2H)-one

### EXAMPLE 72A

### 3-((6-bromopyridin-3-yl)methylene)-4,5,6,7-tetrahydroisobenzofuran-1(3H)-one

The title compound was prepared according to the procedure for EXAMPLE 1C, substituting 6-bromonicotinaldehyde for 2-fluoro-5-formylbenzonitrile. MS (DCI/NH₃) m/z 306 (M+H)⁺.

### EXAMPLE 72B

### 4-((6-bromopyridin-3-yl)methyl)-5,6,7,8-tetrahydrophthalazin-1(2H)-one

The title compound was prepared according to the procedure for EXAMPLE 2C, substituting EXAMPLE 72A for EXAMPLE 2B. MS (DCI/NH₃) m/z 321 (M+H)⁺.

### EXAMPLE 73

### 4-((2-bromopyridin-3-yl)methyl)-5,6,7,8-tetrahydrophthalazin-1(2H)-one

### EXAMPLE 73A

### 3-((2-bromopyridin-3-yl)methylene)-4,5,6,7-tetrahydroisobenzofuran-1(3H)-one

The title compound was prepared according to the procedure for EXAMPLE 1C, substituting 2-bromonicotinaldehyde for 2-fluoro-5-formylbenzonitrile. MS (DCI/NH₃) m/z 306 (M+H)⁺.

### EXAMPLE 73B

### 4-((2-bromopyridin-3-yl)methyl)-5,6,7,8-tetrahydrophthalazin-1(2H)-one

The title compound was prepared according to the procedure for EXAMPLE 2C, substituting EXAMPLE 72A for EXAMPLE 2B. MS (DCI/NH₃) m/z 321 (M+H)⁺.

### EXAMPLE 74

### methyl 6-((4-oxo-3,4,5,6,7,8-hexahydrophthalazin-1-yl)methyl)pyridine-2-carboxylate

### EXAMPLE 74A

### 3-((6-bromopyridin-2-yl)methylene)-4,5,6,7-tetrahydroisobenzofuran-1(3H)-one

The title compound was prepared according to the procedure for EXAMPLE 1C, substituting 6-bromo-pyridine-2-carbaldehyde for 2-fluoro-5-formylbenzonitrile. MS (DCI/NH₃) m/z 300 (M+H)⁺.

### EXAMPLE 74B

### 4-((6-bromopyridin-2-yl)methyl)-5,6,7,8-tetrahydrophthalazin-1(2H)-one

The title compound was prepared according to the procedure for EXAMPLE 2C, substituting EXAMPLE 74A for EXAMPLE 2B. MS (DCI/NH₃) m/z 321 (M+H)⁺.

### EXAMPLE 74C

### methyl 6-((4-oxo-3,4,5,6,7,8-hexahydrophthalazin-1-yl)methyl)picolinate

The title compound was prepared according to the procedure for EXAMPLE 66C, substituting EXAMPLE 74B for EXAMPLE 66B. MS (DCI/NH₃) m/z 300 (M+H)⁺.

### EXAMPLE 75

### N-ethyl-4-((4-oxo-3,4,5,6,7,8-hexahydrophthalazin-1-yl)methyl)pyridine-2-carboxamide

The title compound was prepared according to procedure for EXAMPLE 67, substituting ethylamine for methylamine. MS (ESI) m/z 313 (M+H)⁺.

### EXAMPLE 76

### N-isopropyl-4-((4-oxo-3,4,5,6,7,8-hexahydrophthalazin-1-yl)methyl)pyridine-2-carboxamide

The title compound was prepared according to procedure for EXAMPLE 67, substituting isopropyl amine for methyl amine. MS (ESI) m/z 327 (M+H)⁺.

### EXAMPLE 77

### N-cyclohexyl-4-((4-oxo-3,4,5,6,7,8-hexahydrophthalazin-1-yl)methyl)pyridine-2-carboxamide

The title compound was prepared according to procedure for EXAMPLE 67, substituting cyclohexanamine for methyl amine. MS (ESI) m/z 367 (M+H)⁺; ¹H NMR (300 MHz, dimethylsulfoxide-*d₆*): δ 1.23 - 1.45 (m, 6 H), 1.60 - 1.64 (m, 4 H), 1.65 - 1.86 (m, 5 H), 2.30 - 2.40 (m, 4 H), 4.03 (s, 2 H), 7.41 (dd, *J*=4.92, 1.86 Hz, 1 H), 7.86 (s, 1 H), 8.41 (d, *J*=8.82 Hz, 1 H), 8.53 (d, *J*=5.09 Hz, 1 H), 12.64 (s, 1 H).

### EXAMPLE 78

### N-methyl-N-((1-methylpiperidin-2-yl)methyl)-3-((4-oxo-3,4,5,6,7,8-hexahydrophthalazin-1-yl)methyl)benzamide

The title compound was prepared as a trifluoroacetic acid salt according to procedure for EXAMPLE 65, substituting EXAMPLE 62 for EXAMPLE 56. MS (DCI/NH₃) m/z 409 (M+H)⁺; ¹H NMR (500 MHz, CD₃OD): δ 1.47 - 1.57 (m, 1H), 1.58 - 1.67 (m, 1H), 1.67-1.77 (m, 6H), 1.77 - 1.87 (m, 1H), 1.85 - 1.95 (m, 1H), 2.43 - 2.51 (m, 2H), 2.53 - 2.63 (m, 2H), 2.99 (s, 3H), 3.08 (s, 3H), 3.26 (dd, *J*=13.73, 3.36 Hz, 2H), 3.51 - 3.61 (m, 1H), 3.95 (dd, *J*=13.27, 11.44 Hz, 1H), 4.13 (s, 2H), 5.11 - 5.24 (m, 1H), 7.35 - 7.40 (m, 2H), 7.41 - 7.47 (m, 2H).

### EXAMPLE 79

### N-((1-methylpiperidin-4-yl)methyl)-3-((4-oxo-3,4,5,6,7,8-hexahydrophthalazin-1-yl)methyl)benzamide

The title compound was prepared as a trifluoroacetic acid salt according to procedure for EXAMPLE 65, substituting EXAMPLE 63 for EXAMPLE 56. MS (DCI/NH₃) m/z 395 (M+H)⁺; ¹H NMR (500 MHz, CD₃OD): δ 1.49 - 1.61 (m, 2H), 1.69 - 1.78 (m, 4H), 1.90-1.98 (m, 1H), 1.97 - 2.08 (m, 2H), 2.43 - 2.53 (m, 2H), 2.54 - 2.65 (m, 2H), 2.85 (s, 3H), 2.95 - 3.04 (m, 2H), 3.32 - 3.38 (m, 2H), 3.53 (dd, *J*=10.53, 1.98 Hz, 2H), 4.15 (s, 2H), 7.39 - 7.41 (m, 1H), 7.43 (t, *J*=7.63 Hz, 1H), 7.68 (s, 1H), 7.70 - 7.73 (m, 1H).

### EXAMPLE 80

### N-methyl-6-((4-oxo-3,4,5,6,7,8-hexahydrophthalazin-1-yl)methyl)pyridine-2-carboxamide

The title compound was prepared according to procedure for EXAMPLE 67, substituting EXAMPLE 74 for EXAMPLE 66. MS (ESI) m/z 299 (M+H)⁺; ¹H NMR (300 MHz, CD₃OD): δ 1.61 - 1.81 (m, 4 H), 2.38 - 2.61 (m, 4 H), 2.95 (s, 3 H), 4.22 (s, 2 H), 7.41 (dd, *J*=7.46, 1.36 Hz, 1 H), 7.88 (t, *J*=7.63 Hz, 1 H), 7.91 - 7.98 (m, 1 H).

### EXAMPLE 81

### N-ethyl-6-((4-oxo-3,4,5,6,7,8-hexahydrophthalazin-1-yl)methyl)pyridine-2-carboxamide

The title compound was prepared according to procedure for EXAMPLE 67, substituting EXAMPLE 74 for EXAMPLE 66, and ethylamine for methylamine. MS (ESI) m/z 313 (M+H)⁺.

### EXAMPLE 82

### N-isopropyl-6-((4-oxo-3,4,5,6,7,8-hexahydrophthalazin-1-yl)methyl)pyridine-2-carboxamide

The title compound was prepared according to procedure for EXAMPLE 67, substituting EXAMPLE 74 for EXAMPLE 66, and isopropylamine for methylamine. MS (ESI) m/z 327 (M+H)⁺.

### EXAMPLE 83

### N-cyclopropyl-6-((4-oxo-3,4,5,6,7,8-hexahydrophthalazin-1-yl)methyl)pyridine-2-carboxamide

The title compound was prepared according to procedure for EXAMPLE 67, substituting EXAMPLE 74 for EXAMPLE 66, and cyclopropylamine for methylamine. MS (ESI) m/z 325 (M+H)⁺.

### EXAMPLE 84

### N-cyclohexyl-6-((4-oxo-3,4,5,6,7,8-hexahydrophthalazin-1-yl)methyl)pyridine-2-carboxamide

The title compound was prepared according to procedure for EXAMPLE 67, substituting EXAMPLE 74 for EXAMPLE 66, and cyclohexylamine for methylamine. MS (ESI) m/z 367 (M+H)⁺.

### EXAMPLE 85

### methyl 3-((4-oxo-3,4,5,6,7,8-hexahydrophthalazin-1-yl)methyl)pyridine-2-carboxylate

The title compound was prepared according to procedure for EXAMPLE 66C, substituting EXAMPLE 73B for EXAMPLE 66B. MS (DCI/NH₃) ml) 300 (M+H)⁺.

### EXAMPLE 86

### methyl 5-((4-oxo-3,4,5,6,7,8-hexahydrophthalazin-1-yl)methyl)pyridine-2-carboxylate

The title compound was prepared according to the procedure for EXAMPLE 66C, substituting EXAMPLE 72B for EXAMPLE 66B. MS (DCI/NH₃) m/z 300 (M+H)⁺.

### EXAMPLE 87

### 4-((5-bromothiophen-2-yl)methyl)-5,6,7,8-tetrahydrophthalazin-1(2H)-one

### EXAMPLE 87A

### 3-((5-bromothiophen-2-yl)methylene)-4,5,6,7-tetrahydroisobenzofuran-1(3H)-one

The title compound was prepared according to procedure for EXAMPLE 1C, substituting 5-bromothiophene-2-carbaldehyde for 2-fluoro-5-formylbenzonitrile. MS (DCI/NH₃) m/z 312 (M+H)⁺.

### EXAMPLE 87B

### 4-((5-bromothiophen-2-yl)methyl)-5,6,7,8-tetrahydrophthalazin-1(2H)-one

The title compound was prepared according to the procedure for EXAMPLE 2C, substituting EXAMPLE 87A for EXAMPLE 2B. MS (DCI/NH₃) m/z 326 (M+H)⁺.

### EXAMPLE 88

### 4-((3-bromothiophen-2-yl)methyl)-5,6,7,8-tetrahydrophthalazin-1(2H)-one

### EXAMPLE 88A

### 3-((3-bromothiophen-2-yl)methylene)-4,5,6,7-tetrahydroisobenzofuran-1(3H)-one

The title compound was prepared according to procedure for EXAMPLE 1C, substituting 3-bromothiophene-2-carbaldehyde for 2-fluoro-5-formylbenzonitrile. MS (DCI/NH₃) m/z 312 (M+H)⁺.

### EXAMPLE 88B

### 4-((3-bromothiophen-2-yl)methyl)-5,6,7,8-tetrahydrophthalazin-1(2H)-one

The title compound was prepared according to the procedure for EXAMPLE 2C substituting EXAMPLE 88A for EXAMPLE 2B. MS (DCI/NH₃) m/z 326 (M+H)⁺.

### EXAMPLE 89

### 4-(3-aminobenzyl)-5,6,7,8-tetrahydrophthalazin-1(2H)-one

The title compound was prepared according to the procedure for EXAMPLE 2, substituting 3-nitrobenzaldehyde for 4-fluoro-3-nitrobenzaldehyde in EXAMPLE 2A. MS (DCI/NH₃) m/z 256 (M+H)⁺; ¹H NMR (300 MHz, CD₃OD): δ 1.62 - 1.75 (m, 4H), 2.37 - 2.44 (m, 2H), 2.46 - 2.54 (m, 2H), 3.86 (s, 2H), 6.46 - 6.54 (m, 2H), 6.57 (dd, *J*=7.93, 1.98 Hz, 1H), 7.01 (t, *J*=7.73 Hz, 1H).

### EXAMPLE 90

### 4-(3-bromobenzyl)-5,6,7,8-tetrahydrophthalazin-1(2H)-one

The title compound was prepared according to the procedure for EXAMPLE 2C, substituting 3-bromobenzaldehyde for 4-fluoro-3-nitrobenzaldehyde. MS (DCI/NH₃) m/z 256 (M+H)⁺; ¹H NMR (300 MHz, CD₃OD): δ 1.64 - 1.77 (m, 4H), 2.37 - 2.46 (m, 2H), 2.47 - 2.55 (m, 2H), 3.96 (s, 2H), 7.13 - 7.18 (m, 1H), 7.18 - 7.24 (m, 1H), 7.35 - 7.40 (m, 2H).

### EXAMPLE 91

### 4-(thien-2-ylmethyl)-5,6,7,8-tetrahydrophthalazin-1(2H)-one

A mixture of EXAMPLE 87 (100 mg, 0.31 mmol) and acetamide (1 g) was stirred at 180 °C overnight. After cooling, the mixture was dissolved in methanol, and separated by HPLC (Zorbax® C-8 packing material [Agilent Technologies, Santa Clara, CA]0.1% trifluoroacetic acid/CH₃CN/H₂O) to provide the title compound. MS (DCI/NH₃) m/z 247 (M+H)⁺.

### EXAMPLE 92

### methyl 5-((4-oxo-3,4,5,6,7,8-hexahydrophthalazin-1-yl)methyl)thiophene-2-carboxylate

The title compound was prepared according to the procedure for EXAMPLE 66C, substituting EXAMPLE 87 for EXAMPLE 66B. MS (DCI/NH₃) m/z 305 (M+H)⁺.

### EXAMPLE 93

### N-methyl-5-((4-oxo-3,4,5,6,7,8-hexahydrophthalazin-1-yl)methyl)pyridine-2-carboxamide

The title compound was prepared according to procedure for EXAMPLE 67, substituting EXAMPLE 86 for 66. MS (ESI) m/z 299 (M+H)⁺, ¹H NMR (300 MHz, dimethylsulfoxide-*d₆*): δ 1.51 - 1.73 (m, 4 H), 2.40 (d, *J*=14.92 Hz, 4 H), 2.81 (d, *J*=5.09 Hz, 3 H), 4.02 (s, 2 H), 7.75 (dd, *J*=7.97, 2.20 Hz, 1 H), 7.96 (d, *J*=8.14 Hz, 1 H), 8.49 (d, *J*=1.70 Hz, 1 H), 8.70 (d, *J*=4.75 Hz, 1 H), 12.60 (s, 1 H).

### EXAMPLE 94

### N-ethyl-5-((4-oxo-3,4,5,6,7,8-hexahydrophthalazin-1-yl)methyl)pyridine-2-carboxamide

The title compound was prepared according to procedure for EXAMPLE 67, substituting EXAMPLE 86 for EXAMPLE 66, and ethylamine for methylamine. MS (ESI) m/z 313 (M+H)⁺.

### EXAMPLE 95

### N-methyl-3-((4-oxo-3,4,5,6,7,8-hexahydrophthalazin-1-yl)methyl)pyridine-2-carboxamide

The title compound was prepared according to procedure for EXAMPLE 67, substituting EXAMPLE 85 for 66. MS (ESI) m/z 299 (M+H)⁺, ¹H NMR (300 MHz, dimethylsulfoxide-*d₆*) δ 1.53 - 1.79 (m, 4 H), 2.29 - 2.44 (m, 4 H), 2.73 (d, *J*=5.16 Hz, 3 H), 4.35 (s, 2 H), 7.50 (dd, *J*=7.73, 4.56 Hz, 1 H), 7.66 (dd, *J*=7.93, 1.59 Hz, 1 H), 8.49 (dd, *J*=4.36, 1.59 Hz, 1 H), 8.65 (d, *J*=5.16 Hz, 1 H), 12.35 (s, 1 H).

### EXAMPLE 96

### N-ethyl-3-((4-oxo-3,4,5,6,7,8-hexahydrophthalazin-1-yl)methyl)pyridine-2-carboxamide

The title compound was prepared according to procedure for EXAMPLE 67, substituting EXAMPLE 85 for EXAMPLE 66, and ethylamine for methylamine. MS (ESI) m/z 313 (M+H)⁺.

### EXAMPLE 97

### N,N-dimethyl-N'-(3-((4-oxo-3,4,5,6,7,8-hexahydrophthalazin-1-yl)methyl)phenyl)sulfamide

To a solution of EXAMPLE 89 (50 mg, 02 mmol) in dichloromethane (4 mL) was added dimethylsulfamoyl chloride (31 mg, 0.22 mmol) and pyridine (17 mL, 0.22 mol). The solution was stirred at room temperature for 16 hours, and was concentrated. The residue was separated by HPLC (Zorbax® C-18 ODS packing material [Agilcnt Technologies, Santa Clara, CA], 0.1% trifluoroacetic acid/CH₃CN/H₂O) to provide the title compound as a trifluoroacetic acid salt. MS (DCT/NH₃) m/z 363 (M+H)⁺; ¹H NMR (400 MHz, CD₃OD): δ 1.64 - 1.76 (m, 4H), 2.37 - 2.46 (m, 2H), 2.47 - 2.54 (m, 2H), 2.72 (s, 6H), 3.95 (s, 2H), 6.91 - 6.96 (m, 1H), 7.02 - 7.06 (m, 2H), 7.19 - 7.24 (m, 1H).

### EXAMPLE 98

### N-(3-((4-oxo-3,4,5,6,7,8-hexahydrophthalazin-1-yl)methyl)phenyl)-3-piperidin-1-ylpropanamide

To a solution of 3-(piperidin-1-yl)propanoic acid (31 mg) in anhydrous dichloromethane (2 mL) was added oxalyl chloride (25.7 µL) and a drop of N,N-dimethylformamide. The solution was stirred for 1 hour, and was concentrated. The residue was re-dissolved in anhydrous dichloromethane (2 mL), and was quickly added to a solution of EXAMPLE 89 (50 mg) in anhydrous tetrahydrofuran (2 mL). Triethylamine (32.8 µL) was added, and the reaction mixture was stirred at room temperature overnight. The mixture was concentrated. The residue was partitioned between ethyl acetate and brine. The organic phase was washed with brine and concentrated. The residual solid was separated on HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 395 (M+H)⁺; ¹H NMR (400 MHz, CD₃OD): δ 1.48 - 1.60 (m, 1H), 1.65 - 1.71 (m, 4H), 1.73 - 1.87 (m, 3H), 1.92 - 2.01 (m, 2H), 2.38 - 2.45 (m, 2H), 2.46 - 2.53 (m, 2H), 2.87 (t, *J*=6.60 Hz, 2H), 2.93 - 3.03 (m, 2H), 3.44 (t, *J*=6.75 Hz, 2H), 3.57 (d, *J*=12.58 Hz, 2H), 3.97 (s, 2H), 6.95 - 7.00 (m, 1H), 7.26 (t, *J*=7.83 Hz, 1H), 7.36 - 7.39 (m, 1H), 7.41 - 7.48 (m, 1H).

### EXAMPLE 99

### 4-chloro-N-(3-((4-oxo-3,4,5,6,7,8-hexahydrophthalazin-1-yl)methyl)phenyl)butanamide

A solution of EXAMPLE 89 (150 mg, 0.59 mmol) and 4-chlorobutanoyl chloride (83 mg, 0.59 mmol) in dichloromethane (5 mL) was stirred at room temperature for 16 hours, and was concentrated. The residue was partitioned between ethyl acetate and brine. The organic phase was washed with brine, and concentrated to provide the title compound. MS (DCI/NH₃) m/z 360 (M+H)⁺; ¹H NMR (400 MHz, CD₃OD): δ 1.66 - 1.73 (m, 4H), 2.07 - 2.15 (m, 2H), 2.40 - 2.46 (m, 2H), 2.48 - 2.51 (m, 2H), 2.50 - 2.56 (m, 2H), 3.63 (t, *J*=6.44 Hz, 2H), 3.96 (s, 2H), 6.93 (d, *J*=7.67 Hz, 1H), 7.21 - 7.26 (m, 1H), 7.36 (s, 1H), 7.38 - 7.46 (m, 1H).

### EXAMPLE 100

### 4-(3-(2-oxopyrrolidin-1-yl)benzyl)-5,6,7,8-tetrahydrophthalazin-1(2H)-one

A suspension of EXAMPLE 90 (150 mg, 0.47 mmol), pyrrolidine-2-one (80 mg, 0.94 mmol), tris(dibenzylideneacetone)dipalladium(0) (43 mg, 0.05 mmol), Xantphos (4,5-bis(diphenylphosphino)-9,9-dimethylxanthene) (4,5-bis(diphenylphosphino)-9,9-dimethylxanthene) (41 mg, 0.07 mmol) and cesium carbonate (214 mg, 0.66 mmol) in anhydrous dioxane (2 mL) was heated in a CEM Explorer® microwave reactor (Matthews, NC) at 200 °C for 30 minutes. After cooling, the reaction mixture was concentrated. The residue was separated by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 324 (M+H)⁺; ¹H NMR (400 MHz, CD₃OD): δ 1.65 - 1.75 (m, 4H), 2.11 - 2.23 (m, 2H), 2.41 - 2.47 (m, 2H), 2.48 - 2.53 (m, 2H), 2.57 (t, *J*=7.98 Hz, 2H), 3.83 - 3.92 (m, 2H), 3.99 (s, 2H), 7.01 (d, *J*=7.67 Hz, 1H), 7.31 (t, *J*=7.98 Hz, 1H), 7.38 - 7.42 (m, 1H), 7.51 (t, *J*=1.69 Hz, 1H).

### EXAMPLE 101

### 4-((2-(2-oxoazetidin-1-yl)pyridin-4-yl)methyl)-5,6,7,8-tetrahydrophthalazin-1(2H)-one

A microwave tube was charged with tris(dibenzylideneacetone)dipalladium(0) (5.4 mg, 0.006 mmol), Xantphos (4,5-bis(diphenylphosphino)-9,9-dimethylxanthene) (5.4 mg, 0.01 mmol), EXAMPLE 103 (50 mg, 0.16 mmol), azetidin-2-one (53 mg, 0.62 mmol) and Cs₂CO₃ (70 mg, 0.21 mmol). Anhydrous dioxane was added, and the suspension was heated in a CEM Explorer® microwave reactor (Matthews, NC)at 200 °C for 30 minutes. After concentration, the residue was partitioned between ethyl acetate and brine. The organic phase was concentrated. The residual solid was separated by flash chromatography on silica gel (100% ethyl acetate) to provide the title compound. MS (DCI/NH₃) m/z 311 (M+H)⁺.

### EXAMPLE 102

### 4-((2-(2-oxopyrrolidin-1-yl)pyridin-4-yl)mcthyl)-5,6,7,8-tetrahydrophthalazin-1 (2H)-one

The title compound was prepared according to procedure for EXAMPLE 101, substituting pyrroline-2-one for azetidin-2-one. MS (ESI) m/z 339 (M+H)⁺.

### EXAMPLE 103

### 4-((2-bromopyridin-4-yl)methyl)-5,6,7,8-tetrahydrophthalazin-1(2H)-one

The title compound was prepared as described in EXAMPLE 66B. MS (DCI/NH₃) m/z 321 (M+H)⁺.

### EXAMPLE 104

### 4-((6-(2-oxopyrrolidin-1-yl)pyridin-3-yl)methyl)-5,6,7,8-tetrahydrophthalazin-1(2H)-one

The title compound was prepared according to procedure for EXAMPLE 101, substituting EXAMPLE 72 for EXAMPLE 103, and pyrroline -2-one for azetidin-2-one. MS (ESI) m/z 325 (M+H)⁺.

### EXAMPLE 105

### 4-((6-(2-oxoazetidin-1-yl)pyridin-3-yl)methyl)-5,6,7,8-tetrahydrophthalazin-1(2H)-one

The title compound was prepared according to procedure for EXAMPLE 101, substituting EXAMPLE 72 for EXAMPLE 103. MS (ESI) m/z 311 (M+H)⁺.

### EXAMPLE 106

### N-(5-((4-oxo-3,4,5,6,7,8-hexahydrophthalazin-1-yl)methyl)pyridin-2-yl)benzamide

The title compound was prepared according to procedure for EXAMPLE 101, substituting EXAMPLE 72 for EXAMPLE 103, and benzamide for azetidin-2-one. MS (ESI) m/z 361 (M+H)⁺; ¹H NMR (300 MHz, dimethylsulfoxide-*d₆*): δ 1.48 - 1.70 (m, 4 H), 2.41 (d, *J*=17.29 Hz, 4 H), 3.92 (s, 2 H), 7.86 (t, *J*=1.86 Hz, 3 H), 7.86 - 7.90 (m, 2 H), 7.99 - 8.06 (m, 1 H), 8.12 (d, *J*=8.48 Hz, 1 H), 8.24 (d, *J*=2.37 Hz, 1 H), 10.72 (s, 1 H) 12.60 (s, 1 H).

### EXAMPLE 107

### N-(5-((4-oxo-3,4,5,6,7,8-hexahydrophthalazin-1-yl)methyl)pyridin-2-yl)isonicotinamide

The title compound was prepared according to procedure for EXAMPLE 101, substituting EXAMPLE 72 for EXAMPLE 103, and isonicotinamide for azetidin-2-one. MS (ESI) m/z 362 (M+H)⁺; ¹H NMR (300 MHz, dimethylsulfoxide-*d₆*): δ 1.65 (d, *J*=5.09 Hz, 4 H), 2.41 (d, *J*=16.28 Hz, 4 H), 3.93 (s, 2 H), 7.68 (dd, *J*=8.48, 2.37 Hz, 1 H), 7.90 - 8.00 (m, 2 H), 8.11 (d, *J*=8.48 Hz, 1 H), 8.27 (d, *J*=2.03 Hz, 1 H), 8.76 - 8.82 (m, 2 H), 11.12 (s, 1 H), 12.60 (s, 1H).

### EXAMPLE 108

### N-(5-((4-oxo-3,4,5,6,7,8-hexahydrophthalazin-1-yl)methyl)pyridin-2-yl)nicotinamide

The title compound was prepared according to procedure for EXAMPLE 101, substituting EXAMPLE 72 for EXAMPLE 103, and nicotinamide for azetidin-2-one. MS (ESI) m/z 362 (M+H)⁺.

### EXAMPLE 109

### 4-((5'-((4-oxo-3,4,5,6,7,8-hexahydrophthalazin-1-yl)methyl)-2,2'-bipyridin-5-yl)methyl)-5,6,7,8-tetrahydrophthalazin-1 (2H)-one

The title compound was a side-product of EXAMPLE 108. MS (ESI) m/z 481 (M+H)⁺.

### EXAMPLE 110

### N-methyl-5-((4-oxo-3,4,5,6,7,8-hexahydrophthalazin-1-yl)methyl)thiophene-2-carboxamide

The title compound was prepared according to procedure for EXAMPLE 67, substituting EXAMPLE 92 for 66. MS (ESI) m/z 304 (M+H)⁺; ¹H NMR (300 MHz, dimethylsulfoxide-*d₆*): δ 1.63 (d, *J*=3.05 Hz, 4 H), 2.29 - 2.46 (m, 4 H), 2.72 (d, *J*=4.41 Hz, 3 H), 4.09 (s, 2 H), 6.88 (d, *J*=3.73 Hz, 1 H), 7.51 (d, *J*=3.73 Hz, 1 H), 8.31 (d, *J*=4.41 Hz, 1 H), 12.66 (s, 1 H).

### EXAMPLE 111

### N¹-(3-((4-oxo-3,4,5,6,7,8-hexahydrophthalazin-1-yl)methyl)phenyl)glycinamide

A solution of EXAMPLE 89 (50 mg, 0.2 mmol) and 2,5-dioxopyrrolidin-1-yl 2-(tert-butoxycarbonylamino)acetate 59 mg, 0.22 mmol) in anhydrous tetrahydrofuran (4 mL) was stirred at room temperature for 16 hours, and concentrated. The residual solid was dissolved in dichloromethane (4 mL) and treated with trifluoroacetic acid (2 mL) at room temperature for 1 hour. The reaction mixture was concentrated and the residue was separated by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 313 (M+H)⁺; ¹H NMR (300 MHz, CD₃OD): δ 1.64 - 1.75 (m, 4H), 2.36-2.45 (m, 2H), 2.46 - 2.54 (m, 2H), 3.80 (s, 2H), 3.98 (s, 2H), 7.00 (d, *J*=7.80 Hz, 1H), 7.28 (t, *J*=7.97 Hz, 1H), 7.37 - 7.40 (m, 1H), 7.42 - 7.47 (m, 1H).

### EXAMPLE 112

### N-(3-((4-oxo-3,4,5,6,7,8-hexahydrophthalazin-1-yl)methyl)phenyl)azetidine-2-carboxamide

### EXAMPLE 112A

### tert-butyl 2-(3-((4-oxo-3,4,5,6,7,8-hexahydrophthalazin-1-yl)methyl)phenylcarbamoyl)azetidine-1-carboxylate

The title compound was prepared according to procedure for EXAMPLE 98, substituting 1-(tert-butoxycarbonyl)azetidine-2-carboxylic acid for 3-(piperidin-1-yl)propanoic acid. MS (DCI/NH₃) m/z 439 (M+H)⁺.

### EXAMPLE 112B

### N-(3-((4-oxo-3,4,5,6,7,8-hexahydrophthalazin-1-yl)methyl)phenyl)azetidine-2-carboxamide

A solution of EXAMPLE 112A (64 mg) in dichloromethane (4 mL) was treated with trifluoroacetic acid (2 mL) at room temperature for 1 hour. The reaction mixture was concentrated and the residue was purified by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 339 (M+H)⁺; ¹H NMR (300 MHz, CD₃OD): δ 1.64 - 1.73 (m, 4H), 2.36 - 2.44 (m, 2H), 2.46 - 2.53 (m, 2H), 2.57-2.69 (m, 1H), 2.81 - 2.93 (m, 1H), 3.94 - 4.04 (m, 1H), 3.98 (s, 2H), 4.08 - 4.20 (m, 1H), 5.07 (dd, *J*=9.49, 7.80 Hz, 1 H), 7.03 (d, *J*=8.14 Hz, 1 H), 7.30 (t, *J*=7.80 Hz, 1 H), 7.41 (t, *J*=1.70 Hz, 1H), 7.49 (dd, *J*=7.97, 1.53 Hz, 1H).

### EXAMPLE 113

### N-(3-((4-oxo-3,4,5,6,7,8-hexahydrophthalazin-1-yl)methyl)phenyl)azetidine-3-carboxamide

The title compound was prepared according to procedure for EXAMPLE 112, substituting 1-(tert-butoxycarbonyl)azetidine-3-carboxylic acid for 1-(tert-butoxycarbonyl)azetidine-2-carboxylic acid. MS (DCI/NH₃) m/z 339 (M+H)⁺; ¹H NMR (300 MHz, CD₃OD): δ1.62 - 1.75 (m, 4H), 2.36 - 2.44 (m, 2H), 2.46 - 2.54 (m, 2H), 3.69 - 3.83 (m, 1H), 3.97 (s, 2H), 4.17 - 4.33 (m, 4H), 7.00 (dd, *J*=7.14, 1.19 Hz, 1H), 7.27 (t, *J*=7.93 Hz, 1H), 7.40 (t, *J*=1.59 Hz, 1H), 7.45 - 7.51 (m, 1H).

### EXAMPLE 114

### N-(3-((4-oxo-3,4,5,6,7,8-hexahydrophthalazin-1-yl)methyl)phenyl)methanesulfonamide

The title compound was prepared according to the procedure for EXAMPLE 97, substituting methanesulfonyl chloride for dimethylsulfamoyl chloride. MS (DCI/NH₃) m/z 334 (M+H)⁺; ¹H NMR (500 MHz, dimethylsulfoxide-*d₆*): δ 1.53 - 1.67 (m, 4H), 2.29 - 2.42 (m, 4H), 2.95 (s, 3H), 3.88 (s, 2H), 6.92 (d, *J*=7.63 Hz, 1H), 7.00 (s, 1H), 7.06 (dd, *J*=7.93, 1.22 Hz, 1H), 7.26 (t, *J*=7.78 Hz, 1H), 9.68 (br s, 1H), 12.63 (br s, 1H).

### EXAMPLE 115

### N-(3-((4-oxo-3,4,5,6,7,8-hexahydrophthalazin-1-yl)methyl)phenyl)propane-2-sulfonamide

The title compound was prepared according to the procedure for EXAMPLE 97, substituting propane-2-sulfonyl chloride for dimethylsulfamoyl chloride. MS (DCI/NH₃) m/z 362 (M+H)⁺; ¹H NMR (500 MHz, dimethylsulfoxide-*d₆*): δ 1.19 (d, *J*=7.02 Hz, 6H), 1.53 - 1.66 (m, 4H), 2.25 - 2.33 (m, 2H), 2.34 - 2.41 (m, 2H), 3.08 - 3.22 (m, 1H), 3.87 (s, 2H), 6.88 - 6.91 (m, 1H), 7.01 (s, 1H), 7.08 (dd, *J*=8.24, 1.22 Hz, 1H), 7.23 (t, *J*=7.78 Hz, 1H), 9.68 (br s, 1H), 12.64 (br s, 1H).

### EXAMPLE 116

### N-(3-((4-oxo-3,4,5,6,7,8-hexahydrophthalazin-1-yl)methyl)phenyl)benzenesulfonamide

The title compound was prepared according to the procedure for EXAMPLE 97, substituting benzenesulfonyl chloride for dimethylsulfamoyl chloride. MS (DCI/NH₃) m/z 396 (M+H)⁺; ¹H NMR (500 MHz, dimethylsulfoxide-*d₆*): δ 1.48 - 1.60 (m, 4H), 2.10 - 2.21 (m, 2H), 2.29 - 2.39 (m, 2H), 3.78 (s, 2H), 6.83 - 6.87 (m, 2H), 6.92 (dd, *J*=8.09, 1.37 Hz, 1H), 7.15 (t, *J*=7.78 Hz, 1H), 7.48 (t, *J*=7.78 Hz, 2H), 7.58 (t, *J*=7.48 Hz, 1H), 7.64 - 7.69 (m, 2H), 10.24 (br s, 1H), 12.64 (br s, 1H).

### EXAMPLE 117

### N-(3-((4-oxo-3,4,5,6,7,8-hexahydrophthalazin-1-yl)methyl)phenyl)pyridine-3-sulfonamide

The title compound was prepared according to the procedure for EXAMPLE 97, substituting pyridine-3-sulfonyl chloride hydrochloride for dimethylsulfamoyl chloride. MS (DCI/NH₃) m/z 397 (M+H)⁺; ¹H NMR (500 MHz, dimethylsulfoxide-*d₆*): δ 1.50 - 1.61 (m, 4H), 2.12 - 2.21 (m, 2H), 2.33 - 2.40 (m, 2H), 3.80 (s, 2H), 6.85 (s, 1H), 6.92 (d, *J*=7.63 Hz, 1H), 6.96 (dd, *J*=7.93, 1.22 Hz, 1H), 7.19 (t, *J*=7.78 Hz, 1H), 7.55 (dd, *J*=8.09, 4.73 Hz, 1H), 8.02 - 8.06 (m, 1H), 8.75 (dd, *J*=4.88, 1.53 Hz, 1H), 8.77 (d, *J*=1.83 Hz, 1H), 10.43 (br s, 1H), 12.63 (br s, 1H).

### EXAMPLE 118

### N-(3-((4-oxo-3,4,5,6,7,8-hexahydrophthalazin-1-yl)methyl)phenyl)furan-2-sulfonamide

The title compound was prepared according to the procedure for EXAMPLE 97, substituting furan-2-sulfonyl chloride for dimethylsulfamoyl chloride. MS (DCI/NH₃) m/z 386 (M+H)⁺; ¹H NMR (500 MHz, dimethylsulfoxide-*d₆*): δ 1.52 - 1.63 (m, 4H), 2.19 - 2.28 (m, 2H), 2.32 - 2.40 (m, 2H), 3.82 (s, 2H), 6.57 (dd, *J*=3.66,1.83 Hz, 1H), 6.89 (d, *J*=1.53 Hz, 1H), 6.91 (d, *J*=7.63 Hz, 1H), 6.95 - 6.99 (m, 1H), 7.04 (d, *J*=3.36 Hz, 1H), 7.20 (t, *J*=7.78 Hz, 1H), 7.90 (dd, *J*=1.83, 0.92 Hz, 1H), 10.60 (br s, 1H), 12.65 (br s, 1H).

### EXAMPLE 119

### 1-methyl-N-(3-((4-oxo-3,4,5,6,7,8-hexahydrophthalazin-1-yl)methyl)phenyl)-1H-imidazole-4-sulfonamide

The title compound was prepared according to the procedure for EXAMPLE 97, substituting 1-methyl-1H-imidazole-4-sulfonyl chloride for dimethylsulfamoyl chloride. MS (DCI/NH₃) m/z 400 (M+H)⁺. ¹H NMR (500 MHz, dimethylsulfoxide-*d₆*): δ 1.55 - 1.61 (m, 4H), 2.24 - 2.32 (m, 2H), 2.32 - 2.40 (m, 2H), 3.63 (s, 3H), 3.80 (s, 2H), 6.80 (d, *J*=7.93 Hz, 1H), 6.92 (s, 1H), 6.99 (dd, *J*=8.09, 1.37 Hz, 1H), 7.13 (t, *J*=7.78 Hz, 1H), 7.70 (d, *J*=1.22 Hz, 1H), 7.73 (d, *J*= 1.22 Hz, 1H), 10.15 (br s, 1H), 12.64 (br s, 1H).

### EXAMPLE 120

### N-(3-((4-oxo-3,4,5,6,7,8-hexahydrophthalazin-1-yl)methyl)phenyl)thiophene-2-sulfonamide

The title compound was prepared according to the procedure for EXAMPLE 97, substituting thiophene-2-sulfonyl chloride for dimethylsulfamoyl chloride. MS (DCI/NH₃) m/z 402 (M+H)⁺; ¹H NMR (500 MHz, dimethylsulfoxide-*d₆*): δ 1.41 - 1.64 (m, 4H), 2.18 - 2.24 (m, 2H), 2.34 - 2.40 (m, 2H), 3.82 (s, 2H), 6.90 - 6.94 (m, 2H), 6.97 (d, *J*=7.93 Hz, 1H), 7.06 (dd, *J*=5.03, 3.81 Hz, 1H), 7.17 - 7.24 (m, 1H), 7.45 (dd, *J*=3.81, 1.37 Hz, 1H), 7.85 (dd, *J*=5.03, 1.37 Hz, 1H), 10.36 (br s, 1H), 12.65 (br s, 1H).

### EXAMPLE 121

### 4-cyano-N-(3-((4-oxo-3,4,5,6,7,8-hexahydrophthalazin-1-yl)methyl)phenyl)benzenesulfonamide

The title compound was prepared according to the procedure for EXAMPLE 97, substituting 4-cyanobenzene-1-sulfonyl chloride for dimethylsulfamoyl chloride. MS (DCI/NH₃) m/z 421 (M+H)⁺; ¹H NMR (500 MHz, dimethylsulfoxide-*d₆*): δ 1.49 - 1.62 (m, 4H), 2.14 - 2.20 (m, 2H), 2.31 - 2.39 (m, 2H), 3.80 (s, 2H), 6.84 (s, 1H), 6.90 (d, *J*=7.63 Hz, 1H), 6.94 (dd, *J*=7.93, 1.22 Hz, 1H), 7.19 (t, *J*=7.93 Hz, 1H), 7.83 (d, *J*=8.85 Hz, 2H), 8.00 (d, *J*=8.54 Hz, 2H), 10.52 (br s, 1H), 12.65 (br s, 1H).

### EXAMPLE 122

### N-(3-((4-oxo-3,4,5,6,7,8-hexahydrophthalazin-1-yl)methyl)phenyl)naphthalene-1-sulfonamide

The title compound was prepared according to the procedure for EXAMPLE 97, substituting naphthalene-1-sulfonyl chloride for dimethylsulfamoyl chloride. MS (DCI/NH₃) m/z 421 (M+H)⁺; ¹H NMR (500 MHz, dimethylsulfoxide-*d₆*): δ 1.41 - 1.47 (m, 2H), 1.47-1.56 (m, 2H), 2.00 - 2.10 (m, 2H), 2.30 - 2.39 (m, 2H), 3.71 (s, 2H), 6.75 - 6.80 (m, 2H), 6.83 - 6.89 (m, 1H), 7.07 (t, *J*=7.78 Hz, 1H), 7.50 - 7.56 (m, 1H), 7.64 (t, *J*=7.02 Hz, 1H), 7.67 - 7.72 (m, 1H), 8.04 (d, *J*=7.63 Hz, 1H), 8.06 - 8.10 (m, 1H), 8.18 (d, *J*=8.24 Hz, 1 H), 8.67 (d, *J*=8.54 Hz, 1H), 10.65 (br s, 1 H), 12.64 (br s, 1H).

### EXAMPLE 123

### 4-((6-bromopyridin-2-yl)methyl)-5,6,7,8-tetrahydrophthalazin-1(2H)-one

The title compound was prepared as described in EXAMPLE 74B. MS (DCI/NH₃) m/z 321 (M+H)⁺.

### EXAMPLE 125

### 4-((6-(2-oxopyrrolidin-1-yl)pyridin-2-yl)methyl)-5,6,7,8-tetrahydrophthalazin-1(2H)-one

The title compound was prepared according to procedure for EXAMPLE 101, substituting EXAMPLE 123 for EXAMPLE 103, and pyrroline -2-one for azetidin-2-one. MS (ESI) m/z 325 (M+H)⁺.

### EXAMPLE 126

### N-(6-((4-oxo-3,4,5,6,7,8-hexahydrophthalazin-1-yl)methyl)pyridin-2-yl)benzamide

The title compound was prepared according to procedure for EXAMPLE 101, substituting EXAMPLE 123 for EXAMPLE 103, and benzylamide for azetidin-2-one. MS (ESI) m/z 361 (M+H)⁺; ¹H NMR (300 MHz, dimethylsulfoxide-*d₆*): δ 1.63 (m, 4 H), 2.40 (m, 4 H), 4.02 (s, 2 H), 6.95 (d, *J*=7.46 Hz, 1 H), 7.38 - 7.54 (m, 2 H), 7.50 - 7.62 (m, 1 H), 7.67 - 7.84 (m, 1 H), 7.90 - 8.12 (m, 3 H), 10.69 (s, 1 H), 12.61 (s, 1 H).

### EXAMPLE 127

### 4-((3'-((isopropylamino)methyl)-1,1'-biphenyl-3-yl)methyl)-5,6,7,8-tetrahydrophthalazin-1(2H)-one

### EXAMPLE 127A

### 3'-((4-oxo-3,4,5,6,7,8-hexahydrophthalazin-1-yl)methyl)biphenyl-3-carbaldehyde

A suspension of EXAMPLE 90 (500 mg, 1.57 mmol), 3-formylphenylboronic acid (352 mg, 2.35 mmol), dichlorobis(triphenylphosphine)palladium(II) (112 mg, 0.16 mmol) and sodium carbonate (2M solution, 3.13 mmol, 1.6 mL) in a 7/3/3 mixture of 1,2-dimethoxyethane/water/ethanol (23 mL) was purged with nitrogen, and heated at 70 °C for 16 hours. After cooling to room temperature, the reaction mixture was concentrated on a rotary evaporator. The crude solid was separated by HPLC (Zorbax® C-18 ODS packing material [Agilent Technologies, Santa Clara, CA], 0.1% trifluoroacetic acid/CH₃CN/H₂O) to provide the title compound. MS (DCI/NH₃) m/z 345 (M+H)⁺.

### EXAMPLE 127B

### 4-((3'-((isopropylamino)methyl)-1,1'-biphenyl-3-yl)methyl)-5,6,7,8-tetrahydrophthalazin-1(2H)-one

The title compound was prepared as a trifluoroacetic acid salt according to procedure for EXAMPLE 65, substituting EXAMPLE 127A for formaldehyde, and propan-2-amine for EXAMPLE 56. MS (DCI/NH₃) m/z 388 (M+H)⁺; ¹H NMR (500 MHz, CD₃OD): δ 1.41 (d, *J*=6.71 Hz, 6H), 1.64 - 1.76 (m, 4H), 2.43 - 2.48 (m, 2H), 2.48 - 2.53 (m, 2H), 3.42 - 3.52 (m, 1H), 4.06 (s, 2H), 4.27 (s, 2H), 7.22 (d, *J*=7.93 Hz, 1H), 7.41 (t, *J*=7.63 Hz, 1H), 7.45 - 7.50 (m, 2H), 7.51 - 7.56 (m, 2H), 7.64 - 7.69 (m, 1H), 7.71 - 7.74 (m, 1H).

### EXAMPLE 128

### 4-((3'-((cyclopentylamino)methyl)-1,1'-biphenyl-3-yl)methyl)-5,6,7,8-tetrahydrophthalazin-1(2H)-one

The title compound was prepared as a trifluoroacetic acid salt according to procedure for EXAMPLE 65, substituting EXAMPLE 127A for formaldehyde, and cyclopentanamine for EXAMPLE 56. MS (DCI/NH₃) m/z 414 (M+H)⁺.

### EXAMPLE 129

### 4-((3'-((2-methylpyrrolidin-1-yl)methyl)-1,1'-biphenyl-3-yl)methyl)-5,6,7,8-tetrahydrophthalazin-1(2H)-one

The title compound was prepared as a trifluoroacetic acid salt according to procedure for EXAMPLE 65, substituting EXAMPLE 127A for formaldehyde and 2-methylpyrrolidine for EXAMPLE 56. MS (DCI/NH₃) m/z 414 (M+H)⁺.

### EXAMPLE 130

### 4-((3'-((cyclopropylamino)methyl)-1,1'-biphenyl-3-yl)methyl)-5,6,7,8-tetrahydrophthalazin-1(2H)-one

The title compound was prepared as a trifluoroacetic acid salt according to procedure for EXAMPLE 65, substituting EXAMPLE 127A for formaldehyde, and cyclopropanamine for EXAMPLE 56. MS (DCI/NH₃) m/z 386 (M+H)⁺.

### EXAMPLE 131

### 4-((3'-((cyclobutylamino)methyl)-1,1'-biphenyl-3-yl)methyl)-5,6,7,8-tetrahydrophthalazin-1(2H)-one

The title compound was prepared as a trifluoroacetic acid salt according to procedure for EXAMPLE 65, substituting EXAMPLE 127A for formaldehyde, and cyclobutanamine for EXAMPLE 56. MS (DCI/NH₃) m/z 400 (M+H)⁺.

### EXAMPLE 132

### 4-((2-bromo-1-oxidopyridin-4-yl)methyl)-5,6,7,8-tetrahydrophthalazin-1(2H)-one

A solution of EXAMPLE 103 (100 mg, 0.31 mmol) in dichloromethane (15 ml) was treated with *meta*-chloroperoxybenzoic acid (100 mg, 0.58 mmol) at room temperature overnight, and concentrated. The residue was dissolved in methanol, and separated by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 336 (M+H)⁺.

### EXAMPLE 133

### 4-((1-oxido-2-(2-oxopyrrolidin-1-yl)pyridin-4-yl)methyl)-5,6,7,8-tetrahydrophthalazin-1(2H)-one

The title compound was prepared according to procedure for EXAMPLE 132, substituting EXAMPLE 102 for EXAMPLE 103. MS (ESI) m/z 341 (M+H)⁺.

### EXAMPLE 134

### methyl 5-((4-oxo-3,4,5,6,7,8-hexahydrophthalazin-1-yl)methyl)thiophene-3-carboxylate

### EXAMPLE 134A

### 3-((4-bromothiophen-2-yl)methylene)-4,5,6,7-tetrahydroisobenzofuran-1(3H)-one

The title compound was prepared according to the procedure for EXAMPLE 1C, substituting 5-bromothiophene-2-carbaldehyde for 2-fluoro-5-formylbenzonitrile. MS (DCI/NH₃) m/z 312 (M+H)⁺.

### EXAMPLE 134B

### 4-((4-bromothiophen-2-yl)methyl)-5,6,7,8-tetrahydrophthalazin-1(2H)-one

The title compound was prepared according to the procedure for EXAMPLE 2C substituting EXAMPLE 134A for EXAMPLE 2B. MS (DCI/NH₃) m/z 326 (M+H)⁺.

### EXAMPLE 134C

### methyl 5-((4-oxo-3,4,5,6,7,8-hexahydrophthalazin-1-yl)methyl)thiophene-3-carboxylate

The title compound was prepared according to the procedure for EXAMPLE 66, substituting EXAMPLE 134B for EXAMPLE 66B. MS (DCI/NH₃) m/z 305 (M+H)⁺.

### EXAMPLE 135

### 4-(3-((4-(2-(2-(2-aminoethoxy)ethoxy)ethyl)-1,4-diazepan-1-yl)carbonyl)-4-fluorobenzyl)phthalazin-1 (2H)-one

### EXAMPLE 135A

### tert-butyl 2-(2-(2-(4-(2-fluoro-5-((4-oxo-3,4,5,6,7,8-hexahydrophthalazin-1-yl)methyl)benzoyl)-1,4-diazepan-1-yl)ethoxy)ethoxy)ethylcarbamate

To a solution of 2-(2-(-t-Boc-aminoethoxy)ethoxy)ethyl bromide (Toronto, 137 mg, 0.44 mmol) in N,N-dimethylformamide (4 mL) was added EXAMPLE 5 (84 mg, 0.22 mmol) and potassium carbonate (91 mg, 0.66 mmol). The reaction mixture was heated at 35°C overnight, and partitioned between ethyl acetate and brine. The organic phase was washed with brine, and concentrated. The residue was separated by HPLC (Zorbax® C-18 ODS packing material [Agilent Technologies, Santa Clara, CA], 250x2.54 column, mobile phase A: 0.1% trifluoroacetic acid in H₂O; B: 0.1% trifluoroacetic acid in CH₃CN; 0-100% gradient) to provide the title compound as a trifluoroacetic acid salt. MS (DCI/NH₃) m/z 612 (M+H)⁺.

### EXAMPLE 135B

### 4-(3-((4-(2-(2-(2-aminoethoxy)ethoxy)ethyl)-1,4-diazepan-1-yl)carbonyl)-4-fluorobenzyl)phthalazin-1 (2H)-one

To a suspension of EXAMPLE 135A (43 mg, 0.06 mmol) in dichloromethane (5 mL) was added trifluoroacetic acid (1 mL) at room temperature. The solution remained at room temperature for 1 hour, and was concentrated. The residue was purified by HPLC (Zorbax® C-18 ODS packing material [Agilent Technologies, Santa Clara, CA], 250x2.54 column, mobile phase A: 0.1% trifluoroacetic acid in H₂O; B: 0.1% trifluoroacetic acid in CH₃CN; 0-100% gradient) to provide the title compound as a trifluoroacetic acid salt. The trifluoroacetic acid salt was dissolved in a mixture of methylene chloride and methanol, and was treated with 1M solution of HCl in ether. Removal of the volatiles afforded the title compound as a HCl salt. MS (DCI/NH₃) m/z 338 (M+H)⁺.

### EXAMPLE 136

### 1-methyl-N-(3-((4-oxo-3,4,5,6,7,8-hexahydrophthalazin-1-yl)methyl)phenyl)cyclopropanecarboxamide

A solution of EXAMPLE 89 (20 mg, 0.08 mmol), 1-methylcyclopropanecarboxylic acid (10 mg, 0.096 mmol), HATU (2-(1H-7-azabenzotriazol-1-yl)-1,1,3,3-tetramethyl uronium hexafluorophosphate methanaminium) (38 mg, 0.1 mmol) and triethylamine (20 mg, 0.2 mmol) in dimethylacetamide (2.5 mL) was stirred at room temperature for 18 hours, and concentrated. The residue was dissolved in a 1:1 mixture of dimethylsulfoxide/methanol, and separated by HPLC (Waters Sunfire® C-8 analytical column [Milford, MA], 0.1% trifluoroacetic acid/water/CH₃CN) to provide the title compound. MS (DCI/NH₃) m/z 338 (M+H)⁺; ¹H NMR (500 MHz, D₂O/dimethylsulfoxide-*d*₆): δ 0.57 - 0.69 (m, 2H), 1.02 - 1.10 (m, 2H), 1.38 (s, 3H), 1.57 - 1.65 (m, 4H), 2.29 - 2.44 (m, 4H), 3.87 (s, 2H), 6.89 (d, *J*=7.63 Hz, 1H), 7.23 (t, *J*=7.93 Hz, 1H), 7.42 (s, 1H), 7.46 (d, *J*= 8.24 Hz, 1H).

### EXAMPLE 137

### 2-methyl-N-(3-((4-oxo-3,4,5,6,7,8-hexahydrophthalazin-1-yl)methyl)phenyl)cyclopropanecarboxamide

The title compound was prepared according to the procedure for EXAMPLE 136, substituting 2-methylcyclopropanecarboxylic acid for 1-methylcyclopropanecarboxylic acid. MS (DCI/NH₃) m/z 338 (M+H)⁺.

### EXAMPLE 138

### 3-ethoxy-N-(3-((4-oxo-3,4,5,6,7,8-hexahydrophthalazin-1-yl)methyl)phenyl)propanamide

The title compound was prepared according to the procedure for EXAMPLE 136, substituting 3-ethoxypropanoic acid for 1-methylcyclopropanecarboxylic acid. MS (DCI/NH₃) m/z 356 (M+H)⁺; ¹H NMR (500 MHz, D₂O/dimethylsulfoxide-*d*₆): δ ¹H NMR (500 MHz, Solvent) δ 1.08 (t, *J*=7.02 Hz, 3H), 1.54 - 1.64 (m, 4H), 2.32 - 2.42 (m, 4H), 2.51 (t, *J*=6.26 Hz, 2H), 3.43 (q, *J*=7.02 Hz, 2H), 3.64 (t, *J*=6.26 Hz, 2H), 3.88 (s, 2H), 6.90 (d, *J*=7.63 Hz, 1H), 7.24 (t, *J*=7.78 Hz, 1H), 7.36 (s, 1H), 7.48 (d, *J*=7.93 Hz, 1H).

### EXAMPLE 139

### 5-oxo-N-(3-((4-oxo-3,4,5,6,7,8-hexahydrophthalazin-1-yl)methyl)phenyl)-L-prolinamide

The title compound was prepared according to the procedure for EXAMPLE 136, substituting (S)-5-oxopyrrolidine-2-carboxylic acid for 1-methylcyclopropanecarboxylic acid. MS (DCI/NH₃) m/z 367 (M+H)⁺; ¹H NMR (500 MHz, D₂O/dimethylsulfoxide-*d*₆): δ ¹H NMR (500 MHz, Solvent) δ 1.56 - 1.66 (m, 4H), 1.93 - 2.03 (m, 1H), 2.14 - 2.27 (m, 2H), 2.32 - 2.43 (m, 5H), 3.96 (s, 2H), 4.19 (dd, *J*=8.70, 4.42 Hz, 1H), 6.94 (d, *J*=7.63 Hz, 1H), 7.27 (t, *J*=7.93 Hz, 1H), 7.40 (s, 1H), 7.49 (d, *J*=7.93 Hz, 1H).

### EXAMPLE 140

### 5-oxo-N-(3-((4-oxo-3,4,5,6,7,8-hexahydrophthalazin-1-yl)methyl)phenyl)-D-prolinamide

The title compound was prepared according to the procedure for EXAMPLE 136, substituting (R)-5-oxopyrrolidine-2-carboxylic acid for 1-methylcyclopropanecarboxylic acid. MS (DCI/NH₃) m/z 367 (M+H)⁺; ¹H NMR (500 MHz, D₂O/dimethylsulfoxide-*d*₆): δ 1.56 - 1.66 (m, 4H), 1.93 - 2.02 (m, 1H), 2.13 - 2.25 (m, 2H), 2.32 - 2.42 (m, 5H), 3.89 (s, 2H), 4.18 (dd, *J*=8.70, 4.42 Hz, 1H), 6.94 (d, *J*=7.63 Hz, 1H), 7.27 (t, *J*=7.93 Hz, 1H), 7.39 (s, 1H), 7.49 (d, *J*=8.24 Hz, 1H).

### EXAMPLE 141

### N¹-(3-((4-oxo-3,4,5,6,7,8-hexahydrophthalazin-1-yl)methyl)phenyl)cyclopropane-1,1-dicarboxamide

The title compound was prepared according to the procedure for EXAMPLE 136, substituting 1-carbamoylcyclopropanecarboxylic acid for 1-methylcyclopropanecarboxylic acid. MS (DCI/NH₃) m/z 367 (M+H)⁺; ¹H NMR (500 MHz, D₂O/dimethylsulfoxide-*d*₆): δ 1.35 - 1.44 (m, 4H), 1.55 - 1.67 (m, 4H), 2.31 - 2.44 (m, 4H), 3.88 (s, 2H), 6.91 (d, *J=*7.63 Hz, 1H), 7.26 (t, *J*=7.78 Hz, 1H), 7.40 (s, 1H), 7.43 (d, *J*=7.93 Hz, 1H).

### EXAMPLE 142

### 2-(benzyloxy)-N-(3-((4-oxo-3,4,5,6,7,8-hexahydrophthalazin-l-yl)methyl)phenyl)acetamide

The title compound was prepared according to the procedure for EXAMPLE 136, substituting 2-(benzyloxy)acetic acid for 1-methylcyclopropanecarboxylic acid. MS (DCI/NH₃) m/z 404 (M+H)⁺; ¹H NMR (500 MHz, D₂O/dimethylsulfoxide-*d*₆): δ 1.56 - 1.64 (m, 4H), 2.32 - 2.42 (m, 4H), 3.89 (s, 2H), 4.06 (s, 2H), 4.60 (s, 2H), 6.93 (d, *J*=7.63 Hz, 1H), 7.26 (t, *J*=7.78 Hz, 1H), 7.31 - 7.34 (m, 1H), 7.36 - 7.42 (m, 5H), 7.50 (d, *J*=7.93 Hz, 1H).

### EXAMPLE 143

### N-(3-((4-oxo-3,4,5,6,7,8-hexahydrophthalazin-1-yl)methyl)phenyl)-3-phenylpropanamide

The title compound was prepared according to the procedure for EXAMPLE 136, substituting 3-phenylpropanoic acid for 1-methylcyclopropanecarboxylic acid. MS (DCI/NH₃) m/z 388 (M+H)⁺; ¹H NMR (500 MHz, D₂O/dimethylsulfoxide-*d*₆): δ 1.55 - 1.65 (m, 4H), 2.32 - 2.42 (m, 4H), 2.60 (t, *J*=7.63 Hz, 2H), 2.89 (t, *J*=7.63 Hz, 2H), 3.87 (s, 2H), 6.89 (d, *J*=7.63 Hz, 1H), 7.18 (t, *J*=7.17 Hz, 1H), 7.21 - 7.26 (m, 3H), 7.28 (t, *J=*7.48 Hz, 2H), 7.32 (s, 1H), 7.45 (d, *J*=8.24 Hz, 1H).

### EXAMPLE 144

### 3-(2,5-dimethoxyphenyl)-N-(3-((4-oxo-3,4,5,6,7,8-hexahydrophthalazin-1-yl)methyl)phenyl)propanamide

The title compound was prepared according to procedure for EXAMPLE 136, substituting 3-(2,5-dimethoxyphenyl)propanoic acid for 1-methylcyclopropanecarboxylic acid. MS (DCI/NH₃) m/z 448 (M+H)⁺.

### EXAMPLE 145

### N-(3-((4-oxo-3,4,5,6,7,8-hexahydrophthalazin-1-yl)methyl)phenyl)-1-phenylcyclopropanecarboxamide

The title compound was prepared according to the procedure for EXAMPLE 136, substituting 1-phenylcyclopropanecarboxylic acid for 1-methylcyclopropanecarboxylic acid. MS (DCI/NH₃) m/z 400 (M+H)⁺.

### EXAMPLE 146

### (2S)-N-(3-((4-oxo-3,4,5,6,7,8-hexahydrophthalazin-1-yl)methyl)phenyl)-2-phenylbutanamide

The title compound was prepared according to the procedure for EXAMPLE 136, substituting (S)-2-phenylbutanoic acid for 1-methylcyclopropanecarboxylic acid. MS (DCI/NH₃) m/z 402 (M+H)⁺.

### EXAMPLE 147

### N-(3-((4-oxo-3,4,5,6,7,8-hexahydrophthalazin-1-yl)methyl)phenyl)-4-phenylbutanamide

The title compound was prepared according to procedure for EXAMPLE 136, substituting 4-phenylbutanoic acid for 1-methylcyclopropanecarboxylic acid. MS (DCI/NH₃) m/z 402 (M+H)⁺.

### EXAMPLE 148

### 2-(3-methylphenoxy)-N-(3-((4-oxo-3,4,5,6,7,8-hexahydrophthalazin-1-yl)methyl)phenyl)acetamide

The title compound was prepared according to the procedure for EXAMPLE 136, substituting 2-(m-tolyloxy)acetic acid for 1-methylcyclopropanecarboxylic acid. MS (DCI/NH₃) m/z 404 (M+H)⁺.

### EXAMPLE 149

### 2-(2-methylphenoxy)-N-(3-((4-oxo-3,4,5,6,7,8-hexahydrophthalazin-1-yl)methyl)phenyl)acetamide

The title compound was prepared according to the procedure for EXAMPLE 136, substituting 2-(o-tolyloxy)acetic acid for 1-methylcyclopropanecarboxylic acid. MS (DCI/NH₃) m/z 404 (M+H)⁺.

### EXAMPLE 150

### 2-(4-methylphenoxy)-N-(3-((4-oxo-3,4,5,6,7,8-hexahydrophthalazin-1-yl)mcthyl)phcnyl)acctamidc

The title compound was prepared according to procedure for EXAMPLE 136, substituting 2-(p-tolyloxy)acetic acid for 1-methylcyclopropanecarboxylic acid. MS (DCI/NH₃) m/z 404 (M+H)⁺; ¹H NMR (500 MHz, D₂O/dimethylsulfoxide-*d*₆): δ 1.56 - 1.65 (m, 4H), 2.23 (s, 3H), 2.33 - 2.44 (m, 4H), 3.89 (s, 2H), 4.61 (s, 2H), 6.88 (d, *J*=8.54 Hz, 2H), 6.94 (d, *J*=7.63 Hz, 1H), 7.11 (d, *J*=8.24 Hz, 2H), 7.27 (t, *J*=7.78 Hz, 1H), 7.41 (s, 1H), 7.50 (d, *J*=8.24 Hz, 1H).

### EXAMPLE 151

### (2R)-2-methoxy-N-(3-((4-oxo-3,4,5,6,7,8-hexahydrophthalazin-1-yl)methyl)phenyl)-2-phenylacetamide

The title compound was prepared according to procedure for EXAMPLE 136, substituting (R)-2-methoxy-2-phenylacetic acid for 1-methylcyclopropanecarboxylic acid. MS (DCI/NH₃) m/z 404 (M+H)⁺; ¹H NMR (500 MHz, D₂O/dimethylsulfoxide-*d*₆): δ 1.53 - 1.66 (m, 4H), 2.29 - 2.44 (m, 4H), 3.35 (s, 3H), 3.87 (s, 2H), 4.81 (s, 1H), 6.91 (d, *J*=7.63 Hz, 1H), 7.25 (t, *J*=7.93 Hz, 1H), 7.33 - 7.36 (m, 1H), 7.39 (t, *J*=7.17 Hz, 2H), 7.45 - 7.49 (m, 3H), 7.52 (d, *J*=8.24 Hz, 1H).

### EXAMPLE 152

### (2S)-2-methoxy-N-(3-((4-oxo-3,4,5,6,7,8-hexahydrophthalazin-1-yl)methyl)phenyl)-2-phenylacetamide

The title compound was prepared according to the procedure for EXAMPLE 136, substituting (S)-2-methoxy-2-phenylacetic acid for 1-methylcyclopropanecarboxylic acid. MS (DCI/NH₃) m/z 404 (M+H)⁺; ¹H NMR (500 MHz, D₂O/dimethylsulfoxide-*d*₆): δ 1.53 - 1.66 (m, 4H), 2.30 - 2.42 (m, 4H), 3.34 (s, 3H), 3.87 (s, 2H), 4.81 (s, 1H), 6.91 (d, *J*=7.63 Hz, 1H), 7.25 (t, *J*=7.93 Hz, 1H), 7.32 - 7.36 (m, 1H), 7.39 (t, *J*=7.17 Hz, 2H), 7.44 - 7.49 (m, 3H), 7.51 (d, *J*=8.24 Hz, 1H).

### EXAMPLE 153

### N-(3-((4-oxo-3,4,5,6,7,8-hexahydrophthalazin-1-yl)methyl)phenyl)-3-phenoxypropanamide

The title compound was prepared according to procedure for EXAMPLE 136, substituting 3-phenoxypropanoic acid for 1-methylcyclopropanecarboxylic acid. MS (DCI/NH₃) m/z 404 (M+H)⁺.

### EXAMPLE 154

### N-(3-((4-oxo-3,4,5,6,7,8-hexahydrophthalazin-1-yl)methyl)phenyl)-4-thien-2-ylbutanamide

The title compound was prepared according to procedure for EXAMPLE 136, substituting 4-(thiophen-2-yl)butanoic acid for 1-methylcyclopropanecarboxylic acid. MS (DCI/NH₃) m/z 408 (M+H)⁺.

### EXAMPLE 155

### 1-acetyl-N-(3-((4-oxo-3,4,5,6,7,8-hexahydrophthalazin-1-yl)methyl)phenyl)piperidine-4-carboxamide

The title compound was prepared according to the procedure for EXAMPLE 136, substituting 1-acetylpiperidine-4-carboxylic acid for 1-methylcyclopropanecarboxylic acid. MS (DCI/NH₃) m/z 409 (M+H)⁺; ¹H NMR (500 MHz, D₂O/dimethylsulfoxide-*d*₆): δ 1.36-1.46 (m, 1H), 1.52 - 1.67 (m, 5H), 1.79 (t, *J*=14.19 Hz, 2H), 2.02 (s, 3H), 2.30 - 2.43 (m, 4H), 2.56 - 2.63 (m, 1H), 3.06 (t, *J*=12.97 Hz, 1H), 3.85 - 3.90 (m, 2H), 3.97 (s, 2H), 4.39 (d, *J*=13.43 Hz, 1H), 6.89 (d, *J*=7.63 Hz, 1H), 7.24 (t, *J*=7.78 Hz, 1H), 7.38 (s, 1H), 7.48 (d, *J*=8.24 Hz, 1H).

### EXAMPLE 156

### 2-(3,5-difluorophenyl)-N-(3-((4-oxo-3,4,5,6,7,8-hexahydrophthalazin-1-yl)methyl)phenyl)acetamide

The title compound was prepared according to procedure for EXAMPLE 136, substituting 2-(3,5-difluorophenyl)acetic acid for 1-methylcyclopropanecarboxylic acid. MS (DCI/NH₃) m/z 410 (M+H)⁺; ¹H NMR (500 MHz, D₂O/dimethylsulfoxide-*d*₆): δ 1.53 - 1.67 (m, 4H), 2.31 - 2.42 (m, 4H), 3.67 (s, 2H), 3.88 (s, 2H), 6.91 (d, *J*=7.63 Hz, 1H), 7.04 (d, *J*=6.41 Hz, 1H), 7.07 - 7.13 (m, 1H), 7.25 (t, *J*=7.93 Hz, 1H), 7.36 (s, 1H), 7.46 (d, *J*=8.24 Hz, 1H).

### EXAMPLE 157

### N²-acetyl-N¹-(3-((4-oxo-3,4,5,6,7,8-hexahydrophthalazin-1-yl)methyl)phenyl)-L-leucinamide

The title compound was prepared according to procedure for EXAMPLE 136, substituting (S)-2-acetamido-4-methylpentanoic acid for 1-methylcyclopropanecarboxylic acid. MS (DCI/NH₃) m/z 411 (M+H)⁺; ¹H NMR (500 MHz, D₂O/dimethylsulfoxide-*d*₆): δ 0.88 (d, *J*= 6.71 Hz, 3H), 0.90 (d, *J*= 6.71 Hz, 3H), 1.43 - 1.53 (m, 2H), 1.56 - 1.66 (m, 5H), 1.87 (s, 3 H), 2.29 - 2.43 (m, 4H), 3.88 (s, 2H), 4.39 (dd, *J*=9.61, 5.34 Hz, 1H), 6.91 (d, *J*=7.63 Hz, 1H), 7.25 (t, *J*=7.78 Hz, 1H), 7.38 (s, 1H), 7.49 (d, *J*=8.24 Hz, 1H).

### EXAMPLE 158

### N¹-(3-((4-oxo-3,4,5,6,7,8-hexahydrophthalazin-1-yl)methyl)phenyl)-N²,N²-dipropyl-L-alaninamide

The title compound was prepared according to procedure for EXAMPLE 136, substituting (S)-2-(dipropylamino)propanoic acid for 1-methylcyclopropanecarboxylic acid. MS (DCI/NH₃) m/z 411 (M+H)⁺.

### EXAMPLE 159

### 4-oxo-N-(3-((4-oxo-3,4,5,6,7,8-hexahydrophthalazin-1-yl)methyl)phenyl)-4-phenylbutanamide

The title compound was prepared according to procedure for EXAMPLE 136, substituting 4-oxo-4-phenylbutanoic acid for 1-methylcyclopropanecarboxylic acid. MS (DCI/NH₃) m/z 411 (M+H)⁺; ¹H NMR (500 MHz, D₂O/dimethylsulfoxide-*d*₆): δ 1.55 - 1.66 (m, 4H), 2.31 - 2.42 (m, 4H), 2.70 (t, *J*=6.26 Hz, 2H), 3.32 (t, *J*=6.26 Hz, 2H), 3.87 (s, 2H), 6.88 (d, *J*=7.63 Hz, 1H), 7.23 (t, *J*=7.93 Hz, 1H), 7.37 (s, 1H), 7.45 (d, *J*=8.24 Hz, 1H), 7.55 (t, *J*=7.63 Hz, 2H), 7.66 (t, *J*=7.32 Hz, 1H), 7.99 (t, *J*=6.41 Hz, 2H).

### EXAMPLE 160

### N-(2-oxo-2-(3-((4-oxo-3,4,5,6,7,8-hexahydrophthalazin-1-yl)methyl)phenylamino)ethyl)benzamide

The title compound was prepared according to procedure for EXAMPLE 136, substituting 2-benzamidoacetic acid for 1-methylcyclopropanecarboxylic acid. MS (DCI/NH₃) m/z 417 (M+H)⁺; ¹H NMR (500 MHz, D₂O/dimethylsulfoxide-*d*₆): δ 1.55 - 1.66 (m, 4H), 2.31 - 2.40 (m, 4H), 3.89 (s, 2H), 4.04 (s, 2H), 6.92 (d, *J*=7.93 Hz, 1H), 7.26 (t, *J*=7.93 Hz, 1H), 7.38 (s, 1H), 7.47 (d, *J*=8.24 Hz, 1H), 7.49 - 7.54 (m, 2H), 7.58 (t, *J*=7.32 Hz, 1H), 7.85 - 7.90 (m, 2H).

### EXAMPLE 161

### 3-(3-methoxyphenyl)-N-(3-((4-oxo-3,4,5,6,7,8-hexahydrophthalazin-1-yl)methyl)phenyl)propanamide

The title compound was prepared according to procedure for EXAMPLE 136, substituting 3-(3-methoxyphenyl)propanoic acid for 1-methylcyclopropanecarboxylic acid. MS (DCI/NH₃) m/z 418 (M+H)⁺.

### EXAMPLE 162

### 3-(4-methoxyphenyl)-N-(3-((4-oxo-3,4,5,6,7,8-hexahydrophthalazin-1-yl)methyl)phenyl)propanamide

The title compound was prepared according to procedure for EXAMPLE 136, substituting 3-(4-methoxyphenyl)propanoic acid for 1-methylcyclopropanecarboxylic acid. MS (DCI/NH₃) m/z 418 (M+H)⁺.

### EXAMPLE 163

### 2-(3,4-dimethylphenoxy)-N-(3-((4-oxo-3,4,5,6,7,8-hexahydrophthalazin-1-yl)methyl)phenyl)acetamide

The title compound was prepared according to procedure for EXAMPLE 136, substituting 2-(3,4-dimethylphenoxy)acetic acid for 1-methylcyclopropanecarboxylic acid. MS (DCI/NH₃) m/z 418 (M+H)⁺.

### EXAMPLE 164

### (2R)-2-hydroxy-N-(3-((4-oxo-3,4,5,6,7,8-hexahydrophthalazin-1-yl)methyl)phenyl)-4-phenylbutanamide

The title compound was prepared according to procedure for EXAMPLE 136, substituting (R)-2-hydroxy-4-phenylbutanoic acid for 1-methylcyclopropanecarboxylic acid. MS (DCI/NH₃) m/z 418 (M+H); ¹H NMR (500 MHz, D₂O/dimethylsulfoxide-*d*₆): δ 1.55 - 1.65 (m, 4H), 1.80 - 1.90 (m, 1H), 1.95 - 2.03 (m, 1H), 2.31 - 2.44 (m, 4H), 2.69 (t, *J*=7.93 Hz, 2H), 3.88 (s, 2H), 3.96 (s, 1H), 4.01 (dd, *J*=8.09, 4.12 Hz, 1H),6.91 (d, *J*=7.63 Hz, 1H), 7.17 - 7.23 (m, 3H), 7.25 (t, *J*=7.78 Hz, 1H), 7.29 (t, *J*=7.48 Hz, 2H), 7.49 (s, 1H), 7.53 (d, *J*=7.93 Hz, 1H).

### EXAMPLE 165

### N-(3-((4-oxo-3,4,5,6,7,8-hexahydrophthalazin-1-yl)methyl)phenyl)-4-phenoxybutanamide

The title compound was prepared according to procedure for EXAMPLE 136, substituting 4-phenoxybutanoic acid for 1-methylcyclopropanecarboxylic acid. MS (DCI/NH₃) m/z 418 (M+H)⁺; ¹H NMR (500 MHz, D₂O/dimethylsulfoxide-*d*₆): δ 1.55 - 1.67 (m, 4H), 1.96 - 2.06 (m, 2H), 2.31 - 2.42 (m, 4H), 2.47 (t, *J*=7.48 Hz, 2H), 3.88 (s, 2H), 3.99 (t, *J*=6.26 Hz, 2H), 6.87 - 6.91 (m, 2H), 6.91 - 6.96 (m, 2H), 7.24 (t, *J*=7.78 Hz, 1H), 7.26-7.30 (m, 2H), 7.36 (s, 1H), 7.48 (d, *J*=8.24 Hz, 1H).

### EXAMPLE 166

### 4-oxo-N-(3-((4-oxo-3,4,5,6,7,8-hexahydrophthalazin-1-yl)methyl)phenyl)-4-thien-2-ylbutanamide

The title compound was prepared according to the procedure for EXAMPLE 136, substituting 4-oxo-4-(thiophen-2-yl)butanoic acid for 1-methylcyclopropanecarboxylic acid. MS (DCI/NH₃) m/z 422 (M+H)⁺; ¹H NMR (500 MHz, D₂O/dimethylsulfoxide-*d₆*): δ 1.55 - 1.65 (m, 4H), 2.32 - 2.42 (m, 4H), 2.69 (t, *J*=6.41 Hz, 2H), 3.26 (t, *J*=6.41 Hz, 2H), 3.87 (s, 2H), 6.88 (d, *J*=7.63 Hz, 1H), 7.23 (t, *J*=7.93 Hz, 1H), 7.25 - 7.29 (m, 1H), 7.37 (s, 1H), 7.44 (d, *J*=8.24 Hz, 1H), 7.97 (d, *J*=4.88 Hz, 1H), 7.99 (d, *J*=2.75 Hz, 1H).

### EXAMPLE 167

### 2-((4-methylpyrimidin-2-yl)thio)-N-(3-((4-oxo-3,4,5,6,7,8-hexahydrophthalazin-1-yl)methyl)phenyl)acetamide

The title compound was prepared according to procedure for EXAMPLE 136, substituting 2-(4-methylpyrimidin-2-ylthio)acetic acid for 1-methylcyclopropanecarboxylic acid. MS (DCI/NH₃) m/z 422 (M+H)⁺.

### EXAMPLE 168

### 3-(2-chlorophenyl)-N-(3-((4-oxo-3,4,5,6,7,8-hexahydrophthalazin-1-yl)methyl)phenyl)propanamide

The title compound was prepared according to procedure for EXAMPLE 136, substituting 3-(2-chlorophenyl)propanoic acid for 1-methylcyclopropanecarboxylic acid. MS (DCI/NH₃) m/z 422 (M+H)⁺.

### EXAMPLE 169

### 3-(4-chlorophenyl)-N-(3-((4-oxo-3,4,5,6,7,8-hexahydrophthalazin-1-yl)methyl)phenyl)propanamide

The title compound was prepared according to procedure for EXAMPLE 136, substituting 3-(4-chlorophenyl)propanoic acid for 1-methylcyclopropanecarboxylic acid. MS (DCI/NH₃) m/z 422 (M+H)⁺.

### EXAMPLE 170

### 3-methyl-N-(3-((4-oxo-3,4,5,6,7,8-hexahydrophthalazin-1-yl)methyl)phenyl)-2-phenylpentanamide

The title compound was prepared according to procedure for EXAMPLE 136, substituting 3-methyl-2-phenylpentanoic acid for 1-methylcyclopropanecarboxylic acid. MS (DCI/NH₃) m/z 430 (M+H)⁺.

### EXAMPLE 171

### 2-(4-chloro-2-methylphenoxy)-N-(3-((4-oxo-3,4,5,6,7,8-hexahydrophthalazin-1-yl)methyl)phenyl)acetamide

The title compound was prepared according to procedure for EXAMPLE 136, substituting 2-(4-chloro-2-methylphenoxy)acetic acid for 1-methylcyclopropanecarboxylic acid. MS (DCI/NH₃) m/z 438 (M+H)⁺.

### EXAMPLE 172

### N-(3-((4-oxo-3,4,5,6,7,8-hexahydrophthalazin-1-yl)methyl)phenyl)-N'-phenylpentanediamide

The title compound was prepared according to procedure for EXAMPLE 136, substituting 5-oxo-5-(phenylamino)pentanoic acid for 1-methylcyclopropanecarboxylic acid. MS (DCI/NH₃) m/z 445 (M+H)⁺; ¹H NMR (500 MHz, D₂O/dimethylsulfoxide-*d*₆): δ 1.55 - 1.65 (m, 4H), 1.84 - 1.94 (m, 2H), 2.31 - 2.42 (m, 8H), 3.87 (s, 2H), 6.89 (d, *J*=7.63 Hz, 1H), 7.05 (t, *J*=7.32 Hz, 1H), 7.24 (t, *J*=7.93 Hz, 1H), 7.30 (t, *J*=8.09 Hz, 2H), 7.36 (s, 1H), 7.48 (d, *J*=8.24 Hz, 1H), 7.57 (d, *J*=7.63 Hz, 2 H).

### EXAMPLE 173

### 4-(4-methoxyphenyl)-4-oxo-N-(3-((4-oxo-3,4,5,6,7,8-hexahydrophthalazin-1-yl)methyl)phenyl)butanamide

The title compound was prepared according to procedure for EXAMPLE 136, substituting 4-(4-methoxyphenyl)-4-oxobutanoic acid for 1-methylcyclopropanecarboxylic acid. MS (DCI/NH₃) m/z 446 (M+H)⁺.

### EXAMPLE 174

### N-(3-((4-oxo-3,4,5,6,7,8-hexahydrophthalazin-1-yl)methyl)phenyl)-2,2-diphenylacetamide

The title compound was prepared according to procedure for EXAMPLE 136, substituting 2,2-diphenylacetic acid for 1-methylcyclopropanecarboxylic acid. MS (DCI/NH₃) m/z 450 (M+H)⁺.

### EXAMPLE 175

### N-(3-((4-oxo-3,4,5,6,7,8-hexahydrophthalazin-1-yl)methyl)phenyl)-3-(phenylsulfonyl)propanamide

The title compound was prepared according to procedure for EXAMPLE 136, substituting 3-(phenylsulfonyl)propanoic acid for 1-methylcyclopropanecarboxylic acid. MS (DCI/NH₃) m/z 452 (M+H)⁺; ¹H NMR (500 MHz, D₂O/dimethylsulfoxide-*d*₆): δ 1.52 - 1.70 (m, 4H), 2.29 - 2.42 (m, 4H), 2.66 (t, *J*=7.32 Hz, 2H), 3.59 (t, *J*=7.32 Hz, 2H), 3.87 (s, 2H), 6.90 (d, *J*=7.32 Hz, 1H), 7.20 - 7.26 (m, 2H), 7.37 (d, *J*=8.54 Hz, 1H), 7.66 (t, *J*=7.63 Hz, 2H), 7.74 (t, *J*=7.48 Hz, 1H), 7.91 (d, *J*=7.32 Hz, 2H).

### EXAMPLE 176

### N-(3-((4-oxo-3,4,5,6,7,8-hexahydrophthalazin-1-yl)methyl)phenyl)-2-(3-phenoxyphenyl)acetamide

The title compound was prepared according to the procedure for EXAMPLE 136, substituting 2-(3-phenoxyphenyl)acetic acid for 1-methylcyclopropanecarboxylic acid. MS (DCI/NH₃) m/z 466 (M+H)⁺; ¹H NMR (500 MHz, D₂O/dimethylsulfoxide-*d*₆): δ 1.54 - 1.65 (m, 4H), 2.29 - 2.42 (m, 4H), 3.60 (s, 2H), 3.87 (s, 2H), 6.86 - 6.92 (m, 2H), 6.98 - 7.03 (m, 3H), 7.10 (d, *J*=7.93 Hz, 1H), 7.16 (t, *J*=7.48 Hz, 1H), 7.24 (t, *J*=7.78 Hz, 1H), 7.32 - 7.37 (m, 2H), 7.38 - 7.42 (m, 2H), 7.46 (d, *J*=8.24 Hz, 1H).

### EXAMPLE 177

### 4-ethyl-N-(3-((4-oxo-3,4,5,6,7,8-hexahydrophthalazin-1-yl)methyl)phenyl)benzamide

The title compound was prepared according to the procedure for EXAMPLE 136, substituting 4-ethylbenzoic acid for 1-methylcyclopropanecarboxylic acid. MS (DCI/NH₃) m/z 388 (M+H)⁺.

### EXAMPLE 178

### 3-fluoro-2-methyl-N-(3-((4-oxo-3,4,5,6,7,8-hexahydrophthalazin-1-yl)mcthyl)phcnyl)bcnzamidc

The title compound was prepared according to the procedure for EXAMPLE 136, substituting 3-fluoro-2-methylbenzoic acid for 1-methylcyclopropanecarboxylic acid. MS (DCI/NH₃) m/z 392 (M+H)⁺.

### EXAMPLE 179

### 5-fluoro-2-methyl-N-(3-((4-oxo-3,4,5,6,7,8-hexahydrophthalazin-1-yl)methyl)phenyl)benzamide

The title compound was prepared according to the procedure for EXAMPLE 136, substituting 5-fluoro-2-methylbenzoic acid for 1-methylcyclopropanecarboxylic acid. MS (DCI/NH₃) m/z 392 (M+H)⁺.

### EXAMPLE 180

### 3-fluoro-4-methyl-N-(3-((4-oxo-3,4,5,6,7,8-hexahydrophthalazin-1-yl)methyl)phenyl)benzamide

The title compound was prepared according to the procedure for EXAMPLE 136, substituting 3-fluoro-4-methylbenzoic acid for 1-methylcyclopropanecarboxylic acid. MS (DCI/NH₃) m/z 392 (M+H)⁺.

### EXAMPLE 181

### 2,3-difluoro-N-(3-((4-oxo-3,4,5,6,7,8-hexahydrophthalazin-1-yl)methyl)phenyl)benzamide

The title compound was prepared according to the procedure for EXAMPLE 136, substituting 2,3-difluorobenzoic acid for 1-methylcyclopropanecarboxylic acid. MS (DCI/NH₃) m/z 396 (M+H)⁺.

### EXAMPLE 182

### 2,4-difluoro-N-(3-((4-oxo-3,4,5,6,7,8-hexahydrophthalazin-1-yl)methyl)phenyl)benzamide

The title compound was prepared according to the procedure for EXAMPLE 136, substituting 2,4-difluorobenzoic acid for 1-methylcyclopropanecarboxylic acid. MS (DCI/NH₃) m/z 396 (M+H)⁺.

### EXAMPLE 183

### 2,5-difluoro-N-(3-((4-oxo-3,4,5,6,7,8-hexahydrophthalazin-1-yl)methyl)phenyl)benzamide

The title compound was prepared according to the procedure for EXAMPLE 136, substituting 2,5-difluorobenzoic acid for 1-methylcyclopropanecarboxylic acid. MS (DCI/NH₃) ml) 396 (M+H)⁺.

### EXAMPLE 184

### 3,5-difluoro-N-(3-((4-oxo-3,4,5,6,7,8-hexahydrophthalazin-1-yl)methyl)phenyl)benzamide

The title compound was prepared according to the procedure for EXAMPLE 136, substituting 3,5-difluorobenzoic acid for 1-methylcyclopropanecarboxylic acid. MS (DCI/NH₃) m/z 396 (M+H)⁺.

### EXAMPLE 185

### N-(3-((4-oxo-3,4,5,6,7,8-hexahydrophthalazin-1-yl)methyl)phenyl)-4-propylbenzamide

The title compound was prepared according to the procedure for EXAMPLE 136, substituting 4-propylbenzoic acid for 1-methylcyclopropanecarboxylic acid. MS (DCI/NH₃) m/z 402 (M+H)⁺.

### EXAMPLE 186

### 4-isopropyl-N-(3-((4-oxo-3,4,5,6,7,8-hexahydrophthalazin-1-yl)methyl)phenyl)benzamide

The title compound was prepared according to the procedure for EXAMPLE 136, substituting 4-isopropylbenzoic acid for 1-methylcyclopropanecarboxylic acid. MS (DCI/NH₃) m/z 402 (M+H)⁺.

### EXAMPLE 187

### 2-ethoxy-N-(3-((4-oxo-3,4,5,6,7,8-hexahydrophthalazin-1-yl)methyl)phenyl)benzamide

The title compound was prepared according to the procedure for EXAMPLE 136, substituting 4-ethoxybenzoic acid for 1-methylcyclopropanecarboxylic acid. MS (DCI/NH₃) m/z 404 (M+H)⁺.

### EXAMPLE 188

### 4-isopropoxy-N-(3-((4-oxo-3,4,5,6,7,8-hexahydrophthalazin-1-yl)methyl)phenyl)benzamide

The title compound was prepared according to the procedure for EXAMPLE 136, substituting 4-isopropoxybenzoic acid for 1-methylcyclopropanecarboxylic acid. MS (DCI/NH₃) m/z 418 (M+H)⁺; ¹H NMR (500 MHz, D₂O/dimethylsulfoxide-*d*₆): δ 1.30 (d, *J*=6.10 Hz, 6H), 1.53 - 1.67 (m, 4H), 2.33 - 2.46 (m, 4H), 3.91 (s, 2H), 4.67 - 4.80 (m, 1H), 6.94 (d, *J*=7.63 Hz, 1H), 7.02 (d, *J*=8.85 Hz, 2H), 7.29 (t, *J*=7.78 Hz, 1H), 7.55 (s, 1H), 7.62 (d, *J*=8.24 Hz, 1H), 7.89 (d, *J*=8.85 Hz, 2H).

### EXAMPLE 189

### 4-(diethylamino)-N-(3-((4-oxo-3,4,5,6,7,8-hexahydrophthalazin-1-yl)methyl)phenyl)benzamide

The title compound was prepared according to the procedure for EXAMPLE 136, substituting 4-(diethylamino)benzoic acid for 1-methylcyclopropanecarboxylic acid. MS (DCI/NH₃) m/z 431 (M+H)⁺; ¹H NMR (500 MHz, D₂O/dimethylsulfoxide-*d*₆): δ 1.11 (t, *J*=7.02 Hz, 6H), 1.57 - 1.66 (m, 4H), 2.34 - 2.44 (m, 4H), 3.45 (q, *J*=7.02 Hz, 4H), 3.91 (s, 2H), 6.87 (d, *J*=8.85 Hz, 2H), 6.91 (d, *J*=7.63 Hz, 1H), 7.27 (t, *J*=7.93 Hz, 1H), 7.55 (s, 1H), 7.62 (d, *J*=8.24 Hz, 1H), 7.85 (d, *J*=8.85 Hz, 2H).

### EXAMPLE 190

### 4-butoxy-N-(3-((4-oxo-3,4,5,6,7,8-hexahydrophthalazin-1-yl)methyl)phenyl)benzamide

The title compound was prepared according to the procedure for EXAMPLE 136, substituting 4-butoxybenzoic acid for 1-methylcyclopropanecarboxylic acid. MS (DCI/NH₃) m/z 432 (M+H)⁺.

### EXAMPLE 191

### 2-fluoro-N-(3-((4-oxo-3,4,5,6,7,8-hexahydrophthalazin-1-yl)methyl)phenyl)-5-(trifluoromethyl)benzamide

The title compound was prepared according to the procedure for EXAMPLE 136, substituting 2-fluoro-5-(trifluoromethyl)benzoic acid for 1-methylcyclopropanecarboxylic acid. MS (DCI/NH₃) m/z 446 (M+H)⁺.

### EXAMPLE 192

### 2-chloro-N-(3-((4-oxo-3,4,5,6,7,8-hexahydrophthalazin-1-yl)methyl)phenyl)-5-(trifluoromethyl)benzamide

The title compound was prepared according to the procedure for EXAMPLE 136, substituting 2-chloro-5-(trifluoromethyl)benzoic acid for 1-methylcyclopropanecarboxylic acid. MS (DCI/NH₃) m/z 461 (M+H)⁺.

### EXAMPLE 193

### N-(3-((4-oxo-3,4,5,6,7,8-hexahydrophthalazin-1-yl)methyl)phenyl)-2-furamide

The title compound was prepared according to the procedure for EXAMPLE 136, substituting furan-2-carboxylic acid for 1-methylcyclopropanecarboxylic acid. MS (DCI/NH₃) m/z 350 (M+H)⁺.

### EXAMPLE 194

### N-(3-((4-oxo-3,4,5,6,7,8-hexahydrophthalazin-1-yl)methyl)phenyl)-3-furamide

The title compound was prepared according to the procedure for EXAMPLE 136, substituting furan-3-carboxylic acid for 1-methylcyclopropanecarboxylic acid. MS (DCI/NH₃) m/z 350 (M+H)⁺.

### EXAMPLE 195

### 2,5-dimethyl-N-(3-((4-oxo-3,4,5,6,7,8-hexahydrophthalazin-1-yl)methyl)phenyl)-3-furamide

The title compound was prepared according to the procedure for EXAMPLE 136, substituting 2,5-dimethylfuran-3-carboxylic acid for 1-methylcyclopropanecarboxylic acid. MS (DCI/NH₃) m/z 378 (M+H)⁺.

### EXAMPLE 196

### N-(3-((4-oxo-3,4,5,6,7,8-hexahydrophthalazin-1-yl)methyl)phenyl)thiophene-2-carboxamide

The title compound was prepared according to the procedure for EXAMPLE 136, substituting thiophene-2-carboxylic acid for 1-methylcyclopropanecarboxylic acid. MS (DCI/NH₃) m/z 366 (M+H)⁺.

### EXAMPLE 197

### N-(3-((4-oxo-3,4,5,6,7,8-hexahydrophthalazin-1-yl)methyl)phenyl)thiophene-3-carboxamide

The title compound was prepared according to the procedure for EXAMPLE 136, substituting thiophene-3-carboxylic acid for 1-methylcyclopropanecarboxylic acid. MS (DCI/NH₃) m/z 366 (M+H)⁺; ¹H NMR (500 MHz, D₂O/dimethylsulfoxide-*d*₆): δ 1.54 - 1.66 (m, 4H), 2.32 - 2.44 (m, 4H), 3.92 (s, 2H), 6.96 (d, *J*=7.63 Hz, 1H), 7.30 (t, *J*=7.93 Hz, 1H), 7.53 (s, 1H), 7.59 - 7.65 (m, 3H), 8.31 (dd, *J*=2.75, 1.53 Hz, 1H).

### EXAMPLE 198

### 3-methyl-N-(3-((4-oxo-3,4,5,6,7,8-hexahydrophthalazin-1-yl)methyl)phenyl)thiophene-2-carboxamide

The title compound was prepared according to the procedure for EXAMPLE 136, substituting 3-methylthiophene-2-carboxylic acid for 1-methylcyclopropanecarboxylic acid. MS (DCI/NH₃) m/z 380 (M+H)⁺.

### EXAMPLE 199

### 5-methyl-N-(3-((4-oxo-3,4,5,6,7,8-hexahydrophthalazin-1-yl)methyl)phenyl)thiophene-2-carboxamide

The title compound was prepared according to the procedure for EXAMPLE 136, substituting 5-methylthiophene-2-carboxylic acid for 1-methylcyclopropanecarboxylic acid. MS (DCI/NH₃) m/z 380 (M+H)⁺.

### EXAMPLE 200

### N-(3-((4-oxo-3,4,5,6,7,8-hexahydrophthalazin-1-yl)methyl)phenyl)-1H-pyrrole-2-carboxamide

The title compound was prepared according to the procedure for EXAMPLE 136, substituting pyrrole-3-carboxylic acid for 1-methylcyclopropanecarboxylic acid. MS (DCI/NH₃) m/z 349 (M+H)⁺; ¹H NMR (500 MHz, D₂O/dimethylsulfoxide-*d*₆): δ 1.56 - 1.66 (m, 4H), 2.33 - 2.44 (m, 4H), 3.91 (s, 2H), 6.18 (dd, *J*=3.51, 2.29 Hz, 1H), 6.92 (d, *J*=7.63 Hz, 1H), 6.98 (d, *J*=1.53 Hz, 1H), 7.03 - 7.07 (m, 1H), 7.27 (t, *J*=7.93 Hz, 1H), 7.53 (s, 1H), 7.60 (d, *J*=7.93 Hz, 1H).

### EXAMPLE 201

### 1-methyl-N-(3-((4-oxo-3,4,5,6,7,8-hexahydrophthalazin-1-yl)methyl)phenyl)-1H-pyrrole-2-carboxamide

The title compound was prepared according to the procedure for EXAMPLE 136, substituting 1-methyl-1H-pyrrole-2-carboxylic acid for 1-methylcyclopropanecarboxylic acid. MS (DCI/NH₃) m/z 363 (M+H)⁺.

### EXAMPLE 202

### 2,5-dimethyl-N-(3-((4-oxo-3,4,5,6,7,8-hexahydrophthalazin-1-yl)methyl)phenyl)-1H-pyrrole-3-carboxamide

The title compound was prepared according to the procedure for EXAMPLE 136, substituting 2,5-dimethyl-1H-pyrrole-3-carboxylic acid for 1-methylcyclopropanecarboxylic acid. MS (DCI/NH₃) m/z 377 (M+H)⁺.

### EXAMPLE 203

### 1,2,5-trimethyl-N-(3-((4-oxo-3,4,5,6,7,8-hexahydrophthalazin-1-yl)methyl)phenyl)-1H-pyrrole-3-carboxamide

The title compound was prepared according to the procedure for EXAMPLE 136, substituting 1-methyl-1H-pyrrole-3-carboxylic acid for 1-methylcyclopropanecarboxylic acid. MS (DCI/NH₃) m/z 363 (M+H)⁺.

### EXAMPLE 204

### N-(3-((4-oxo-3,4,5,6,7,8-hexahydrophthalazin-1-yl)methyl)phenyl)-1,3-thiazole-2-carboxamide

The title compound was prepared according to the procedure for EXAMPLE 136, substituting thiazole-2-carboxylic acid for 1-methylcyclopropanecarboxylic acid. MS (DCI/NH₃) m/z 367 (M+H)⁺.

### EXAMPLE 205

### N-(3-((4-oxo-3,4,5,6,7,8-hexahydrophthalazin-1-yl)methyl)phenyl)-1,3-thiazole-4-carboxamide

The title compound was prepared according to the procedure for EXAMPLE 136, substituting thiazole-4-carboxylic acid for 1-methylcyclopropanecarboxylic acid. MS (DCI/NH₃) m/z 367 (M+H)⁺.

### EXAMPLE 206

### N-(3-((4-oxo-3,4,5,6,7,8-hexahydrophthalazin-1-yl)methyl)phenyl)-1,3-thiazole-5-carboxamide

The title compound was prepared according to the procedure for EXAMPLE 136, substituting thiazole-5-carboxylic acid for 1-methylcyclopropanecarboxylic acid. MS (DCI/NH₃) m/z 367 (M+H)⁺; ¹H NMR (500 MHz, D₂O/dimethylsulfoxide-*d*₆): δ 1.54 - 1.67 (m, 2H), 2.32 - 2.44 (m, 2H), 3.92 (s, 2H), 7.00 (d, *J*=7.63 Hz, 1H), 7.32 (t, *J*=7.93 Hz, 1H), 7.49 (s, 1H), 7.59 (d, *J*=8.24 Hz, 1H), 8.66 (s, 1H), 9.27 (s, 1H).

### EXAMPLE 208

### N-(3-((4-oxo-3,4,5,6,7,8-hexahydrophthalazin-1-yl)methyl)phenyl)isoxazole-5-carboxamide

The title compound was prepared according to the procedure for EXAMPLE 136, substituting isoxazole-5-carboxylic acid for 1-methylcyclopropanecarboxylic acid. MS (DCI/NH₃) m/z 351 (M+H)⁺; ¹H NMR (500 MHz, D₂O/dimethylsulfoxide-*d*₆): δ 1.54 - 1.68 (m, 4H), 2.33 - 2.45 (m, 4H), 3.93 (s, 2H), 7.03 (d, *J*=7.63 Hz, 1H), 7.22 (d, *J*= 2.14 Hz, 1H), 7.34 (t, *J*=7.93 Hz, 1H), 7.54 (s, 1H), 7.63 (d, *J*=7.93 Hz, 1H), 8.77 (d, *J*=1.83 Hz, 1H).

### EXAMPLE 209

### 3,5-dimethyl-N-(3-((4-oxo-3,4,5,6,7,8-hexahydrophthalazin-1-yl)methyl)phenyl)isoxazole-4-carboxamide

The title compound was prepared according to the procedure for EXAMPLE 136, substituting 3,5-dimethylisoxazole-4-carboxylic acid for 1-methylcyclopropanecarboxylic acid. MS (DCI/NH₃) m/z 379 (M+H)⁺.

### EXAMPLE 210

### N-(3-((4-oxo-3,4,5,6,7,8-hexahydrophthalazin-1-yl)methyl)phenyl)nicotinamide

The title compound was prepared according to the procedure for EXAMPLE 136, substituting nicotinic acid for 1-methylcyclopropanecarboxylic acid. MS (DCI/NH₃) m/z 361 (M+H)⁺.

### EXAMPLE 211

### N-(3-((4-oxo-3,4,5,6,7,8-hexahydrophthalazin-1-yl)methyl)phenyl)isonicotinamide

The title compound was prepared according to the procedure for EXAMPLE 136, substituting isonicotinic acid for 1-methylcyclopropanecarboxylic acid. MS (DCI/NH₃) m/z 361 (M+H)⁺; ¹H NMR (500 MHz, D₂O/dimethylsulfoxide-*d*₆): δ 1.57 - 1.68 (m, 4H), 2.33 - 2.45 (m, 4H), 3.94 (s, 2H), 7.04 (d, *J*=7.63 Hz, 1H), 7.36 (t, *J*=7.78 Hz, 1H), 7.56 (s, 1H), 7.66 (d, *J*=8.24 Hz, 1H), 8.10 (d, *J*=6.41 Hz, 2H), 8.90 (d, *J*=6.10 Hz, 2H).

### EXAMPLE 212

### 3-hydroxy-N-(3-((4-oxo-3,4,5,6,7,8-hexahydrophthalazin-1-yl)methyl)phenyl)pyridine-2-carboxamide

The title compound was prepared according to the procedure for EXAMPLE 136, substituting 3-hydroxypicolinic acid for 1-methylcyclopropanecarboxylic acid. MS (DCI/NH₃) m/z 377 (M+H)⁺.

### EXAMPLE 213

### 2-hydroxy-N-(3-((4-oxo-3,4,5,6,7,8-hexahydrophthalazin-1-yl)methyl)phenyl)nicotinamide

The title compound was prepared according to the procedure for EXAMPLE 136, substituting 2-hydroxynicotinic acid for 1-methylcyclopropanecarboxylic acid. MS (DCI/NH₃) m/z 377 (M+H)⁺.

### EXAMPLE 214

### 6-hydroxy-N-(3-((4-oxo-3,4,5,6,7,8-hexahydrophthalazin-1-yl)methyl)phenyl)nicotinamide

The title compound was prepared according to the procedure for EXAMPLE 136, substituting 6-hydroxynicotinic acid for 1-methylcyclopropanecarboxylic acid. MS (DCI/NH₃) m/z 377 (M+H)⁺; ¹H NMR (500 MHz, D₂O/dimethylsulfoxide-*d*₆): δ 1.53 - 1.65 (m, 4H), 2.32 - 2.43 (m, 4H), 3.91 (s, 2H), 6.45 (d, *J*=10.07 Hz, 1H), 6.95 (d, *J*=7.63 Hz, 1H), 7.29 (t, *J*=7.93 Hz, 1H), 7.46 (s, 1H), 7.57 (d, *J*=8.24 Hz, 1H), 7.98 (dd, *J*=9.76, 2.75 Hz, 1H), 8.16 (d, *J*=2.14 Hz, 1H).

### EXAMPLE 215

### N-(3-((4-oxo-3,4,5,6,7,8-hexahydrophthalazin-1-yl)methyl)phenyl)-2-pyridin-2-ylacetamide

The title compound was prepared according to the procedure for EXAMPLE 136, substituting 2-(pyridin-2-yl)acetic acid for 1-methylcyclopropanecarboxylic acid. MS (DCI/NH₃) m/z 375 (M+H)⁺.

### EXAMPLE 216

### N-(3-((4-oxo-3,4,5,6,7,8-hexahydrophthalazin-1-yl)methyl)phenyl)-2-pyridin-3-ylacetamide

The title compound was prepared according to the procedure for EXAMPLE 136, substituting 2-(pyridin-3-yl)acetic acid for 1-methylcyclopropanecarboxylic acid. MS (DCI/NH₃) m/z 375 (M+H)⁺; ¹H NMR (500 MHz, D₂O/dimethylsulfoxide-*d*₆): δ 1.54 - 1.66 (m, 4H), 2.30 - 2.42 (m, 4H), 3.88 (s, 2H), 3.98 (s, 2H), 6.94 (d, *J*=7.32 Hz, 1H), 7.27 (t, *J*=7.93 Hz, 1H), 7.38 (s, 1H), 7.46 (d, *J*=8.85 Hz, 1H), 8.04 (dd, *J*=7.93, 5.80 Hz, 1H), 8.52 (d, *J*=8.24 Hz, 1H), 8.81 (d, *J*=5.49 Hz, 1H), 8.85 (s, 1H).

### EXAMPLE 217

### 5-methyl-N-(3-((4-oxo-3,4,5,6,7,8-hexahydrophthalazin-1-yl)methyl)phenyl)pyrazine-2-carboxamide

The title compound was prepared according to the procedure for EXAMPLE 136, substituting 5-methylpyrazine-2-carboxylic acid for 1-methylcyclopropanecarboxylic acid. MS (DCI/NH₃) m/z 376 (M+H)⁺; ¹H NMR (500 MHz, D₂O/dimethylsulfoxide-*d*₆): δ 1.54-1.69 (m, 4H), 2.34 - 2.46 (m, 4H), 2.63 (s, 3H), 3.93 (s, 2H), 7.00 (d, *J*=7.63 Hz, 1H), 7.33 (t, *J*=8.09 Hz, 1H), 7.68 - 7.74 (m, 2H), 8.68 (s, 1H), 9.13 (d, *J*=1.22 Hz, 1H).

### EXAMPLE 218

### N-(3-((4-oxo-3,4,5,6,7,8-hexahydrophthalazin-1-yl)methyl)phenyl)-1H-indole-3-carboxamide

The title compound was prepared according to the procedure for EXAMPLE 136, substituting 1H-indole-3-carboxylic acid for 1-methylcyclopropanecarboxylic acid. MS (DCI/NH₃) m/z 399 (M+H)⁺.

### EXAMPLE 219

### 5-methyl-N-(3-((4-oxo-3,4,5,6,7,8-hexahydrophthalazin-1-yl)methyl)phenyl)-1-phenyl-1H-pyrazole-4-carboxamide

The title compound was prepared according to the procedure for EXAMPLE 136, substituting 5-methyl-1-phenyl-1H-pyrazole-4-carboxylic acid for 1-methylcyclopropanecarboxylic acid. MS (DCI/NH₃) m/z 440 (M+H)⁺.

### EXAMPLE 220

### 6-chloro-N-(3-((4-oxo-3,4,5,6,7,8-hexahydrophthalazin-1-yl)methyl)phenyl)-2H-chromene-3-carboxamide

The title compound was prepared according to the procedure for EXAMPLE 136, substituting 6-chloro-2H-chromene-3-carboxylic acid for 1-methylcyclopropanecarboxylic acid. MS (DCI/NH₃) m/z 448 (M+H)⁺.

### EXAMPLE 221

### N³,N³-dimethyl-N¹-(3-((4-oxo-3,4,5,6,7,8-hexahydrophthalazin-1-yl)methyl)phenyl)-beta-alaninamide

The title compound was prepared according to the procedure for EXAMPLE 136, substituting 3-(dimethylamino)propanoic acid for 1-methylcyclopropanecarboxylic acid. MS (DCI/NH₃) m/z 355 (M+H)⁺; ¹H NMR (500 MHz, D₂O/dimethylsulfoxide-*d*₆): δ 1.55 - 1.66 (m, 4H), 2.30 - 2.43 (m, 4H), 2.77 - 2.93 (m, 10H), 3.93 (s, 2H), 6.74 (s, 1H), 6.90 (dd, *J*=8.09, 1.37 Hz, 1H), 7.06 (d, *J*=7.63 Hz, 1H), 7.37 (t, *J*=7.78 Hz, 1H).

### EXAMPLE 222

### 4-(2-(3-bromophenyl)ethyl)-5,6,7,8-tetrahydrophthalazin-1(2H)-one

### EXAMPLE 222A

### 2-(3-bromophenyl)-N-methoxy-N-methylacetamide

To a solution of 2-(3-bromophenyl)acetic acid (4.4 g, 20.56 mmol) in N,N-dimethylformamide (125 ml) was successively added N,O-dimethylhydroxyamine (4.5 g, 46.26 mmol), triethylamine (10 ml), 1-ethyl-3-(3-dimethylaminopropyl) carbodiimide hydrochloride (8.9 g, 46.26 mmol) and 1-hydroxybenzotriazole (6.24 g, 46.26 mmol). The reaction mixture was stirred at room temperature overnight, and partitioned between ethyl acetate and brine. The organic phase was washed with brine, and concentrated. The residue was purified by flash chromatography on silica gel (50% ethyl acetate in hexane) to provide the title compound. MS (DCI/NH₃) m/z 258 (M+H)⁺.

### EXAMPLE 222B

### 2-(3-bromophenyl)acetaldehyde

A solution of EXAMPLE 222A (2.5 g, 9.7 mmol) in anhydrous tetrahydrofuran (50 ml) was treated with LiAlH₄ (0.37 g, 9.7 mmol) at 0°C for 10 minutes, and quenched with water. The mixture was partitioned between ethyl acetate and saturated ammonium chloride. The organic phase was washed with water and concentrated. The residue was purified by flash chromatography on silica gel (20% ethyl acetate in hexane) to provide the title compound. MS (DCI/NH₃) m/z 199 (M+H)⁺.

### EXAMPLE 222C

### 3-(2-(3-bromophenyl)ethylidene)-4,5,6,7-tetrahydroisobenzofuran-1(3H)-one

The title compound was prepared according to the procedure for EXAMPLE 1C, substituting EXAMPLE 222B for 2-fluoro-5-formylbenzonitrile. MS (DCI/NH₃) m/z 319 (M+H)⁺.

### EXAMPLE 222D

### 4-(2-(3-bromophenyl)ethyl)-5,6,7,8-tetrahydrophthalazin-1(2H)-one

The title compound was prepared according to the procedure for EXAMPLE 2C, substituting EXAMPLE 222C for EXAMPLE 2B. MS (DCI/NH₃) m/z 333 (M+H)⁺.

### EXAMPLE 223

### 4-(2-(3-bromo-4-fluorophenyl)ethyl)-5,6,7,8-tetrahydrophthalazin-1(2H)-one

### EXAMPLE 223A

### 2-(3-bromo-4-fluorophenyl)acetaldehyde

The title compound was prepared according to the procedure for EXAMPLE 222, substituting 2-(3-bromo-4-fluorophenyl)acetic acid for 2-(3-bromophenyl)acetic acid in EXAMPLE 223B. MS (DCI/NH₃) m/z 216 (M+H)⁺.

### EXAMPLE 223B

### 4-(2-(3-bromo-4-fluorophenyl)ethyl)-5,6,7,8-tetrahydrophthalazin-1(2H)-one

The title compound was prepared according to the procedure for EXAMPLE 1C, substituting EXAMPLE 223A for 2-fluoro-5-formylbenzonitrile. MS (DCI/NH₃) m/z 351 (M+H)⁺.

### EXAMPLE 224

### 4-(2,2,2-trifluoro-1-phenylethyl)-5,6,7,8-tetrahydrophthalazin-1(2H)-one

### EXAMPLE 224A

### 3-(2,2,2-trifluoro-1-phenylethylidene)-4,5,6,7-tetrahydroisobenzofuran-1(3H)-one

The title compound was prepared according to the procedure for EXAMPLE 1C, substituting 2,2,2-trifluoro-1-phenylethanone for 2-fluoro-5-formylbenzonitrile. MS (DCI/NH₃) m/z 295 (M+H)⁺.

### EXAMPLE 224B

### 4-(2,2,2-trifluoro-l-phenylethyl)-5,6,7,8-tetrahydrophthalazin-1(2H)-one

The title compound was prepared according to the procedure for EXAMPLE 2C, substituting EXAMPLE 224A for EXAMPLE 2B. MS (DCI/NH₃) m/z 309 (M+H)⁺.

### EXAMPLE 225

### 2-hydroxy-4-methyl-N-(3-((4-oxo-3,4,5,6,7,8-hexahydrophthalazin-1-yl)methyl)phenyl)benzamide

The title compound was prepared according to the procedure for EXAMPLE 136, substituting 2-hydroxy-4-methylbenzoic acid for 1-methylcyclopropanecarboxylic acid. MS (DCI/NH₃) m/z 390 (M+H)⁺.

### EXAMPLE 226

### 4-acetyl-N-(3-((4-oxo-3,4,5,6,7,8-hexahydrophthalazin-1-yl)methyl)phenyl)benzamide

The title compound was prepared according to the procedure for EXAMPLE 136, substituting 4-acetylbenzoic acid for 1-methylcyclopropanecarboxylic acid. MS (DCI/NH₃) m/z 402 (M+H)⁺ ; ¹H NMR (500 MHz, D₂O/dimethylsulfoxide-*d*₆): δ 1.54 - 1.69 (m, 4H), 2.34 - 2.46 (m, 4H), 2.64 (s, 3H), 3.93 (s, 2H), 6.99 (d, *J*=7.63 Hz, 1H), 7.32 (t, *J*=7.93 Hz, 1 H), 7.57 (s, 1 H), 7.65 (d, *J*=7.93 Hz, 1 H), 8.01 - 8.05 (m, 2H), 8.05 - 8.10 (m, 2H).

### EXAMPLE 227

### 3-methoxy-4-methyl-N-(3-((4-oxo-3,4,5,6,7,8-hexahydrophthalazin-1-yl)methyl)phenyl)benzamide

The title compound was prepared according to the procedure for EXAMPLE 136, substituting 3-methoxy-4-methylbenzoic acid for 1-methylcyclopropanecarboxylic acid. MS (DCI/NH₃) m/z 404 (M+H)⁺.

### EXAMPLE 228

### 4-ethoxy-N-(3-((4-oxo-3,4,5,6,7,8-hexahydrophthalazin-1-yl)methyl)phenyl)benzamide

The title compound was prepared according to the procedure for EXAMPLE 136, substituting 4-ethoxybenzoic acid for 1-methylcyclopropanecarboxylic acid. MS (DCI/NH₃) m/z 404 (M+H)⁺.

### EXAMPLE 229

### 3-fluoro-4-methoxy-N-(3-((4-oxo-3,4,5,6,7,8-hexahydrophthalazin-1-yl)methyl)phenyl)benzamide

The title compound was prepared according to the procedure for EXAMPLE 136, substituting 3-fluoro-4-methoxybenzoic acid for 1-methylcyclopropanecarboxylic acid. MS (DCI/NH₃) m/z 408 (M+H)⁺.

### EXAMPLE 230

### N-(3-((4-oxo-3,4,5,6,7,8-hexahydrophthalazin-1-yl)methyl)phenyl)-1-naphthamide

The title compound was prepared according to the procedure for EXAMPLE 136, substituting 1-naphthoic acid for 1-methylcyclopropanecarboxylic acid. MS (DCI/NH₃) m/z 410 (M+H)⁺.

### EXAMPLE 231

### N-(3-((4-oxo-3,4,5,6,7,8-hexahydrophthalazin-1-yl)methyl)phenyl)-2-naphthamide

The title compound was prepared according to the procedure for EXAMPLE 136, substituting 2-naphthoic acid for 1-methylcyclopropanecarboxylic acid. MS (DCI/NH₃) m/z 410 (M+H)⁺.

### EXAMPLE 232

### 5-chloro-2-hydroxy-N-(3-((4-oxo-3,4,5,6,7,8-hexahydrophthalazin-1-yl)methyl)phenyl)benzamide

The title compound was prepared according to the procedure for EXAMPLE 136, substituting 2-hydroxy-5-methylbenzoic acid for 1-methylcyclopropanecarboxylic acid. MS (DCI/NH₃) m/z 390 (M+H)⁺.

### EXAMPLE 233

### 4-tert-butyl-N-(3-((4-oxo-3,4,5,6,7,8-hexahydrophthalazin-1-yl)methyl)phenyl)benzamide

The title compound was prepared according to the procedure for EXAMPLE 136, substituting 4-tert-butylbenzoic acid for 1-methylcyclopropanecarboxylic acid. MS (DCI/NH₃) m/z 416 (M+H)⁺.

### EXAMPLE 234

### 4-(acetylamino)-N-(3-((4-oxo-3,4,5,6,7,8-hexahydrophthalazin-1-yl)methyl)phenyl)benzamide

The title compound was prepared according to the procedure for EXAMPLE 136, substituting 4-acetamidobenzoic acid for 1-methylcyclopropanecarboxylic acid. MS (DCI/NH₃) m/z 417 (M+H)⁺; ¹H NMR (500 MHz, D₂O/dimethylsulfoxide-*d*₆): δ 1.53 - 1.68 (m, 4H), 2.10 (s, 3H), 2.33 - 2.44 (m, 4H), 3.92 (s, 2H), 6.95 (d, *J*=7.93 Hz, 1H), 7.30 (t, *J*=7.93 Hz, 1H), 7.56 (s, 1H), 7.63 (d, *J*=7.63 Hz, 1H), 7.70 (d, *J*=8.85 Hz, 2H), 7.90 (d, *J*=8.85 Hz, 2H).

### EXAMPLE 235

### N-(3-((4-oxo-3,4,5,6,7,8-hexahydrophthalazin-1-yl)methyl)phenyl)-4-propoxybenzamide

The title compound was prepared according to the procedure for EXAMPLE 136, substituting 4-propoxybenzoic acid for 1-methylcyclopropanecarboxylic acid. MS (DCI/NH₃) m/z 418 (M+H)⁺.

### EXAMPLE 236

### 1-hydroxy-N-(3-((4-oxo-3,4,5,6,7,8-hexahydrophthalazin-1-yl)methyl)phenyl)-2-naphthamide

The title compound was prepared according to the procedure for EXAMPLE 136, substituting 1-hydroxy-2-naphthoic acid for 1-methylcyclopropanecarboxylic acid. MS (DCI/NH₃) m/z 426 (M+H)⁺.

### EXAMPLE 237

### 2-chloro-5-(methylthio)-N-(3-((4-oxo-3,4,5,6,7,8-hexahydrophthalazin-1-yl)methyl)phenyl)benzamide

The title compound was prepared according to the procedure for EXAMPLE 136, substituting 2-chloro-5-(methylthio)benzoic acid for 1-methylcyclopropanecarboxylic acid. MS (DCI/NH₃) m/z 440 (M+H)⁺.

### EXAMPLE 238

### 3,4-diethoxy-N-(3-((4-oxo-3,4,5,6,7,8-hexahydrophthalazin-1-yl)methyl)phenyl)benzamide

The title compound was prepared according to the procedure for EXAMPLE 136, substituting 3,4-diethoxybenzoic acid for 1-methylcyclopropanecarboxylic acid. MS (DCI/NH₃) m/z 448 (M+H)⁺.

### EXAMPLE 239

### 2-benzyl-N-(3-((4-oxo-3,4,5,6,7,8-hexahydrophthalazin-1-yl)methyl)phenyl)benzamide

The title compound was prepared according to the procedure for EXAMPLE 136, substituting 2-benzylbenzoic acid for 1-methylcyclopropanecarboxylic acid. MS (DCI/NH₃) m/z 450 (M+H)⁺.

### EXAMPLE 240

### 2-anilino-N-(3-((4-oxo-3,4,5,6,7,8-hexahydrophthalazin-1-yl)methyl)phenyl)benzamide

The title compound was prepared as a trifluoroacetic acid salt according to the procedure for EXAMPLE 136, substituting 2-(phenylamino)benzoic acid for 1-methylcyclopropanecarboxylic acid. MS (DCI/NH₃) m/z 451 (M+H)⁺; ¹H NMR (500 MHz, D₂O/dimethylsulfoxide-*d*₆): δ 1.55 - 1.68 (m, 4H), 2.31 - 2.45 (m, 4H), 3.91 (s, 2H), 6.91 - 6.99 (m, 3H), 7.13 (d, *J*=7.63 Hz, 2H), 7.27 - 7.34 (m, 4H), 7.38 - 7.42 (m, 1H), 7.49 (s, 1H), 7.58 (d, *J*=8.85 Hz, 1H), 7.71 - 7.75 (m, 1H).

### EXAMPLE 241

### 2-benzoyl-N-(3-((4-oxo-3,4,5,6,7,8-hexahydrophthalazin-1-yl)methyl)phenyl)benzamide

The title compound was prepared as according to the procedure for EXAMPLE 136, substituting 2-benzoylbenzoic acid for 1-methylcyclopropanecarboxylic acid. MS (DCI/NH₃) m/z 464 (M+H)⁺.

### EXAMPLE 242

### N-(3-((4-oxo-3,4,5,6,7,8-hexahydrophthalazin-1-yl)methyl)phenyl)-2-(2-phenylethyl)benzamide

The title compound was prepared according to the procedure for EXAMPLE 136, substituting 2-phenethylbenzoic acid for 1-methylcyclopropanecarboxylic acid. MS (DCI/NH₃) m/z 464 (M+H)⁺.

### EXAMPLE 243

### 5-bromo-2-chloro-N-(3-((4-oxo-3,4,5,6,7,8-hexahydrophthalazin-1-yl)methyl)phenyl)benzamide

The title compound was prepared according to the procedure for EXAMPLE 136, substituting 5-bromo-2-chlorobenzoic acid for 1-methylcyclopropanecarboxylic acid. MS (DCI/NH₃) m/z 472 (M+H)⁺.

### EXAMPLE 244

### 2-(4-methylbenzoyl)-N-(3-((4-oxo-3,4,5,6,7,8-hexahydrophthalazin-1-yl)methyl)phenyl)benzamide

The title compound was prepared according to the procedure for EXAMPLE 136, substituting 2-(4-methylbenzoyl)benzoic acid for 1-methylcyclopropanecarboxylic acid. MS (DCI/NH₃) m/z 478 (M+H)⁺.

### EXAMPLE 245

### 2-iodo-N-(3-((4-oxo-3,4,5,6,7,8-hexahydrophthalazin-1-yl)methyl)phenyl)benzamide

The title compound was prepared according to the procedure for EXAMPLE 136, substituting 2-iodobenzoic acid for 1-methylcyclopropanecarboxylic acid. MS (DCI/NH₃) m/z 486 (M+H)⁺.

### EXAMPLE 246

### 3-iodo-N-(3-((4-oxo-3,4,5,6,7,8-hexahydrophthalazin-1-yl)methyl)phenyl)benzamide

The title compound was prepared according to the procedure for EXAMPLE 136, substituting 3-iodobenzoic acid for 1-methylcyclopropanecarboxylic acid. MS (DCI/NH₃) m/z 486 (M+H)⁺.

### EXAMPLE 247

### 4-iodo-N-(3-((4-oxo-3,4,5,6,7,8-hexahydrophthalazin-1-yl)methyl)phenyl)benzamide

The title compound was prepared according to the procedure for EXAMPLE 136, substituting 4-iodobenzoic acid for 1-methylcyclopropanecarboxylic acid. MS (DCI/NH₃) m/z 486 (M+H)⁺.

### EXAMPLE 248

### N-(2'-fluoro-5'-((4-oxo-3,4,5,6,7,8-hexahydrophthalazin-1-yl)methyl)-1,1'-biphenyl-3-yl)acetamide

The title compound was prepared as a free base according to the procedure for EXAMPLE 39, substituting 3-acetamidophenylboronic acid for 3-pyridineboronic acid, but eliminating the last HCl salt formation step. MS (DCI/NH₃) m/z 392 (M+H)⁺; ¹H NMR (500 MHz, dimethylsulfoxide-*d*₆): δ 1.58 - 1.67 (m, 4H), 2.05 (s, 3H), 2.33 - 2.39 (m, 2H), 2.40 - 2.46 (m, 2H), 3.95 (s, 2H), 7.16 (d, *J*=7.02 Hz, 1H), 7.18 - 7.21 (m, 1H), 7.22 - 7.27 (m, 1H), 7.30 (dd, *J*=7.63, 2.14 Hz, 1H), 7.38 (t, *J*=7.93 Hz, 1H), 7.59 (d, *J*=7.32 Hz, 1H), 7.76 (s, 1H), 10.04 (br s, 1H), 12.61 (br s, 1H).

### EXAMPLE 249

### 4-((6-fluoro-3'-(methylsulfonyl)-1,1'-biphenyl-3-yl)methyl)-5,6,7,8-tetrahydrophthalazin-1(2H)-one

The title compound was prepared as free base according to the procedure for EXAMPLE 39, substituting 3-(methylsulfonyl)phenylboronic acid for 3-pyridineboronic acid, but eliminating the last HCl salt formation step. MS (DCI/NH₃) m/z 413 (M+H)⁺; ¹H NMR (500 MHz, dimethylsulfoxide-*d*₆): δ 1.57 - 1.73 (m, 4H), 2.34 - 2.41 (m, 2H), 2.41-2.48 (m, 2H), 3.28 (s, 3H), 3.98 (s, 2H), 7.24 - 7.28 (m, 1H), 7.28 - 7.33 (m, 1H), 7.47 (dd, *J*=7.63, 2.14 Hz, 1H), 7.77 (t, *J*=7.78 Hz, 1H), 7.90 (d, *J*=7.93 Hz, 1H), 7.96 - 8.00 (m, 1H), 8.04 (s, 1H), 12.61 (br s, 1H).

### EXAMPLE 250

### 4-((6-fluoro-3'-(pyrrolidin-1-ylcarbonyl)-1,1'-biphenyl-3-yl)methyl)-5,6,7,8-tetrahydrophthalazin-1(2H)-one

The title compound was prepared as free base according to the procedure for EXAMPLE 39, substituting 3-(pyrrolidine-1-carbonyl)phenylboronic acid for 3-pyridineboronic acid, but eliminating the last HCl salt formation step. MS (DCI/NH₃) m/z 432 (M+H)⁺.

### EXAMPLE 251

### 4-((6-fluoro-4'-(pyrrolidin-1-ylcarbonyl)-1,1'-biphenyl-3-yl)methyl)-5,6,7,8-tetrahydrophthalazin-1(2H)-one

The title compound was prepared as free base according to the procedure for EXAMPLE 39, substituting 4-(pyrrolidine-1-carbonyl)phenylboronic acid for 3-pyridineboronic acid, but eliminating the last HCl salt formation step. MS (DCI/NH₃) m/z 432 (M+H)⁺; ¹H NMR (500 MHz, dimethylsulfoxide-*d*₆): δ 1.57 - 1.68 (m, 4H), 1.78 - 1.93 (m, 4H), 2.32 - 2.39 (m, 2H), 2.40 - 2.47 (m, 2H), 3.39 - 3.53 (m, 4H), 3.95 (s, 2H), 7.21-7.24 (m, 1H), 7.24 - 7.31 (m, 1H), 7.39 (dd, *J*=7.63, 1.86 Hz, 1H), 7.55 - 7.59 (m, 2H), 7.60-7.64 (m, 2H), 12.60 (br s, 1H).

### EXAMPLE 252

### 2'-fluoro-5'-((4-oxo-3,4,5,6,7,8-hexahydrophthalazin-1-yl)methyl)-1,1'-biphenyl-3-carboxamide

The title compound was prepared as free base according to the procedure for EXAMPLE 39, substituting 3-carbamoylphenylboronic acid for 3-pyridineboronic acid, but eliminating the last HCl salt formation step. MS (DCl/NH₃) m/z 378 (M+H)⁺; ¹H NMR (500 MHz, dimethylsulfoxide-*d*₆): δ 1.55 - 1.72 (m, 4H), 2.33 - 2.41 (m, 2H), 2.41 - 2.47 (m, 2H), 3.97 (s, 2H), 7.19 - 7.24 (m, 1H), 7.24 - 7.30 (m, 1H), 7.42 (dd, *J*=7.63, 2.14 Hz, 1H), 7.44 (s, 1H), 7.56 (t, *J*=7.78 Hz, 1H), 7.68 (d, *J*=7.63 Hz, 1H), 7.88 - 7.92 (m, 1H), 8.02 (s, 1H), 8.07 (s, 1H), 12.61 (s, 1H).

### EXAMPLE 253

### 2'-fluoro-N,N-dimethyl-5'-((4-oxo-3,4,5,6,7,8-hexahydrophthalazin-1-yl)methyl)-1,1'-biphenyl-4-carboxamide

The title compound was prepared as free base according to the procedure for EXAMPLE 39, substituting 4-(dimethylcarbamoyl)phenylboronic acid for 3-pyridineboronic acid, but eliminating the last HCl salt formation step. MS (DCI/NH₃) m/z 406 (M+H)⁺. ¹H NMR (500 MHz, dimethylsulfoxide-*d*₆): δ 1.56 - 1.69 (m, 4H), 2.31 - 2.40 (m, 2H), 2.40-2.47 (m, 2H), 2.95 (s, 3H), 3.00 (s, 3H), 3.96 (s, 2H), 7.20 - 7.24 (m, 1H), 7.24 - 7.30 (m, 1H), 7.40 (dd, *J*=7.48, 1.98 Hz, 1H), 7.49 - 7.52 (m, 1H), 7.56 - 7.59 (m, 2H), 7.60 - 7.65 (m, 1 H), 12.61 (br s, 1H).

### EXAMPLE 254

### 4-(3,3,3-trifluoro-2-phenylpropyl)-5,6,7,8-tetrahydrophthalazin-1(2H)-one

### EXAMPLE 254A

### 3-(1,1,1-trifluoro-3-phenylpropan-2-ylidene)-4,5,6,7-tetrahydroisobenzofuran-1(3H)-one

The title compound was prepared according to the procedure for EXAMPLE 1C, substituting 1,1,1-trifluoro-3-phenylpropan-2-one for 2-fluoro-5-formylbenzonitrile. MS (DCI/NH₃) ml) 309 (M+H)⁺.

### EXAMPLE 254B

### 4-(3,3,3-trifluoro-2-phenylpropyl)-5,6,7,8-tetrahydrophthalazin-1(2H)-one

The title compound was prepared according to the procedure for EXAMPLE 2C, substituting EXAMPLE 254A for EXAMPLE 2B. MS (DCI/NH₃) m/z 323 (M+H)⁺.

### EXAMPLE 255

### 4-(2-phenylethyl)-5,6,7,8-tetrahydrophthalazin-1(2H)-one

The title compound was prepared as a side product according to the procedure for EXAMPLE 101, substituting EXAMPLE 222 for EXAMPLE 103. MS (DCI/NH₃) m/z 255 (M+H)⁺.

### EXAMPLE 256

### 4-(2-(3-bromophenyl)propyl)-5,6,7,8-tetrahydrophthalazin-1(2H)-one

### EXAMPLE 256A

### 2-(3-bromophenyl)-N-methoxy-N-methylpropanamide

A solution of EXAMPLE 222A (3.5 g, 13.56 mmol) in anhydrous tetrahydrofuran (50 ml) was treated with 1N sodium dicyanamide v solution in tetrahydrofuran (16 ml, 16.27 mmol) at - 78 °C for 1 hour. Iodomethane (3.85 g, 27.1 mmol) was added through a syringe, and the mixture was allowed to warm up to room temperature for 2 hours. The mixture was concentrated, and the residue was partitioned between ethyl acetate and brine. The organic phase was concentrated, the residue was purified by flash column chromatography (30% ethyl acetate in hexane) to provide the title compound. MS (DCI/NH₃) m/z 273 (M+H)⁺.

### EXAMPLE 256B

### 2-(3-bromophenyl)propanal

The title compound was prepared according to the procedure for EXAMPLE 222B, substituting EXAMPLE 256A for EXAMPLE 222A. MS (DCI/NH₃) m/z 214 (M+H)⁺.

### EXAMPLE 256C

### 3-(2-(3-bromophenyl)propylidene)-4,5,6,7-tetrahydroisobenzofuran-1(3H)-one

The title compound was prepared according to the procedure for EXAMPLE 1C, substituting 256B for 2-fluoro-5-formylbenzonitrile. MS (DCI/NH₃) m/z 334 (M+H)⁺.

### EXAMPLE 256D

### 4-(2-(3-bromophenyl)propyl)-5,6,7,8-tetrahydrophthalazin-1(2H)-one

The title compound was prepared according to the procedure for EXAMPLE 2C, substituting EXAMPLE 256C for EXAMPLE 2B. MS (DCI/NH₃) m/z 348 (M+H)⁺.

### EXAMPLE 257

### tert-butyl 2-((4-oxo-3,4,5,6,7,8-hexahydrophthalazin-1-yl)methyl)piperazine-1-carboxylate

### EXAMPLE 257A

### 4-benzyl 1-tert-butyl 2-(methoxy(methyl)carbamoyl)piperazine-1,4-dicarboxylate

The title compound was prepared according to the procedure for EXAMPLE 222A, substituting 4-(benzyloxycarbonyl)-1-(tert-butoxycarbonyl)piperazine-2-carboxylic acid for 2-(3-bromophenyl)acetic acid. MS (DCI/NH₃) m/z 408 (M+H)⁺.

### EXAMPLE 257B

### 4-benzyl 1-tert-butyl 2-formylpiperazine-1,4-dicarboxylate

The title compound was prepared according to the procedure for EXAMPLE 222B, substituting EXAMPLE 257A for EXAMPLE 222A. MS (DCI/NH₃) m/z 349 (M+H)⁺.

### EXAMPLE 257C

### 4-benzyl 1-tert-butyl 2-((3-oxo-4,5,6,7-tetrahydroisobenzofuran-1(3H)-ylidene)methyl)piperazine-1,4-dicarboxylate

The title compound was prepared according to the procedure for EXAMPLE 1C, substituting EXAMPLE 257B for 2-fluoro-5-formylbenzonitrile. MS (DCI/NH₃) m/z 469 (M+H)⁺.

### EXAMPLE 257D

### 4-benzyl 1-tert-butyl 2-((4-oxo-3,4,5,6,7,8-hexahydrophthalazin-1-yl)methyl)piperazine-1,4-dicarboxylate

The title compound was prepared according to the procedure for EXAMPLE 2C, substituting EXAMPLE 257C for EXAMPLE 2B. MS (DCI/NH₃) m/z 483 (M+H)⁺.

### EXAMPLE 257E

### tert-butyl 2-((4-oxo-3,4,5,6,7,8-hexahydrophthalazin-1-yl)methyl)piperazine-1-carboxylate

A solution of EXAMPLE 257D (0.77 g, 1.6 mmol) in tetrahydrofuran (100 ml) was treated with 10% palladium on carbon (85 mg, 0.8 mmol) at room temperature under hydrogen (balloon) overnight. The catalyst was removed by filtration, and the filtrate was concentrated. The residue was purified by flash chromatography (0-15% gradient of methanol in CH₂Cl₂) to provide the title compound. MS (DCI/NH₃) m/z 349 (M+H)⁺.

### EXAMPLE 258

### 4-benzyl 1-tert-butyl 2-((4-oxo-3,4,5,6,7,8-hexahydrophthalazin-1-yl)methyl)piperazine-1,4-dicarboxylate

The title compound was prepared as described in EXAMPLE 257D. MS (DCI/NH₃) m/z 483 (M+H)⁺.

### EXAMPLE 259

### 4-(2-(3-nitrophenyl)ethyl)-5,6,7,8-tetrahydrophthalazin-1(2H)-one

### EXAMPLE 259A

### N-methoxy-N-methyl-2-(3-nitrophenyl)acetamide

The title compound was prepared according to the procedure for EXAMPLE 222A, substituting 3-nitrobenzoic acid for 2-(3-bromophenyl)acetic acid. MS (DCI/NH₃) m/z 225 (M+H)⁺.

### EXAMPLE 259B

### 2-(3-nitrophenyl)acetaldehyde

The title compound was prepared according to the procedure for EXAMPLE 222B, substituting EXAMPLE 259A for EXAMPLE 222A.

### EXAMPLE 259C

### 3-(2-(3-nitrophenyl)ethylidene)-4,5,6,7-tetrahydroisobenzofuran-1(3H)-one

The title compound was prepared according to the procedure for EXAMPLE 1C, substituting EXAMPLE 259B for 2-fluoro-5-formylbenzonitrile. MS (DCI/NH₃) m/z 286 (M+H)⁺.

### EXAMPLE 259D

### 4-(2-(3-nitrophenyl)ethyl)-5,6,7,8-tetrahydrophthalazin-1(2H)-one

The title compound was prepared according to the procedure for EXAMPLE 2C, substituting EXAMPLE 259C for EXAMPLE 2B. MS (DCI/NH₃) m/z 300 (M+H)⁺.

### EXAMPLE 260

### 4-(2-(3-aminophenyl)ethyl)-5,6,7,8-tetrahydrophthalazin-1(2H)-one

A suspension of EXAMPLE 259 (110 mg, 0.17 mmol) in methanol (20 ml) was treated with Raney Nickel (20 mg) at room temperature under hydrogen (balloon) overnight. The solid material was filtered off, and the filtrate was concentrated to give the title compound. MS (DCI/NH₃) m/z 270 (M+H)⁺.

### EXAMPLE 261

### 4-(piperazin-2-ylmethyl)-5,6,7,8-tetrahydrophthalazin-1(2H)-one

A solution of EXAMPLE 258 (35 mg, 0.1 mmol) in trifluoroacetic acid (5 ml) was stirred at room temperature for 1 hour, and was concentrated. The residue was purified by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 249 (M+H)⁺.

### EXAMPLE 262

### 4-(2-(3-(2-oxopyrrolidin-1-yl)phenyl)ethyl)-5,6,7,8-tetrahydrophthalazin-1(2H)-one

To a solution of EXAMPLE 260 (100 mg, 0.37 mmol) in methylene chloride (5 mL) was added 4-chlorobutyrlchloride (52.3 mg, 0.37 mmol) and triethylamine (0.12 mL, 0.45 mmol). The mixture was stirred at room temperature overnight, and was concentrated. The residue was dissolved in absolute ethanol (5 mL), and was treated with sodium ethoxide (0.2 mL, 21 wt% in ethanol) at room temperature for 16 hours. 1 mL of 2N HCl was added, and the mixture was concentrated. The residue was separated by HPLC (Zorbax® C-18 ODS packing material [Agilent Technologies, Santa Clara, CA], 0.1 % trifluoroacetic acid/CH₃CN/H₂O) to provide the title compound as a trifluoroacetic acid salt. MS (DCI/NH₃) m/z 338 (M+H)⁺.

### EXAMPLE 263

### N-(3-(2-(4-oxo-3,4,5,6,7,8-hexahydrophthalazin-1-yl)ethyl)phenyl)-2-phenoxyacetamide

A solution of 2-phenoxyacetic acid (28 mg, o.186 mmol) in anhydrous dichloromethane (3 ml) was treated with oxalyl chloride (35.3 mg, 0.186 mmol) and a drop of N,N-dimethylformamide at room temperature for 1 hour, and was concentrated. The residue was re-dissolved in anhydrous dichloromethane (5 ml). A suspension of EXAMPLE 260 (50 mg, 0.186 mmol) in anhydrous tetrahydrofuran (2 ml) was then added. The reaction mixture was stirred at room temperature overnight, and was concentrated. The residue was purified by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 404 (M+H)⁺; ¹H NMR (300 MHz, dimethylsulfoxide-*d*₆): δ 1.60-1.70 (m, 4 H), 2.35 - 2.39 (m, 2 H), 2.42 - 2.50 (m, 2 H), 2.66 - 2.93 (m, 4 H), 4.68 (s, 2 H), 6.82 - 7.09 (m, 4 H), 7.23 (t, *J*=7.80 Hz, 1 H), 7.24 - 7.38 (m, 2 H), 7.40 - 7.60 (m, 2 H), 10.01 (s, 1 H) 12.54 (s, 1 H).

### EXAMPLE 264

### 4-(2-(6-fluoro-3'-(morpholin-4-ylcarbonyl)-1,1'-biphenyl-3-yl)ethyl)-5,6,7,8-tetrahydrophthalazin-1(2H)-one

A microwave vial charged with EXAMPLE 223 (50 mg, 0.14 mmol), dichlorobis(triphenylphosphine)palladium (II) (10 mg, 0.014 mmol), 3-(morpholine-4-carbonyl)phenylboronic acid (40 mg, 0.17 mmol), a mixture of DME(7)/water(3) /ethanol(2) (3 ml), and sodium carbonate solution (2M, 0.1 ml) was heated in a CEM Explorer® microwave reactor (Matthews, NC) at 150 °C for 15 minutes. After cooling, the reaction mixture was diluted with methanol (20 ml), and filtered. The filtrate was concentrated, and the residue was separated by HPLC (Zorbax® C-18 ODS packing material [Agilent Technologies, Santa Clara, CA], 0.1% trifluoroacetic acid/CH₃CN/H₂O) to provide the title compound. MS (DCI/NH₃) m/z 462 (M+H)⁺; ¹H NMR (300 MHz, dimethylsulfoxide-*d*₆): δ 1.60 - 1.67 (m, 4 H), 2.35 - 2.39 (m, 2 H), 2.44 - 2.50 (m, 2 H), 2.75 - 3.01 (m, 4 H), 3.44 - 3.73 (m, 8 H), 7.17 - 7.28 (m, 1 H), 7.27 - 7.34 (m, 1 H), 7.38 - 7.47 (m, 1 H), 7.50 - 7.67 (m, 4 H), 12.55 (s, 1 H).

### EXAMPLE 265

### methyl 3-(2-(4-oxo-3,4,5,6,7,8-hexahydrophthalazin-1-yl)ethyl)benzoate

The title compound was prepared according to the procedure for EXAMPLE 66, substituting EXAMPLE 222 for EXAMPLE 66B. MS (DCI/NH₃) m/z 313 (M+H)⁺.

### EXAMPLE 266

### methyl 3-(1-methyl-2-(4-oxo-3,4,5,6,7,8-hexahydrophthalazin-1-yl)ethyl)benzoate

The title compound was prepared according to the procedure for EXAMPLE 66, substituting EXAMPLE 256 for EXAMPLE 66B. MS (DCI/NH₃) m/z 237 (M+H)⁺.

### EXAMPLE 267

### 4-(2-(6-fluoro-4'-(morpholin-4-ylcarbonyl)-1,1'-biphenyl-3-yl)ethyl)-5,6,7,8-tetrahydrophthalazin-1(2H)-one

The title compound was prepared according to the procedure for EXAMPLE 264, substituting 4-(morpholine-4-carbonyl)phenylboronic acid for 3-(morpholine-4-carbonyl)phenylboronic acid. MS (DCI/NH₃) m/z 462 (M+H)⁺.

### EXAMPLE 268

### 4-(2-(6-fluoro-2'-(pyrrolidin-1-ylcarbonyl)-1,1'-biphenyl-3-yl)ethyl)-5,6,7,8-tetrahydrophthalazin-1(2H)-one

The title compound was prepared according to the procedure for EXAMPLE 264 substituting 2-(pyrrolidine-1-carbonyl)phenylboronic acid for 3-(morpholine-4-carbonyl)phenylboronic acid. MS (DCI/NH₃) m/z 446 (M+H)⁺.

### EXAMPLE 269

### 4-(2-(6-fluoro-3'-(pyrrolidin-1-ylcarbonyl)-1,1'-biphenyl-3-yl)ethyl)-5,6,7,8-tetrahydrophthalazin-1(2H)-one

The title compound was prepared according to the procedure for EXAMPLE 264, substituting 3-(pyrrolidine-1-carbonyl)phenylboronic acid for 3-(morpholine-4-carbonyl)phenylboronic acid. MS (DCI/NH₃) m/z 446 (M+H)⁺; ¹H NMR (300 MHz, CDCl₃): δ 1.64 - 1.81 (m, 4 H), 1.83 - 2.03 (m, 4 H), 2.43 - 2.47 (m, 2 H), 2.56 - 2.59 (m, 2 H), 2.76 - 2.88 (m, 2 H), 2.93 - 3.06 (m, 2 H), 3.48 (t, *J*=6.54 Hz, 2 H), 3.67 (t, *J*=6.74 Hz, 2 H), 7.01 - 7.11 (m, 1 H), 7.11 - 7.21 (m, 1 H), 7.29 (dd, *J*=7.54, 2.38 Hz, 1 H), 7.39 - 7.54 (m, 2 H), 7.55 - 7.62 (m, 1 H), 7.69 (s, 1 H), 10.10 (s, 1 H).

### EXAMPLE 270

### N-cyclopropyl-2'-fluoro-5'-(2-(4-oxo-3,4,5,6,7,8-hexahydrophthalazin-1-yl)ethyl)-1,1'-biphenyl-3-carboxamide

The title compound was prepared according to the procedure for EXAMPLE 264, substituting 3-(cyclopropylcarbamoyl)phenylboronic acid for 3-(morpholine-4-carbonyl)phenylboronic acid. MS (DCI/NH₃) m/z 432 (M+H)⁺.

### EXAMPLE 271

### N-(2-(dimethylamino)ethyl)-2'-fluoro-5'-(2-(4-oxo-3,4,5,6,7,8-hexahydrophthalazin-1-yl)ethyl)-1,1'-biphenyl-3-carboxamide

The title compound was prepared according to the procedure for EXAMPLE 264, substituting 3-(2-(dimethylamino)ethylcarbamoyl)phenylboronic acid for 3-(morpholine-4-carbonyl)-phenylboronic acid. MS (DCI/NH₃) m/z 463 (M+H)⁺; ¹H NMR (300 MHz, CDCl₃): δ 2.28 - 2.39 (m, 2 H), 2.35 (m, 3 H), 2.45 (s, 6 H), 2.60 - 2.69 (m, 2 H), 2.73 - 2.82 (m, 2 H), 2.87 (t, *J*=7.14 Hz, 2 H), 3.01 (t, *J*=7.14 Hz, 2 H), 3.54 - 3.64 (m, 1 H), 3.69 (q, *J*=5.29 Hz, 2 H), 6.95 - 7.10 (m, 1 H), 7.10 - 7.20 (m, 1 H), 7.35 - 7.53 (m, 2 H), 7.65 - 7.80 (m, 2 H), 7.79 - 7.88 (m, 1 H).

### EXAMPLE 272

### 2'-fluoro-5'-(2-(4-oxo-3,4,5,6,7,8-hexahydrophthalazin-1-yl)ethyl)-1,1'-biphenyl-3-carboxamide

The title compound was prepared according to the procedure for EXAMPLE 264, substituting 3-carbamoylphenylboronic acid for 3-(morpholine-4-carbonyl)phenylboronic acid. MS (DCI/NH₃) m/z 392 (M+H)⁺; ¹H NMR (500 MHz, pyridine-*d₅*): δ 1.54 (s, 4 H), 2.27 (s, 2 H), 2.70 (s, 2 H), 2.76 - 2.95 (m, 2 H), 2.98 - 3.21 (m, 2 H), 7.19 - 7.27 (m, 2 H), 7.31 (s, 1 H), 7.49 (d, *J*=7.02 Hz, 1 H), 7.82 (d, *J*=7.32 Hz, 1 H), 8.42 (d, *J*=7.63 Hz, 1 H), 8.47 (s, 1 H), 8.68 (s, 1 H), 9.02 (s, 1 H), 14.05 (s, 1 H).

### EXAMPLE 273

### N-(2'-fluoro-5'-(2-(4-oxo-3,4,5,6,7,8-hexahydrophthalazin-1-yl)ethyl)-1,1'-biphenyl-3-yl)methanesulfonamide

The title compound was prepared according to the procedure for EXAMPLE 264, substituting 3-(methylsulfonamido)phenylboronic acid for 3-(morpholine-4-carbonyl)phenylboronic acid. MS (DCI/NH₃) m/z 442 (M+H)⁺; ¹H NMR (300 MHz, CDCl₃): δ 2.31 - 2.48 (m, 4 H), 2.59 (m, 4 H), 2.77 - 2.96 (m, 4 H), 3.02 (t, *J*=7.80 Hz, 3 H), 6.92 - 7.03 (m, 1 H), 7.02 - 7.12 (m, 1 H), 7.10 - 7.22 (m, 2 H), 7.27 - 7.33 (m, 1 H), 7.32-7.47 (m, 2 H), 10.96 (s, 1 H).

### EXAMPLE 274

### N-(2'-fluoro-5'-(2-(4-oxo-3,4,5,6,7,8-hexahydrophthalazin-1-yl)ethyl)-1,1'-biphenyl-3-yl)acetamide

The title compound was prepared according to the procedure for EXAMPLE 264, substituting 3-acetamidophenylboronic acid for 3-(morpholine-4-carbonyl)phenylboronic acid. MS (DCI/NH₃) m/z 406 (M+H)⁺; ¹H NMR (300 MHz, CDCl₃): δ 2.06 - 2.21 (m, 4 H), 2.25 (s, 3 H), 2.34 (m, 2 H), 2.58 (m, 2 H), 2.80 - 2.94 (m, 2 H), 2.92 - 3.06 (m, 2 H), 6.93-7.10 (m, 2 H), 7.10 - 7.19 (m, 1 H), 7.23 (d, *J*=4.36 Hz, 1 H), 7.27 - 7.33 (m, 1 H), 7.38 (t, *J*=7.73 Hz, 1 H), 7.67 - 7.76 (m, 1 H), 11.25 (s, 1 H).

### EXAMPLE 275

### 4-(2-(6-fluoro-3'-(morpholin-4-ylcarbonyl)-1,1'-biphenyl-3-yl)propyl)-5,6,7,8-tetrahydrophthalazin-1(2H)-one

The title compound was prepared according to the procedure for EXAMPLE 264 substituting EXAMPLE 293 for EXAMPLE 223. MS (DCI/NH₃) m/z 476 (M+H)⁺.

### EXAMPLE 276

### 4-(2-(6-fluoro-3'-(pyrrolidin-1-ylcarbonyl)-1,1'-biphenyl-3-yl)propyl)-5,6,7,8-tetrahydrophthalazin-1(2H)-one

The title compound was prepared according to the procedure for EXAMPLE 264 substituting EXAMPLE 293 for EXAMPLE 223, and 3-(pyrrolidine-1-carbonyl)phenylboronic acid for 3-(morpholine-4-carbonyl)phenylboronic acid. MS (DCI/NH₃) m/z 460 (M+H)⁺; ¹H NMR (300 MHz, CDCl₃): δ 1.36 (d, *J*=6.74 Hz, 3 H), 1.61-1.78 (m, 4 H), 1.76 - 2.06 (m, 4 H), 2.26 - 2.45 (m, 2 H), 2.49 - 2.67 (m, 2 H), 2.84 (m, 2 H), 3.21 - 3.36 (m, 1 H), 3.40 - 3.57 (m, 2 H), 3.59 - 3.83 (m, 2 H), 7.13 - 7.23 (m, 1 H), 7.36 (t, *J*=7.93 Hz, 1 H), 7.40 - 7.51 (m, 3 H), 7.55 - 7.64 (m, 1 H), 7.73 (s, 1 H) 9.98 (s, 1 H).

### EXAMPLE 277

### N-cyclopropyl-2'-fluoro-5'-(1-methyl-2-(4-oxo-3,4,5,6,7,8-hexahydrophthalazin-1-yl)ethyl)-1,1 '-biphenyl-3-carboxamide

The title compound was prepared according to the procedure for EXAMPLE 264, substituting EXAMPLE 293 for EXAMPLE 223, and 3-(cyclopropylcarbamoyl)phenylboronic acid for 3-(morpholine-4-carbonyl)phenylboronic acid. MS (DCI/NH₃) m/z 446 (M+H)⁺.

### EXAMPLE 278

### 4-(3-amino-4-chlorobenzyl)-5,6,7,8-tetrahydrophthalazin-1(2H)-one

The title compound was prepared according to the procedure for EXAMPLE 2, substituting 4-chloro-3-nitrobenzaldehyde for 4-fluoro-3-nitrobenzaldehyde. MS (DCI/NH₃) m/z 290 (M+H)⁺.

### EXAMPLE 279

### 4-(3-amino-4-methoxybenzyl)-5,6,7,8-tetrahydrophthalazin-1(2H)-one

The title compound was prepared according to the procedure for EXAMPLE 2, substituting 4-methoxy-3-nitrobenzaldehyde for 4-fluoro-3-nitrobenzaldehyde. MS (DCI/NH₃) m/z 286 (M+H)⁺.

### EXAMPLE 280

### 4-(3-amino-4-hydroxybenzyl)-5,6,7,8-tetrahydrophthalazin-1(2H)-one

The title compound was prepared according to the procedure for EXAMPLE 2, substituting 4-hydroxy-3-nitrobenzaldehyde for 4-fluoro-3-nitrobenzaldehyde. MS (DCI/NH₃) m/z 272 (M+H)⁺.

### EXAMPLE 281

### 4-(3-amino-4-methylbenzyl)-5,6,7,8-tetrahydrophthalazin-1(2H)-one

The title compound was prepared according to the procedure for EXAMPLE 2, substituting 4-methyl-3-nitrobenzaldehyde for 4-fluoro-3-nitrobenzaldehyde. MS (DCI/NH₃) m/z 270 (M+H)⁺.

### EXAMPLE 282

### N-(2-(dimethylamino)ethyl)-3'-(1-methyl-2-(4-oxo-3,4,5,6,7,8-hexahydrophthalazin-1-yl)ethyl)-1,1'-biphenyl-3-carboxamide

The title compound was prepared according to the procedure for EXAMPLE 264, substituting EXAMPLE 256 for EXAMPLE 223, and 3-(2-(dimethylamino)ethylcarbamoyl)phenylboronic acid for 3-(morpholine-4-carbonyl)phenylboronic acid. MS (DCI/NH₃) m/z 459 (M+H)⁺.

### EXAMPLE 283

### 3'-(1-methyl-2-(4-oxo-3,4,5,6,7,8-hexahydrophthalazin-1-yl)ethyl)-1,1'-biphenyl-3-carboxamide

The title compound was prepared according to the procedure for EXAMPLE 264, substituting EXAMPLE 256 for EXAMPLE 223, and 3-carbamoylphenylboronic acid for 3-(morpholine-4-carbonyl)phenylboronic acid. MS (DCI/NH₃) m/z 388 (M+H)⁺; ¹H NMR (300 MHz, CD₃OD): δ 1.40 (d, *J*=7.14 Hz, 3 H), 1.53 - 1.81 (m, 4 H), 2.25 - 2.59 (m, 4 H), 2.91 (d, *J*=7.14 Hz, 2 H), 3.31 - 3.41 (m, 1 H), 7.23 (d, *J*=7.54 Hz, 1 H), 7.36 (t, *J*=7.93 Hz, 1 H), 7.42 - 7.51 (m, 2 H), 7.50 - 7.59 (m, 1 H), 7.73 (d, *J*=7.93 Hz, 1 H), 7.79 - 7.91 (m, 1 H), 8.09 (s, 1 H).

### EXAMPLE 284

### N-(3'-(1-methyl-2-(4-oxo-3,4,5,6,7,8-hexahydrophthalazin-1-yl)ethyl)-1,1'-biphenyl-3-yl)acetamide

The title compound was prepared according to the procedure for EXAMPLE 264, substituting EXAMPLE 256 for EXAMPLE 223, and 3-acetamidophenylboronic acid for 3-(morpholine-4-carbonyl)phenylboronic acid. MS (DCI/NH₃) m/z 402 (M+H)⁺; ¹H NMR (300 MHz, CD₃OD): δ 1.40 (d, *J*=7.12 Hz, 3 H), 1.50 - 1.78 (m, 4 H), 2.16 (s, 3 H), 2.36-2.53 (m, 4 H), 2.83 (m, 1 H), 2.88 (d, *J*=7.46 Hz, 2 H), 7.16 - 7.23 (m, 1 H), 7.22 - 7.29 (m, 1 H), 7.29 - 7.36 (m, 2 H), 7.36 - 7.45 (m, 2 H), 7.47 - 7.67 (m, 1 H), 7.72 (t, *J*=1.86 Hz, 1 H).

### EXAMPLE 285

### 3'-(1-(4-oxo-3,4,5,6,7,8-hexahydrophthalazin-1-yl)ethyl)-1,1'-biphenyl-3-carboxamide

### EXAMPLE 285A

### 1-bromo-3-(1-bromoethyl)benzene

A solution of 3-bromoethyl benzene (2 g, 11 mmol), N-bromosuccinimide (91.9 g, 11 mmol) and azobisisobutyronitrile (10 mg, 0.06 mmol) in chloroform (30 ml) was stirred at 65 °C under nitrogen for 18 hours. After cooling, the reaction mixture was concentrated, and the residue was partitioned between ethyl acetate and brine. The organic layer was washed with brine, and was concentrated. The residue was separated by flash chromatography on silica gel (10% ethyl acetate in hexane) to provide the title compound. MS (DCI/NH₃) m/z 262 (M+H)⁺.

### EXAMPLE 285B

### (1-(3-bromophenyl)ethyl)triphenylphosphonium bromide

A solution of EXAMPLE 285A (1.0 g, 3.8 mmol) and triphenylphosphine (1.1 g, 4.2 mmol) in toluene (15 ml) was heated at 120°C under nitrogen for three days. After cooling to room temperature, the solid material was collected by filtration, washed with toluene, and dried to provide the title compound.

### EXAMPLE 285C

### 3-(1-(3-bromophenyl)ethylidene)-4,5,6,7-tetrahydroisobenzofuran-1(3H)-one

A suspension of EXAMPLE 285B (1.88g, 3.4 mmol) in tetrahydrofuran (100 ml) was treated with potassium t-butoxide (1N solution in tetrahydrofuran, 3.4 ml, 3.4 mmol) at -78 °C for 1 hour, and was allowed to warm up to 0 °C over 30 minutes. A solution of 4,5,6,7-tetrahydroisobenzofuran-1,3-dione (0.54 g, 3.4 mmol) in tetrahydrofuran (10 ml) was then added. The reaction mixture was warmed up to room temperature, and stirred at room temperature for additional 4 hours. After quenching with water, the reaction mixture was partitioned between ethyl acetate and brine. The organic phase was washed with brine, and concentrated. The residue was separated by flash chromatography (20% ethyl acetate in hexane) to provide the title compound. MS (DCI/NH₃) m/z 320 (M+H)⁺.

### EXAMPLE 285D

### 4-(1-(3-bromophenyl)ethyl)-5,6,7,8-tetrahydrophthalazin-1(2H)-one

The title compound was prepared according to the procedure for EXAMPLE 2C, substituting EXAMPLE 285C for EXAMPLE 2B. MS (DCI/NH₃) m/z 334 (M+H)⁺.

### EXAMPLE 285E

### 3'-(1-(4-oxo-3,4,5,6,7,8-hexahydrophthalazin-1-yl)ethyl)-1,1'-biphenyl-3-carboxamide

The title compound was prepared according to the procedure for EXAMPLE 264, substituting EXAMPLE 285D for EXAMPLE 223, and 3-carbamoylphenylboronic acid for 3-(morpholine-4-carbonyl)phenylboronic acid. MS (DCI/NH₃) m/z 374 (M+H)⁺; ¹H NMR (300 MHz, CD₃OD): δ 1.59 (d, *J*=7.12 Hz, 3 H), 1.61 - 1.81 (m, 4 H), 2.10 - 2.22 (m, 1H), 2.39 - 2.55 (m, 2 H), 2.61 - 2.73 (m, 1H), 4.32 (q, *J*=6.78 Hz, 1 H), 7.08 - 7.21 (m, 1 H), 7.35 - 7.43 (m, 1 H), 7.48 - 7.59 (m, 3 H), 7.75 (d, *J*=7.80 Hz, 1 H), 7.80 - 7.87 (m, 1 H), 8.06-8.11 (m, 1H).

### EXAMPLE 286

### N-(3'-(1-(4-oxo-3,4,5,6,7,8-hexahydrophthalazin-1-yl)ethyl)-1,1'-biphenyl-3-yl)acetamide

The title compound was prepared according to the procedure for EXAMPLE 264, substituting EXAMPLE 285D for EXAMPLE 223, and 3-acetamidophenylboronic acid for 3-(morpholine-4-carbonyl)phenylboronic acid. MS (DCI/NH₃) m/z 388 (M+H)⁺; ¹H NMR (300 MHz, CD₃OD): δ 1.58 (d, *J*=7.12 Hz, 3 H), 1.60 - 1.82 (m, 4 H), 2.14 (s, 3 H), 2.10-2.23 (m, 1H), 2.47 - 2.55 (m, 2 H), 2.60 - 2.73 (m, 1H), 4.30 (q, *J*=6.78 Hz, 1 H), 7.15 (d, *J*=7.46 Hz, 1 H), 7.23 - 7.30 (m, 1 H), 7.31 - 7.40 (m, 2 H), 7.41 - 7.47 (m, 1 H), 7.47 - 7.56 (m, 1 H), 7.56 - 7.69 (m, 1 H), 7.77 (t, *J*=1.86 Hz, 1 H).

### EXAMPLE 287

### N-(2-(dimethylamino)ethyl)-3'-(1-(4-oxo-3,4,5,6,7,8-hexahydrophthalazin-1-yl)ethyl)-1,1'-biphenyl-3-carboxamide

The title compound was prepared according to the procedure for EXAMPLE 264, substituting EXAMPLE 285D for EXAMPLE 223, and 3-(2-(dimethylamino)ethylcarbamoyl)phenylboronic acid for 3-(morpholine-4-carbonyl)phenylboronic acid. MS (DCI/NH₃) m/z 445 (M+H)⁺.

### EXAMPLE 288

### 3-(1-methyl-2-(4-oxo-3,4,5,6,7,8-hexahydrophthalazin-1-yl)ethyl)benzoic acid

A solution of EXAMPLE 266 (50 mg, 0.16 mmol) in tetrahydrofuran (10 ml) was treated with a solution of LiOH·H₂O (100 mg, 4 mmol) in water (4 ml) at 50 °C overnight. The mixture was concentrated, and the residue was purified by HPLC (Zorbax® C-18 ODS packing material [Agilent Technologies, Santa Clara, CA], 0.1% trifluoroacetic acid/CH₃CN/H₂O) to provide the title compound. MS (DCI/NH₃) m/z 313 (M+H)⁺.

### EXAMPLE 289

### N-(2-fluoro-5-((4-oxo-3,4,5,6,7,8-hexahydrophthalazin-1-yl)methyl)phenyl)-4-(4-methoxyphenyl)-4-oxobutanamide

To a solution of 4-(4-methoxyphenyl)-4-oxobutanoic acid (29 mg, 0.14 mmol) in dioxane (1.5 mL) was added 2-(1H-7-azabenzotriazol-1-yl)-1,1,3,3-tetramethyl uronium hexafluorophosphate methanaminium (HATU) (42 mg) and N,N'-diisopropylethylamine (32 µL). The mixture was stirred at room temperature for 15 minutes, and EXAMPLE 2 (25 mg, 0.091 mmol) was added. The reaction mixture was stirred at room temperature for 16 hours, and was concentrated. The crude was separated by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 464 (M+H)⁺; ¹H NMR (500 MHz, dimethylsulfoxide-*d*₆): δ 1.53 - 1.66 (m, 4H), 2.29 - 2.40 (m, 4H), 2.74 (t, *J*=6.41 Hz, 2H), 3.25 (t, *J*=6.56 Hz, 2H), 3.84 (s, 2H), 3.85 (s, 3H), 6.88 - 6.96 (m, 1H), 7.05 (d, *J*=8.85 Hz, 2H), 7.12 - 7.20 (m, 1H), 7.74 (d, *J*=6.41 Hz, 1H), 7.97 (d, *J*=9.15 Hz, 2H), 9.75 (br s, 1H), 12.60 (br s, 1H).

### EXAMPLE 290

### 1-(2-fluoro-5-((4-oxo-3,4,5,6,7,8-hexahydrophthalazin-1-yl)methyl)phenyl)-3,4-dimethyl-1H-pyrrole-2,5-dione

To a solution of EXAMPLE 2 (100 mg, 0.37 mmol) in acetic acid (8 mL) was added 3,4-dimethylfuran-2,5-dione (46 mg, 0.37 mmol). The reaction mixture was heated at 80 °C for 16 hours, and concentrated. The residue was separated by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 382 (M+H)⁺; ¹H NMR (500 MHz, dimethylsulfoxide-*d*₆): δ 1.56 - 1.68 (m, 4H), 1.98 (s, 6H), 2.30 - 2.43 (m, 4H), 3.93 (s, 2H), 7.18 (dd, *J*=7.02, 1.53 Hz, 1H), 7.31-7.35 (m, 2H), 12.63 (br s, 1H).

### EXAMPLE 291

### 3-(2-fluoro-5-((4-oxo-3,4,5,6,7,8-hexahydrophthalazin-1-yl)methyl)phenyl)-3-azabicyclo(3.1.0)hexane-2,4-dione

To a suspension of EXAMPLE 2 (210 mg, 0.77 mmol) in acetonitrile (8 mL) was added 3-oxabicyclo(3.1.0)hexane-2,4-dione (95 mg, 0.85 mmol) and stirred at 80 °C for 16 hours. The reaction mixture was cooled and concentrated on a rotary evaporator. The residual solid was dissolved in dioxane (4 mL), and treated with O-(benzotriazol-1-yl, N, N, N', N'-tetramethyluronium hexafluorophosphate (380 mg, 0.99 mmol) and N,N'-diisopropylethylamine (0.3 mL, 1.69 mmol) at room temperature for an additional 16 hours. The reaction mixture was concentrated, and separated by HPLC (Zorbax® C-18 ODS packing material [Agilent Technologies, Santa Clara, CA], 0.1% trifluoroacetic acid/CH₃CN/H₂O) to provide the title compound as a trifluoroacetic acid salt. MS (DCI/NH₃) m/z 368 (M+H)⁺; ¹H NMR (400 MHz, dimethylsulfoxide-*d*₆): δ 1.57 - 1.64 (m, 4H), 1.66 - 1.72 (m, 2H), 2.32 - 2.41 (m, 4H), 2.75 (dd, *J*=7.82, 3.22 Hz, 2H), 3.91 (s, 1H), 7.16 (d, *J*=7.36 Hz, 1H), 7.27 - 7.29 (m, 1H), 7.29 - 7.32 (m, 1H), 12.61 (br s, 1H).

### EXAMPLE 292

### 4-((4-(phenoxyacetyl)piperazin-2-yl)methyl)-5,6,7,8-tetrahydrophthalazin-1(2H)-one

A solution of EXAMPLE 258 (138 mg, 0.29 mmol) in methylene chloride (10 ml) was treated with trifluoroacetic acid (2 ml) at 40 °C for 2 hours, and concentrated. The residue was purified by HPLC (Zorbax® C-18 ODS packing material [Agilent Technologies, Santa Clara, CA], 0.1% trifluoroacetic acid/CH₃CN/H₂O) to provide the title compound as TFA salt. MS (DCI/NH₃) m/z 383 (M+H)⁺.

### EXAMPLE 293

### 4-(2-(3-bromo-4-fluorophenyl)propyl)-5,6,7,8-tetrahydrophthalazin-1(2H)-one

### EXAMPLE 293A

### 2-(3-bromo-4-fluorophenyl)-N-methoxy-N-methylacetamide

The title compound was prepared according to the procedure for EXAMPLE 222A, substituting 2-(3-bromo-4-fluorophenyl)acetic acid for 2-(3-bromophenyl)acetic acid. MS (DCI/NH₃) m/z 276 (M+H)⁺.

### EXAMPLE 293B

### 2-(3-bromo-4-fluorophenyl)-N-methoxy-N-methylpropanamide

The title compound was prepared according to the procedure for EXAMPLE 256A, substituting EXAMPLE 293A for EXAMPLE 222A. MS (DCI/NH₃) m/z 291 (M+H)⁺.

### EXAMPLE 293C

### 2-(3-bromo-4-fluorophenyl)propanal

The title compound was prepared according to the procedure for EXAMPLE 256B, substituting EXAMPLE 293B for EXAMPLE 256A. MS (DCI/NH₃) m/z 232 (M+H)⁺.

### EXAMPLE 293D

### 3-(2-(3-bromo-4-fluorophenyl)propylidene)-4,5,6,7-tetrahydroisobenzofuran-1(3H)-one

The title compound was prepared according to the procedure for EXAMPLE 1C, substituting 293C for 2-fluoro-5-formylbenzonitrile. MS (DCI/NH₃) m/z 352 (M+H)⁺.

### EXAMPLE 293E

### 4-(2-(3-bromo-4-fluorophenyl)propyl)-5,6,7,8-tetrahydrophthalazin-1(2H)-one

The title compound was prepared according to the procedure for EXAMPLE 2C, substituting EXAMPLE 293D for EXAMPLE 2B. MS (DCI/NH₃) m/z 366 (M+H)⁺.

### EXAMPLE 294

### 4-oxo-N-(3-(2-(4-oxo-3,4,5,6,7,8-hexahydrophthalazin-1-yl)ethyl)phenyl)-4-phenylbutanamide

A mixture of 4-oxo-4-phenylbutanoic acid (50 mg, 0.28 mmol), 2-(3H-(1,2,3)triazolo(4,5-b)pyridin-3-yl)-1,1,3,3-tetramethylisouroniumhexafluorophosphate (V) (106 mg, 0.28 mmol) and Hunig's base (120 mg, 0.9 mmol) in anhydrous N,N-dimethylformamide (0.5 ml) was stirred at room temperature for 10 minutes, and EXAMPLE 260 (50 mg, 0.18 mmol) was added in one portion. The reaction mixture was stirred at room temperature for another 1 hour, and was diluted with 5 mL of methanol. The solid material was collected by filtration, washed with methanol, and dried to provide the title compound. MS (DCI/NH₃) m/z 430 (M+H)⁺. ¹H NMR (300 MHz, dimethylsulfoxide-*d₆*): δ 1.54 - 1.77 (m, 4 H), 2.30 - 2.42 (m, 2 H), 2.42 - 2.49 (m, 2 H), 2.65 - 2.79 (m, 4 H), 2.79 - 2.89 (m, 2 H), 3.30 - 3.38 (m, 2 H), 6.90 (d, *J*=7.80 Hz, 1 H), 7.19 (t, *J*=7.80 Hz, 1 H), 7.38 - 7.49 (m, 2 H), 7.54 (t, *J*=7.46 Hz, 2 H), 7.61 - 7.69 (m, 1 H), 7.99 (t, *J*=6.61 Hz, 2 H), 9.96 (s, 1 H), 12.52 (s, 1 H).

### EXAMPLE 295

### 2'-fluoro-5'-(1-methyl-2-(4-oxo-3,4,5,6,7,8-hexahydrophthalazin-1-yl)ethyl)-1,1'-biphenyl-3-carboxamide

The title compound was prepared according to the procedure for EXAMPLE 264, substituting EXAMPLE 293 for EXAMPLE 223, and 3-carbamoylphenylboronic acid for 3-(morpholine-4-carbonyl)phenylboronic acid. MS (DCI/NH₃) m/z 406 (M+H)⁺.

### EXAMPLE 296

### N-(2'-fluoro-5'-(1-methyl-2-(4-oxo-3,4,5,6,7,8-hexahydrophthalazin-1-yl)ethyl)-1,1'-biphenyl-3-yl)acetamide

The title compound was prepared according to the procedure for EXAMPLE 264, substituting EXAMPLE 293 for EXAMPLE 223, and 3-acetamidophenylboronic acid for 3-(morpholine-4-carbonyl)phenylboronic acid. MS (DCI/NH₃) m/z 420 (M+H)⁺.

### EXAMPLE 297

### N-((2'-fluoro-5'-(1-methyl-2-(4-oxo-3,4,5,6,7,8-hexahydrophthalazin-1-yl)ethyl)-1,1'-biphenyl-3-yl)methyl)methanesulfonamide

The title compound was prepared according to the procedure for EXAMPLE 264, substituting EXAMPLE 293 for EXAMPLE 223, and 3-(methylsulfonamidomethyl)phenylboronic acid for 3-(morpholine-4-carbonyl)phenylboronic acid. MS (DCI/NH₃) ml) 470 (M+H)⁺.

### EXAMPLE 298

### 2-(2-fluoro-5-((4-oxo-3,4,5,6,7,8-hexahydrophthalazin-1-yl)methyl)phenyl)hexahydro-1H-isoindole-1,3(2H)-dione

The title compound was prepared according to the procedure for EXAMPLE 291, substituting hexahydroisobenzofuran-1,3-dione for oxabicyclo(3.1.0)hexane-2,4-dione. MS (DCI/NH₃) m/z 410 (M+H)⁺; ¹H NMR (300 MHz, dimethylsulfoxide-*d*₆): δ 1.31 1.53 (m, 5H), 1.57 - 1.68 (m, 4H), 1.66 - 1.78 (m, 3H), 1.76 - 1.92 (m, 2H), 2.29 - 2.43 (m, 4H), 3.93 (s, 2H), 7.12 - 7.17 (m, 1H), 7.28 - 7.33 (m, 1H), 7.33 - 7.37 (m, 1H), 12.63 (br s, 1H).

### EXAMPLE 299

### 1-(2-fluoro-5-((4-oxo-3,4,5,6,7,8-hexahydrophthalazin-1-yl)methyl)phenyl)-3,3-dimethylpyrrolidine-2,5-dione

The title compound was prepared according to the procedure for EXAMPLE 291, substituting 3,3-dimethyldihydrofuran-2,5-dione for oxabicyclo(3.1.0)hexane-2,4-dione. MS (DCI/NH₃) m/z 384 (M+H)⁺; ¹H NMR (300 MHz, dimethylsulfoxide-*d*₆): δ 1.31 (s, 6H), 1.54 - 1.70 (m, 4H), 2.30 - 2.44 (m, 4H), 2.78 (s, 2H), 3.94 (s, 2H), 7.19 (d, *J*=7.46 Hz, 1H), 7.30-7.33 (m, 1H), 7.35 (s, 1H), 12.62 (br s, 1H).

### EXAMPLE 300

### 4-(4-fluoro-3-(2-methyl-5-oxopyrrolidin-1-yl)benzyl)-5,6,7,8-tetrahydrophthalazin-1(2H)-one

### EXAMPLE 300A

### 4-fluoro-3-(2-methyl-5-oxopyrrolidin-1-yl)benzaldehyde

A 100 mL round bottom flask was charged with 3-bromo-4-fluorobenzaldehyde (1.0 g, 4.93 mmol), tris(dibenzylideneacetone)dipalladium(0)(450 mg, 0.493 mmol), Xantphos (4,5-bis(diphenylphosphino)-9,9-dimethylxanthene) (428 mg, 0.739 mmol), and cesium carbonate (2.4 g, 7.39 mmol). The mixture was purged with nitrogen, and anhydrous dioxane (15 mL), and 5-methylpyrrolidinone (0.586 g, 5.91 mmol) were added. The reaction mixture was purged with nitrogen again, and heated at 100 °C for 20 hours. After cooling to room temperature, the reaction mixture was partitioned between ethyl acetate and brine. The organic phase was dried over MgSO₄, filtered and concentrated. The residue was separated by flash chromatography (50% ethyl acetate in hexane) to provide the title compound. MS (DCI/NH₃) m/z 222 (M+H)⁺.

### EXAMPLE 300B

### 4-(4-fluoro-3-(2-methyl-5-oxopyrrolidin-1-yl)benzyl)-5,6,7,8-tetrahydrophthalazin-1(2H)-one

A solution of EXAMPLE 1B (486 mg, 1.16 mmol), EXAMPLE 300A (265 mg) and triethylamine (0.16 mL) in dichloromethane (8 mL) was stirred at room temperature for 16 hours, and concentrated. The residue was dissolved in ethanol (5 mL) and treated with hydrazine monohydrate (0.11 mL) at 80 °C for 2 hours. The mixture was allowed to cool and the precipitated solid was filtered and dried to provide the title compound. MS (DCI/NH₃) m/z 356 (M+H)⁺; ¹H NMR (400 MHz, dimethylsulfoxide-*d*₆): δ 1.02 (d, *J*=6.14 Hz, 3H), 1.57 - 1.63 (m, 4H), 1.64 - 1.72 (m, 1H), 2.27 - 2.34 (m, 1H), 2.34 - 2.40 (m, 4H), 2.41 - 2.46 (m, 2H), 3.90 (s, 2H), 4.08 (q, *J*=6.44 Hz, 1H), 7.10 - 7.14 (m, 1H), 7.14 - 7.18 (m, 1H), 7.20-7.27 (m, 1H), 12.61 (s, 1H).

### EXAMPLE 301

### 4-(4-fluoro-3-(2-oxo-1,3-oxazolidin-3-yl)benzyl)-5,6,7,8-tetrahydrophthalazin-1(2H)-one

### EXAMPLE 301A

### 4-fluoro-3-(2-oxooxazolidin-3-yl)benzaldehyde

The title compound was prepared according to the procedure for EXAMPLE 300A, substituting oxazolidin-2-one for 5-methylpyrrolidinone. MS (DCI/NH₃) m/z 210 (M+H)⁻.

### EXAMPLE 301B

### 4-(4-fluoro-3-(2-oxo-1,3-oxazolidin-3-yl)benzyl)-5,6,7,8-tetrahydrophthalazin-1(2H)-one

The title compound was prepared according to the procedure for EXAMPLE 300B, substituting EXAMPLE 301A for EXAMPLE 300A. MS (DCI/NH₃) m/z 344 (M+H)⁺; ¹H NMR (300 MHz, dimethylsulfoxide-*d*₆): δ 1.55 - 1.75 (m, 4H), 2.30 - 2.45 (m, 4H), 3.90 (s, 2H), 3.98 (t, *J*=7.93 Hz, 2H), 4.45 (dd, *J*=8.72, 7.14 Hz, 2H), 7.11 - 7.17 (m, 1H), 7.25 (dd, *J*=10.91, 8.53 Hz, 1H), 7.36 (dd, *J*=7.54, 2.38 Hz, 1H), 12.61 (br s, 1H).

### EXAMPLE 302

### 4-(4-fluoro-3-(2-oxoazepan-1-yl)benzyl)-5,6,7,8-tetrahydrophthalazin-1(2H)-one

### EXAMPLE 302A

### 4-fluoro-3-(2-oxoazepan-1-yl)benzaldehyde

The title compound was prepared according to the procedure for EXAMPLE 300A, substituting azepan-2-one for 5-methylpyrrolidinone. MS (DCI/NH₃) m/z 236 (M+H)⁺.

### EXAMPLE 302B

### 4-(4-fluoro-3-(2-oxoazepan-1-yl)benzyl)-5,6,7,8-tetrahydrophthalazin-1(2H)-one

The title compound was prepared according to the procedure for EXAMPLE 300B, substituting EXAMPLE 302A for EXAMPLE 300A. MS (DCI/NH₃) m/z 370 (M+H)⁺; ¹H NMR (300 MHz, dimethylsulfoxide-*d*₆): δ 1.54 - 1.65 (m, 4H), 1.65 - 1.80 (m, 6H), 2.32-2.45 (m, 4H), 2.54 - 2.63 (m, 2H), 3.57 - 3.72 (m, 2H), 3.88 (s, 2H), 7.04 - 7.12 (m, 2H), 7.13 - 7.22 (m, 1H), 12.61 (br s, 1H).

### EXAMPLE 303

### 1-(2-fluoro-5-((4-oxo-3,4,5,6,7,8-hexahydrophthalazin-1-yl)methyl)phenyl)piperidine-2,6-dione

The title compound was prepared according to the procedure for EXAMPLE 291, substituting dihydro-2H-pyran-2,6(3H)-dione for oxabicyclo(3.1.0)hexane-2,4-dione. MS (DCI/NH₃) m/z 370 (M+H)⁺; ¹H NMR (300 MHz, dimethylsulfoxide-*d*₆): δ 1.53 - 1.69 (m, 4H), 1.84 - 1.97 (m, 1H), 1.98 - 2.11 (m, 1H), 2.29 - 2.42 (m, 4H), 2.75 (t, *J*=6.44 Hz, 4H), 3.90 (s, 2H), 7.04 (d, *J*=7.80 Hz, 1H), 7.24 (s, 1H), 7.26 (d, *J*=1.36 Hz, 1H), 12.63 (s, 1H).

### EXAMPLE 304

### 4-(4-fluoro-3-(2-oxoimidazolidin-1-yl)benzyl)-5,6,7,8-tetrahydrophthalazin-1(2H)-one

### EXAMPLE 304A

### 4-fluoro-3-(2-oxoimidazolidin-1-yl)benzaldehyde

The title compound was prepared according to the procedure for EXAMPLE 300A, substituting imidazolidin-2-onc for 5-methylpyrrolidinone. MS (DCI/NH₃) m/z 209 (M+H)⁻.

### EXAMPLE 304B

### 4-(4-fluoro-3-(2-oxoimidazolidin-1-yl)benzyl)-5,6,7,8-tetrahydrophthalazin-1(2H)-one

The title compound was prepared according to the procedure for EXAMPLE 300B, substituting EXAMPLE 304A for EXAMPLE 300A. MS (DCI/NH₃) m/z 343 (M+H)⁺; ¹H NMR (300 MHz, dimethylsulfoxide-*d*₆): δ 1.55 - 1.69 (m, 4H), 2.31 - 2.44 (m, 4H), 3.39 (t, *J*=7.97 Hz, 2H), 3.75 - 3.83 (m, 2H), 3.86 (s, 2H), 6.86 (br s, 1H), 6.94 - 7.03 (m, 1H), 7.16 (dd, J=11.19, 8.48 Hz, 1H), 7.31 (dd, *J*=7.63, 2.20Hz, 1H), 12.61 (brs, 1H).

### EXAMPLE 305

### 4-(3-(1,1-dioxidoisothiazolidin-2-yl)-4-fluorobenzyl)-5,6,7,8-tetrahydrophthalazin-1(2H)-one

To a solution of EXAMPLE 2 (150 mg, 0.55 mmol) in dichloromethane (5 mL) was added 3-chloropropane-1-sulfonyl chloride (97 mg, 0.55 mmol), and the mixture stirred for 16 hours. The reaction mixture was concentrated, and the residual solid was dissolved in dioxane (3 mL). Sodium ethoxide (0.14 mL, 21 wt% in ethyl alcohol) was then added, and the solution was heated at 80 °C for 16 hours. After cooling, the reaction mixture was concentrated. The residue was separated by HPLC (Zorbax^{®} C-18 ODS packing material [Agilent Technologies, Santa Clara, CA], 0.1% trifluoroacetic acid/CH₃CN/H₂O) to provide the title compound as free base. MS (DCI/NH₃) m/z 378 (M+H)¹; ¹H NMR (300 MHz, dimethylsulfoxide-*d*₆): δ 1.56 - 1.70 (m, 4H), 2.33 - 2.47 (m, 6H), 3.40 (t, *J*=7.29 Hz, 2H), 3.72 (t, *J*=6.44 Hz, 2H), 3.90 (s, 2H), 7.09 - 7.16 (m, 1H), 7.23 (d, *J*=8.48 Hz, 1H), 7.25-7.28 (m, 1H), 12.61 (br s, 1H).

### EXAMPLE 306

### 4-(4-fluoro-3-(2-oxoazetidin-1-yl)benzyl)-5,6,7,8-tetrahydrophthalazin-1(2H)-one

### EXAMPLE 306A

### 4-fluoro-3-(2-oxoazetidin-1-yl)benzaldehyde

The title compound was prepared according to the procedure for EXAMPLE 300A, substituting azetidin-2-one for 5-methylpyrrolidinone. MS (DCI/NH₃) m/z 194 (M+H)⁺.

### EXAMPLE 306B

### 4-(4-fluoro-3-(2-oxoazetidin-1-yl)benzyl)-5,6,7,8-tetrahydrophthalazin-1(2H)-one

The title compound was prepared according to the procedure for EXAMPLE 300B, substituting EXAMPLE 306A for EXAMPLE 300A. MS (DCI/NH₃) m/z 328 (M+H)⁺. ¹H NMR (300 MHz, dimethylsulfoxide-*d*₆): δ 1.55 - 1.68 (m, 4H), 2.31 - 2.43 (m, 4H), 3.11 (t, *J*=4.58 Hz, 2H), 3.82 (q, *J*=4.41 Hz, 2H), 3.86 (s, 2H), 6.86 - 6.94 (m, 1H), 7.18 (dd, *J*=11.87, 8.48 Hz, 1H), 7.74 (dd, *J*=7.63, 2.20 Hz, 1H), 12.60 (br s, 1H).

### EXAMPLE 307

### 4-(4-fluoro-3-(2-oxopiperidin-1-yl)benzyl)-5,6,7,8-tetrahydrophthalazin-1(2H)-one

### EXAMPLE 307A

### 4-fluoro-3-(2-oxopiperidin-1-yl)benzaldehyde

The title compound was prepared according to the procedure for EXAMPLE 300A, substituting piperidin-2-one for 5-methylpyrrolidinone. MS (DCI/NH₃) m/z 222 (M+H)⁺.

### EXAMPLE 307B

### 4-(4-fluoro-3-(2-oxopiperidin-1-yl)benzyl)-5,6,7,8-tetrahydrophthalazin-1(2H)-one

The title compound was prepared according to the procedure for EXAMPLE 300B, substituting EXAMPLE 307A for EXAMPLE 300A. MS (DCI/NH₃) m/z 356 (M+H)⁺; ¹H NMR (300 MHz, dimethylsulfoxide-*d₆*): δ 1.54 - 1.67 (m, 4H), 1.77 - 1.93 (m, 4H), 2.31 - 2.44 (m, 6H), 3.44 - 3.53 (m, 2H), 3.88 (s, 2H), 7.10 - 7.14 (m, 1H), 7.15 (d, *J*=6.35 Hz, 1H), 7.17 - 7.23 (m, 1H), 12.62 (s, 1H).

### EXAMPLE 308

### N-(3-furylmethyl)-3-((4-oxo-3,4,5,6,7,8-hexahydrophthalazin-1-yl)methyl)benzamide

### EXAMPLE 308A

### methyl 3-((3-oxo-4,5,6,7-tetrahydroisobenzofuran-1(3H)-ylidene)methyl)benzoate

A solution of EXAMPLE 1B (25.8 g, 61.5 mmol), methyl-3-formylbenzoate (10.01 g, 61.0 mmol), and triethylamine (8.7 mL, 62.4 mmol) in dichloromethane (125 mL) was stirred at room temperature for 16 hours, and concentrated. The residue was stirred with a mixture of ethyl acetate and water. The precipitated solid was filtered, washed with water, and dried to provide the title compound. MS (DCI/NH₃) m/z 285 (M+H)⁺.

### EXAMPLE 308B

### 3-((3-oxo-4,5,6,7-tetrahydroisobenzofuran-1(3H)-ylidene)methyl)benzoic acid

A solution of EXAMPLE 308A (9.9 g, 35 mmol) in 1:1 mixture of tetrahydrofuran/water (100 mL) was treated with lithium hydroxide monohydrate (2.93 g, 70 mmol) at room temperature for 16 hours. Ethyl acetate was added (100 mL) and the mixture washed with 2M HCl (100 mL). The combined organics were concentrated and dried under vacuum to provide the title compound. MS (DCI/NH₃) m/z 271 (M+H)⁺.

### EXAMPLE 308C

### 3-((4-oxo-3,4,5,6,7,8-hexahydrophthalazin-1-yl)methyl)benzoic acid

A solution of EXAMPLE 308B (9.0 g, 33.33 mmol) in absolute ethanol (120 mL) was heated with hydrazine monohydrate (3.3 mL, 66.66 mmol) at 80 °C for 16 hours. After cooling to room temperature, the precipitated solid was filtered, and dried to provide the title compound. MS (DCI/NH₃) m/z 285 (M+H)⁺.

### EXAMPLE 308D

### 3-((4-oxo-3,4,5,6,7,8-hexahydrophthalazin-1-yl)methyl)benzoyl chloride

A solution of EXAMPLE 308C (2.73 g, 9.6 mmol) in anhydrous tetrahydrofuran (30 mL) was treated with oxalyl chloride (1.3 mL, 14.4 mmol) and a couple of drops ofN,N-dimethylformamide at room temperature for 10 minutes and at 50 °C for 1 hour. The reaction mixture was concentrated and dried to provide the title compound. MS (DCI/NH₃) m/z 303 (M+H)⁺.

### EXAMPLE 308E

### N-(3-furylmethyl)-3-((4-oxo-3,4,5,6,7,8-hexahydrophthalazin-1-yl)methyl)benzamide

A solution of EXAMPLE 308D (19 mg, 0.06mmol), furan-3-ylmethanamine (0.07 mmol) and triethylamine (14.6 mg, 0.14 mmol) in tetrahydrofuran (1.0 mL) was stirred at room temperature for 16 hours. The reaction mixture was concentrated. The residue was dissolved in 1:1 mixture of dimethylsulfoxide/methanol and purified by HPLC (Waters Sunfire^{®} C-8 analytical column [Milford, MA]/0.1% trifluoroacetic acid/water/100% CH₃CN) to provide the title compound. MS (DCI/NH₃) m/z 363 (M+H)⁺; ¹H NMR (500 MHz, D₂O/dimethylsulfoxide-*d*₆): δ 1.54 - 1.69 (m, 4H), 2.32 - 2.45 (m, 4H), 3.96 (s, 2H), 4.29 (s, 2H), 6.41 - 6.49 (m, 1H), 7.32 - 7.37 (m, 1H), 7.41 (t, *J*=7.63 Hz, 1H), 7.52 - 7.60 (m, 2H), 7.64 - 7.72 (m, 2H).

### EXAMPLE 309

### 3-((4-oxo-3,4,5,6,7,8-hexahydrophthalazin-1-yl)methyl)-N-(thien-2-ylmethyl)benzamide

The title compound was prepared according to the procedure for EXAMPLE 308, substituting thiophen-2-ylmethanamine for furan-3-ylmethanamine. MS (DCI/NH₃) m/z 380 (M+H)⁺; ¹H NMR (500 MHz, D₂O/dimethylsulfoxide-*d*₆): δ 1.56 - 1.70 (m, 4H), 2.32 - 2.43 (m, 4H), 3.97 (s, 2H), 4.61 (s, 2H), 6.97 (dd, *J*=5.03, 3.51 Hz, 1H), 7.02 (d, *J*=2.44 Hz, 1H), 7.34 - 7.39 (m, 2H), 7.42 (t, *J*=7.63 Hz, 1H), 7.67 (s, 1H), 7.70 (d, *J*=7.93 Hz, 1H).

### EXAMPLE 310

### 3-((4-oxo-3,4,5,6,7,8-hexahydrophthalazin-1-yl)methyl)-N-(thien-3-ylmethyl)benzamide

The title compound was prepared according to the procedure for EXAMPLE 308, substituting thiophen-3-ylmethanamine for furan-3-ylmethanamine. MS (DCI/NH₃) m/z 380 (M+H)⁺.

### EXAMPLE 311

### 3-((4-oxo-3,4,5,6,7,8-hexahydrophthalazin-1-yl)methyl)-N-(pyridin-3-ylmethyl)benzamide

The title compound was prepared according to the procedure for EXAMPLE 308, substituting pyridin-3-ylmethanamine for furan-3-ylmethanamine. MS (DCI/NH₃) m/z 375 (M+H)⁺; ¹H NMR (500 MHz, D₂O/dimethylsulfoxide-*d*₆): δ 1.53 - 1.70 (m, 4H), 2.31 - 2.45 (m, 4H), 3.98 (s, 2H), 4.61 (s, 2H), 7.37 - 7.41 (m, 1H), 7.45 (t, *J*=7.63 Hz, 1H), 7.69 (s, 1H), 7.74 (d, *J*=7.63 Hz, 1H), 7.91 (dd, *J*=7.93, 5.49 Hz, 1H), 8.37 (d, *J*=7.93 Hz, 1H), 8.72 (d, *J*=75.19 Hz, 1H), 8.78 (s, 1H).

### EXAMPLE 312

### 3-((4-oxo-3,4,5,6,7,8-hexahydrophthalazin-1-yl)methyl)-N-(pyridin-4-ylmethyl)benzamide

The title compound was prepared according to the procedure for EXAMPLE 308, substituting pyridin-4-ylmethanamine for furan-3-ylmethanamine. MS (DCI/NH₃) m/z 375 (M+H)⁺.

### EXAMPLE 313

### N-(2-(dimethylamino)ethyl)-3-((4-oxo-3,4,5,6,7,8-hexahydrophthalazin-1-yl)methyl)benzamide

The title compound was prepared according to the procedure for EXAMPLE 308, substituting N¹,N¹-dimethylethane-1,2-diamine for furan-3-ylmethanamine. MS (DCI/NH₃) m/z 355 (M+H)⁺; ¹H NMR (500 MHz, D₂O/dimethylsulfoxide-*d*₆): δ 1.56 - 1.66 (m, 4H), 2.33 - 2.44 (m, 4H), 2.84 (s, 6H), 3.26 (t, *J*=5.95 Hz, 2H), 3.60 (t, *J*=5.95 Hz, 2H), 3.98 (s, 2H), 7.38 - 7.41 (m, 1H), 7.45 (t, *J*=7.63 Hz, 1H), 7.67 (s, 1H), 7.71 (d, *J*=7.93 Hz, 1H).

### EXAMPLE 314

### N-(3-(dimethylamino)propyl)-3-((4-oxo-3,4,5,6,7,8-hexahydrophthalazin-1-yl)methyl)benzamide

The title compound was prepared according to the procedure for EXAMPLE 308, substituting N¹,N¹-dimethylpropane-1,3-diamine for furan-3-ylmethanamine. MS (DCI/NH₃) m/z 369 (M+H)⁺.

### EXAMPLE 315

### 3-((4-oxo-3,4,5,6,7,8-hexahydrophthalazin-1-yl)methyl)-N-(3-pyrrolidin-1-ylpropyl)benzamide

The title compound was prepared according to the procedure for EXAMPLE 308, substituting 3-(pyrrolidin-1-yl)propan-1-amine for furan-3-ylmethanamine. MS (DCI/NH₃) m/z 395 (M+H)⁺.

### EXAMPLE 316

### 3-((4-oxo-3,4,5,6,7,8-hexahydrophthalazin-1-yl)methyl)-N-(3-piperidin-1-ylpropyl)benzamide

The title compound was prepared according to the procedure for EXAMPLE 308, substituting 3-(piperidin-1-yl)propan-1-amine for furan-3-ylmethanamine. MS (DCI/NH₃) m/z 409 (M+H)⁺.

### EXAMPLE 317

### N-(3-morpholin-4-ylpropyl)-3-((4-oxo-3,4,5,6,7,8-hexahydrophthalazin-1-yl)methyl)benzamide

The title compound was prepared according to the procedure for EXAMPLE 308, substituting 3-morpholinopropan-1-amine for furan-3-ylmethanamine. MS (DCI/NH₃) m/z 411 (M+H)⁺; ¹H NMR (500 MHz, D₂O/dimethylsulfoxide-*d₆*): δ 1.56 - 1.68 (m, 4H), 1.87 - 1.97 (m, 2H), 2.31 - 2.45 (m, 4H), 3.08 (t, *J*=12.05 Hz, 2H), 3.11 - 3.17 (m, 2H), 3.33 (t, *J*=6.71 Hz, 2H), 3.42 (d, *J*=12.51 Hz, 2H), 3.65 (t, *J*=12.05 Hz, 2H), 3.96 - 4.02 (m, 2H), 3.97 (s, 2H), 7.35 - 7.39 (m, 1H), 7.43 (t, *J*=7.63 Hz, 1H), 7.65 (s, 1H), 7.69 (d, *J*=7.93 Hz, 1H).

### EXAMPLE 318

### N-(2-(1H-indol-3-yl)ethyl)-3-((4-oxo-3,4,5,6,7,8-hexahydrophthalazin-1-yl)methyl)benzamide

The title compound was prepared according to the procedure for EXAMPLE 308, substituting 2-(1H-indol-3-yl)ethanamine for furan-3-ylmethanamine. MS (DCI/NH₃) m/z 427 (M+H)⁺.

### EXAMPLE 319

### 3-((4-oxo-3,4,5,6,7,8-hexahydrophthalazin-1-yl)methyl)-N-1,3-thiazol-2-ylbenzamide

The title compound was prepared according to the procedure for EXAMPLE 308, substituting thiazol-2-amine for furan-3-ylmethanamine. MS (DCI/NH₃) m/z 367 (M+H)⁺; ¹H NMR (500 MHz, D₂O/dimethylsulfoxide-*d*₆): δ 1.59 - 1.68 (m, 4H), 2.35 - 2.46 (m, 4H), 4.01 (s, 2H), 7.28 (d, *J*=3.66 Hz, 1H), 7.45 - 7.48 (m, 1H), 7.50 (t, *J*=7.48 Hz, 1H), 7.56 (d, *J*=3.66 Hz, 1H), 7.87 (s, 1H), 7.93 (d, *J*=7.63 Hz, 1H).

### EXAMPLE 320

### benzyl 2-oxo-2-(3-(2-(4-oxo-3,4,5,6,7,8-hexahydrophthalazin-1-yl)cthyl)phcnylamino)cthylcarbamatc

The title compound was prepared according to the procedure for EXAMPLE 294, substituting 2-(benzyloxycarbonylamino)acetic acid for 4-oxo-4-phenylbutanoic acid. MS (DCI/NH₃) m/z 461 (M+H)⁺.

### EXAMPLE 321

### 4-oxo-N-(3-(2-(4-oxo-3,4,5,6,7,8-hexahydrophthalazin-1-yl)ethyl)phenyl)-4-(4-phenoxyphenyl)butanamide

The title compound was prepared according to the procedure for EXAMPLE 294, substituting 4-oxo-4-(4-phenoxyphenyl)butanoic acid for 4-oxo-4-phenylbutanoic acid. MS (DCI/NH₃) m/z 522 (M+H)⁺.

### EXAMPLE 322

### benzyl 3-[({3-[2-(4-oxo-3,4,5,6,7,8-hexahydrophthalazin-1-yl)ethyl]phenyl} amino)carbonyl]piperidine-1-carboxylate

The title compound was prepared according to the procedure for EXAMPLE 294, substituting 1-(benzyloxycarbonyl)piperidine-3-carboxylic acid for 4-oxo-4-phenylbutanoic acid. MS (DCI/NH₃) m/z 515 (M+H)⁺.

### EXAMPLE 323

### 2-(4-methylphenoxy)-N-{3-[2-(4-oxo-3,4,5,6,7,8-hexahydrophthalazin-1-yl)ethyl]phenyl} acetamide

The title compound was prepared according to the procedure for EXAMPLE 294, substituting 2-(p-tolyloxy)acetic acid for 4-oxo-4-phenylbutanoic acid. MS (DCI/NH₃) m/z 418 (M+H)⁺.

### EXAMPLE 324

### 2-(4-methoxyphenoxy)-N-(3-(2-(4-oxo-3,4,5,6,7,8-hexahydrophthalazin-1-yl)ethyl)phenyl)acetamide

The title compound was prepared according to the procedure for EXAMPLE 294, substituting 2-(4-methoxyphenoxy)acetic acid for 4-oxo-4-phenylbutanoic acid. MS (DCI/NH₃) m/z 434 (M+H)⁺.

### EXAMPLE 325

### 4-[4-fluoro-3-(3-methyl-2-oxoimidazolidin-1-yl)benzyl]-5,6,7,8-tetrahydrophthalazin-1(2H)-one

### EXAMPLE 325A

### 4-fluoro-3-(3-methyl-2-oxoimidazolidin-1-yl)benzaldehyde

The title compound was prepared according to the procedure for EXAMPLE 300A, substituting 1-methylimidazolidin-2-one for 5-methylpyrrolidinone. MS (DCI/NH₃) m/z 223 (M+H)⁺.

### EXAMPLE 325B

### 4-[4-fluoro-3-(3-methyl-2-oxoimidazolidin-1-yl)benzyl]-5,6,7,8-tetrahydrophthalazin-1 (2H)-one

The title compound was prepared according to the procedure for EXAMPLE 300B, substituting EXAMPLE 325A for EXAMPLE 300A. MS (DCI/NH₃) m/z 357 (M+H)⁺; ¹H NMR (300 MHz, DMSO-*d*₆): δ 1.54 - 1.69 (m, 4H), 2.32 - 2.43 (m, 4H), 2.74 (s, 3H), 3.39 - 3.44 (m, 2H), 3.67 - 3.76 (m, 2H), 3.86 (s, 2H), 6.97 - 7.05 (m, 1H), 7.17 (dd,*J*=11.19, 8.48 Hz, 1H), 7.31 (dd, *J*=7.63, 2.20 Hz, 1H), 12.60 (s, 1H).

### EXAMPLE 326

### 4-[4-fluoro-3-(2-oxotetrahydropyrimidin-1(2H)-yl)benzyl]-5,6,7,8-tetrahydrophthalazin-1(2H)-one

### EXAMPLE 326A

### 4-fluoro-3-(2-oxotetrahydropyrimidin-1 (2H)-yl)benzaldehyde

The title compound was prepared according to the procedure for EXAMPLE 300A, substituting tetrahydropyrimidin-2(1H)-one for 5-methylpyrrolidinone. MS (DCI/NH₃) m/z 223 (M+H)⁺.

### EXAMPLE 326B

### 4-[4-fluoro-3-(2-oxotetrahydropyrimidin-1(2H)-yl)benzyl]-5,6,7,8-tetrahydrophthalazin-1(2H)-one

The title compound was prepared according to the procedure for EXAMPLE 300B, substituting EXAMPLE 326A for EXAMPLE 300A. MS (DCI/NH₃) m/z 357 (M+H)⁺; ¹H NMR (300 MHz, DMSO-*d*₆): δ 1.57 - 1.68 (m, 4H), 1.87 - 2.00 (m, 2H), 2.33 - 2.43 (m, 4H), 3.23 (t, *J*=5.76 Hz, 2H), 3.44 - 3.52 (m, 2H), 3.86 (s, 2H), 6.60 (s, 1H), 7.00 - 7.07 (m, 1H), 7.09 - 7.18 (m, 2H), 12.61 (s, 1H).

### EXAMPLE 327

### 4-[3-(3-tert-butyl-2-oxoimidazolidin-1-yl)-4-fluorobenzyl]-5,6,7,8-tetrahydrophthalazin-1(2H)-one

### EXAMPLE 327A

### 3-(3-tert-butyl-2-oxoimidazolidin-1-yl)-4-fluorobenzaldehyde

The title compound was prepared according to the procedure for EXAMPLE 300A, substituting 1-tert-butylimidazolidin-2-one for 5-methylpyrrolidinone. MS (DCI/NH₃) m/z 265 (M+H)⁺.

### EXAMPLE 327B

### 4-[3-(3-tert-butyl-2-oxoimidazolidin-1-yl)-4-fluorobenzyl]-5,6,7,8-tetrahydrophthalazin-1(2H)-one

The title compound was prepared according to the procedure for EXAMPLE 300B, substituting EXAMPLE 327A for EXAMPLE 300A. MS (DCI/NH₃) m/z 399 (M+H)⁺; ¹H NMR (300 MHz, DMSO-*d*₆): δ 1.3 (s, 9H), 1.53 - 1.68 (m, 4H), 2.31 - 2.45 (m, 4H), 3.43-3.48 (m, 2H), 3.58 - 3.69 (m, 2H), 3.86 (s, 2H), 6.95 - 7.02 (m, 1H), 7.15 (dd, *J*=11.36, 8.31 Hz, 1H), 7.28 (dd, *J*=7.46, 2.03 Hz, 1H), 12.59 (s, 1H).

### EXAMPLE 328

### 4-{4-fluoro-3-[(1S,4R)-3-oxo-2-azabicyclo[2.2.1]hept-2-yl]benzyl}-5,6,7,8-tetrahydrophthalazin-1 (2H)-one

### EXAMPLE 328A

### 4-fluoro-3-((1S,4R)-3-oxo-2-azabicyclo[2.2.1]heptan-2-yl)benzaldehyde

The title compound was prepared according to the procedure for EXAMPLE 300A, substituting (1 S,4R)-2-azabicyclo[2.2.1]heptan-3-one for 5-methylpyrrolidinone. MS (DCI/NH₃) m/z 234 (M+H)⁺.

### EXAMPLE 328B

### 4-{4-fluoro-3-[(1S,4R)-3-oxo-2-azabicyclo[2.2.1]hept-2-yl]benzyl}-5,6,7,8-tetrahydrophthalazin-1 (2H)-one

The title compound was prepared according to the procedure for EXAMPLE 300B, substituting EXAMPLE 328A for EXAMPLE 300A. MS (DCI/NH₃) m/z 368 (M+H)⁺ ; ¹H NMR (300 MHz, DMSO-*d*₆): δ 1.49 - 1.56 (m, 2H), 1.57 - 1.65 (m, 4H), 1.69 - 1.76 (m, 1H), 1.79 - 1.86 (m, 1H), 1.89 - 1.96 (m, 1H), 1.97 - 2.03 (m, 1H), 2.32 - 2.45 (m, 4H), 2.74 - 2.82 (m, 1H), 3.87 (s, 2H), 4.25 (s, 1H), 7.01 - 7.08 (m, 1H), 7.16 - 7.23 (m, 1H), 7.23 - 7.28 (m, 1H), 12.59 (br s, 1H).

### EXAMPLE 329

### N-(2-ethylphenyl)-3-[(4-oxo-3,4,5,6,7,8-hexahydrophthalazin-1-yl)methyl]benzamide

The title compound was prepared according to the procedure for EXAMPLE 308, substituting 2-ethylaniline for furan-3-ylmethanamine. MS (DCI/NH₃) m/z 388 (M+H)⁺.

### EXAMPLE 330

### N-(3-ethylphenyl)-3-[(4-oxo-3,4,5,6,7,8-hexahydrophthalazin-1-yl)methyl]benzamide

The title compound was prepared according to the procedure for EXAMPLE 308, substituting 3-ethylaniline for furan-3-ylmethanamine. MS (DCI/NH₃) m/z 388 (M+H)⁺.

### EXAMPLE 331

N-(4-ethylphenyl)-3-[(4-oxo-3,4,5,6,7,8-hexahydrophthalazin-1-yl)methyl]benzamide The title compound was prepared according to the procedure for EXAMPLE 308, substituting 4-ethylaniline for furan-3-ylmethanamine. MS (DCI/NH₃) m/z 388 (M+H)⁺.

### EXAMPLE 332

### 3-[(4-oxo-3,4,5,6,7,8-hexahydrophthalazin-1-yl)methyl]-N-(2-propylphenyl)benzamide

The title compound was prepared according to the procedure for EXAMPLE 308, substituting 2-propylaniline for furan-3-ylmethanamine. MS (DCI/NH₃) m/z 402 (M+H)⁺.

### EXAMPLE 333

### N-(2-isopropylphenyl)-3-[(4-oxo-3,4,5,6,7,8-hexahydrophthalazin-1-yl)methyl]benzamide

The title compound was prepared according to the procedure for EXAMPLE 308, substituting 2-isopropylaniline for furan-3-ylmethanamine. MS (DCI/NH₃) m/z 402 (M+H)⁺.

### EXAMPLE 334

### N-(4-isopropylphenyl)-3-[(4-oxo-3,4,5,6,7,8-hexahydrophthalazin-1-yl)methyl]benzamide

The title compound was prepared according to the procedure for EXAMPLE 308, substituting 4-isopropylaniline for furan-3-ylmethanamine. MS (DCI/NH₃) m/z 402 (M+H)⁺; ¹H NMR (500 MHz, D₂O/DMSO-d₆): δ 1.20 (d, *J*=7.02 Hz, 6H), 1.55 - 1.72 (m, 4H), 2.34 - 2.47 (m, 4H), 2.82 - 2.96 (m, 1H), 4.01 (s, 2H), 7.23 (d, *J*=8.24 Hz, 2H), 7.39 (d, *J*=7.63 Hz, 1H), 7.47 (t, *J*=7.63 Hz, 1H), 7.63 (d, *J*=8.54 Hz, 2H), 7.74 (s, 1H), 7.80 (d, *J*=7.93 Hz, 1H).

### EXAMPLE 335

### N-(3-tert-butylphenyl)-3-[(4-oxo-3,4,5,6,7,8-hexahydrophthalazin-1-yl)methyl]benzamide

The title compound was prepared according to the procedure for EXAMPLE 308, substituting 3-tert-butylaniline for furan-3-ylmethanamine. MS (DCI/NH₃) m/z 416 (M+H)⁻.

### EXAMPLE 336

### N-(4-tert-butylphenyl)-3-[(4-oxo-3,4,5,6,7,8-hexahydrophthalazin-1-yl)methyl]benzamide

The title compound was prepared according to the procedure for EXAMPLE 308, substituting 4-tert-butylaniline for furan-3-ylmethanamine. MS (DCI/NH₃) m/z 416 (M+H)⁻.

### EXAMPLE 337

### N-1,1'-biphenyl-4-yl-3-[(4-oxo-3,4,5,6,7,8-hexahydrophthalazin-1-yl)methyl]benzamide

The title compound was prepared according to the procedure for EXAMPLE 308, substituting biphenyl-4-amine for furan-3-ylmethanamine. MS (DCI/NH₃) m/z 436 (M+H)⁺; ¹H NMR (500 MHz, D₂O/DMSO-*d*₆): δ 1.57 - 1.70 (m, 4H), 2.34 - 2.48 (m, 4H), 4.02 (s, 2H), 7.36 (t, *J*=7.32 Hz, 1H), 7.42 (d, *J*=7.93 Hz, 1H), 7.45 - 7.48 (m, 2H), 7.49 - 7.52 (m, 1H), 7.66 - 7.71 (m, 4H), 7.78 (s, 1H), 7.81 - 7.87 (m, 3H).

### EXAMPLE 338

### N-(2-fluoro-4-methylphenyl)-3-[(4-oxo-3,4,5,6,7,8-hexahydrophthalazin-1-yl)methyl]benzamide

The title compound was prepared according to the procedure for EXAMPLE 308, substituting 2-fluoro-4-methylaniline for furan-3-ylmethanamine. MS (DCI/NH₃) m/z 392 (M+H)⁺.

### EXAMPLE 339

### N-(3-fluoro-4-methylphenyl)-3-[(4-oxo-3,4,5,6,7,8-hexahydrophthalazin-1-yl)methyl]benzamide

The title compound was prepared according to the procedure for EXAMPLE 308, substituting 3-fluoro-4-methylaniline for furan-3-ylmethanamine. MS (DCI/NH₃) m/z 392 (M+H)⁺.

### EXAMPLE 340

### N-(4-fluoro-2-methylphenyl)-3-[(4-oxo-3,4,5,6,7,8-hexahydrophthalazin-1-yl)methyl]benzamide

The title compound was prepared according to the procedure for EXAMPLE 308, substituting 4-fluoro-2-methylaniline for furan-3-ylmethanamine. MS (DCI/NH₃) m/z 392 (M+H)⁺.

### EXAMPLE 341

### N-(4-fluoro-3-methylphenyl)-3-[(4-oxo-3,4,5,6,7,8-hexahydrophthalazin-1-yl)methyl]benzamide

The title compound was prepared according to the procedure for EXAMPLE 308, substituting 4-fluoro-3-methylaniline for furan-3-ylmethanamine. MS (DCI/NH₃) m/z 392 (M+H)⁺.

### EXAMPLE 342

### N-(3-chloro-4-methylphenyl)-3-[(4-oxo-3,4,5,6,7,8-hexahydrophthalazin-1-yl)methyl]benzamide

The title compound was prepared according to the procedure for EXAMPLE 308, substituting 3-chloro-4-methylaniline for furan-3-ylmethanamine. MS (DCI/NH₃) m/z 408 (M+H)⁺.

### EXAMPLE 343

### N-(4-chloro-3-methylphenyl)-3-[(4-oxo-3,4,5,6,7,8-hexahydrophthalazin-1-yl)methyl]benzamide

The title compound was prepared according to the procedure for EXAMPLE 308, substituting 4-chloro-3-methylaniline for furan-3-ylmethanamine. MS (DCI/NH₃) m/z 408 (M+H)⁺.

### EXAMPLE 344

### N-(3-bromo-4-methylphenyl)-3-[(4-oxo-3,4,5,6,7,8-hexahydrophthalazin-1-yl)methyl]benzamide

The title compound was prepared according to the procedure for EXAMPLE 308, substituting 3-bromo-4-methylaniline for furan-3-ylmethanamine. MS (DCI/NH₃) m/z 452 (M+H)⁺.

### EXAMPLE 345

### N-(4-bromo-3-methylphenyl)-3-[(4-oxo-3,4,5,6,7,8-hexahydrophthalazin-1-yl)methyl]benzamide

The title compound was prepared according to the procedure for EXAMPLE 308, substituting 4-bromo-3-methylaniline for furan-3-ylmethanamine. MS (DCI/NH₃) m/z 452 (M+H)⁺.

### EXAMPLE 346

### N-(3-fluoro-4-methoxyphenyl)-3-[(4-oxo-3,4,5,6,7,8-hexahydrophthalazin-1-yl)mcthyl]bcnzamidc

The title compound was prepared according to the procedure for EXAMPLE 308, substituting 3-fluoro-4-methoxyaniline for furan-3-ylmethanamine. MS (DCI/NH₃) m/z 408 (M+H)⁺; ¹H NMR (500 MHz, D₂O/DMSO-*d₆*): δ 1.55 - 1.69 (m, 4H), 2.33 - 2.49 (m, 4H), 3.83 (s, 3H), 4.01 (s, 2H), 7.16 (t, *J*=9.31 Hz, 1H), 7.40 (d, *J*=7.93 Hz, 1H), 7.44 - 7.50 (m, 2H), 7.69 (dd, *J*=13.58, 2.59 Hz, 1H), 7.73 (s, 1H), 7.79 (d, *J*=7.93 Hz, 1H).

### EXAMPLE 347

### N-[3-methoxy-5-(trifluoromethyl)phenyl]-3-[(4-oxo-3,4,5,6,7,8-hexahydrophthalazin-1-yl)methyl]benzamide

The title compound was prepared according to the procedure for EXAMPLE 308, substituting 3-methoxy-5-(trifluoromethyl)aniline for furan-3-ylmethanamine. MS (DCI/NH₃) m/z 458 (M+H)⁺.

### EXAMPLE 348

### N-(2-hydroxy-6-methylphenyl)-3-[(4-oxo-3,4,5,6,7,8-hexahydrophthalazin-1-yl)methyl]benzamide

The title compound was prepared according to the procedure for EXAMPLE 308, substituting 2-amino-3-methylphenol for furan-3-ylmethanamine. MS (DCI/NH₃) m/z 390 (M+H)⁺.

### EXAMPLE 349

### N-(3-hydroxy-2-methylphenyl)-3-[(4-oxo-3,4,5,6,7,8-hexahydrophthalazin-1-yl)methyl]benzamide

The title compound was prepared according to the procedure for EXAMPLE 308, substituting 3-amino-2-methylphenol for furan-3-ylmethanamine. MS (DCI/NH₃) m/z 390 (M+H)⁺.

### EXAMPLE 350

### N-(3-hydroxy-4-methylphenyl)-3-[(4-oxo-3,4,5,6,7,8-hexahydrophthalazin-1-yl)methyl]benzamide

The title compound was prepared according to the procedure for EXAMPLE 308, substituting 2-amino-5-methylphenol for furan-3-ylmethanamine. MS (DCI/NH₃) m/z 390 (M+H)⁺.

### EXAMPLE 351

### N-(2-methoxy-5-methylphenyl)-3-[(4-oxo-3,4,5,6,7,8-hexahydrophthalazin-1-yl)mcthyl]bcnzamidc

The title compound was prepared according to the procedure for EXAMPLE 308, substituting 2-methoxy-5-methylaniline for furan-3-ylmethanamine. MS (DCI/NH₃) m/z 404 (M+H)⁺.

### EXAMPLE 352

### N-(3-methoxy-4-methylphenyl)-3-[(4-oxo-3,4,5,6,7,8-hexahydrophthalazin-1-yl)methyl]benzamide

The title compound was prepared according to the procedure for EXAMPLE 308, substituting 5-methoxy-2-methylaniline for furan-3-ylmethanamine. MS (DCI/NH₃) m/z 404 (M+H)⁺.

### EXAMPLE 353

### N-(3-hydroxy-4-methoxyphenyl)-3-[(4-oxo-3,4,5,6,7,8-hexahydrophthalazin-1-yl)methyl]benzamide

The title compound was prepared according to the procedure for EXAMPLE 308, substituting 5-amino-2-methoxyphenol for furan-3-ylmethanamine. MS (DCI/NH₃) m/z 406 (M+H)⁺.

### EXAMPLE 354

### N-(2-ethoxyphenyl)-3-[(4-oxo-3,4,5,6,7,8-hexahydrophthalazin-1-yl)methyl]benzamide

The title compound was prepared according to the procedure for EXAMPLE 308, substituting 2-ethoxyaniline for furan-3-ylmethanamine. MS (DCI/NH₃) m/z 404 (M+H)⁺.

### EXAMPLE 355

### 3-[(4-oxo-3,4,5,6,7,8-hexahydrophthalazin-1-yl)methyl]-N-(4-propoxyphenyl)benzamide

The title compound was prepared according to the procedure for EXAMPLE 308, substituting 4-propoxyaniline for furan-3-ylmethanamine. MS (DCI/NH₃) m/z 418 (M+H)⁺.

### EXAMPLE 356

### N-(5-tert-butyl-2-methoxyphenyl)-3-[(4-oxo-3,4,5,6,7,8-hexahydrophthalazin-1-yl)methyl]benzamide

The title compound was prepared according to the procedure for EXAMPLE 308, substituting 5-tert-butyl-2-methoxyaniline for furan-3-ylmethanamine. MS (DCI/NH₃) m/z 446 (M+H)⁺.

### EXAMPLE 357

### N-[5-(acetylamino)-2-methoxyphenyl]-3-[(4-oxo-3,4,5,6,7,8-hexahydrophthalazin-1-yl)methyl]benzamide

The title compound was prepared according to the procedure for EXAMPLE 308, substituting N-(3-amino-4-methoxyphenyl)acetamide for furan-3-ylmethanamine. MS (DCI/NH₃) m/z 447 (M+H)⁺.

### EXAMPLE 358

### N-2,3-dihydro-1,4-benzodioxin-6-yl-3-[(4-oxo-3,4,5,6,7,8-hexahydrophthalazin-1-yl)methyl]benzamide

The title compound was prepared according to the procedure for EXAMPLE 308, substituting 2,3-dihydrobenzo[b][1,4]dioxin-6-amine for furan-3-ylmethanamine. MS (DCI/NH₃) m/z 418 (M+H)⁺; ¹H NMR (500 MHz, D₂O/DMSO-*d₆*): δ 1.56 - 1.68 (m, 4H), 2.34 - 2.48 (m, 4H), 4.00 (s, 2H), 4.15 - 4.32 (m, 4H), 6.84 (d, *J*=8.85 Hz, 1H), 7.16 (dd, *J*=8.85, 2.44 Hz, 1H), 7.34 (d, *J*=2.44 Hz, 1H), 7.38 (d, *J*=7.93 Hz, 1H), 7.46 (t, *J*=7.63 Hz, 1 H), 7.72 (s, 1H), 7.77 (d, *J*=7.63 Hz, 1 H).

### EXAMPLE 359

### N-(5-chloro-2,4-dimethoxyphenyl)-3-[(4-oxo-3,4,5,6,7,8-hexahydrophthalazin-1-yl)methyl]benzamide

The title compound was prepared according to the procedure for EXAMPLE 308, substituting 5-chloro-2,4-dimethoxyaniline for furan-3-ylmethanamine. MS (DCI/NH₃) m/z 454 (M+H)⁺.

### EXAMPLE 360

### N-[3-(methylthio)phenyl]-3-[(4-oxo-3,4,5,6,7,8-hexahydrophthalazin-1-yl)methyl]benzamide

The title compound was prepared according to the procedure for EXAMPLE 308, substituting 3-(methylthio)aniline for furan-3-ylmethanamine. MS (DCI/NH₃) m/z 406 (M+H)⁺.

### EXAMPLE 361

### N-[4-(methylthio)phenyl]-3-[(4-oxo-3,4,5,6,7,8-hexahydrophthalazin-1-yl)methyl]benzamide

The title compound was prepared according to the procedure for EXAMPLE 308, substituting 4-(methylthio)aniline for furan-3-ylmethanamine. MS (DCI/NH₃) m/z 406 (M+H)⁺.

### EXAMPLE 362

### 3-[(4-oxo-3,4,5,6,7,8-hexahydrophthalazin-1-yl)methyl]-N-(4-piperidin-1-ylphenyl)benzamide

The title compound was prepared according to the procedure for EXAMPLE 308, substituting 4-(piperidin-1-yl)aniline for furan-3-ylmethanamine. MS (DCI/NH₃) m/z 443 (M+H)⁺.

### EXAMPLE 363

### N-(4-morpholin-4-ylphenyl)-3-[(4-oxo-3,4,5,6,7,8-hexahydrophthalazin-1-yl)methyl]benzamide

The title compound was prepared according to the procedure for EXAMPLE 308, substituting 4-morpholinoaniline for furan-3-ylmethanamine. MS (DCI/NH₃) m/z 445 (M+H)⁺; ¹H NMR (500 MHz, D₂O/DMSO-*d₆*): δ 1.57 - 1.68 (m, 4H), 2.34 - 2.46 (m, 4H), 3.15 - 3.23 (m, 4H), 3.79 - 3.82 (m, 4H), 4.01 (s, 2H), 7.10 (d, *J*=9.15 Hz, 2H), 7.39 (d, *J*=7.63 Hz, 1H), 7.45 - 7.50 (m, 1H), 7.66 (d, *J*=9.15 Hz, 2H), 7.72 - 7.77 (m, 1H), 7.80 (d, *J*=7.93 Hz, 1H).

### EXAMPLE 364

### N-(2-anilinophenyl)-3-[(4-oxo-3,4,5,6,7,8-hexahydrophthalazin-1-yl)methyl]benzamide

The title compound was prepared according to the procedure for EXAMPLE 308, substituting N¹-phenylbenzene-1,2-diamine for furan-3-ylmethanamine. MS (DCI/NH₃) m/z 451 (M+H)⁺; ¹H NMR (500 MHz, D₂O/DMSO-*d*₆): δ 1.54 - 1.69 (m, 4H), 2.31 - 2.44 (m, 4H), 3.95 (s, 2H), 6.78 (t, *J*=7.32 Hz, 1H), 6.86 (d, *J*=7.63 Hz, 2H), 7.02 - 7.09 (m, 1H), 7.15 - 7.23 (m, 3H), 7.28 - 7.32 (m, 1H), 7.35 - 7.39 (m, 1H), 7.42 (t, *J*=7.63 Hz, 1H), 7.59 (d, *J*=7.32 Hz, 1H), 7.63 (s, 1H), 7.69 (d, *J*=7.63 Hz, 1H).

### EXAMPLE 365

### N-{4-[(4-methoxyphenyl)amino]phenyl}-3-[(4-oxo-3,4,5,6,7,8-hexahydrophthalazin-1-yl)methyl]benzamide

The title compound was prepared according to the procedure for EXAMPLE 308, substituting N¹-(4-methoxyphenyl)benzene-1,2-diamine for furan-3-ylmethanamine. MS (DCI/NH₃) m/z 481 (M+H)⁺.

### EXAMPLE 366

### 3-[(4-oxo-3,4,5,6,7,8-hexahydrophthalazin-1-yl)methyl]-N-quinolin-6-ylbenzamide

The title compound was prepared according to the procedure for EXAMPLE 308, substituting quinolin-7-amine for furan-3-ylmethanamine. MS (DCI/NH₃) m/z 411 (M+H)⁺; ¹H NMR (500 MHz, D₂O/DMSO-*d₆*): δ1.57-1.72 (m, 4H), 2.36 - 2.49 (m, 4H), 4.04 (s, 2H), 7.44 - 7.50 (m, 1H), 7.54 (t, *J*=7.63 Hz, 1H), 7.82 (s, 1H), 7.88 (dd, *J*=8.24, 5.19 Hz, 2H), 8.19 (d, *J*=9.15 Hz, 1H), 8.27 (dd, *J*=9.15, 2.14 Hz, 1H), 8.74 (d, *J*=2.44 Hz, 1H), 8.88 (d, *J*=7.93 Hz, 1H), 9.04 (d, *J*=4.88 Hz, 1H).

### EXAMPLE 367

### N-(5-hydroxy-1-naphthyl)-3-[(4-oxo-3,4,5,6,7,8-hexahydrophthalazin-1-yl)methyl]benzamide

The title compound was prepared according to the procedure for EXAMPLE 308, substituting 5-aminonaphthalen-1-ol for furan-3-ylmethanamine. MS (DCI/NH₃) m/z 426 (M+H)⁺.

### EXAMPLE 368

### N-1H-indazol-6-yl-3-[(4-oxo-3,4,5,6,7,8-hexahydrophthalazin-1-yl)methyl]benzamide

The title compound was prepared according to the procedure for EXAMPLE 308, substituting 1H-indazol-6-amine for furan-3-ylmethanamine. MS (DCI/NH₃) m/z 400 (M+H)⁺.

### EXAMPLE 369

### 8-(4-fluorobenzyl)pyrido[3,2-d]pyridazin-5(6H)-one

### EXAMPLE 369A

### methyl 2-(2-(4-fluorophenyl)acetyl)nicotinate

To a solution of dimethyl pyridine-2,3-dicarboxylate (1.0 g, 5.1 mmol) in tetrahydrofuran (50 ml) was added (4-fluorobenzyl)magnesium chloride (0.25 M in tetrahydrofuran, 20 ml, 5.1 mmol) through a syringe at -78°C. The reaction mixture was stirred at the same temperature for 30 minutes and was quenched with addition of water. After warming up to room temperature, the reaction mixture was partitioned between ethyl acetate and brine. The organic phase was washed with brine and concentrated. The residue was purified by flash chromatography (15% ethyl acetate in hexane) to give the title compound. MS (DCI/NH₃) m/z 274 (M+H)⁺.

### EXAMPLE 369B

### 8-(4-fluorobenzyl)pyrido[3,2-d]pyridazin-5(6H)-one

A solution of EXAMPLE 369A (0.46 g, 1.68 mmol) in ethanol (20 ml) was treated with hydrazine (108 mg, 3.37 mmol) at room temperature for 5 hours. The reaction mixture was concentrated to about 5 mL. The solid was collected by filtration, washed with ethanol and dried to provide the title compound. MS (DCI/NH₃) m/z 256 (M+H)⁻.

### EXAMPLE 370

### 8-(3-chloro-4-fluorobenzyl)pyrido[3,2-d]pyridazin-5(6H)-one

### EXAMPLE 370A

### methyl 2-(2-(3-chloro-4-fluorophcnyl)acetyl)nicotinate

The title compound was prepared according to the procedure for EXAMPLE 369A, substituting (2-chloro-4-fluorobenzyl)magnesium chloride for (4-fluorobenzyl)magnesium chloride. MS (DCI/NH₃) m/z 308 (M+H)⁺.

### EXAMPLE 370B

### 8-(3-chloro-4-fluorobenzyl)pyrido[3,2-d]pyridazin-5(6H)-one

The title compound was prepared according to the procedure for EXAMPLE 369B, substituting EXAMPLE 370A for EXAMPLE 369A. MS (DCI/NH₃) m/z 290 (M+H)⁺.

### EXAMPLE 371

### (3aR)-8-[(4-oxo-3,4,5,6,7,8-hexahydrophthalazin-1-yl)methyl]-2,3,3a,4-tetrahydro-1H-pyrrolo[2,1-c][1,4]benzoxazin-1-one

### EXAMPLE 371A

### (R)-1-oxo-2,3,3a,4-tetrahydro-1H-benzo[b]pyrrolo[1,2-d][1,4]oxazine-8-carbaldehyde

The title compound was prepared according to the procedure for EXAMPLE 300A, substituting (R)-5-(hydroxymethyl)pyrrolidin-2-one for 5-methylpyrrolidinone. MS (DCI/NH₃) m/z 232 (M+H)⁺.

### EXAMPLE 371B

### (3aR)-8-[(4-oxo-3,4,5,6,7,8-hexahydrophthalazin-1-yl)methyl]-2,3,3a,4-tetrahydro-1H-pyrrolo[2,1-c][1,4]benzoxazin-1-one

The title compound was prepared according to the procedure for EXAMPLE 300B, substituting EXAMPLE 371A for EXAMPLE 300A. MS (DCI/NH₃) m/z 352 (M+H)⁺; ¹H NMR (300 MHz, DMSO-*d*₆): δ 1.54 - 1.63 (m, 4H), 1.64 - 1.72 (m, 1H), 2.13 - 2.22 (m, 1H), 2.23 - 2.31 (m, 1H), 2.33 - 2.44 (m, 4H), 2.54 - 2.64 (m, 1H), 3.72 (t, *J*=10.17 Hz, 1H), 3.78 - 3.84 (m, 2H), 3.91 - 4.05 (m, 1H), 4.48 (dd, *J*=10.51, 3.05 Hz, 1H), 6.77 - 6.82 (m, 1H), 6.84 - 6.89 (m, 1H), 8.26 (d, *J*=2.03 Hz, 1H), 12.58 (br s, 1H).

### EXAMPLE 372

### N-{2-fluoro-5-[(4-oxo-3,4,5,6,7,8-hexahydrophthalazin-1-yl)methyl]phenyl}-N-methylmethanesulfonamide

### EXAMPLE 372A

### N-(2-fluoro-5-formylphenyl)-N-methylmethanesulfonamide

The title compound was prepared according to the procedure for EXAMPLE 300A, substituting N-methylmethanesulfonamide for 5-methylpyrrolidinone. MS (DCI/NH₃) m/z 232 (M+H)⁺.

### EXAMPLE 372B

### N-{2-fluoro-5-[(4-oxo-3,4,5,6,7,8-hexahydrophthalazin-1-yl)methyl]phenyl}-N-methylmethanesulfonamide

The title compound was prepared according to the procedure for EXAMPLE 300B, substituting EXAMPLE 372A for EXAMPLE 300A. MS (DCI/NH₃) m/z 366 (M+H)⁺; ¹H NMR (300 MHz, DMSO-*d*₆)_{:} δ 1.57 - 1.71 (m, 4H), 2.34 - 2.47 (m, 4H), 3.13 (s, 6H), 3.93 (s, 2H), 7.25 (dd, *J*=8.33, 1.98 Hz, 1H), 7.51 (d, *J*=7.93 Hz, 1H), 7.58 (d, *J*=1.98 Hz, 1H), 12.62 (br s, 1H).

### EXAMPLE 373

### N-{2-fluoro-5-[(4-oxo-3,4,5,6,7,8-hexahydrophthalazin-1-yl)methyl]phenyl}-2-hydroxy-2-mcthylpropanamidc

### EXAMPLE 373A

### N-(2-fluoro-5-formylphenyl)-2-hydroxy-2-methylpropanamide

The title compound was prepared according to the procedure for EXAMPLE 300A, substituting 5,5-dimethyloxazolidine-2,4-dione for 5-methylpyrrolidinone. MS (DCI/NH₃) m/z 226 (M+H)⁺.

### EXAMPLE 373B

### N-{2-fluoro-5-[(4-oxo-3,4,5,6,7,8-hexahydrophthalazin-1-yl)methyl]phenyl}-2-hydroxy-2-methylpropanamide

The title compound was prepared according to the procedure for EXAMPLE 300B, substituting EXAMPLE 373A for EXAMPLE 300A. MS (DCI/NH₃) m/z 360 (M+H)⁺; ¹H NMR (300 MHz, DMSO-*d*₆)_{:} δ 1.34 (s, 6H), 1.53 - 1.70 (m, 4H), 2.29 - 2.45 (m, 4H), 3.87 (s, 2H), 6.85 - 7.02 (m, 1H), 7.20 (dd, *J*=10.91, 8.53 Hz, 1H), 7.91 (dd, *J*=7.54, 1.98 Hz, 1H), 9.24 (s, 1H), 12.62 (s, 1H).

### EXAMPLE 374

### (3aS)-8-[(4-oxo-3,4,5,6,7,8-hexahydrophthalazin-1-yl)methyl]-2,3,3a,4-tetrahydro-1H-pyrrolo[2,1-c][1,4]benzoxazin-1-one

### EXAMPLE 374A

### (S)-1-oxo-2,3,3a,4-tetrahydro-1H-benzo[b]pyrrolo[1,2-d][1,4]oxazine-8-carbaldehyde

The title compound was prepared according to the procedure for EXAMPLE 300A, substituting (S)-5-(hydroxymethyl)pyrrolidin-2-one for 5-methylpyrrolidinone. MS (DCI/NH₃) m/z 232 (M+H)⁺.

### EXAMPLE 374B

### (3aS)-8-[(4-oxo-3,4,5,6,7,8-hexahydrophthalazin-1-yl)methyl]-2,3,3a,4-tetrahydro-1H-pyrrolo[2,1-c][1,4]benzoxazin-1-one

The title compound was prepared according to the procedure for EXAMPLE 300A, substituting EXAMPLE 374A for EXAMPLE 300B. MS (DCI/NH₃) m/z 352 (M+H)⁺; ¹H NMR (300 MHz, DMSO-*d*₆)_{:} δ 1.54 - 1.62 (m, 4H), 1.63 - 1.76 (m, 1H), 2.13 - 2.22 (m, 1H), 2.23 - 2.31 (m, 2H), 2.32 - 2.40 (m, 4H), 3.72 (t, *J*=10.31 Hz, 1H), 3.81 (s, 2H), 3.90 - 4.04 (m, 1H), 4.48 (dd, *J*=10.71, 3.17 Hz, 1H), 6.77 - 6.83 (m, 1H), 6.84 - 6.91 (m, 1H), 8.26 (d, *J*=1.98 Hz, 1H), 12.58 (br s, 1H).

### EXAMPLE 375

### 3-[(4-oxo-3,4,5,6,7,8-hexahydrophthalazin-1-yl)methyl]-N-(2-phenylethyl)benzamide

The title compound was prepared according to the procedure for EXAMPLE 308, substituting 2-phenylethanamine for furan-3-ylmethanamine. MS (DCI/NH₃) m/z 388 (M+H)⁺; ¹H NMR (500 MHz, D₂O/DMSO-*d*₆): δ 1.56 - 1.69 (m, 4H), 2.33 - 2.45 (m, 4H), 2.84 (t, *J*=7.48 Hz, 2H), 3.43 - 3.51 (m, 2H), 3.96 (s, 2H), 7.21 (t, *J*=7.17 Hz, 1H), 7.23 - 7.26 (m, 2H), 7.27 - 7.32 (m, 2H), 7.32 - 7.36 (m, 1H), 7.40 (t, *J*=7.63 Hz, 1H), 7.60 (s, 1H), 7.64 (d, *J*=7.93 Hz, 1H).

### EXAMPLE 376

### N-[2-(2-methylphenyl)ethyl]-3-[(4-oxo-3,4,5,6,7,8-hexahydrophthalazin-1-yl)methyl]benzamide

The title compound was prepared according to the procedure for EXAMPLE 308, substituting 2-o-tolylethanamine for furan-3-ylmethanamine. MS (DCI/NH₃) m/z 402 (M+H)⁺.

### EXAMPLE 377

### N-[2-(3-methylphenyl)ethyl]-3-[(4-oxo-3,4,5,6,7,8-hexahydrophthalazin-1-yl)methyl]benzamide

The title compound was prepared according to the procedure for EXAMPLE 308, substituting 2-m-tolylethanamine for furan-3-ylmethanamine. MS (DCI/NH₃) m/z 402 (M+H)⁺.

### EXAMPLE 378

### N-[2-(4-methylphenyl)ethyl]-3-[(4-oxo-3,4,5,6,7,8-hexahydrophthalazin-1-yl)methyl]benzamide

The title compound was prepared according to the procedure for EXAMPLE 308, substituting 2-p-tolylethanamine for furan-3-ylmethanamine. MS (DCI/NH₃) m/z 402 (M+H)⁺.

### EXAMPLE 379

### 3-[(4-oxo-3,4,5,6,7,8-hexahydrophthalazin-1-yl)methyl]-N-(2-pyridin-2-ylethyl)benzamide

The title compound was prepared according to the procedure for EXAMPLE 308, substituting 2-(pyridin-2-yl)ethanamine for furan-3-ylmethanamine. MS (DCI/NH₃) m/z 389 (M+H)⁺; ¹H NMR (500 MHz, D₂O/DMSO-*d*₆): δ 1.57 - 1.68 (m, 4H), 2.31 - 2.43 (m, 4H), 3.24 (t, *J*=6.56 Hz, 2H), 3.69 (t, *J*=6.41 Hz, 2H), 3.95 (s, 2H), 7.33 - 7.37 (m, 1H), 7.40 (t, *J*=7.63 Hz, 1H), 7.53 (s, 1H), 7.57 (d, *J*=7.63 Hz, 1H), 7.86 - 7.89 (m, 1H), 7.90 - 7.94 (m, 1H), 8.40 - 8.49 (m, 1H), 8.75 (d, *J*=4.88 Hz, 1H).

### EXAMPLE 380

### 3-[(4-oxo-3,4,5,6,7,8-hexahydrophthalazin-1-yl)methyl]-N-(2-pyridin-3-ylethyl)benzamide

The title compound was prepared according to the procedure for EXAMPLE 308, substituting 3-(pyridin-2-yl)ethanamine for furan-3-ylmethanamine. MS (DCI/NH₃) m/z 389 (M+H)⁺.

### EXAMPLE 381

### 3-[(4-oxo-3,4,5,6,7,8-hexahydrophthalazin-1-yl)methyl]-N-(2-pyridin-4-ylethyl)benzamide

The title compound was prepared according to the procedure for EXAMPLE 308, substituting 4-(pyridin-2-yl)ethanamine for furan-3-ylmethanamine. MS (DCI/NH₃) m/z 389 (M+H)⁺.

### EXAMPLE 382

### N-[2-(2-methoxyphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(2-methoxyphenyl)ethanamine for furan-3-ylmethanamine. MS (DCI/NH₃) m/z 418 (M+H)⁺.

### EXAMPLE 383

### N-[2-(3-methoxyphenyl)ethyl]-3-[(4-oxo-3,4,5,6,7,8-hexahydrophthalazin-1-yl)methyl]benzamide

The title compound was prepared according to the procedure for EXAMPLE 308, substituting 3-(2-methoxyphenyl)ethanamine for furan-3-ylmethanamine. MS (DCI/NH₃) m/z 418(M+H)⁺.

### EXAMPLE 384

### N-[2-(4-methoxyphenyl)ethyl]-3-[(4-oxo-3,4,5,6,7,8-hexahydrophthalazin-1-yl)mcthyl]bcnzamidc

The title compound was prepared according to the procedure for EXAMPLE 308, substituting 4-(2-methoxyphenyl)ethanamine for furan-3-ylmethanamine. MS (DCI/NH₃) m/z 418 (M+H)⁺; ¹H NMR (500 MHz, D₂O/DMSO-*d₆*): δ 1.55 - 1.68 (m, 4H), 2.32 - 2.45 (m, 4H), 2.77 (t, *J*=7.48 Hz, 2H), 3.37 - 3.47 (m, 2H), 3.71 (s, 3H), 3.96 (s, 2H), 6.83 - 6.88 (m, 2H), 7.14 - 7.20 (m, 2H), 7.32 - 7.36 (m, 1H), 7.40 (t, *J*=7.63 Hz, 1H), 7.61 (s, 1H), 7.64 (d, *J*=7.63 Hz, 1H).

### EXAMPLE 385

### N-[2-(2-fluorophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(2-fluorophenyl)ethanamine for furan-3-ylmethanamine. MS (DCI/NH₃) m/z 406 (M+H)⁺.

### EXAMPLE 386

### N-[2-(3-fluorophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(3-fluorophenyl)ethanamine for furan-3-ylmethanamine. MS (DCI/NH₃) m/z 406 (M+H)⁺.

### EXAMPLE 387

### N-[2-(4-fluorophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(4-fluorophenyl)ethanamine for furan-3-ylmethanamine. MS (DCI/NH₃) m/z 406 (M+H)⁺.

### EXAMPLE 388

### N-[2-(2-chlorophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(2-chlorophenyl)ethanamine for furan-3-ylmethanamine. MS (DCI/NH₃) m/z 422 (M+H)⁺.

### EXAMPLE 389

### N-[2-(3-chlorophenyl)ethyl]-3-[(4-oxo-3,4,5,6,7,8-hexahydrophthalazin-1-yl)mcthyl]bcnzamidc

The title compound was prepared according to the procedure for EXAMPLE 308, substituting 2-(3-chlorophenyl)ethanamine for furan-3-ylmethanamine. MS (DCI/NH₃) m/z 422 (M+H)⁺.

### EXAMPLE 390

### N-[2-(4-chlorophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(4-chlorophenyl)ethanamine for furan-3-ylmethanamine. MS (DCI/NH₃) m/z 422 (M+H)⁺.

### EXAMPLE 391

### N-[2-(3-bromophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(3-bromophenyl)ethanamine for furan-3-ylmethanamine. MS (DCI/NH₃) m/z 467 (M+H)⁺.

### EXAMPLE 392

### N-[2-(4-bromophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(4-bromophenyl)ethanamine for furan-3-ylmethanamine. MS (DCI/NH₃) m/z 467 (M+H)⁺.

### EXAMPLE 393

### N-[2-(1,1'-biphenyl-4-yl)ethyl]-3-[(4-oxo-3,4,5,6,7,8-hexahydrophthalazin-1-yl)methyl]benzamide

The title compound was prepared according to the procedure for EXAMPLE 308, substituting 2-(biphenyl-4-yl)ethanamine for furan-3-ylmethanamine. MS (DCI/NH₃) m/z 464 (M+H)⁺; ¹H NMR (500 MHz, D₂O/DMSO-*d₆*): δ 1.54 - 1.67 (m, 4H), 2.30 - 2.44 (m, 4H), 2.89 (t, *J*=7.48 Hz, 2H), 3.52 (t, *J*=7.32 Hz, 2H), 3.96 (s, 2H), 7.33 - 7.37 (m, 4H), 7.41 (t, *J*=7.63 Hz, 1H), 7.44 - 7.50 (m, 2H), 7.59 (d, *J*=8.24 Hz, 2H), 7.64 (d, *J*=8.24 Hz, 2H), 7.64 - 7.68 (m, 2H).

### EXAMPLE 394

### 3-[(4-oxo-3,4,5,6,7,8-hexahydrophthalazin-1-yl)methyl]-N-{2-[3-(trifluoromethyl)phenyl]ethyl}benzamide

The title compound was prepared according to the procedure for EXAMPLE 308, substituting 2-(3-(trifluoromethyl)phenyl)ethanamine for furan-3-ylmethanamine. MS (DCI/NH₃) ml) 456 (M+H)⁺.

### EXAMPLE 395

### 3-[(4-oxo-3,4,5,6,7,8-hexahydrophthalazin-1-yl)methyl]-N-{2-[4-(trifluoromethyl)phenyl] ethyl} benzamide

The title compound was prepared according to the procedure for EXAMPLE 308, substituting 2-(4-(trifluoromethyl)phenyl)ethanamine for furan-3-ylmethanamine. MS (DCI/NH₃) m/z 456 (M+H)⁺.

### EXAMPLE 396

### 3-[(4-oxo-3,4,5,6,7,8-hexahydrophthalazin-1-yl)methyl]-N-[2-(4-phenoxyphenyl)ethyl]benzamide

The title compound was prepared according to the procedure for EXAMPLE 308, substituting 2-(4-phenoxyphenyl)ethanamine for furan-3-ylmethanamine. MS (DCI/NH₃) m/z 480 (M+H)⁺; ¹H NMR (500 MHz, D₂O/DMSO-*d₆*): δ 1.55 - 1.66 (m, 4H), 2.32 - 2.43 (m, 4H), 2.83 (t, *J*=7.32 Hz, 2H), 3.48 (t, *J*=7.32 Hz, 2H), 3.96 (s, 2H), 6.91 - 6.94 (m, 2H), 6.96 (d, *J*=7.63 Hz, 2H), 7.12 (t, *J*=7.48 Hz, 1H), 7.26 (d, *J*=8.54 Hz, 2H), 7.32 - 7.35 (m, 1H), 7.35 - 7.38 (m, 2H), 7.38 - 7.43 (m, 1H), 7.62 (s, 1H), 7.64 (d, *J*=7.63 Hz, 1H).

### EXAMPLE 397

### N-[2-(3,4-dimethylphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(3,4-dimethylphenyl)ethanamine for furan-3-ylmethanamine. MS (DCI/NH₃) m/z 416 (M+H)⁺.

### EXAMPLE 398

### N-[2-(2,4-dimethylphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(2,4-dimethylphenyl)ethanamine for furan-3-ylmethanamine. MS (DCI/NH₃) m/z 416 (M+H)⁺.

### EXAMPLE 399

### N-[2-(2,5-dimethylphenyl)ethyl]-3-[(4-oxo-3,4,5,6,7,8-hexahydrophthalazin-1-yl)mcthyl]bcnzamidc

The title compound was prepared according to the procedure for EXAMPLE 308, substituting 2-(2,5-dimethylphenyl)ethanamine for furan-3-ylmethanamine. MS (DCI/NH₃) m/z 416 (M+H)⁺.

### EXAMPLE 400

### N-[2-(3-ethoxy-4-methoxyphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(3-ethoxy-4-methoxyphenyl)ethanamine for furan-3-ylmethanamine. MS (DCI/NH₃) m/z 462 (M+H)⁺.

### EXAMPLE 401

### N-[2-(4-ethoxy-3-methoxyphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(4-ethoxy-3-methoxyphenyl)ethanamine for furan-3-ylmethanamine. MS (DCI/NH₃) m/z 462 (M+H)⁺.

### EXAMPLE 402

### N-[2-(2,3-dimethoxyphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(2,3-dimethoxyphenyl)ethanamine for furan-3-ylmethanamine. MS (DCI/NH₃) m/z 448 (M+H)⁺.

### EXAMPLE 403

### N-[2-(2,4-dimethoxyphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(2,4-dimethoxyphenyl)ethanamine for furan-3-ylmethanamine. MS (DCI/NH₃) m/z 448 (M+H)⁺.

### EXAMPLE 404

### N-[2-(2,5-dimethoxyphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(2,5-dimethoxyphenyl)ethanamine for furan-3-ylmethanamine. MS (DCI/NH₃) m/z 448 (M+H)⁺.

### EXAMPLE 405

### N-[2-(3,4-dimethoxyphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(3,4-dimethoxyphenyl)ethanamine for furan-3-ylmethanamine. MS (DCI/NH₃) m/z 448 (M+H)⁺.

### EXAMPLE 406

### N-[2-(3,5-dimethoxyphenyl)ethyl]-3-[(4-oxo-3,4,5,6,7,8-hexahydrophthalazin-1-yl)methyl]benzamide

The title compound was prepared according to the procedure for EXAMPLE 308, substituting 2-(3,5-dimethoxyphenyl)ethanamine for furan-3-ylmethanamine. MS (DCI/NH₃) m/z 448 (M+H)⁺.

### EXAMPLE 407

### N-[2-(1,3-benzodioxol-5-yl)ethyl]-3-[(4-oxo-3,4,5,6,7,8-hexahydrophthalazin-1-yl)methyl]benzamide

The title compound was prepared according to the procedure for EXAMPLE 308, substituting 2-(benzo[d][1,3]dioxol-5-yl)ethanamine for furan-3-ylmethanamine. MS (DCI/NH₃) m/z 432 (M+H)⁺; ¹H NMR (500 MHz, D₂O/DMSO-*d₆*): δ 1.54 - 1.72 (m, 4H), 2.32 - 2.44 (m, 4H), 2.75 (t, *J*=7.32 Hz, 2H), 3.43 (t, *J*=7.32 Hz, 2H), 3.96 (s, 2H), 5.94 (s, 2H), 6.70 (dd, *J*=7.93, 1.53 Hz, 1H), 6.80 (s, 1H), 6.81 - 6.83 (m, 1H), 7.32 - 7.36 (m, 1H), 7.40 (t, *J*=7.63 Hz, 1H), 7.61 (s, 1H), 7.64 (d, *J*=7.93 Hz, 1H).

### EXAMPLE 408

### N-[2-(2,3-dichlorophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(2,3-dichlorophenyl)ethanamine for furan-3-ylmethanamine. MS (DCI/NH₃) m/z 457 (M+H)⁺.

### EXAMPLE 409

### N-[2-(3,4-dichlorophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(3,4-dichlorophenyl)ethanamine for furan-3-ylmethanamine. MS (DCI/NH₃) m/z 457 (M+H)⁺.

### EXAMPLE 410

### N-[2-(2,6-dichlorophenyl)ethyl]-3-[(4-oxo-3,4,5,6,7,8-hexahydrophthalazin-1-yl)methyl]benzamide

The title compound was prepared according to the procedure for EXAMPLE 308, substituting 2-(2,6-dichlorophenyl)ethanamine for furan-3-ylmethanamine. MS (DCI/NH₃) m/z 457 (M+H)⁺.

### EXAMPLE 411

### (3aS,4R,7S,7aR)-5-{2-fluoro-5-[(4-oxo-3,4,5,6,7,8-hexahydrophthalazin-1-yl)methyl]phenyl}-2,2-dimethyltetrahydro-4,7-methano[1,3]dioxolo[4,5-c]pyridin-6(3aH)-one

### EXAMPLE 411A

### 3-[(3aS,4R,7S,7aR)-2,2-dimethyl-6-oxotetrahydro-4,7-methano[1,3]dioxolo[4,5-c]pyridin-5(4H)-yl]-4-fluorobenzaldehyde

The title compound was prepared according to the procedure for EXAMPLE 300A, substituting (1S, 2R, 6S, 7R)-4,4-dimethyl-3,5-dioxa-8-azatricyclo[5.2.1.0(2,6)]decan-9-one for 5-methylpyrrolidinone. MS (DCI/NH₃) m/z 306 (M+H)⁺.

### EXAMPLE 411B

### (3aS,4R,7S,7aR)-5-{2-fluoro-5-[(4-oxo-3,4,5,6,7,8-hexahydrophthalazin-1-yl)methyl]phenyl}-2,2-dimethyltetrahydro-4,7-methano[1,3]dioxolo[4,5-c]pyridin-6(3aH)-one

The title compound was prepared according to the procedure for EXAMPLE 300B, substituting EXAMPLE 411A for EXAMPLE 300B. MS (DCI/NH₃) m/z 440 (M+H)⁺; ¹H NMR (300 MHz, DMSO-*d*₆)_{:} δ 1.29 - 1.34 (m, 3H), 1.42 (s, 3H), 1.55 - 1.67 (m, 4H), 2.01 - 2.11 (m, 1H), 2.12 - 2.21 (m, 1H), 2.32 - 2.45 (m, 4H), 2.77 - 2.84 (m, 1H), 3.88 (s, 2H), 4.16 - 4.24 (m, 1H), 4.58 - 4.64 (m, 1H), 4.64 - 4.69 (m, 1H), 7.02 - 7.09 (m, 1H), 7.22 (dd, *J*=11.19, 8.48 Hz, 1H), 7.31 (dd, *J*=7.46, 2.03 Hz, 1H), 12.59 (s, 1H).

### EXAMPLE 412

### 4-(1-(3-bromo-4-fluorophenyl)ethyl)-5,6,7,8-tetrahydrophthalazin-1(2H)-one

### EXAMPLE 412A

### 1-(3-bromo-4-fluorophenyl)ethanol

A solution of 1-(3-bromo-4-fluorophenyl)ethanone (15.0 g, 69 mmol) in tetrahydrofuran (200 mL) was treated with sodium borohydride (5.3 g, 138 mmol) at 0 °C. After the addition, the ice bath was removed, and the mixture was stirred at room temperature for 30 minutes and at reflux overnight. After cooling, 1N HC1 (10 mL) was slowly added and the reaction mixture was concentrated. The residue was partitioned between ethyl acetate and brine. The organic phase was washed with water, and concentrated. The residue was purified by flash chromatography (30% ethyl acetate in hexane) to provide the title compound. MS (DCI/NH₃) m/z 220 (M+H)⁺.

### EXAMPLE 412B

### 2-bromo-4-(1-bromoethyl)-1-fluorobenzene

To a solution of EXAMPLE 412A (1.5 g, 6.8 mmol) and triphenyl phosphine (1.9 g, 7.2 mmol) in dimethylformamide (20 ml) was added bromine (1.1 g, 6.8 mmol) through a syringe. After the addition, the reaction mixture was stirred at room temperature for additional 15 minutes, and partitioned between water (100 ml) and ethyl acetate (200 ml). The organic phase was washed with brine and concentrated. The residue was purified by flash chromatography (2.6% ethyl acetate in hexane) to provide the title compound. MS (DCI/NH₃) m/z 282 (M+H)⁺.

### EXAMPLE 412B

### (1-(3-bromo-4-fluorophenyl)ethyl)triphenylphosphonium bromide

The title compound was prepared according to the procedure for EXAMPLE 285B, substituting EXAMPLE 412A for EXAMPLE 285A.

### EXAMPLE 412C

### 3-(1-(3-bromo-4-fluorophenyl)ethylidene)-4,5,6,7-tetrahydroisobenzofuran-1(3H)-one

The title compound was prepared according to the procedure for EXAMPLE 285C, substituting EXAMPLE 412B for EXAMPLE 285B. MS (DCI/NH₃) m/z 338 (M+H)⁺.

### EXAMPLE 412D

### 4-(1-(3-bromophenyl)ethyl)-5,6,7,8-tetrahydrophthalazin-1(2H)-one

The title compound was prepared according to the procedure for EXAMPLE 2C, substituting EXAMPLE 412C for EXAMPLE 2B. MS (DCI/NH₃) m/z 352 (M+H)⁺.

### EXAMPLE 413

### 4-(1-(4-fluoro-3-(2-oxopyrrolidin-1-yl)phenyl)ethyl)-5,6,7,8-tetrahydrophthalazin-1(2H)-one

The title compound was prepared according to procedure for EXAMPLE 101, substituting EXAMPLE 412 for EXAMPLE 103, and pyrrolinc-2-onc for azetidin-2-one. MS (ESI) m/z 356 (M+H)⁺; ¹H NMR (300 MHz, DMSO-*d*₆)_{:} δ 1.42 (d, *J*=6.74 Hz, 3 H), 1.46 - 1.70 (m, 4 H), 1.93 - 2.16 (m, 4 H), 2.29 - 2.67 (m, 6 H), 4.25 (q, *J*=6.74 Hz, 1 H), 7.07 - 7.15 (m, 1 H), 7.18 (s, 1 H), 7.19 - 7.29 (m, 1 H), 12.70 (s, 1 H).

### EXAMPLE 414

### 8-(4-fluorobenzyl)-1,2,3,4-tetrahydropyrido[3,2-d]pyridazin-5(6H)-one

A mixture of EXAMPLE 369 (150 mg, 0.6 mmol), 5% platinum on carbon (30 mg), concentrated aqueous HCl (50 µL) and dimethylformamide (5 ml) in a pressure vessel was stirred at room temperature under 50 psi of hydrogen for 16 hours. The mixture was filtered, and the filtrate was concentrated. The residual solid was purified by HPLC (Zorbax® C-18 ODS packing material [Agilent Technologies, Santa Clara, CA], 0.1% TFA/CH₃CN/H₂O) to provide the title product as TFA salt. MS (ESI) m/z 260 (M+H)⁺.

### EXAMPLE 415

### 8-(3-bromo-4-fluorobenzyl)pyrido[3,2-d]pyridazin-5(6H)-one

### EXAMPLE 415A

### methyl 2-(2-(3-bromo-4-fluorophenyl)acetyl)nicotinate

A mixture of magnesium turnings (880 mg, 37 mmol) and 2-bromo-4-(bromomethyl)-1-fluorobenzene (1.0 g, 3.7 mmol) in anhydrous diethyl ether (15 ml) was treated with a piece of iodine. The mixture was then heated to gentle reflux until the color of the mixture disappeared, after which the heating continued for additional hour. The suspension was cooled to room temperature, and cannulated into a solution of dimethyl pyridine-2,3-dicarboxylate (1.0 g, 5.1 mmol) in tetrahydrofuran (50 ml) at - 78 °C. The reaction mixture was maintained at the same temperature for 30 minutes, and was quenched with addition of water. After warming up to room temperature, the reaction mixture was partitioned between ethyl acetate and brine. The organic phase was washed with brine and concentrated. The residue was purified by flash chromatography (15% ethyl acetate in hexane) to provide the title compound. MS (DCI/NH₃) m/z 353 (M+H)⁺.

### EXAMPLE 415B

### 8-(3-bromo-4-fluorobenzyl)pyrido[3,2-d]pyridazin-5(6H)-one

The title compound was prepared according to the procedure for EXAMPLE 369B substituting EXAMPLE 415A for EXAMPLE 369A. MS (DCI/NH₃) m/z 335 (M+H)⁺.

### EXAMPLE 418

### N-[2-(dimethylamino)ethyl]-N-ethyl-3-[(4-oxo-3,4,5,6,7,8-hexahydrophthalazin-1-yl)mcthyl]bcnzamidc

The title compound was prepared according to the procedure for EXAMPLE 308, substituting N¹-ethyl-N²,N²-dimethylethane-1,2-diamine for furan-3-ylmethanamine. MS (DCI/NH₃) m/z 383 (M+H)⁺.

### EXAMPLE 419

### N-[2-(diethylamino)ethyl]-N-methyl-3-[(4-oxo-3,4,5,6,7,8-hexahydrophthalazin-1-yl)methyl]benzamide

The title compound was prepared according to the procedure for EXAMPLE 308, substituting N¹,N¹-diethyl-N²-methylethane-1,2-diamine for furan-3-ylmethanamine. MS (DCI/NH₃) ml) 397 (M+H)⁺.

### EXAMPLE 420

### N-benzyl-N-ethyl-3-[(4-oxo-3,4,5,6,7,8-hexahydrophthalazin-1-yl)methyl]benzamide

The title compound was prepared according to the procedure for EXAMPLE 308, substituting N-benzylethanamine for furan-3-ylmethanamine. MS (DCI/NH₃) m/z 402 (M+H)⁺; ¹H NMR (500 MHz, D₂O/pyridine-*d₅*): δ 0.97 - 1.11 (m, 3H), 1.36 - 1.57 (m, 4H), 2.22 - 2.30 (m, 2H), 2.50 - 2.66 (m, 2H), 3.27 - 3.45 (m, 2H), 3.98 (s, 2H), 4.61 - 4.74 (m, 2H), 7.26 - 7.31 (m, 1H), 7.32 - 7.40 (m, 6H), 7.42 - 7.47 (m, 1H), 7.52 (s, 1H).

### EXAMPLE 421

### N-benzyl-N-isopropyl-3-[(4-oxo-3,4,5,6,7,8-hexahydrophthalazin-1-yl)methyl]benzamide

The title compound was prepared according to the procedure for EXAMPLE 308, substituting N-benzylpropan-2-amine for furan-3-ylmethanamine. MS (DCI/NH₃) m/z 416 (M+H)⁺.

### EXAMPLE 422

### N-benzyl-N-butyl-3-[(4-oxo-3,4,5,6,7,8-hexahydrophthalazin-1-yl)methyl]benzamide

The title compound was prepared according to the procedure for EXAMPLE 308, substituting N-benzylbutan-1-amine for furan-3-ylmethanamine. MS (DCI/NH₃) m/z 430 (M+H)⁺.

### EXAMPLE 423

### N,N-dibenzyl-3-[(4-oxo-3,4,5,6,7,8-hexahydrophthalazin-1-yl)methyl]benzamide

The title compound was prepared according to the procedure for EXAMPLE 308, substituting dibenzylamine for furan-3-ylmethanamine. MS (DCI/NH₃) m/z 464 (M+H)⁺.

### EXAMPLE 424

### N-benzyl-N-(2-hydroxyethyl)-3-[(4-oxo-3,4,5,6,7,8-hexahydrophthalazin-1-yl)methyl]benzamide

The title compound was prepared according to the procedure for EXAMPLE 308, substituting 2-(benzylamino)ethanol for furan-3-ylmethanamine. MS (DCI/NH₃) m/z 418 (M+H)⁺; ¹H NMR (500 MHz, D₂O/pyridine-*d₅*): δ 1.40 - 1.54 (m, 4H), 2.21 - 2.33 (m, 2H), 2.50 - 2.61 (m, 2H), 3.62 - 3.78 (m, 2H), 3.89 - 4.02 (m, 2H), 3.96 (s, 2H), 4.82 - 4.97 (m, 2H), 7.25 - 7.29 (m, 1H), 7.30 - 7.36 (m, 5H), 7.36 - 7.43 (m, 1H), 7.43 - 7.47 (m, 1H), 7.50 (d, *J*=7.32 Hz, 1H).

### EXAMPLE 426

### N-methyl-3-[(4-oxo-3,4,5,6,7,8-hexahydrophthalazin-1-yl)methyl]-N-(2-pyridin-2-ylethyl)benzamide

The title compound was prepared according to the procedure for EXAMPLE 308, substituting N-methyl-2-(pyridin-2-yl)ethanamine for furan-3-ylmethanamine. MS (DCI/NH₃) m/z 403 (M+H)⁺; ¹H NMR (500 MHz, D₂O/pyridine-*d₅*): δ 1.42 - 1.52 (m, 4H), 2.24 - 2.34 (m, 2H), 2.51 - 2.62 (m, 2H), 2.97 (s, 3H), 3.07 - 3.21 (m, 2H), 3.83 - 3.94 (m, 2H), 3.99 (s, 2H), 7.12 (dd, *J*=7.32, 5.49 Hz, 1H), 7.14 - 7.20 (m, 1H), 7.28 - 7.32 (m, 2H), 7.32 - 7.37 (m, 1H), 7.37 - 7.45 (m, 1H), 7.57 (t, *J*=7.63 Hz, 1H), 8.55 (d, *J*=3.66 Hz, 1H).

### EXAMPLE 427

### N-[2-(3,4-dimethoxyphenyl)ethyl]-N-methyl-3-[(4-oxo-3,4,5,6,7,8-hexahydrophthalazin-1-yl)methyl]benzamide

The title compound was prepared according to the procedure for EXAMPLE 308, substituting 2-(3,4-dimethoxyphenyl)-N-methylethanamine for furan-3-ylmethanamine. MS (DCI/NH₃) m/z 462 (M+H)⁺.

### EXAMPLE 428

### 4-{3-[(4-hydroxypiperidin-1-yl)carbonyl]benzyl}-5,6,7,8-tetrahydrophthalazin-1(2H)-one

The title compound was prepared according to the procedure for EXAMPLE 308, substituting piperidin-4-ol for furan-3-ylmethanamine. MS (DCI/NH₃) m/z 368 (M+H)⁺; ¹H NMR (500 MHz, D₂O/pyridine-*d₅*): δ 1.42 - 1.52 (m, 4H), 1.63 - 1.75 (m, 2H), 1.87 - 1.98 (m, 2H), 2.27 - 2.35 (m, 2H), 2.54 - 2.62 (m, 2H), 3.25 - 3.40 (m, 2H), 4.01 (s, 2H), 4.01 - 4.04 (m, 2H), 4.05 - 4.07 (m, 1H), 7.35 (t, *J*=7.17 Hz, 1H), 7.37 - 7.40 (m, 1H), 7.40 - 7.44 (m, 1H), 7.51 (s, 1H).

### EXAMPLE 429

### 1-{3-[(4-oxo-3,4,5,6,7,8-hexahydrophthalazin-1-yl)methyl]benzoyl}piperidine-3-carboxamide

The title compound was prepared according to the procedure for EXAMPLE 308, substituting piperidine-3-carboxamide for furan-3-ylmethanamine. MS (DCI/NH₃) m/z 395 (M+H)⁺.

### EXAMPLE 430

### 1-{3-[(4-oxo-3,4,5,6,7,8-hexahydrophthalazin-1-yl)methyl]benzoyl}piperidine-4-carboxamide

The title compound was prepared according to the procedure for EXAMPLE 308, substituting piperidine-4-carboxamide for furan-3-ylmethanamine. MS (DCI/NH₃) m/z 395 (M+H)⁺; ¹H NMR (500 MHz, D₂O/pyridine-*d₅*): δ 1.41 - 1.55 (m, 4H), 1.82 - 1.97 (m, 4H), 2.27 - 2.37 (m, 2H), 2.54 - 2.61 (m, 2H), 2.63 - 2.73 (m, 1H), 2.94 - 3.06 (m, 2H), 4.00 (s, 2H), 4.16 - 4.32 (m, 2H), 7.32 - 7.36 (m, 1H), 7.36 - 7.40 (m, 2H), 7.48 - 7.51 (m, 1H).

### EXAMPLE 434

### 4-(3-{[4-(2-oxo-2,3-dihydro-1H-benzimidazol-1-yl)piperidin-1-yl]carbonyl}benzyl)-5,6,7,8-tetrahydrophthalazin-1(2H)-one

The title compound was prepared according to the procedure for EXAMPLE 308, substituting 1-(piperidin-4-yl)-1H-benzo[d]imidazol-2(3H)-one for furan-3-ylmethanamine. MS (DCI/NH₃) m/z 484 (M+H)⁺.

### EXAMPLE 435

### 4-{3-[(4-methylpiperazin-1-yl)carbonyl]benzyl}-5,6,7,8-tetrahydrophthalazin-1(2H)-one

The title compound was prepared according to the procedure for EXAMPLE 308, substituting 1-methylpiperazine for furan-3-ylmethanamine. MS (DCI/NH₃) m/z 367 (M+H)⁺; ¹H NMR (500 MHz, D₂O/pyridine-*d₅*): δ 1.46 - 1.52 (m, 4H), 2.24 (s, 3H), 2.29-2.34 (m, 2H), 2.37 - 2.42 (m, 4H), 2.54 - 2.62 (m, 2H), 3.61 - 3.73 (m, 4H), 4.02 (s, 2H), 7.35 - 7.39 (m, 1H), 7.39 - 7.43 (m, 2H), 7.51 (s, 1H).

### EXAMPLE 436

### 4-{3-[(4-ethylpiperazin-1-yl)carbonyl]benzyl}-5,6,7,8-tetrahydrophthalazin-1(2H)-one

The title compound was prepared according to the procedure for EXAMPLE 308, substituting 1-ethylpiperazine for furan-3-ylmethanamine. MS (DCI/NH₃) m/z 381 (M+H)⁺.

### EXAMPLE 437

### 4-{3-[(4-oxo-3,4,5,6,7,8-hexahydrophthalazin-1-yl)methyl]benzoyl}piperazine-1-carbaldehyde

The title compound was prepared according to the procedure for EXAMPLE 308, substituting piperazine-1-carbaldehyde for furan-3-ylmethanamine. MS (DCI/NH₃) m/z 381 (M+H)⁺.

### EXAMPLE 438

### 4-{3-[(4-acetylpiperazin-1-yl)carbonyl]benzyl}-5,6,7,8-tetrahydrophthalazin-1(2H)-one

The title compound was prepared according to the procedure for EXAMPLE 308, substituting 1-(piperazin-1-yl)ethanone for furan-3-ylmethanamine. MS (DCI/NH₃) m/z 395 (M+H)⁺; ¹H NMR (500 MHz, D₂O/pyridine-*d₅*): δ 1.44 - 1.57 (m, 4H), 2.09 (s, 3H), 2.28 - 2.39 (m, 2H), 2.51 - 2.66 (m, 2H), 3.39 - 3.73 (m, 8H), 4.03 (s, 2H), 7.36 - 7.39 (m, 1H), 7.43 (t, *J*=7.02 Hz, 2H), 7.54 (s, 1H).

### EXAMPLE 439

### 4-(3-{[4-(2-hydroxyethyl)piperazin-1-yl]carbonyl}benzyl)-5,6,7,8-tetrahydrophthalazin-1(2H)-one

The title compound was prepared according to the procedure for EXAMPLE 308, substituting 2-(piperazin-1-yl)ethanol for furan-3-ylmethanamine. MS (DCI/NH₃) m/z 397 (M+H)⁺; ¹H NMR (500 MHz, D₂O/pyridine-*d₅*): δ 1.42 - 1.55 (m, 4H), 2.27 - 2.37 (m, 2H), 2.50 - 2.62 (m, 6H), 2.69 (t, *J*=5.80 Hz, 2H), 3.54 - 3.75 (m, 4H), 3.89 (t, *J*=5.80 Hz, 2H), 4.02 (s, 2H), 7.35 - 7.38 (m, 1H), 7.39 - 7.43 (m, 2H), 7.50 (s, 1H).

### EXAMPLE 440

### 4-{3-[(4-phenylpiperazin-1-yl)carbonyl]benzyl}-5,6,7,8-tetrahydrophthalazin-1(2H)-one

The title compound was prepared according to the procedure for EXAMPLE 308, substituting 1-phenylpiperazine for furan-3-ylmethanamine. MS (DCI/NH₃) m/z 429 (M+H)⁺; ¹H NMR (500 MHz, D₂O/pyridine-*d₅*): δ 1.44 - 1.57 (m, 4H), 2.27 - 2.42 (m, 2H), 2.52 - 2.66 (m, 2H), 3.08 - 3.20 (m, 4H), 3.68 - 3.83 (m, 4H), 4.04 (s, 2H), 6.91 (t, *J*=7.32 Hz, 1H), 6.98 (d, *J*=7.93 Hz, 2H), 7.28 - 7.34 (m, 2H), 7.37 - 7.41 (m, 1H), 7.44 (t, J=7.48 Hz, 1H), 7.46 - 7.49 (m, 1H), 7.57 (s, 1H).

### EXAMPLE 441

### 4- {3-[(4-pyridin-2-ylpiperazin-1-yl)carbonyl]benzyl}-5,6,7,8-tetrahydrophthalazin-1(2H)-one

The title compound was prepared according to the procedure for EXAMPLE 308, substituting 1-(pyridin-2-yl)piperazine for furan-3-ylmethanamine. MS (DCI/NH₃) m/z 430 (M+H)⁺; ¹H NMR (500 MHz, D₂O/pyridine-*d₅*): δ 1.46 - 1.55 (m, 4H), 2.29 - 2.40 (m, 2H), 2.52 - 2.65 (m, 2H), 3.53 - 3.63 (m, 4H), 3.64 - 3.78 (m, 4H), 4.03 (s, 2H), 6.66 (dd, *J*=6.71, 4.58 Hz, 1H), 6.73 (d, *J*=8.54 Hz, 1 H), 7.37 - 7.41 (m, 1 H), 7.43 (d, *J*=7.63 Hz, 1H), 7.45 - 7.48 (m, 1H), 7.49 - 7.54 (m, 1H), 7.57 (s, 1H), 8.29 (dd, *J*=4.88, 1.22 Hz, 1H).

### EXAMPLE 442

### 4-{3-[(4-pyrimidin-2-ylpiperazin-1-yl)carbonyl]benzyl}-5,6,7,8-tetrahydrophthalazin-1(2H)-one

The title compound was prepared according to the procedure for EXAMPLE 308, substituting 2-(piperazin-1-yl)pyrimidine for furan-3-ylmethanamine. MS (DCI/NH₃) m/z 431 (M+H)⁺; ¹H NMR (500 MHz, D₂O/pyridine-*d*₅): δ 1.42 - 1.59 (m, 4H), 2.26 - 2.40 (m, 2H), 2.50 - 2.66 (m, 2H), 3.62 - 3.74 (m, 4H), 3.82 - 3.91 (m, 4H), 4.03 (s, 2H), 6.55 (t, *J*=4.73 Hz, 1H), 7.38 (d, *J*=5.80 Hz, 1H), 7.40 - 7.45 (m, 1H), 7.45 - 7.49 (m, 1H), 7.57 (s, 1H), 8.38 (d, *J*=4.88 Hz, 2H).

### EXAMPLE 443

### 4-[3-({4-[2-(2-hydroxyethoxy)ethyl]piperazin-1-yl}carbonyl)benzyl]-5,6,7,8-tetrahydrophthalazin-1(2H)-one

The title compound was prepared according to the procedure for EXAMPLE 308, substituting 2-(2-(piperazin-1-yl)ethoxy)ethanol for furan-3-ylmethanamine. MS (DCI/NH₃) m/z 441 (M+H)⁺.

### EXAMPLE 444

### 4-(3-{[4-(2-fluorophenyl)piperazin-1-yl]carbonyl}benzyl)-5,6,7,8-tetrahydrophthalazin-1(2H)-one

The title compound was prepared according to the procedure for EXAMPLE 308, substituting 1-(2-fluorophenyl)piperazine for furan-3-ylmethanamine. MS (DCI/NH₃) m/z 447 (M+H)⁺.

### EXAMPLE 445

### 4-(3-{[4-(4-fluorophenyl)piperazin-1-yl]carbonyl}benzyl)-5,6,7,8-tetrahydrophthalazin-1(2H)-one

The title compound was prepared according to the procedure for EXAMPLE 308, substituting 1-(4-fluorophenyl)piperazine for furan-3-ylmethanamine. MS (DCI/NH₃) m/z 447 (M+H)⁺; ¹H NMR (500 MHz, D₂O/pyridine-*d₅*): δ 1.44 - 1.56 (m, 4H), 2.30 - 2.39 (m, 2H), 2.53 - 2.63 (m, 2H), 3.02 - 3.12 (m, 4H), 3.68 - 3.81 (m, 4H), 4.05 (s, 2H), 6.93 - 6.97 (m, 2H), 7.04 - 7.09 (m, 2H), 7.37 - 7.41 (m, 1H), 7.44 (t, *J*=7.32 Hz, 1H), 7.46 - 7.51 (m, 1H), 7.57 (s, 1H).

### EXAMPLE 446

### 4-(3-{[4-(2-chlorophenyl)piperazin-1-yl]carbonyl}benzyl)-5,6,7,8-tetrahydrophthalazin-1(2H)-one

The title compound was prepared according to the procedure for EXAMPLE 308, substituting 1-(2-chlorophenyl)piperazine for furan-3-ylmethanamine. MS (DCI/NH₃) m/z 463 (M+H)⁺.

### EXAMPLE 447

### 4-{3-[(4-methyl-1,4-diazepan-1-yl)carbonyl]benzyl}-5,6,7,8-tetrahydrophthalazin-1(2H)-one

The title compound was prepared according to the procedure for EXAMPLE 308, substituting 1-methyl-4-(piperazin-1-yl)azepane for furan-3-ylmethanamine. MS (DCI/NH₃) m/z 381 (M+H)⁺; ¹H NMR (500 MHz, D₂O/pyridine-*d₅*): δ 1.44 - 1.48 (m, 1H), 1.48 - 1.51 (m, 4H), 1.92 - 2.01 (m, 2H), 2.28 - 2.36 (m, 2H), 2.51 - 2.55 (m, 2H), 2.58 (s, 3H), 2.64 (t, *J*=5.65 Hz, 1H), 2.84 - 2.94 (m, 2H), 3.64 - 3.71 (m, 3H), 3.88 - 3.92 (m, 1H), 4.01 (s, 2H), 7.33 - 7.39 (m, 1H), 7.39 - 7.41 (m, 1H), 7.41 - 7.45 (m, 1H), 7.51 (s, 1H).

### EXAMPLE 448

### 4-[3-(1,1-dioxido-1,2-thiazinan-2-yl)-4-fluorobenzyl]-5,6,7,8-tetrahydrophthalazin-1(2H)-one

### EXAMPLE 448A

### 3-(1,1-dioxido-1,2-thiazinan-2-yl)-4-fluorobenzaldehyde

The title compound was prepared according to the procedure for EXAMPLE 300A, substituting 1,4-butanesultam for 5-methylpyrrolidinone. MS (DCI/NH₃) m/z 258 (M+H)⁺.

### EXAMPLE 448B

### 4-[3-(1,1-dioxido-1,2-thiazinan-2-yl)-4-fluorobenzyl]-5,6,7,8-tetrahydrophthalazin-1(2H)-one

The title compound was prepared according to the procedure for EXAMPLE 300B, substituting EXAMPLE 448A for EXAMPLE 300A. MS (DCI/NH₃) m/z 392 (M+H)⁺; ¹H NMR (300 MHz, DMSO-*d*₆)_{:} δ 1.57 - 1.71 (m, 4H), 1.75 - 1.87 (m, 2H), 2.13 - 2.24 (m, 2H), 2.34 - 2.46 (m, 4H), 3.16 - 3.28 (m, 2H), 3.46 - 3.58 (m, 2H), 3.92 (s, 2H), 7.23 (dd, *J*=8.13, 2.18 Hz, 1H), 7.48 (d, *J*=7.93 Hz, 1H), 7.57 (d, *J*=1.59 Hz, 1H), 12.61 (br s, 1H).

### EXAMPLE 449

### 8-[4-fluoro-3-(2-oxoazetidin-1-yl)benzyl]pyrido[2,3-d]pyridazin-5(6H)-one

The title compound was prepared according to procedure for EXAMPLE 101, substituting EXAMPLE 415 for EXAMPLE 103. MS (ESI) m/z 325 (M+H)⁺.

### EXAMPLE 450

### 8-(3-chloro-4-fluorobenzyl)-2,3,4,6-tetrahydropyrido[2,3-d]pyridazin-5(1H)-one

The title compound was prepared as TFA salt according to procedure for EXAMPLE 414, substituting EXAMPLE 370 for EXAMPLE 369. MS (ESI) m/z 294 (M+H)⁺.

### EXAMPLE 451

### 4-(1-(4-fluoro-3-(2-oxoazetidin-1-yl)phenyl)ethyl)-5,6,7,8-tetrahydrophthalazin-1(2H)-one

The title compound was prepared according to procedure for EXAMPLE 101, substituting EXAMPLE 412 for EXAMPLE 103. MS (ESI) m/z 342 (M+H)⁺; ¹H NMR (300 MHz, DMSO-d₆): 1.41 (d, *J*=7.12 Hz, 3 H), 1.44 - 1.67 (m, 4 H), 1.84 - 2.08 (m, 1 H), 2.34 (m, 2 H), 2.53 - 2.74 (m, 1 H), 3.11 (t, *J*=4.58 Hz, 2 H), 3.72 - 3.88 (m, 2 H), 4.22 (q, *J*=6.78 Hz, 1 H), 6.81 - 6.95 (m, 1 H), 7.18 (dd, *J*=11.87, 8.48 Hz, 1 H), 7.76 (dd, *J*=7.46, 2.37 Hz, 1 H), 12.70 (s, 1 H).

### EXAMPLE 452

### 1-{3-[(4-oxo-3,4,5,6,7,8-hexahydrophthalazin-1-yl)methyl]phenyl}pyrrolidine-2,5-dione

The title compound was prepared according to the procedure for EXAMPLEs 2, 3 and 4, substituting 3-nitrobenzaldehyde for 4-fluoro-3-nitrobenzaldehyde. MS (DCI/NH₃) m/z 256 (M+H)⁺.

### EXAMPLE 453

### N-{2-fluoro-5-[(4-oxo-3,4,5,6,7,8-hexahydrophthalazin-1-yl)methyl]phenyl}-2-(2-oxopyrrolidin-1-yl)acetamide

The title compound was prepared as TFA salt according to the procedure for EXAMPLE 136, substituting EXAMPLE 2 for EXAMPLE 89, and 2-(2-oxopyrrolidin-1-yl)acetic acid for 1-methylcyclopropanecarboxylic acid. MS (DCI/NH₃) m/z 356 (M+H)⁺. MS (DCI/NH₃) m/z 399 (M+H)⁺; ¹H NMR (300 MHz, DMSO-*d*₆)_{:} δ 1.53 - 1.67 (m, 4H), 1.89 - 2.06 (m, 2H), 2.26 (t, *J*=7.97 Hz, 2H), 2.31 - 2.43 (m, 4H), 3.39 - 3.47 (m, 2H), 3.86 (s, 2H), 4.07 (s, 2H), 6.93 - 6.99 (m, 1H), 7.18 (dd, *J*=10.85, 8.48 Hz, 1H), 7.71 (dd, *J*=7.46, 2.03 Hz, 1H), 9.82 (br s, 1H), 12.61 (br s, 1H).

### EXAMPLE 454

### N-{2-fluoro-5-[(4-oxo-3,4,5,6,7,8-hexahydrophthalazin-1-yl)methyl]phenyl}-5-methyl-1-phenyl-1 H-pyrazole-4-carboxamide

The title compound was prepared as TFA salt according to the procedure for EXAMPLE 136, substituting EXAMPLE 2 for EXAMPLE 89, and 5-methyl-1-phenyl-1H-pyrazole-4-carboxylic acid for 1-methylcyclopropanecarboxylic acid. MS (DCI/NH₃) m/z 356 (M+H)⁺. MS (DCI/NH₃) m/z 458 (M+H)⁺.

### EXAMPLE 455

### N-{2-fluoro-5-[(4-oxo-3,4,5,6,7,8-hexahydrophthalazin-1-yl)methyl]phenyl}-5-oxohexanamide

The title compound was prepared as TFA salt according to the procedure for EXAMPLE 136, substituting EXAMPLE 2 for EXAMPLE 89, and 5-oxohexanoic acid for 1-methylcyclopropanecarboxylic acid. MS (DCI/NH₃) m/z 386 (M+H)⁺; ¹H NMR (300 MHz, DMSO-*d*₆): δ 1.53 - 1.65 (m, 4H), 1.67 - 1.80 (m, 2H), 2.08 (s, 3H), 2.28 - 2.34 (m, 2H), 2.34 - 2.41 (m, 4H), 2.42 - 2.49 (m, 2H), 3.85 (s, 2H), 6.82 - 6.96 (m, 1H), 7.15 (dd, *J*=10.85, 8.48 Hz, 1H), 7.67 (dd, *J*=7.46, 1.70 Hz, 1H), 9.60 (br s, 1H), 12.61 (br s, 1H).

### EXAMPLE 456

### N-{2-fluoro-5-[(4-oxo-3,4,5,6,7,8-hexahydrophthalazin-1-yl)methyl]phenyl}-3-methoxypropanamide

The title compound was prepared as TFA salt according to the procedure for EXAMPLE 136, substituting EXAMPLE 2 for EXAMPLE 89, and 3-methoxypropanoic acid for 1-methylcyclopropanecarboxylic acid. MS (DCI/NH₃) m/z 360 (M+H)⁺; ¹H NMR (300 MHz, DMSO-*d₆*): δ 1.55 - 1.66 (m, 4H), 2.30 - 2.43 (m, 4H), 2.60 (t, *J*=6.10 Hz, 2H), 3.23 (s, 3H), 3.59 (t, *J*=6.27 Hz, 2H), 3.86 (s, 2H), 6.85 - 6.99 (m, 1H), 7.16 (dd, *J*=10.85, 8.48 Hz, 1H), 7.74 (dd, *J*=7.46, 1.70 Hz, 1H), 9.64 (br s, 1H), 12.61 (br s, 1H).

### EXAMPLE 457

### N-{2-fluoro-5-[(4-oxo-3,4,5,6,7,8-hexahydrophthalazin-1-yl)methyl]phenyl}-N'-phenylpentanediamide

The title compound was prepared as TFA salt according to the procedure for EXAMPLE 136, substituting EXAMPLE 2 for EXAMPLE 89, and 5-oxo-5-(phenylamino)pentanoic acid for 1-methylcyclopropanecarboxylic acid. MS (DCI/NH₃) m/z 463 (M+H)⁺; ¹H NMR (300 MHz, DMSO-*d₆*): δ 1.55 - 1.67 (m, 4H), 1.83 - 1.94 (m, 2H), 2.31 - 2.40 (m, 6H), 2.40 - 2.45 (m, 2H), 3.85 (s, 2H), 6.89 - 6.96 (m, 1H), 6.98 - 7.06 (m, 1H), 7.15 (dd, *J*=10.85, 8.48 Hz, 1H), 7.24 - 7.32 (m, 2H), 7.59 (d, *J*=7.46 Hz, 2H), 7.71 (dd, *J*=7.97, 1.53 Hz, 1H), 9.67 (br s, 1H), 9.88 (br s, 1H), 12.62 (br s, 1H).

### EXAMPLE 458

### benzyl 2-(dimethylamino)-5-[(4-oxo-3,4,5,6,7,8-hexahydrophthalazin-1-yl)methyl]phenylcarbamate

### EXAMPLE 458A

### 4-(dimethylamino)-N-methoxy-N-methyl-3-nitrobenzamide

To a solution of 4-fluoro-3-nitrobenzoic acid (5 g, 27.0 mmol) in dimethylformamide (100 mL) was added N,O-dimethylhydroxylamine hydrochloride (5.93 g, 60.8 mmol) and triethylamine (17.0 mL, 122 mmol). 1-Ethyl-3-(3-dimethylaminopropyl) carbodiimide hydrochloride (11.65 g, 60.8 mmol) and hydroxybenzotriazole (9.31 g, 60.8 mmol) were added and the reaction mixture was stirred at room temperature for 3 days. The reaction mixture was concentrated and partitioned between ethyl acetate (150 mL) and brine (150 mL). The organics were concentrated on rotary evaporator and the crude was purified by flash chromatography eluting with 40% ethyl acetate in hexanes to provide the title product. MS (DCI/NH₃) m/z 254 (M+H)⁺.

### EXAMPLE 458B

### 3-amino-4-(dimethylamino)-N-methoxy-N-methylbenzamide

A solution of EXAMPLE 458A (2.34 g, 9.24 mmol) in tetrahydrofuran (40 mL) was treated with Raney Ni (2.0 g, Raney 2800, slurry in water) at room temperature under a hydrogen (balloon) for 16 hours. The catalyst was filtered off, and the filtrate was concentrated. The residue was used the subsequent step without further purification.

### EXAMPLE 458C

### benzyl 2-(dimethylamino)-5-(methoxy(methyl)carbamoyl)phenylcarbamate

To a solution of EXAMPLE 458B in a mixture of tetrahydrofuran (20 mL) and water (20 mL) was added cesium carbonate (6.02 g, 18.58 mmol) and benzyl chloroformate (1.5 mL, 10.16 mmol). The reaction mixture was stirred at room temperature for 16 hours, and concentrated. The residue was partitioned between ethyl acetate (100 mL) and brine (75 mL). The organic layer was washed with brine, and concentrated. The residual oil was purified by flash chromatography eluting with 40% ethyl acetate in hexanes to provide the title product. MS (DCI/NH₃) m/z 358 (M+H)⁺.

### EXAMPLE 458D

### benzyl 2-(dimethylamino)-5-formylphenylcarbamate

A solution of EXAMPLE 458C (2.89 g, 8.1 mmol) in anhydrous tetrahydrofuran (20 mL) was treated with lithium aluminum hydride (1.0 M solution in tetrahydrofuran, 8.1 mL, 8.1 mmol) at 0 °C for 10 minutes. The reaction was quenched with water, and the mixture was partitioned between ethyl acetate and diluted HCl solution. The organic layer was washed with brine, and concentrated on a rotary evaporator. The residual oil was purified by flash chromatography eluting with 20% ethyl acetate in hexanes to provide the title product. MS (DCI/NH₃) m/z 299 (M+H)⁺.

### EXAMPLE 458E

### benzyl 2-(dimethylamino)-5-[(4-oxo-3,4,5,6,7,8-hexahydrophthalazin-1-yl)methyl]phenylcarbamate

The title compound was prepared according to the procedure for EXAMPLE 300B, substituting EXAMPLE 458D for EXAMPLE 300A. MS (DCI/NH₃) m/z 433 (M+H)⁺.

### EXAMPLE 459

### 8-(4-fluoro-3-(2-oxoazetidin-1-yl)benzyl)-1,2,3,4-tetrahydropyrido[3,2-d]pyridazin-5(6H)-one

The title compound was prepared according to procedure for EXAMPLE 414, substituting EXAMPLE 449 for EXAMPLE 369. MS (ESI) m/z 329 (M+H)⁺; ¹H NMR (300 MHz, DMSO-*d₆*): δ 1.61 - 1.76 (m, 2 H), 2.33 (t, *J*=6.35 Hz, 2 H), 3.12 (t, *J*=4.56 Hz, 2 H), 3.17 (m, 2 H), 3.77 (s, 2 H), 3.81 (q, *J*=4.36 Hz, 2 H), 6.32 (s, 1 H), 6.87 - 7.01 (m, 1 H), 7.17 (dd, *J*=11.90, 8.33 Hz, 1 H), 7.78 (dd, *J*=7.54, 2.38 Hz, 1 H), 11.80 (s, 1 H).

### EXAMPLE 460

### 4-(3-bromo-4-fluorophenyl)-5,6,7,8-tetrahydrophthalazin-1(2H)-one

### EXAMPLE 460A

### 3-(3-bromo-4-fluorophenyl)-3-methoxy-4,5,6,7-tetrahydroisobenzofuran-1(3H)-one

To a solution of 2-bromo-1-fluoro-4-iodobenzene (13.23 g, 44 mmol) in anhydrous tetrahydrofuran (30 mL) was added isopropylmagnesium chloride (2.0 M solution in tetrahydrofuran, 24.18 mL, 48.4 mmol) at - 20 °C. After the addition, the reaction mixture was stirred at 0 °C for 3 hours, and was added to a solution of 3,4,5,6-tetrahydrophthalic anhydride (6.08 g, 40 mmol) in anhydrous tetrahydrofuran (60 mL) at -78 °C. The mixture was stirred for 2 hours, and a saturated aqueous ammonium chloride solution was added to the reaction mixture, which then was stirred at room temperature for 30 minutes. Anhydrous magnesium sulfate was added to the reaction mixture, and the mixture was filtered. The filtrate was concentrated. Thionyl chloride (10.4 mL, 142 mol) was added dropwise to methanol (40 mL) at -10 °C., and the solution was stirred at 0 °C for 30 minutes. To the thionyl chloride solution was then added the residue from the filtrate in anhydrous methanol (15 mL). The reaction mixture was stirred at room temperature overnight, and was concentrated. The residue was dissoved in methylene chloride (40 mL), and was treated with triethylamine (5.58 mL) at 0 °C for 1 hour. Water was added, and the mixture was washed with sodium bicarbonate, brine and water. The organic phase was dried over magnesium sulfate, filtered and concentrated. The residue was separated by flash chromatography (10-35% gradient ethyl acetate in hexane) to provide the title compound. MS (DCI/NH₃) m/z 341, 343 (M+H)⁺.

### EXAMPLE 460B

### 4-(3-bromo-4-fluorophenyl)-5,6,7,8-tetrahydrophthalazin-1(2H)-one

A solution of EXAMPLE 460A (9.5 g, 27.8 mmol) and hydrazine monohydrate (1.76 mL, 36.2 mmol) in ethanol (70 mL) was heated under reflux for 4 hours. After cooling to room temperature, the solids were collected by filtration, washed with ethanol and dried to provide the title compound. MS (DCI/NH₃) m/z 323, 325 (M+H)⁺; ¹H NMR (300 MHz, DMSO-D₆): δ 1.57 - 1.65 (m, 2 H), 1.66 - 1.74 (m, 2 H), 2.34 (t, *J*=5.75 Hz, 2 H), 2.45 (t, *J*=6.15 Hz, 2 H), 7.45 (t, *J*=8.72 Hz, 1 H), 7.49 - 7.55 (m, 1 H), 7.80 (dd, *J*=6.74, 2.38 Hz, 1 H), 12.85 (br s, 1H).

### EXAMPLE 461

### 4-[4-fluoro-3-(2-oxoazetidin-1-yl)phenyl]-5,6,7,8-tetrahydrophthalazin-1(2H)-one

### EXAMPLE 461A

### 2-(benzyloxymethyl)-4-(3-bromo-4-fluorophenyl)-5,6,7,8-tetrahydrophthalazin-1(2H)-one

To a solution of EXAMPLE 460 (2.0 g, 6.19 mmol) in anhydrous dimethylformamide (30 mL) was added potassium t-butoxide (1 M solution in tetrahydrofuran, 6.50 mL, 6.5 mmol). The solution was stirred at room temperature for 30 minutes, and benzyl chloromethylether (1.163 g, 7.43 mmol) was added. The reaction mixture was stirred at room temperature overnight. After quenching with water, the reaction mixture was partitioned between water and ethyl acetate. The organic phase was washed with water, and concentrated. The residue was separated by flash chromatography (20-60% gradient ethyl acetate in hexane) to provide the title compound. MS (DCI/NH₃) m/z 443 (M+H)⁺.

### EXAMPLE 461B

### 2-(benzyloxymethyl)-4-(4-fluoro-3-(2-oxoazetidin-1-yl)phenyl)-5,6,7,8-tetrahydrophthalazin-1(2H)-one

A microwave reactor tube was charged with EXAMPLE 461A (137 mg, 0.309 mmol), tris(dibenzylideneacetone)dipalladium(0) (28.3 mg, 0.031 mmol), 4,5-bis(diphenylphosphino)-9,9-dimethylxanthene (Xantphos) (26.9 mg, 0.046 mmol), 2-azetidinone (44 mg, 0.619 mmol), and potassium phosphate tribasic (98 mg, 0.464 mmol). Anhydrous dioxane (3 mL) was added. The suspension was purged with nitrogen, and was capped with a microwave septum. The reaction mixture was heated in a CEM Explorer® microwave reactor (Matthews, NC) at 200 °C for 50 minutes. After cooling, the reaction mixture was partitioned between ethyl acetate and brine. The organic phase was washed with water, and concentrated. The residue was separated by flash chromatography (20-70% gradient ethyl acetate in hexane) to provide the title compound. MS (DCI/NH₃) m/z 434 (M+H)⁺.

### EXAMPLE 461C

### 4-[4-fluoro-3-(2-oxoazetidin-1-yl)phenyl]-5,6,7,8-tetrahydrophthalazin-1(2H)-one

To a solution of EXAMPLE 461B (140 mg, 0.323 mmol) in methanol (10 mL) was added 20% palladium hydroxide on carbon (80 mg) under nitrogen. This suspension was purged with hydrogen, and stirred under hydrogen (balloon) at 50 °C for 4 hours. The mixture was filtered, and the filtrate was concentrated. The residue was recrystallized from methanol (4 mL) to provide the title compound. The mother liquor was separated by HPLC (Zorbax® C-18 ODS packing material [Agilent Technologies, Santa Clara, CA], 250x2.54 column, Mobile phase A: 0.1% TFA in H₂O; B: 0.1% TFA in CH₃CN; 0-100% gradient) to provide additional title compound. MS (DCI/NH₃) m/z 314 (M+H)⁺; ¹H NMR (400 MHz, DMSO-d₆), δ 1.57 - 1.63 (m, 2 H), 1.67 - 1.74 (m, 2 H), 2.33 (t, *J*=5.83 Hz, 2 H), 2.45 (t, *J*=6.14 Hz, 2 H), 3.14 - 3.18 (m, 2 H), 3.86 - 3.90 (m, 2 H), 7.16 - 7.21 (m, 1 H), 7.34 (dd, *J*=11.66, 8.59 Hz, 1 H), 7.93 (dd, *J*=7.52, 2.30 Hz, 1 H), 12.89 (s, 1 H).

### EXAMPLE 462

### 2-fluoro-5-[(5-oxo-5,6-dihydropyrido[2,3-d]pyridazin-8-yl)methyl]benzamide

### EXAMPLE 462A

### methyl 2-fluoro-5-((5-oxo-5,6-dihydropyrido[3,2-d]pyridazin-8-yl)methyl)benzoate

The title compound was prepared according to the procedure for EXAMPLE 66C, substituting EXAMPLE 415 for EXAMPLE 66B. MS (DCI/NH₃) m/z 314 (M+H)⁺.

### EXAMPLE 462B

### 2-fluoro-5-[(5-oxo-5,6-dihydropyrido[2,3-d]pyridazin-8-yl)methyl]benzamide

A solution of EXAMPLE 462A (1 g, 3.2 mmol) in 7N ammonia in methanol (5 ml) was heated at 70 °C overnight, and cooled to room temperature. The solid was collected by filtration, washed with methanol and dried to provide the title compound. MS (DCI/NH₃) m/z 299 (M+H)⁺.

### EXAMPLE 463

### 8-(3-amino-4-fluorobenzyl)pyrido[2,3-d]pyridazin-5(6H)-one

A mixture of 1.5 N aqueous KOH solution (2 ml) and 3 g of ice was treated with bromine (80 mg, 0.5 mmol) at -10 °C for 10 minutes. EXAMPLE 462 (100 mg, 0.3 mmol) was added. The reaction mixture was stirred at -10 °C for an additional 10 minutes, and was then allowed to warm up to 65 °C for 1 hour. After cooling, the mixture was partitioned between ethyl acetate and brine. The organic phase was washed with brine, and concentrated to about 10 mL. The solid was collected by filtration, washed with methanol, and dried to provide the title compound. MS (DCI/NH₃) m/z 271 (M+H)⁺.

### EXAMPLE 464

### 8-[4-fluoro-3-(2-oxopyrrolidin-1-yl)benzyl]-2,3,4,6-tetrahydropyrido[2,3-d]pyridazin-5(1H)-one

### EXAMPLE 464A

### 6-(benzyloxymethyl)-8-(3-bromo-4-fluorobenzyl)pyrido[3,2-d]pyridazin-5(6H)-one

A solution of EXAMPLE 415 (1 g, 3 mmol) in anhydrous dimethylformamide (100 ml) was treated with potassium t-butoxide (1N solution in tetrahydrofuran, 3 mL, 3 mmol) at room temperature for 30 minutes. Benzyloxychloromethane (0.6 g, 3.6 mmol) was then added, and the mixture was stirred at room temperature overnight. After quenching with water, the reaction mixture was partitioned between ethyl acetate and brine. The organic layer was washed with brine, and concentrated. The residue was purified by flash chromatography (85% ethyl acetate in hexane) to provide the title compound. MS (DCI/NH₃) m/z 454 (M+H)⁺.

### EXAMPLE 464B

### 6-(benzyloxymethyl)-8-(4-fluoro-3-(2-oxopyrrolidin-1-yl)benzyl)pyrido[3,2-d]pyridazin-5(6H)-one

The title compound was prepared according to procedure for EXAMPLE 101, substituting EXAMPLE 464A for EXAMPLE 103. MS (ESI) m/z 459 (M+H)⁺.

### EXAMPLE 464C

### 8-[4-fluoro-3-(2-oxopyrrolidin-1-yl)benzyl]-2,3,4,6-tetrahydropyrido[2,3-d]pyridazin-5(1H)-one

A mixture of EXAMPLE 464B (130 mg, 0.28 mmol), 5% platinum on carbon (25 mg), 5% Pd(OH)₂ on carbon (25 mg), concentrated aqueous HCl (66 µL) and dimethylformamide (10 ml) was stirred in a pressure vessel at room temperature under 40 psi of hydrogen for 48 hours. The volatiles were removed, the residue was separated by HPLC (Zorbax® C-18 ODS packing material [Agilent Technologies, Santa Clara, CA], 0.1% TFA/CH₃CN/H₂O) to provide the title product as TFA salt. MS (ESI) m/z 343 (M+H)⁺; ¹H NMR (300 MHz, DMSO-*d₆*): δ 1.57 - 1.81 (m, 2 H), 2.01 - 2.18 (m, 2 H), 2.26 - 2.46 (m, 4 H), 3.17 (m, 2 H), 3.72 (t, *J*=6.94 Hz, 2 H), 3.84 (s, 2 H), 6.39 (s, 1 H), 7.16 - 7.19 (m, 1 H), 7.18 - 7.25 (m, 1 H), 7.29 (dd, *J*=7.54, 1.98 Hz, 1 H), 11.89 (s, 1 H).

### EXAMPLE 465

### methyl 2-fluoro-5-[(5-oxo-1,2,3,4,5,6-hexahydropyrido[2,3-d]pyridazin-8-yl)methyl]benzoate

The title compound was prepared as TFA salt according to procedure for EXAMPLE 414, substituting EXAMPLE 462A for EXAMPLE 369. MS (ESI) m/z 318 (M+H)⁻; ¹H NMR (300 MHz, DMSO-*d₆*): δ 1.61 - 1.75 (m, 2 H), 2.34 (t, *J*=6.15 Hz, 2 H), 3.17 (m, 2 H), 3.44 (s, 3 H), 3.84 (s, 2 H), 6.39 (s, 1 H), 7.27 (dd, *J*=10.91, 8.53 Hz, 1 H), 7.46 - 7.56 (m, 1 H), 7.76 (dd, *J*=7.14, 2.38 Hz, 1 H), 11.84 (s, 1 H).

### EXAMPLE 467

### 8-(3-amino-4-fluorobenzyl)-2,3,4,6-tetrahydropyrido[2,3-d]pyridazin-5(1H)-one

The title compound was prepared as TFA salt according to procedure for EXAMPLE 414, substituting EXAMPLE 463 for EXAMPLE 369. MS (ESI) m/z 275 (M+H)⁺; ¹H NMR (300 MHz, DMSO-*d₆*): 1.62 - 1.74 (m, 2 H), 2.35 (t, *J*=6.27 Hz, 2 H), 3.10 - 3.23 (m, 2 H), 3.69 (s, 2 H), 4.91 (s, 2 H), 6.25 (s, 1 H), 6.45 - 6.54 (m, 1 H), 6.64 (dd, *J*=8.82, 2.03 Hz, 1 H), 6.92 (dd, *J*=11.53, 8.48 Hz, 1 H), 11.93 (s, 1 H).

### EXAMPLE 468

### 2-fluoro-5-[(5-oxo-1,2,3,4,5,6-hexahydropyrido[2,3-d]pyridazin-8-yl)methyl]benzoic acid

The title compound was prepared according to procedure for EXAMPLE 288, substituting EXAMPLE 465 for EXAMPLE 266. MS (ESI) m/z 304 (M+H)⁺; ¹H NMR (300 MHz, DMSO-*d₆*): δ 1.61 - 1.77 (m, 2 H), 2.34 (t, *J*=6.10 Hz, 2 H), 3.06 - 3.25 (m, 2 H), 3.84 (s, 2 H), 6.36 (s, 1 H), 7.22 (dd, *J*=10.85, 8.48 Hz, 1 H), 7.39 - 7.52 (m, 1 H), 7.73 (dd, *J*=7.12, 2.37 Hz, 1 H), 11.82 (s, 1 H) 13.19 (s, 1 H).

### EXAMPLE 470

### N-ethyl-2-fluoro-5-[(5-oxo-1,2,3,4,5,6-hexahydropyrido[2,3-d]pyridazin-8-yl)methyl]benzamide

The title compound was prepared as TFA salt according to procedure for EXAMPLE 48, substituting EXAMPLE 468 for EXAMPLE 48C, and ethylamine for 1-(3-dimethylaminopropyl)-3-ethylcarbodiimide. MS (ESI) m/z 331 (M+H)⁺; ¹H NMR (300 MHz, DMSO-*d₆*): 1.09 (t, *J*=7.14 Hz, 3 H), 1.58 - 1.74 (m, 2 H), 2.34 (t, *J*=6.15 Hz, 2 H), 3.12 - 3.20 (m, 2 H), 3.20 - 3.29 (m, 2 H), 3.82 (s, 2 H), 6.39 (s, 1 H), 7.19 (dd, *J*=10.31, 8.33 Hz, 1 H), 7.30 - 7.38 (m, 1 H), 7.47 (dd, *J*=6.74, 2.38 Hz, 1 H), 8.17 - 8.29 (m, 1 H), 11.88 (s, 1 H).

### EXAMPLE 471

### N-cyclobutyl-2-fluoro-5-[(5-oxo-1,2,3,4,5,6-hexahydropyrido[2,3-d]pyridazin-8-yl)methyl]benzamide

The title compound was prepared as TFA salt according to procedure for EXAMPLE 48, substituting EXAMPLE 468 for EXAMPLE 48C, and cyclobutanamine for 1-(3-dimethylaminopropyl)-3-ethylcarbodiimide. MS (ESI) m/z 357 (M+H)⁺; ¹H NMR (300 MHz, DMSO-*d₆*): δ 1.56 - 1.77 (m, 4 H), 1.90 - 2.10 (m, 2 H), 2.12 - 2.28 (m, 2 H), 2.33 (t, *J*=6.35 Hz, 2 H), 3.05 - 3.25 (m, 2 H), 3.81 (s, 2 H), 4.27 - 4.45 (m, 1 H), 6.35 (s, 1 H), 7.18 (dd, *J*=10.31, 8.33 Hz, 1 H), 7.26 - 7.37 (m, 1 H), 7.42 (dd, *J*=6.74, 2.38 Hz, 1 H), 8.49 (d, *J*=7.54 Hz, 1 H), 11.84 (s, 1 H).

### EXAMPLE 472

### 2-fluoro-5-[(5-oxo-1,2,3,4,5,6-hexahydropyrido[2,3-d]pyridazin-8-yl)methyl]-N-(2-pyrrolidin-1-ylethyl)benzamide

The title compound was prepared as TFA salt according to procedure for EXAMPLE 48, substituting EXAMPLE 468 for EXAMPLE 48C, and 2-(pyrrolidin-1-yl)ethanamine for 1-(3-dimethylaminopropyl)-3-ethylcarbodiimide. MS (ESI) m/z 400 (M+H)⁺; ¹H NMR (300 MHz, DMSO-*d₆*): δ 1.63 - 1.76 (m, 2 H), 1.76 - 1.93 (m, 2 H), 1.93 - 2.10 (m, 2 H), 2.34 (t, *J*=6.10 Hz, 2 H), 2.61 - 2.76 (m, 2 H), 2.96 - 3.12 (m, 2 H), 3.12 - 3.22 (m, 2 H), 3.25 - 3.40 (m, 2 H), 3.52 - 3.68 (m, 2 H), 3.84 (s, 2 H), 6.35 (s, 1 H,) 7.25 (dd, *J*=10.85, 8.48 Hz, 1 H), 7.33 - 7.49 (m, 1 H), 7.57 (dd, *J*=7.12, 2.37 Hz, 1 H), 8.31 - 8.50 (m, 1 H), 11.84 (s, 1 H).

### EXAMPLE 473

### 8-(4-fluoro-3-{[4-(morpholin-4-ylcarbonyl)piperazin-1-yl]carbonyl}benzyl)-2,3,4,6-tetrahydropyrido[2,3-d]pyridazin-5(1H)-one

The title compound was prepared as TFA salt according to procedure for EXAMPLE 48, substituting EXAMPLE 468 for EXAMPLE 48C, and morpholino(piperazin-1-yl)methanone for 1-(3-dimethylaminopropyl)-3-ethylcarbodiimide. MS (ESI) m/z 485 (M+H)⁺; ¹H NMR (300 MHz, DMSO-*d₆*): δ 1.60 - 1.78 (m, 2 H), 2.35 (t, *J*=6.15 Hz, 2 H), 3.05 - 3.28 (m, 12 H), 3.51 - 3.58 (m, 4 H), 3.60 - 3.70 (m, 2 H), 3.82 (s, 2 H), 6.41 (s, 1 H), 7.16 - 7.29 (m, 2 H), 7.29 - 7.37 (m, 1 H), 11.92 (s, 1 H).

### EXAMPLE 474

### N-{2-fluoro-5-[(5-oxo-1,2,3,4,5,6-hexahydropyrido[2,3-d]pyridazin-8-yl)methyl]phenyl}-N'-phenylpentanediamide

The title compound was prepared as TFA salt according to the procedure for EXAMPLE 136, substituting EXAMPLE 467 for EXAMPLE 89, and 5-oxo-5-(phenylamino)pentanoic acid for 1-methylcyclopropanecarboxylic acid. MS (ESI) m/z 464 (M+H)⁺; ¹H NMR (300 MHz, DMSO-*d₆*): δ 1.62 - 1.75 (m, 2 H), 1.81 - 1.96 (m, 2 H), 2.34 (t, *J*=7.12 Hz, 4 H), 2.42 (t, *J*=8.14 Hz, 2 H), 3.09 - 3.22 (m, 2 H), 3.77 (s, 2 H), 6.30 (s, 1 H), 6.93 - 7.07 (m, 1 H), 7.14 (dd, *J*=10.85, 8.48 Hz, 1 H), 7.22 - 7.34 (m, 3 H), 7.59 (d, *J*=7.80 Hz, 2 H), 7.68 - 7.77 (m, 1 H), 9.62 (s, 1 H), 9.87 (s, 1 H), 11.82 (s, 1 H).

### EXAMPLE 475

### 1-{2-fluoro-5-[(5-oxo-1,2,3,4,5,6-hexahydropyrido[2,3-d]pyridazin-8-yl)methyl]phenyl}pyrrolidine-2,5-dione

### EXAMPLE 475A

### 4-(2-fluoro-5-((5-oxo-5,6-dihydropyrido[3,2-d]pyridazin-8-yl)methyl)phenylamino)-4-oxobutanoic acid

The title compound was prepared according to procedure for EXAMPLE 3, substituting EXAMPLE 463 for EXAMPLE 2. MS (ESI) m/z 371 (M+H)⁺.

### EXAMPLE 475B

### 1-(2-fluoro-5-((5-oxo-5,6-dihydropyrido[3,2-d]pyridazin-8-yl)methyl)phenyl)pyrrolidine-2,5-dione

The title compound was prepared according to procedure for EXAMPLE 4, substituting EXAMPLE 475A for EXAMPLE 3. MS (ESI) m/z 353 (M+H)⁺.

### EXAMPLE 475C

### 1-{2-fluoro-5-[(5-oxo-1,2,3,4,5,6-hexahydropyrido[2,3-d]pyridazin-8-yl)methyl]phenyl}pyrrolidine-2,5-dione

The title compound was prepared according to procedure for EXAMPLE 414, substituting EXAMPLE 475B for EXAMPLE 369. MS (ESI) m/z 357 (M+H)⁺; ¹H NMR (300 MHz, DMSO-*d₆*): δ 1.58 - 1.78 (m, 2 H), 2.33 (t, *J*=6.27 Hz, 2 H), 2.72 - 2.90 (m, 4 H), 3.07 - 3.23 (m, 2 H), 3.84 (s, 2 H), 6.34 (s, 1 H), 7.13 (dd, *J*=6.95, 2.20 Hz, 1 H), 7.27 - 7.37 (m, 1 H), 7.37 - 7.43 (m, 1 H), 11.83 (s, 1 H).

### EXAMPLE 476

### N-{2-fluoro-5-[(5-oxo-1,2,3,4,5,6-hexahydropyrido[2,3-d]pyridazin-8-yl)methyl]phenyl}-3-methoxypropanamide

The title compound was prepared as TFA salt according to the procedure for EXAMPLE 136, substituting EXAMPLE 467 for EXAMPLE 89, and 3-methoxypropanoic acid for 1-methylcyclopropanecarboxylic acid. MS (ESI) m/z 361 (M+H)⁺; ¹H NMR (300 MHz, DMSO-*d₆*): δ 1.63 - 1.74 (m, 2 H), 2.33 (t, *J*=6.15 Hz, 2 H), 2.60 (t, *J*=6.15 Hz, 2 H), 3.09 - 3.21 (m, 2 H), 3.24 (s, 3 H), 3.59 (t, *J*=6.15 Hz, 2 H), 3.77 (s, 2 H), 6.33 (s, 1 H), 6.93 - 7.05 (m, 1 H), 7.14 (dd, *J*=10.91, 8.53 Hz, 1 H), 7.69 - 7.80 (m, 1 H), 9.63 (s, 1 H), 11.85 (s, 1 H).

### EXAMPLE 477

### N-{2-fluoro-5-[(5-oxo-1,2,3,4,5,6-hexahydropyrido[2,3-d]pyridazin-8-yl)methyl]phenyl}-5-oxohexanamide

The title compound was prepared as TFA salt according to the procedure for EXAMPLE 136, substituting EXAMPLE 467 for EXAMPLE 89, and 5-oxohexanoic acid for 1-methylcyclopropanecarboxylic acid. MS (ESI) m/z 387 (M+H)⁺; ¹H NMR (300 MHz, DMSO-*d₆*): 1.64 - 1.80 (m, 4 H), 2.08 (s, 3 H), 2.27 - 2.39 (m, 4 H), 2.42 - 2.50 (m, 2 H), 3.10 - 3.23 (m, 2 H), 3.77 (s, 2 H), 6.34 (s, 1 H), 6.94 - 7.04 (m, 1 H), 7.13 (dd, *J*=10.85, 8.48 Hz, 1 H), 7.66 - 7.71 (m, 1 H), 9.58 (s, 1 H), 11.86 (s, 1 H).

### EXAMPLE 478

### N-{2-fluoro-5-[(5-oxo-1,2,3,4,5,6-hexahydropyrido[2,3-d]pyridazin-8-yl)methyl]phenyl}-3-phenoxypropanamide

The title compound was prepared as TFA salt according to the procedure for EXAMPLE 136, substituting EXAMPLE 467 for EXAMPLE 89, and 3-phenoxypropanoic acid for 1-methylcyclopropanecarboxylic acid. MS (ESI) m/z 423 (M+H)⁺; ¹H NMR (300 MHz, DMSO-*d₆*): δ 1.55 - 1.74 (m, 2 H), 2.33 (t, *J*=6.15 Hz, 2 H), 2.84 (t, *J*=6.15 Hz, 2 H), 3.08 - 3.21 (m, 2 H), 3.78 (s, 2 H), 4.24 (t, *J*=6.15 Hz, 2 H), 6.36 (s, 1 H), 6.88 - 6.96 (m, 3 H), 6.97 - 7.05 (m, 1 H), 7.16 (dd, *J*=10.91, 8.53 Hz, 1 H), 7.25 - 7.31 (m, 2 H), 7.77 (dd, *J*=7.54, 1.98 Hz, 1 H), 9.79 (s, 1 H), 11.89 (s, 1 H).

### EXAMPLE 479

### N-{2-fluoro-5-[(5-oxo-1,2,3,4,5,6-hexahydropyrido[2,3-d]pyridazin-8-yl)methyl]phenyl}-4-oxo-4-phenylbutanamide

The title compound was prepared according to the procedure for EXAMPLE 136, substituting EXAMPLE 467 for EXAMPLE 89, and 4-oxo-4-phenylbutanoic acid for 1-methylcyclopropanecarboxylic acid. MS (ESI) m/z 435 (M+H)⁺; ¹H NMR (300 MHz, DMSO-*d₆*): δ 1.63 - 1.73 (m, 2 H), 2.32 (t, *J*=5.95 Hz, 2 H), 2.75 - 2.79 (m, 2 H), 3.08 - 3.19 (m, 2 H), 3.27 - 3.36 (m, 2 H), 3.75 (s, 2 H), 6.27 (s, 1 H), 6.91 - 7.04 (m, 1 H), 7.14 (dd, *J*=10.91, 8.53 Hz, 1 H), 7.54 (t, *J*=7.54 Hz, 2 H), 7.59 - 7.69 (m, 1 H), 7.70 - 7.77 (m, 1 H), 7.94 - 8.03 (m, 2 H), 9.74 (s, 1 H), 11.78 (s, 1 H).

### EXAMPLE 481

### 2-[4-(benzyloxy)phenoxy]-N-{2-fluoro-5-[(5-oxo-1,2,3,4,5,6-hexahydropyrido[2,3-d]pyridazin-8-yl)methyl]phenyl}acetamide

The title compound was prepared as TFA salt according to the procedure for EXAMPLE 136, substituting EXAMPLE 467 for EXAMPLE 89, and 2-(4-(benzyloxy)phenoxy)acetic acid for 1-methylcyclopropanecarboxylic acid. MS (ESI) m/z 515 (M+H)⁺; ¹H NMR (300 MHz, DMSO-*d₆*): δ 1.53 - 1.81 (m, 2 H), 2.22 - 2.39 (m, 2 H), 3.07 - 3.21 (m, 2 H), 3.78 (s, 2 H), 4.66 (s, 2 H), 5.04 (s, 2 H), 6.34 (s, 1 H), 6.83 - 6.99 (m, 4 H), 7.02 - 7.10 (m, 1 H), 7.14 - 7.23 (m, 1 H), 7.30 - 7.37 (m, 2 H), 7.37 - 7.46 (m, 3 H), 7.65 (dd, *J*=7.54, 1.98 Hz, 1 H), 9.77 (s, 1 H), 11.84 (s, 1 H).

### EXAMPLE 483

### N-{2-fluoro-5-[(5-oxo-1,2,3,4,5,6-hexahydropyrido[2,3-d]pyridazin-8-yl)methyl]phenyl}-2-(4-methoxyphenoxy)acetamide

The title compound was prepared as TFA salt according to the procedure for EXAMPLE 136, substituting EXAMPLE 467 for EXAMPLE 89, and 2-(4-methoxyphenoxy)acetic acid for 1-methylcyclopropanecarboxylic acid. MS (ESI) m/z 438 (M+H)⁺; ¹H NMR (300 MHz, DMSO-*d₆*): 1.63 - 1.75 (m, 2 H), 2.33 (t, *J*=6.27 Hz, 2 H), 3.08 - 3.24 (m, 2 H), 3.70 (s, 3 H), 3.79 (s, 2 H), 4.66 (s, 2 H), 6.35 (s, 1 H), 6.84 - 6.96 (m, 4 H), 7.01 - 7.11 (m, 1 H), 7.18 (dd, *J*=10.85, 8.48 Hz, 1 H), 7.66 (dd, *J*=7.63, 2.20 Hz, 1 H), 9.73 (s, 1 H), 11.85 (s, 1 H).

### EXAMPLE 484

### N-cyclopropyl-2-fluoro-5-[(5-oxo-1,2,3,4,5,6-hexahydropyrido[2,3-d]pyridazin-8-yl)methyl]benzamide

The title compound was prepared as TFA salt according to procedure for EXAMPLE 48, substituting EXAMPLE 468 for EXAMPLE 48C, and cyclopropanamine for 1-(3-dimethylaminopropyl)-3-ethylcarbodiimide. MS (ESI) m/z 343 (M+H)⁺; ¹H NMR (300 MHz, DMSO-*d₆*): 0.44 - 0.59 (m, 2 H), 0.63 - 0.76 (m, 2 H), 1.60 - 1.78 (m, 2 H), 2.34 (t, *J*=6.35 Hz, 2 H), 2.74 - 2.90 (m, 1 H), 3.09 - 3.22 (m, 2 H), 3.81 (s, 2 H), 6.39 (s, 1 H), 7.03 - 7.25 (m, 1 H), 7.25 - 7.37 (m, 1 H), 7.42 (dd, *J*=6.74, 2.38 Hz, 1 H), 8.33 (d, *J*=3.97 Hz, 1 H), 11.89 (s, 1 H).

### EXAMPLE 485

### 8-(3-{[4-(2-ethoxyethyl)piperazin-1-yl]carbonyl}-4-fluorobenzyl)-2,3,4,6-tetrahydropyrido[2,3-d]pyridazin-5(1H)-one

The title compound was prepared as TFA salt according to procedure for EXAMPLE 48, substituting EXAMPLE 468 for EXAMPLE 48C, and 1-(2-ethoxyethyl)piperazine for 1-(3-dimethylaminopropyl)-3-ethylcarbodiimide. MS (ESI) m/z 444 (M+H)⁺; ¹H NMR (300 MHz, DMSO-*d₆*): 1.09 (t, *J*=9.0 Hz, 3 H), 1.61 - 1.76 (m, 2 H), 2.56 - 2.69 (m, 2 H), 3.01 - 3.11 (m, 2 H), 3.11 - 3.24 (m, 4 H), 3.35 - 3.43 (m, 4 H), 3.43 - 3.61 (m, 4 H), 3.82 (s, 2 H), 6.35 (s, 1 H), 7.18 - 7.26 (m, 1 H), 7.28 - 7.34 (m, 1 H), 7.34 - 7.41 (m, 1 H), 11.84 (s, 1 H).

### EXAMPLE 486

### 2-fluoro-5-[(5-oxo-1,2,3,4,5,6-hexahydropyrido[2,3-d]pyridazin-8-yl)methyl]-N-(2-piperidin-1-ylethyl)benzamide

The title compound was prepared according to procedure for EXAMPLE 48, substituting EXAMPLE 468 for EXAMPLE 48C, and 2-(piperidin-1-yl)ethanamine for 1-(3-dimethylaminopropyl)-3-ethylcarbodiimide. MS (ESI) m/z 414 (M+H)⁺; ¹H NMR (300 MHz, DMSO-*d₆*): 1.33 - 1.49 (m, 2 H), 1.50 - 1.64 (m, 4 H), 1.62 - 1.75 (m, 2 H), 2.33 (t, *J*=6.35 Hz, 2 H), 2.54 - 2.82 (m, 4 H), 3.10 - 3.20 (m, 2 H), 3.20 - 3.35 (m, 2 H), 3.37 - 3.55 (m, 2 H), 3.82 (s, 2 H), 6.35 (s, 1 H), 7.20 (dd, *J*=10.51, 8.53 Hz, 1 H), 7.33 - 7.44 (m, 1 H), 7.53 (dd, *J*=7.14, 2.38 Hz, 1 H), 8.51 (dd, *J*=4.36, 1.59 Hz, 1 H,) 11.83 (s, 1 H).

### EXAMPLE 487

### 2-fluoro-5-((5-oxo-1,2,3,4,5,6-hexahydropyrido[3,2-d]pyridazin-8-yl)methyl)-N-(2-oxo-2-(piperidin-1-yl)ethyl)benzamide

The title compound was prepared according to procedure for EXAMPLE 48, substituting EXAMPLE 468 for EXAMPLE 48C, and 2-amino-1-(piperidin-1-yl)ethanone for 1-(3-dimethylaminopropyl)-3-ethylcarbodiimide. MS (ESI) m/z 428 (M+H)⁺; ¹H NMR (300 MHz, DMSO-*d₆*): 1.44 (m, 2 H), 1.48 - 1.65 (m, 4 H), 1.65 - 1.79 (m, 2 H), 2.33 (t, *J*=6.27 Hz, 2 H), 3.10 - 3.24 (m, 2 H), 3.34 - 3.42 (m, 2 H), 3.41 - 3.50 (m, 2 H), 3.84 (s, 2 H), 4.13 (d, *J*=5.09 Hz, 2 H), 6.00 - 6.50 (m, 1 H), 7.05 - 7.28 (m, 1 H), 7.32 - 7.53 (m, 1 H), 7.63 (dd, *J*=7.12, 2.37 Hz, 1 H), 8.17 (q, *J*=5.09 Hz, 1 H), 11.82 (s, 1 H).

### EXAMPLE 490

### 4-{4-fluoro-3-[(4-pyrimidin-2-ylpiperazin-1-yl)carbonyl]benzyl}-5,6,7,8-tetrahydrophthalazin-1 (2H)-one

To a solution of EXAMPLE 1 (100 mg, 0.33 mmol) in dimethlyacetamide (5 mL) was added 2-(1H-7-azabenzotriazol-1-yl)-1,1,3,3-tetramethyl uronium hexafluorophosphate methanaminium (HATU) (126 mg, 0.33 mmol) and triethylamine (92 µL, 0.66 mmol) and stirred for 20 minutes at room temperature. (Piperazin-1-yl)pyrimidine dihydrochloride (78 mg, 0.33 mmol) was then added and the reaction mixture was stirred at room temperature for 16 hours. After concentration, the residual oil was purified by HPLC (Zorbax® C-18 ODS packing material [Agilent Technologies, Santa Clara, CA], 0.1% TFA/CH₃CN/H₂O) to provide the title product. MS (DCI/NH₃) m/z 449 (M+H)⁺; ¹H NMR (300 MHz, DMSO-*d₆*): δ 1.53 - 1.71 (m, 4H), 2.32 - 2.44 (m, 4H), 3.24 - 3.39 (m, 2H), 3.67 - 3.78 (m, 4H), 3.79 - 3.88 (m, 2H), 3.93 (s, 2H), 6.67 (t, *J=*4.75 Hz, 1H), 7.21 - 7.23 (m, 1H), 7.24 - 7.28 (m, 1H), 7.30 - 7.35 (m, 1H), 8.39 (d, *J*=4.75 Hz, 2H), 12.62 (br s, 1H).

### EXAMPLE 491

### 4-(4-fluoro-3-(2-oxopyrrolidin-1-yl)phenyl)-5,6,7,8-tetrahydrophthalazin-1(2H)-one

The title compound was prepared according to the procedure for EXAMPLE 461, substituting 2-pyrrolidinone for 2-azetidinone in EXAMPLE 461 B. MS (DCI/NH₃) m/z 328 (M+H)⁺; ¹H NMR (400 MHz, CD₃OD): δ 1.73 - 1.79 (m, 2 H), 1.83 - 1.90 (m, 2 H), 2.22-2.29 (m, 2 H), 2.55 - 2.60 (m, 4 H), 2.69 (t, *J*=5.83 Hz, 2 H), 3.91 (t, *J*=7.06 Hz, 2 H), 7.35 - 7.41 (m, 1 H), 7.48 - 7.52 (m, 1 H), 7.60 - 7.64 (m, 1 H).

The foregoing is meant to be illustrative of the invention and not meant to limit it to disclosed embodiments. Variations and changes obvious to one skilled in the art are intended to be within the scope and nature of the invention as defined in the appended claims.

The following embodiments 1.-35., listed as items 1.-35., are embodiments of the invention.
1. A compound having formula I 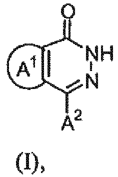 or a pharmaceutically acceptable salt thereof, wherein
   A¹ is R¹ or R², wherein A¹ is unsubstituted or substituted with one or two OH, CN, C₁-alkyl, C₂-alkyl, C₃-alkyl, C₄-alkyl, C₅-alkyl, cycloalkane, OR^{A} or NR^{A}R^{A};
   R^{A} is H or alkyl;
   R¹ is cycloalkane or cycloalkene each of which is unfused or fused with R^{1A};
   R^{1A} is benzene, heteroarene, cycloalkane, cycloalkene, heterocycloalkane or heterocycloalkene;
   R² is heterocycloalkane or heterocycloalkene; each of which is unfused or fused with R^{2A};
   R^{2A} is benzene, heteroarene, cycloalkane, cycloalkene, heterocycloalkane or heterocycloalkene;
   A² is OR⁴, NHR⁴, N(R⁴)₂, SR⁴, S(O)R⁴, SO₂R⁴ or R⁵;
   wherein each R⁴ is C₁-alkyl, C₂-alkyl or C₃-alkyl; each of which is substituted with R¹⁰;
   R⁵ is C₁-alkyl, C₂-alkyl, C₃-alkyl, C₄-alkyl or C₅-alkyl; each of which is substituted with R¹⁰, and further unsubstituted or substituted with one or two or three of independently selected OR¹⁰, NHR¹⁰, N(R¹⁰)₂, SR¹⁰, S(O)R¹⁰, SO₂R¹⁰ or CF₃;
   wherein each R¹⁰ is R^{10A}, R^{10B} or R^{10C}; each of which must be attached at a carbon atom;
   R^{10A} is phenyl which is unfused or fused with benzene, heteroarene, cycloalkane, cycloalkene, heterocycloalkane or heterocycloalkene; each of which are unfused or fused with benzene, heteroarene, cycloalkane, cycloalkene, heterocycloalkane or heterocycloalkene; 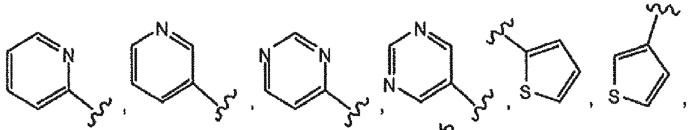
   R^{10B} is 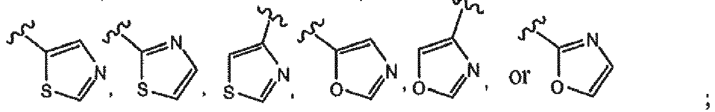 each of which is unfused or fused with benzene, heteroarene, cycloalkane, cycloalkene, heterocycloalkane or heterocycloalkene; each of which are unfused or fused with benzene, heteroarene, cycloalkane, cycloalkene, heterocycloalkane or heterocycloalkene;
   R^{10C} is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene;
   wherein each R¹⁰ is independently unsubstituted or substituted with one or two or three of independently selected, R¹¹, OR¹¹, SR¹¹, S(O)R¹¹, SO₂R¹¹, NH₂, NHR¹¹, N(R¹¹)₂, C(O)R¹¹, C(O)OR¹¹, C(O)NH₂, C(O)NHR¹¹, C(O)N(R¹¹)₂, NHC(O)R¹¹, NR¹¹C(O)R¹¹, NHSO₂R¹¹, NR¹¹SO₂R¹¹, NHC(O)OR¹¹, NR¹¹C(O)OR¹¹, NHSO₂NH₂, NHSO₂NHR¹¹, NHSO₂N(R¹¹)₂, SO₂NH₂, SO₂NHR¹¹, SO₂N(R¹¹)₂, NHC(O)NH₂, NHC(O)NHR¹¹, NHC(O)N(R¹¹)₂, NR¹¹C(O)N(R¹¹)₂, NO₂, OH, (O), C(O)H, C(O)OH, CN, CF₃, OCF₃, CF₂CF₃, F, Cl, Br or I;
   wherein each R¹¹ is R¹², R¹³, R¹⁴ or R¹⁵;
   R¹² is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R¹³ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R¹⁴ is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R¹⁵ is alkyl, alkenyl or alkynyl; each of which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NH₂, C(O)NHR¹⁶, C(O)N(R¹⁶)₂, NHC(O)R¹⁶, NR¹⁶C(O)R¹⁶, NHC(O)OR¹⁶, NR¹⁶C(O)OR¹⁶, OH, F, Cl, Br or I;
   wherein each R¹⁶ is R¹⁷ or R^{17A};
   R¹⁷ is alkyl, alkenyl or alkynyl; each of which is unsubstituted or substituted with one or two of independently selected R¹⁸, C(O)OH, NH₂, NHR¹⁸ or N(R¹⁸)₂, C(O)R¹⁸, C(O)NH₂, C(O)NHR¹⁸, C(O)N(R¹⁸)₂, NHC(O)R¹⁸, NR¹⁸C(O)R¹⁸, F, Cl, Br or I;
   R^{17A} is phenyl, heteroaryl, cycloalkyl, cycloalkenyl, heterocycloalkyl, heterocycloalkenyl each of which is unfused or fused with benzene, heteroarene, cycloalkane, cycloalkene, heterocycloalkane or heterocycloalkene;
   wherein each R¹⁸ is phenyl, heteroaryl, cycloalkyl, cycloalkenyl, heterocycloalkyl or heterocycloalkenyl;
   wherein each of the moieties represented by R¹², R¹³, R¹⁴, R^{17A}, and R¹⁸ are independently unsubstituted or substituted with one or two or three or four of independently selected R¹⁹, OR¹⁹, SR¹⁹, S(O)R¹⁹, SO₂R¹⁹, C(O)R¹⁹, CO(O)R¹⁹, OC(O)R¹⁹, OC(O)OR¹⁹, NH₂, NHR¹⁹, N(R¹⁹)₂, NHC(O)R¹⁹, NR¹⁹C(O)R¹⁹, NHS(O)₂R¹⁹, NR¹⁹S(O)₂R¹⁹, NHC(O)OR¹⁹, NR¹⁹C(O)OR¹⁹, NHC(O)NH₂, NHC(O)NHR¹⁹, NHC(O)N(R¹⁹)₂, NR¹⁹C(O)NHR¹⁹, NR¹⁹C(O)N(R¹⁹)₂, C(O)NH₂, C(O)NHR¹⁹, C(O)N(R¹⁹)₂, C(O)NHOH, C(O)NHOR¹⁹, C(O)NHSO₂R¹⁹, C(O)NR¹⁹SO₂R¹⁹, SO₂NH₂, SO₂NHR¹⁹, SO₂N(R¹⁹)₂, C(O)H, C(O)OH, C(N)NH₂, C(N)NHR¹⁹, C(N)N(R¹⁹)₂, CNOH, CNOCH₃, OH, (O), CN, N₃, NO₂, CF₃, CF₂CF₃, OCF₃, OCF₂CF₃, F, Cl, Br or I;
   wherein each R¹⁹ is R²⁰, R²¹, R²² or R²³;
   R²⁰ is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R²¹ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R²² is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R²³ is alkyl, alkenyl or alkynyl; each of which is unsubstituted or substituted with one or two of independently selected R²⁴, OR²⁴, SR²⁴, S(O)₂R²⁴, C(O)OH, NH₂, NHR²⁴ N(R²⁴)₂, C(O)R²⁴, C(O)NH₂, C(O)NHR²⁴, C(O)N(R²⁴)₂, NHC(O)R²⁴, NR²⁴C(O)R²⁴, NHC(O)OR⁴, NR²⁴C(O)OR²⁴, NHS(O)₂R²⁴, NR²⁴S(O)₂R²⁴, OH, F, Cl, Br or I;
   wherein each R²⁴ is R^{24A} or R^{24B};
   R^{24A} is phenyl, heteroaryl, cycloalkyl, cycloalkenyl, heterocycloalkyl or heterocycloalkenyl each of which is unfused or fused with benzene, heteroarene, cycloalkane, cycloalkene, heterocycloalkane or heterocycloalkene;
   R^{24B} is alkyl, alkenyl or alkynyl each of which is unsubstituted or substituted with one or two of independently selected R²⁵, OR²⁵, SR²⁵, S(O)₂R²⁵, C(O)OH, NH₂, NHR²⁵ N(R²⁵)₂, C(O)R²⁵, C(O)NH₂, C(O)NHR²⁵, C(O)N(R²⁵)₂, NHC(O)R²⁵, NR²⁵C(O)R²⁵, NHC(O)OR²⁵, NR²⁵C(O)OR²⁵, OH, F, Cl, Br or I;
   wherein each R²⁵ is alkyl, phenyl, heteroaryl, cycloalkyl, cycloalkenyl, heterocycloalkyl or heterocycloalkenyl ; each of which is unsubstituted or substituted with NH₂, NH(CH₃), N(CH₃)₂, OH or OCH₃;
   wherein each of the moieties represented by R²⁰, R²¹, R²², and R^{24A} are independently unsubstituted or substituted with one or two of independently selected R²⁶, OR²⁶, alkenyl, alkynyl, phenyl, OH, (O), C(O)OH, CN, CF₃, OCF₃, CF₂CF₃, F, Cl, Br or I; and
   R²⁶ is alkyl.
2. The compound of item 1, wherein A¹ is unsubstituted R¹ or unsubstituted R²;
   R¹ is cycloalkane, which is unfused;
   R² is heterocycloalkane, which is unfused; and
   A² is R⁵;
   or a pharmaceutically acceptable salt thereof.
3. The compound of item 2, wherein A¹ is unsubstituted R¹; and
   R¹ is cyclohexane, which is unfused;
   or a pharmaceutically acceptable salt thereof.
4. The compound of item 3, wherein R⁵ is C₁-alkyl, C₂-alkyl or C₃-alkyl;
   or a pharmaceutically acceptable salt thereof.
5. The compound of item 4, wherein
   R^{10A} is phenyl which is unfused or fused with heterocycloalkane, which is fused heterocycloalkane;
   R^{10B} is 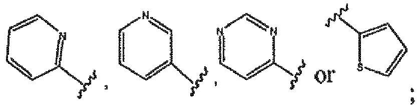
   R^{10C} is heterocycloalkyl, which is unfused;
   wherein R¹⁰ is substituted with F and further unsubstituted or substituted with one or two of independently selected R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹NHC(O)R¹¹, NHSO2R¹¹, NR¹¹SO₂R¹¹, NHC(O)OR¹¹, NHSO₂N(R¹¹)₂, NO₂, OH, (O), C(O)OH, F, Cl or Br;
   wherein each R¹¹ is R¹², R¹³, R¹⁴ or R¹⁵;
   R¹² is phenyl which is unfused or fused with benzene, heteroarene, heterocycloalkane or heterocycloalkene;
   R¹³ is heteroaryl, which is unfused;
   R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene;
   R¹⁵ is alkyl which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I;
   wherein each R¹⁶ is R¹⁷ or R^{17A};
   R¹⁷ is alkyl which is unsubstituted or substituted with R¹⁸;
   R^{17A} is phenyl, heteroaryl, cycloalkyl, heterocycloalkyl or heterocycloalkenyl each of which is unfused or fused with benzene or heterocycloalkane;
   R¹⁸ is phenyl or heterocycloalkyl, which is unfused;
   wherein the moieties represented by R¹², R¹³, R¹⁴, R^{17A}, and R¹⁸ are independently unsubstituted or substituted with one or two of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I;
   wherein each R¹⁹ is R²⁰, R²¹, R²² or R²³;
   R²⁰ is phenyl, which is unfused;
   R²¹ is heteroaryl, which is unfused;
   R²² is cycloalkyl or heterocycloalkyl; each of which is unfused or fused with benzene;
   R²³ is alkyl which is unsubstituted or substituted with R²⁴, OR²⁴, NHR²⁴ N(R²⁴)₂, NHS(O)₂R²⁴ or OH;
   wherein each R²⁴ is R^{24A} or R^{24B};
   R^{24A} is phenyl, cycloalkyl, heterocycloalkyl or heterocycloalkenyl, which is unfused or fused with heterocycloalkane;
   R^{24B} is alkyl, which is unsubstituted or substituted with OR²⁵, OH, F, Cl, Br or I;
   R²⁵ is alkyl, which is unsubstituted or substituted with NH₂;
   wherein the moieties represented by R²⁰, R²¹, R²², and R^{24A} are independently unsubstituted or substituted with one or two of independently selected R²⁶, OR²⁶ (O), F, Cl, Br or I; and
   R²⁶ is alkyl;
   or a pharmaceutically acceptable salt thereof.
6. The compound of item 4, wherein R¹⁰ is phenyl which is unfused;
   or a pharmaceutically acceptable salt thereof.
7. The compound of item 6, wherein R¹⁰ is substituted with C(O)R¹¹, C(O)NH₂, C(O)NHR¹¹, C(O)N(R¹¹)₂, NHC(O)R¹¹ or NR¹¹C(O)R¹¹ and is further unsubstituted or substituted with one or two or three of independently selected R¹¹, OR¹¹, SR¹¹, S(O)R¹¹, SO₂R¹¹, NH₂, NHR¹¹, N(R¹¹)₂, C(O)OR¹¹, C(O)NH₂, C(D)NHR¹¹, C(O)N(R¹¹)₂, NHC(O)R¹¹, NR¹¹C(O)R¹¹, NHSO₂R¹¹, NR¹¹SO₂R¹¹, NHC(O)OR¹¹, NR¹¹C(O)OR¹¹, NHSO₂NH₂, NHSO₂NHR¹¹, NHSO₂N(R¹¹)₂, SO₂NH₂, SO₂NHR¹¹, SO₂N(R¹¹)₂, NHC(O)NH₂, NHC(O)NHR¹¹, NHC(O)N(R¹¹)₂, NR¹¹C(O)N(R¹¹)₂, NO₂, OH, (O), C(O)H, C(O)OH, CN, CF₃, OCF₃, CF₂CF₃, F, Cl, Br or I;
   or a pharmaceutically acceptable salt thereof.
8. The compound of item 3, wherein
   R⁵ is C₁-alkyl which is substituted with R¹⁰, and further unsubstituted or substituted with CF₃;
   R¹⁰ is R^{10A} , R^{10B} or R^{10C}; each of which must be attached at a carbon atom;
   R^{10A} is phenyl which is unfused or fused with heterocycloalkane, which is fused with heterocycloalkane;
   R^{10B} is 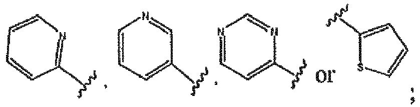
   R^{10C} is heterocycloalkyl, which is unfused;
   wherein R¹⁰ is substituted with C(O)R¹¹, C(O)NHR¹¹, C(O)N(R¹¹)₂ or NHC(O)R¹¹, and is further unsubstituted or substituted with one or two or three of independently selected R¹¹, OR¹¹, SR¹¹, S(O)R¹¹, SO₂R¹¹, NH₂,N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂ NO₂, OH, (O), C(O)OH, F, Cl, Br or I;
   wherein each R¹¹ is R¹², R¹³, R¹⁴ or R¹⁵;
   R¹² is phenyl which is unfused or fused with benzene, heteroarene, heterocycloalkane or heterocycloalkene;
   R¹³ is heteroaryl, which is unfused;
   R¹⁴ is cycloalkyl, heterocycloalkyl or heterocycloalkenyl; each of which is unfused or fused with benzene, cycloalkane, heterocycloalkane or heterocycloalkene; each of which is unfused or fused with benzene;
   R¹⁵ is alkyl, which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I;
   wherein each R¹⁶ is R¹⁷ or R^{17A};
   R¹⁷ is alkyl, which is unsubstituted or substituted with R¹⁸;
   R^{17A} is phenyl, heteroaryl, cycloalkyl, heterocycloalkyl, heterocycloalkenyl each of which is unfused or fused with benzene or heterocycloalkane;
   R¹⁸ is phenyl or heterocycloalkyl, which is unfused;
   wherein the moieties represented by R¹², R¹³, R¹⁴, R^{17A}, and R¹⁸ are independently unsubstituted or substituted with one or two independently selected R¹⁹, OR¹⁹, SR¹⁹, , SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I;
   wherein each R¹⁹ is R²⁰, R²¹, R²² or R²³;
   R²⁰ is phenyl, which is unfused;
   R²¹ is heteroaryl, which is unfused;
   R²² is cycloalkyl,or heterocycloalkyl each of which is unfused or fused with benzene;
   R²³ is alkyl which is unsubstituted or substituted with one or two of independently selected R²⁴, OR²⁴, NHR²⁴ N(R²⁴)₂, NHS(O)₂R²⁴, OH, F, Cl, Br or I;
   wherein each R²⁴ is R^{24A} or R^{24B};
   R^{24A} is phenyl, cycloalkyl, heterocycloalkyl or heterocycloalkenyl; each of which is infused or fused with heterocycloalkane;
   R^{24B} is alkyl which is unsubstituted or substituted with OR²⁵, OH, F, Cl, Br or I;
   R²⁵ is alkyl each of which is unsubstituted or substituted with NH₂;
   wherein the moieties represented by R²⁰, R²¹, R²², and R^{24A} are independently unsubstituted or substituted with one or two of independently selected R²⁶, OR²⁶, (O), F, Cl, Br or I; and
   R²⁶ is alkyl;
   or a pharmaceutically acceptable salt thereof.
9. The compound of item 3, wherein R¹⁰ is R^{10A}, wherein is R^{10A} is phenyl which is unfused and substituted with F, and further substituted with C(O)R¹¹, C(O)NH₂, C(O)NHR¹¹, C(O)N(R¹¹)₂, NHC(O)R¹¹ or NR¹¹C(O)R¹¹ and is further unsubstituted or substituted with one or two or three of independently selected R¹¹, OR¹¹, SR¹¹, S(O)R¹¹, SO₂R¹¹, NH₂, NHR¹¹, N(R¹¹)2, C(O)R¹¹, C(O)OR¹¹, C(O)NH₂, C(O)NHR¹¹, C(O)N(R¹¹)₂, NHC(O)R¹¹, NR¹¹C(O)R¹¹, NHSO₂R¹¹, NR¹¹SO₂R¹¹, NHC(O)OR¹¹, NR¹¹C(O)OR¹¹, NHSO₂NH₂, NHSO₂NHR¹¹, NHSO₂N(R¹¹)₂, SO₂NH₂, SO₂NHR¹¹, SO₂N(R¹¹)₂, NHC(O)NH₂, NHC(O)NHR¹¹, NHC(O)N(R¹¹)₃, NR¹¹C(O)N(R¹¹)₂, NO₂, OH, (O), C(O)H, C(O)OH, CN, CF₃, OCF₃, CF₂CF₃, F, Cl, Br or I;
   or a pharmaceutically acceptable salt thereof.
10. The compound of item 4, wherein R¹⁰ is R^{10A}, wherein is R^{10A} is phenyl which is unfused and substituted with F, and further substituted with NHC(O)R¹¹, wherein R¹¹ is R¹⁵;
   or a pharmaceutically acceptable salt thereof.
11. The compound of item 10, wherein R¹⁵ is alkyl, which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NHR¹⁶, NHC(O)R¹⁶, NHC(O)OR¹⁶, OH, F, Cl, Br or I;
   wherein each R¹⁶ is R¹⁷ or R^{17A};
   R¹⁷ is alkyl, which is unsubstituted or substituted with one or two of independently selected R¹⁸;
   R^{17A} is phenyl, heteroaryl, cycloalkyl, heterocycloalkyl, heterocycloalkenyl each of which is unfused or fused with benzene or heterocycloalkane;
   wherein each R¹⁸ is phenyl or heterocycloalkyl;
   wherein each of the moieties represented by R^{17A} and R¹⁸ are independently unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I;
   wherein each R¹⁹ is R²⁰, R²¹, R²² or R²³;
   R²⁰ is phenyl which is unfused;
   R²¹ is heteroaryl which is unfused;
   R²² is cycloalkyl or heterocycloalkyl; each of which are unfused or fused with benzene;
   R²³ is alkyl, which is unsubstituted or substituted with one or two of independently selected R²⁴, OR²⁴, NHR²⁴N(R²⁴)₂, NHS(O)₂R²⁴ or OH;
   wherein each R²⁴ is R^{24A} or R^{24B};
   R^{24A} is unsubstituted phenyl, cycloalkyl, heterocycloalkyl or heterocycloalkenyl each of which is unfused or fused with heterocycloalkane;
   R^{24B} is alkyl, which is unsubstituted or substituted with one or two of independently selected OR²⁵ or OH;
   wherein each R²⁵ is alkyl unsubstituted or substituted with NH₂;
   wherein each R²⁰ is unsubstituted or substituted with one or two of independently selected R²⁶, OR²⁶, (O), F, Cl, Br or I; and
   R²⁶ is alkyl;
   or a pharmaceutically acceptable salt thereof.
12. The compound of item 4, wherein R¹⁰ is R^{10A}, wherein R^{10A} is phenyl which is unfused and substituted with F, and further substituted with R¹¹, wherein R¹¹ is phenyl, pyrrolyl, azabicylclo[3.1.0]hexanyl, hexahydro-1H-isoindolyl, 1,3-oxazolidinyl, azepanyl, piperidinyl, imidazolidinyl, thiazolidinyl, thiazinyl, azetidinyl, 1,6-dihydropyridazyl, tetrahydropyrimidin(2H)-yl or azabicylo[2.2.1]hept-2-yl; each of which are independently unsubstituted or substituted with one or two or three of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I;
   wherein each R¹⁹ is R²⁰, R²¹, R²² or R²³;
   R²⁰ is phenyl, which is unfused;
   R²¹ is heteroaryl, which is unfused;
   R²² is cycloalkyl,or heterocycloalkyl each of which is unfused or fused with benzene;
   R²³ is alkyl which is unsubstituted or substituted with one or two of independently selected R²⁴, OR²⁴, NHR²⁴ N(R²⁴)₂, NHS(O)₂R¹⁹, OH, F, Cl, Br or I;
   wherein each R²⁴ is R^{24A} or R^{24B};
   R^{24A} is phenyl, cycloalkyl, heterocycloalkyl or heterocycloalkenyl; each of which is unfused or fused with heterocycloalkane;
   R^{24B} is alkyl which is unsubstituted or substituted with OR²⁵, OH, F, Cl, Br or I;
   R²⁵ is alkyl each of which is unsubstituted or substituted with NH₂;
   wherein the moieties represented by R²⁰, R²¹, R²², and R^{24A} are independently unsubstituted or substituted with one or two of independently selected R²⁶, OR²⁶, (O), F, Cl, Br or I; and
   R²⁶ is alkyl,
   or a pharmaceutically acceptable salt thereof.
13. The compound of item 4, wherein R¹⁰ is substituted with F, and further substituted with R¹⁴ wherein each R¹⁰ is independently unsubstituted or substituted with one or two or three of independently selected R¹¹, OR¹¹, SR¹¹, S(O)R¹¹, SO₂R¹¹, NH₂, NHR¹¹, N(R¹¹)₂, C(O)R¹¹, C(O)OR¹¹, C(O)NH₂, C(O)NHR¹¹, C(O)N(R¹¹)₂, NHC(O)R¹¹, NR¹¹C(O)R¹¹, NHSO₂R¹¹, NR¹¹SO₂R¹¹, NHC(O)OR¹¹, NR¹¹C(O)OR¹¹, NHSO₂NH₂, NHSO₂NHR¹¹, NHSO₂N(R¹¹)₂, SO₂NH₂, SO₂NHR¹¹, SO₂N(R¹¹)₂, NHC(O)NH₂, NHC(O)NHR¹¹, NHC(O)N(R¹¹)₂, NR¹¹C(O)N(R¹¹)₂, NO₂, OH, (O), C(O)H, C(O)OH, CN, CF₃, OCF₃, CF₂CF₃, F, Cl, Br or I;
   wherein R¹⁴ is pyrrolidinyl, azetidinyl, pyrrolyl, 1,3-oxazolidinyl, azepanyl, piperidinyl, imidazolidinyl, tetrahydropyrimidin(2H)-yl, azabicyclo[2.2.1]heptyl or 1,6-dihydropyridazyl; each of which unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene; and
   wherein the moiety represented by R¹⁴ is substituted with one or two (O) substituents;
   or a pharmaceutically acceptable salt thereof.
14. A compound having formula (Ik), 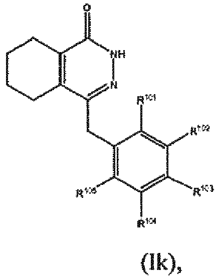
   or a pharmaceutically acceptable salt thereof, wherein R¹⁰¹, R¹⁰², R¹⁰³, R¹⁰⁴, and R¹⁰⁵, are independently selected from H, R¹¹, OR¹¹, SR¹¹, S(O)R¹¹, SO₂R¹¹, NH₂, N(R¹¹)₂, C(O)R¹¹, C(O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein each R¹¹ is R¹², R¹³, R¹⁴ or R¹⁵;
   R¹² is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R¹³ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R¹⁴ is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R¹⁵ is alkyl, alkenyl or alkynyl; each of which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NH₂, C(O)NHR¹⁶, C(O)N(R¹⁶)₂, NHC(O)R¹⁶, NR¹⁶C(O)R¹⁶, NHC(O)OR¹⁶, NR¹⁶C(O)OR¹⁶, OH, F, Cl, Br or I;
   wherein each R¹⁶ is R¹⁷ or R^{17A};
   R¹⁷ is alkyl, alkenyl or alkynyl; each of which is unsubstituted or substituted with one or two of independently selected R¹⁸, C(O)OH, NH₂,NHR¹⁸ or N(R¹⁸)₂, C(O)R¹⁸, C(O)NH₂, C(O)NHR¹⁸, C(O)N(R¹⁸)₂, NHC(O)R¹⁸, NR¹⁸C(O)R¹⁸, F, Cl, Br or 1;
   R^{17A} is phenyl, heteroaryl, cycloalkyl, cycloalkenyl, heterocycloalkyl, heterocycloalkenyl each of which is unfused or fused with benzene, heteroarene, cycloalkane, cycloalkene, heterocycloalkane or heterocycloalkene;
   wherein each R¹⁸ is phenyl, heteroaryl, cycloalkyl, cycloalkenyl, heterocycloalkyl or heterocycloalkenyl;
   wherein each of the moieties represented by R¹², R¹³, R¹⁴, R^{17A}, and R¹⁸ are independently unsubstituted or substituted with one or two or three or four of independently selected R¹⁹, OR¹⁹, SR¹⁹, S(O)R¹⁹, SO₂R¹⁹, C(O)R¹⁹, CO(O)R¹⁹, OC(O)R¹⁹, OC(O)OR¹⁹, NH₂, NHR¹⁹, N(R¹⁹)₂, NHC(O)R¹⁹, NR¹⁹C(O)R¹⁹, NHS(O)₂R¹⁹, NR¹⁹S(O)₂R¹⁹, NHC(O)OR¹⁹, NR¹⁹C(O)OR¹⁹, NHC(O)NH₂, NHC(O)NHR¹⁹, NHC(O)N(R¹⁹)₂, NR¹⁹C(O)NHR¹⁹, NR¹⁹C(O)N(R¹⁹)₂, C(O)NH₂, C(O)NHR¹⁹, C(O)N(R¹⁹)₂, C(O)NHOH, C(O)NHOR¹⁹, C(O)NHSO₂R¹⁹, C(O)NR¹⁹SO₂R¹⁹, SO₂NH₂, SO₂NHR¹⁹, SO₂N(R¹⁹)₂, C(O)H, C(O)OH, C(N)NH₂, C(N)NHR¹⁹, C(N)N(R¹⁹)₂, CNOH, CNOCH₃, OH, (O), CN, N₃, NO₂, CF₃, CF₂CF₃, OCF₃, OCF₂CF₃, F, Cl, Br or I;
   wherein each R¹⁹ is R²⁰, R²¹, R²² or R²³;
   R²⁰ is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R²¹ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R²² is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R²³ is alkyl, alkenyl or alkynyl; each of which is unsubstituted or substituted with one or two of independently selected R²⁴, OR²⁴, SR²⁴, S(O)₂R²⁴, C(O)OH, NH₂, NHR²⁴ N(R²⁴)₂, C(O)R²⁴, C(O)NH₂, C(O)NHR²⁴, C(O)N(R²⁴)₂, NHC(O)R²⁴, NR²⁴C(O)R²⁴, NHC(O)OR²⁴, NR²⁴C(O)OR²⁴, NHS(O)₂R²⁴, NR²⁴S(O)₂R²⁴, OH, F, Cl, Br or I;
   wherein each R²⁴ is R^{24A} or R^{24B};
   R^{24A} is phenyl, heteroaryl, cycloalkyl, cycloalkenyl, heterocycloalkyl or heterocycloalkenyl each of which is unfused or fused with benzene, heteroarene, cycloalkane, cycloalkene, heterocycloalkane or heterocycloalkene;
   R^{24B} is alkyl, alkenyl or alkynyl each of which is unsubstituted or substituted with one or two of independently selected R²⁵, OR²⁵, SR²⁵, S(O)₂R²⁵, C(O)OH, NH₂, NHR²⁵ N(R²⁵)₂, C(O)R²⁵, C(O)NH₂, C(O)NHR²⁵, C(O)N(R²⁵)₂, NHC(O)R²⁵, NR²⁵C(O)R²⁵, NHC(O)OR²⁵, NR²⁵C(O)OR²⁵, OH, F, Cl, Br or I;
   wherein each R²⁵ is alkyl, phenyl, heteroaryl, cycloalkyl, cycloalkenyl, heterocycloalkyl or heterocycloalkenyl ; each of which is unsubstituted or substituted with NH₂, NH(CH₃), N(CH₃)₂, OH or OCH₃;
   wherein each of the moieties represented by R²⁰, R²¹, R²², and R^{24A} are independently unsubstituted or substituted with one or two of independently selected R²⁶, OR²⁶, alkenyl, alkynyl, phenyl, OH, (O), C(O)OH, CN, CF₃, OCF₃, CF₂CF₃, F, Cl, Br or I; and
   R²⁶ is alkyl.
15. The compound of item 14, wherein R¹⁰³ is F; or a pharmaceutically acceptable salt thereof.
16. The compound of item 15, wherein R¹⁰¹, R¹⁰⁴ and R¹⁰⁵ are H; or a pharmaceutically acceptable salt thereof.
17. The compound of item 15, wherein R¹⁰² is NHC(O)R¹¹; or a pharmaceutically acceptable salt thereof.
18. The compound of item 17, wherein R¹¹ is R¹⁵, wherein R¹⁵ is alkyl, unsubstituted or substituted with one or two of independently selected R¹⁶, OR¹⁶, SR¹⁶, S(O)₂R₁₆, C(O)OH, NH₂, NHR¹⁶ N(R¹⁶)₂, C(O)R¹⁶, C(O)NHR¹⁶, NHC(O)R¹⁶, NHC(O)OR¹⁶, OH or Cl;
   wherein each R¹⁶ is R¹⁷ or R^{17A};
   R¹⁷ is alkyl, which is unsubstituted or substituted with one or two of independently selected R¹⁸;
   R^{17A} is phenyl, heteroaryl, cycloalkyl, heterocycloalkyl, heterocycloalkenyl each of which is unfused or fused with benzene or heterocycloalkane;
   wherein each R¹⁸ is phenyl or heterocycloalkyl;
   wherein each of the moieties represented by R^{17A} and R¹⁸ are independently unsubstituted or substituted with one or two or three or four of independently selected R¹⁹, OR¹⁹, SR¹⁹, SO₂R¹⁹, C(O)R¹⁹, CO(O)R¹⁹, NHR¹⁹, N(R¹⁹)₂, NHC(O)R¹⁹, NHS(O)₂R¹⁹, C(O)NH₂, C(O)NHR¹⁹, C(O)N(R¹⁹)₂, C(O)H, OH, (O), CN, CF₃, F, Cl, Br or I;
   wherein each R¹⁹ is R²⁰, R²¹, R²² or R²³;
   R²⁰ is phenyl which is unfused;
   R²¹ is heteroaryl which is unfused;
   R²² is cycloalkyl or heterocycloalkyl; each of which are unfused or fused with benzene;
   R²³ is alkyl, which is unsubstituted or substituted with one or two of independently selected R²⁴, OR²⁴, NHR²⁴ N(R²⁴)₂, NHS(O)₂R²⁴ or OH;
   wherein each R²⁴ is R^{24A} or R^{24B};
   R^{24A} is unsubstituted phenyl, cycloalkyl, heterocycloalkyl or heterocycloalkenyl each of which is unfused or fused with heterocycloalkane;
   R^{24B} is alkyl, which is unsubstituted or substituted with one or two of independently selected OR²⁵ or OH;
   wherein each R²⁵ is alkyl unsubstituted or substituted with NH₂;
   wherein each R²⁰ is unsubstituted or substituted with one or two of independently selected R²⁶, OR²⁶, (O), F or Cl; and
   R²⁶ is alkyl; or a pharmaceutically acceptable salt thereof.
19. The compound of item 16, wherein R¹⁰² is NHC(O)R¹¹.
20. The compound of item 15, wherein R¹⁰² is R¹¹, wherein R¹¹ is selected from pyrrolidinyl, oxazolyl, imidazolidinyl, isothiazolidinyl, piperidinyl, piperazinyl and azepanyl, wherein R¹⁰⁴ is substituted with one or two (O) substituents; or a pharmaceutically acceptable salt thereof.
21. The compound of item 20, wherein R¹¹ is pyrrolidinyl; or a pharmaceutically acceptable salt thereof.
22. The compound of item 16, wherein R¹⁰² is R¹¹, wherein R¹¹ is selected from pyrrolidinyl, oxazolyl, imidazolidinyl, isothiazolidinyl, piperidinyl, and azepanyl, wherein R¹⁰⁴ is substituted with one or two (O) substituents; or a pharmaceutically acceptable salt thereof.
23. The compound of item 22, wherein R¹¹ is pyrrolidinyl; or a pharmaceutically acceptable salt thereof.
24. A compound according to item 15 selected from
   2-fluoro-5-((4-oxo-3,4,5,6,7,8-hexahydrophthalazin-1-yl)methyl)benzoic acid;
   4-(3-amino-4-fluorobenzyl)-5,6,7,8-tetrahydrophthalazin-1(2H)-one;
   4-((2-fluoro-5-((4-oxo-3,4,5,6,7,8-hexahydrophthalazin-1-yl)methyl)phenyl)amino)-4-oxobutanoic acid;
   1-(2-fluoro-5-((4-oxo-3,4,5,6,7,8-hexahydrophthalazin-1-yl)methyl)phenyl)pyrrolidine-2,5-dione;
   4-(3-(1,4-diazepan-1-ylcarbonyl)-4-fluorobenzyl)-5,6,7,8-tetrahydrophthalazin-1(2H)-one;
   4-(3-(aminomethyl)-4-fluorobenzyl)-5,6,7,8-tetrahydrophthalazin-1(2H)-one;
   4-(3-((dimethylamino)methyl)-4-fluorohenzyl)-5,6,7,8-tetrahydrophthalazin-1(2H)-one;
   4-(4-fluoro-3-((isopropylamino)methyl)benzyl)-5,6,7,8-tetrahydrophthalazin-1(2H)-one;
   4-(3-((cyclohexylamino)methyl)-4-fluorobenzyl)-5,6,7,8-tetrahydrophthalazin-1(2H)-one;
   4-(4-fluoro-3-((tetrahydro-2H-pyran-4-ylamino)methyl)benzyl)-5,6,7,8-tetrahydrophthalazin-1(2H)-one;
   4-(4-fluoro-3-((methyl((1-methylpyrrolidin-3-yl)methyl)amino)methyl)benzyl)-5,6,7,8-tetrahydrophthalazin-1(2H)-one;
   4-(4-fluoro-3-((methyl(((2R)-1-methylpyrrolidin-2-yl)methyl)amino)methyl)benzyl)-5,6,7,8-tetrahydrophthalazin-1(2H)-one;
   4-(4-fluoro-3-pyrimidin-2-ylbenzyl)-5,6,7,8-tetrahydrophthalazin-1(2H)-one;
   4-(4-fluoro-3-pyridin-3-ylbenzyl)-5,6,7,8-tetiahydrophthalazin-1(2H)-one;
   4-(4-fluoro-3-pyridin-4-ylbenzyl)-5,6,7,8-tetrahydrophthalazin-1(2H)-one;
   N,N-diethyl-2'-fluoro-5'-((4-oxo-3,4,5,6,7,8-hexahydrophthalazin-1-yl)methyl)-1,1'-biphenyl-2-carboxamide;
   N-(2-fluoro-5-((4-oxo-3,4,5,6,7,8-hexahydrophthalazin-1-yl)methyl)phenyl)-3-piperidin-1-ylpropanamide;
   N-(2-fluoro-5-((4-oxo-3,4,5,6,7,8-hexahydrophthalazin-1-yl)methyl)phenyl)-3-(4-methylpiperazin-1-yl)propanamide;
   2-amino-N-(2-fluoro-5-((4-oxo-3,4,5,6,7,8-hexahydrophthalazin-1-yl)methyl)phenyl)acetamide;
   3-cyclohexyl-N-(2-fluoro-5-((4-oxo-3,4,5,6,7,8-hexahydrophthalazin-1-yl)methyl)phenyl)propanamide;
   N-(2-fluoro-5-((4-oxo-3,4,5,6,7,8-hexahydrophthalazin-1-yl)methyl)phenyl)piperidine-3-carboxamide;
   4-(4-fluoro-3-(2-oxopyrrolidin-1-yl)benzyl)-5,6,7,8-tetrahydrophthalazin-1(2H)-one;
   N-(2-fluoro-5-((4-oxo-3,4,5,6,7,8-hexahydrophthalazin-1-yl)methyl)phenyl)azetidine-3-carboxamide;
   N-(2-fluoro-5-((4-oxo-3,4,5,6,7,8-hexahydrophthalazin-1-yl)methyl)phenyl)-2-morpholin-4-ylacetamide;
   N-(2'-fluoro-5'-((4-oxo-3,4,5,6,7,8-hexahydrophthalazin-1-yl)methyl)-1,1'-biphenyl-3-yl)acetamide;
   4-((6-fluoro-3'-(methylsulfonyl)-1,1'-biphenyl-3-yl)methyl)-5,6,7,8-tetrahydrophthalazin-1(2H)-one;
   4-((6-fluoro-3'-(pyrrolidin-1-ylcarbonyl)-1,1'-biphenyl-3-yl)methyl)-5,6,7,8-tetrahydrophthalazin-1(2H)-one;
   4-((6-fluoro-4'-(pyrrolidin-1-ylcarbonyl)-1,1'-biphenyl-3-yl)methyl)-5,6,7,8-tetrahydrophthalazin-1(2H)-one;
   N,N-diethyl-2'-fluoro-5'-((4-oxo-3,4,5,6,7,8-hexahydiophthalazin-1-yl)methyl)-1,1' biphenyl-3-carboxamide;
   2'-fluoro-N(N-dimethyl-5'-((4-oxo-3,4)5,6,7,8-hexahydrophthalazin-1-yl)methyl)-1,1'-biphenyl-4-carboxamide;
   N-(2-fluoro-5-((4-oxo-3,4,5,6,7,8-hexahydrophthalazin-1-yl)methyl)phenyl)-4-(4-methoxyphenyl)-4-oxobutanamide;
   1-(2-fluoro-5-((4-oxo-3,4,5,6,7,8-hexahydroplithalazin-1-yl)methyl)phenyl)-3,4-dimethyl-1 H-pyrrole-2,5-dione;
   3-(2-fluoro-5-((4-oxo-3,4,5,6,7,8-hexahydrophthalazin-1-yl)methyl)phenyl)-3-azabicyclo(3.1.0)hexane-2,4-dione;
   2-(2-fluoro-5-((4-oxo-3,4,5,6,7,8-hexahydrophthalazin-1-yl)methyl)phenyl)hexahydro-1H-isoindole-1,3(2H)-dione;
   1-(2-fluoro-5-((4-oxo-3,4,5,6,7,8-hexahydiophthalazin-1-yl)methyl)phenyl)-3,3-dimethylpyrrolidine-2,5-dione;
   4-(4-fluoro-3-(2-methyl-5-oxopyrrolidin-1-yl)benzyl)-5,6,7,8-tetrahydrophthalazin-1(2H)-one;
   4-(4-fluoro-3-(2-oxo-1,3-oxazolidin-3-yl)benzyl)-5,6,7,8-tetrahydrophthalazin-1(2H)-one;
   4-(4-fluoro-3-(2-oxoazepan-1-yl)benzyl)-5,6,7,8-tetrahydrophthalazin-1(2H)-one;
   1-(2-fluoro-5-((4-oxo-3,4,5,6,7,8-hexahydrophthalazin-1-yl)methyl)phenyl)piperidine-2,6-dione;
   4-(4-fluoro-3-(2-oxoimidazolidin-1-yl)benzyl)-5,6,7,8-tetrahydrophthalazin-1(2H)-one;
   4-(3-(1,1-dioxidoisothiazolidin-2-yl)-4-fluorobenzyl)-5,6,7,8-tetrahydrophthalazin-1(2H)-one;
   4-(4-fluoro-3-(2-oxoazetidin-1-yl)benzyl)-5,6,7,8-tetrahydrophthalazin-1(2H)-one;
   4-(4-ftuoro-3-(2-oxopiperidin-1-yl)benzyl)-5,6,7,8-tetrahydrophthalazin-1 (2H)-one;
   4-(4-fluoro-3-(3-methyl-2-oxoimidazolidin-1-yl)benzyl)-5,6,7,8-tetrahydrophthalazin-1 (2H)-one;
   4-(4-fluoro-3-(2-oxotetrahydropyrimidin-1(2H)-yl)benzyl)-5,6,7,8-tetrahydrophthalazin-1(2H)-one;
   4-(3-(3-tert-butyl-2-oxoimidazolidin-1-yl)-4-fluorobenzyl)-5,6,7,8-tetrahydrophthalazin-1(2H)-one;
   4-(4-fluoro-3-((1S,4R)-3-oxo-2-azabicyclo(2.2.1)hept-2-yl)benzyl)-5,6,7,8-tetrahydrophthalazin-1(2H)-one;
   N-(2-fluoro-5-((4-oxo-3,4,5,6,7,8-hexahydrophthalazin-1-yl)methyl)phenyl)-N-methylmethanesulfonamide;
   N-(2-fluoro-5-((4-oxo-3,4,5,6,7,8-hexahydiophthalazin-1-yl)methyl)phenyl)-2-hydroxy-2-methylpropanamide;
   (3aS,4R,7S,7aR)-5-(2-fluoro-5-((4-oxo-3,4,5,6,7,8-hexahydrophthalazin-1-yl)methyl)phenyl)-2,2-dimethyltetrahydro-4,7-methano(1,3)dioxolo(4,5-c)pyridin-6(3aH)-one;
   4-(3-(1,1-dioxido-1,2-thiazinan-2-yl)-4-fluorobenzyl)-5,6,7,8-tetrahydrophthalazin-1(2H)-one;
   N-(2-fluoro-5-((4-oxo-3,4,5,6,7,8-hexahydrophthalazin-1-yl)methyl)phenyl)-2-(2-oxopyrrolidin-1-yl)acetamide;
   N-(2-fluoro-5-((4-oxo-3,4,5,6,7,8-hexahydrophthalazin-1-yl)methyl)phenyl)-5-methyl-1-phenyl-1H-pyrazole-4-carboxamide;
   N-(2-fluoro-5-((4-oxo-3,4,5,6,7,8-hexahydrophthalazin-1-yl)methyl)phenyl)-5-oxohexanamide;;
   N-(2-fluoro-5-((4-oxo-3,4,5,6,7,8-hexahydrophthalazin-1-yl)methyl)phenyl)-3-methoxypropanamide;
   N-(2-fluoro-5-((4-oxo-3,4,5,6,7,8-hexahydrophthalazin-1-yl)methyl)phenyl)-N'-phenylpentanediamide;
   4-(4-fluoro-3-((4-pyrimidin-2-ylpiperazin-1-yl)carbonyl)benzyl)-5,6,7,8-tetrahydrophthalazin-1(2H)-one; or
   4-(4-fluoro-3-(2-oxopyrrolidin-1-yl)phenyl)-5,6,7,8-tetrahydrophthalazin-1(2H)-one;
   or a pharmaceutically acceptable salt thereof.
25. The compound of item 15 selected from
   4-((2-fluoro-5-((4-oxo-3,4,5,6,7,8-hexahydrophthalazin-1-yl)methyl)phenyl)amino)-4-oxobutanoic acid;
   1-(2-fluoro-5-((4-oxo-3,4,5,6,7,8-hexahydrophthalazin-1-yl)methyl)phenyl)pyrrolidine-2,5-dione;
   4-(4-fluoro-3-(2-oxopyrrolidin-1-yl)benzyl)-5,6,7,8-tetrahydrophthalazin-1(2H)-one;
   N-(2-fluoro-5-((4-oxo-3,4,5,6,7,8-hexahydrophthalazin-1-yl)methyl)phenyl)-3-(4-methylpiperazin-1-yl)propanamide;
   3-(2-fluoro-5-((4-oxo-3,4,5,6,7,8-hexahydrophthalazin-1-yl)methyl)phenyl)-3-azabicyclo(3.1.0)hexane-2,4-dione;
   1-(2-fluoro-5-((4-oxo-3,4,5,6,7,8-hexahydrophthalazin-1-yl)methyl)phenyl)-3,3-dimethylpyrrolidine-2,5-dione;
   4-(4-fluoro-3-(2-oxo-1,3-oxazolidin-3-yl)benzyl)-5,6,7,8-tetrahydrophthalazin-1(2H)-one;
   4-(4-fluoro-3-(3-methyl-2-oxoimidazolidin-1-yl)benzyl)-5,6,7,8-tetrahydrophthalazin-1(2H)-one;
   4-(4-fluoro-3-(2-oxoazepan-1-yl)benzyl)-5,6,7,8-tetrahydrophthalazin-1(2H)-one;
   1-(2-fluoro-5-((4-oxo-3,4,5,6,7,8-hexahydrophthalazin-1-yl)methyl)phenyl)piperidine-2,6-dione;
   4-(3-(1,1-dioxidoisothiazolidin-2-yl)-4-fluorobenzyl)-5,6,7,8-tetrahydrophthalazin-1(2H)-one;
   4-(4-fluoro-3-(2-oxopiperidin-1-yl)benzyl)-5,6,7,8-tetrahydrophthalazin-1(2H)-one; or
   4-(4-fluoro-3-((4-pyrimidin-2-ylpiperazin-1-yl)carbonyl)benzyl)-5,6,7,8-tetrahydrophthalazin-1(2H)-one;
   or a pharmaceutically acceptable salt thereof.
26. A compound having formula (Iv), 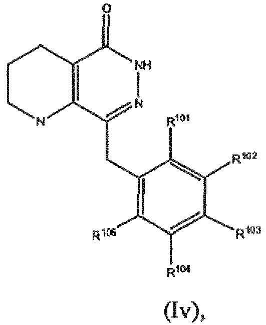
   or a pharmaceutically acceptable salt thereof, wherein R¹⁰¹, R¹⁰², R¹⁰³, R¹⁰⁴, and R¹⁰⁵, are independently selected from H, R¹¹, OR¹¹, SR¹¹, S(O)R¹¹, SO₂R¹¹, NH₂, N(R¹¹)₂, C(O)R¹¹, C{O)OR¹¹, C(O)NHR¹¹, C(O)N(R¹¹)₂, NHC(O)R¹¹, NHSO₂R¹¹, NR¹¹SO₂R¹¹, NHC(O)OR¹¹, NHSO₂N(R¹¹)₂, NO₂, OH, (O), C(O)OH, F, Cl or Br; wherein each R¹¹ is R¹², R¹³, R¹⁴ or R¹⁵;
   R¹² is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R¹³ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R¹⁴ is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R¹⁵ is alkyl, alkenyl or alkynyl; each of which is unsubstituted or substituted with one or two of independently selected R¹⁶, OR¹⁶, SR¹⁶, S(O)₂R¹⁶, C(O)OH, NH₂, NHR¹⁶ N(R¹⁶)₂, C(O)R¹⁶, C(O)NH₂, C(O)NHR¹⁶, C(O)N(R¹⁶)₂, NHC(O)R¹⁶, NR¹⁶C(O)R¹⁶, NHC(O)OR¹⁶, NR¹⁶C(O)OR¹⁶ OH, F, Cl, Br or I;
   wherein each R¹⁶ is R¹⁷ or R^{17A};
   R¹⁷ is alkyl, alkenyl or alkynyl; each of which is unsubstituted or substituted with one or two of independently selected R¹⁸, C(O)OH, NH₂, NHR¹⁸ or N(R¹⁸)₂, C(O)R¹⁸, C(O)NH₂, C(O)NHR¹⁸, C(O)N(R¹⁸)₂, NHC(O)R¹⁸, NR¹⁸C(O)R¹⁸, F, Cl, Br or I;
   R^{17A} is phenyl, heteroaryl, cycloalkyl, cycloalkenyl, heterocycloalkyl, heterocycloalkenyl each of which is unfused or fused with benzene, heteroarene, cycloalkane, cycloalkene, heterocycloalkane or heterocycloalkene;
   wherein each R¹⁸ is phenyl, heteroaryl, cycloalkyl, cycloalkenyl, heterocycloalkyl or heterocycloalkenyl;
   wherein each of the moieties represented by R¹², R¹³, R¹⁴, R^{17A}, and R¹⁸ are independently unsubstituted or substituted with one or two or three or four of independently selected R¹⁹, OR¹⁹, SR¹⁹, S(O)R¹⁹, SO₂R¹⁹, C(O)R¹⁹, CO(O)R¹⁹, OC(O)R¹⁹, OC(O)OR¹⁹, NH₂, NHR¹⁹, N(R¹⁹)₂, NHC(O)R¹⁹, NR¹⁹C(O)R¹⁹, NHS(O)₂R¹⁹, NR¹⁹S(O)₂R¹⁹, NHC(O)OR¹⁹, NR¹⁹C(O)OR¹⁹, NHC(O)NH₂, NHC(O)NHR¹⁹, NHC(O)N(R¹⁹)₂, NR¹⁹C(O)NHR¹⁹,NR¹⁹C(O)N(R¹⁹)₂, C(O)NH₂, C(O)NHR¹⁹, C(O)N(R¹⁹)₂, C(O)NHOH, C(O)NHOR¹⁹, C(O)NHSO₂R¹⁹, C(O)NR¹⁹SO₂R¹⁹, SO₂NH₂, SO₂NHR¹⁹, SO₂N(R¹⁹)₂, C(O)H, C(O)OH, C(N)NH₂, C(N)NHR¹⁹, C(N)N(R¹⁹)₂, CNOH, CNOCH₃, OH, (O), CN, N₃, NO₂, CF₃, CF₂CF₃, OCF₃, OCF₂CF₃, F, Cl, Br or I;
   wherein each R¹⁹ is R²⁰, R²¹, R²² or R²³;
   R²⁰ is phenyl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R²¹ is heteroaryl which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R²² is cycloalkyl, cycloalkenyl, heterocycloalkyl or heterocycloalkenyl; each of which is unfused or fused with benzene, heteroarene, cycloalkane, cycloalkene, heterocycloalkane or heterocycloalkene; each of which is unfused or fused with benzene, heteroarene, cycloalkane, cycloalkene, heterocycloalkane or heterocycloalkene;
   R²³ is alkyl, alkenyl or alkynyl; each of which is unsubstituted or substituted with one or two of independently selected R²⁴, OR²⁴, SR²⁴, S(O)₂R²⁴, C(O)OH, NN₂, NHR²⁴ N(R²⁴)₂, C(O)R²⁴, C(O)NH₂, C(O)NHR²⁴, C(O)N(R²⁴)₂, NHC(O)R²⁴, NR²⁴C(O)R²⁴, NHC(O)OR²⁴, NR²⁴C(O)OR²⁴, NHS(O)₂R²⁴, NR²⁴S(O)₂R²⁴, OH, F, Cl, Br or I;
   wherein each R²⁴ is R^{24A} or R^{24B};
   R^{24A} is phenyl, heteroaryl, cycloalkyl, cycloalkenyl, heterocycloalkyl or heterocycloalkenyl each of which is unfused or fused with benzene, heteroarene, cycloalkane, cycloalkene, heterocycloalkane or heterocyctaalkene;
   R^{24B} is alkyl, alkenyl or alkynyl each of which is unsubstituted or substituted with one or two of independently selected R²⁵, OR²⁵, SR²⁵, S(O)₂R²⁵, C(O)OH, NH₂, NHR²⁵ N(R²⁵)₂, C(O)R²⁵, C(O)NH₂, C(O)NHR²⁵, C(O)N(R²⁵)₂, NHC(O)R²⁵, NR²⁵C(O)R²⁵, NHC(O)OR²⁵, NR²⁵C(O)OR²⁵, OH, F, Cl, Br or I;
   wherein each R²⁵ is alkyl, phenyl, heteroatyl, cycloalkyl, cycloalkenyl, heterocycloalkyl or heterocycloalkenyl ; each of which is unsubstituted or substituted with NH₂, NH(CH₃), N(CH₃)₂, OH or OCH₃;
   wherein each of the moieties represented by R²⁰, R²¹, R²², and R^{24A} are independently unsubstituted or substituted with one or two of independently selected R²⁶, OR²⁶, alkenyl, alkynyl, phenyl, OH, (O), C(O)OH, CN, CF₃, OCF₃, CF₂CF₃, F, Cl, Br or I; and
   R²⁶ is alkyl.
27. The compound of item 26, wherein R¹⁰³ is F; or a pharmaceutically acceptable salt thereof.
28. The compound of item 27 selected from
   8-(4-fluorobenzyl)-2,3,4,6-tetrahydropyrido(2,3-d)pyridazin-5(1H)-one;
   8-(4-fluoro-3-(2-oxoazetidin-1-yl)benzyl)-2,3,4,6-tetrahydropyrido(2,3-d)pyridazin-5(1H)-one;
   8-(3-chloro-4-fluorobenzyl)-2,3,4,6-tetrahydropyrido(2,3-d)pyridazin-5(1H)-one
   8-(4-fluoro-3-(2-oxopyrrolidin-1-yl)benzyl)-2,3,4,6-tetrahydropyrido(2,3-d)pyridazin-5(1H)-one;
   methyl 2-fluoro-5-((5-oxo-1,2,3,4,5,6-hexahydropyrido(2,3-d)pyridazin-8-yl)methyl)benzoate;
   8-(3-amino-4-fluorobenzyl)-2,3,4,6-tetrahydropyrido(2,3-d)pyridazin-5(1H)-one;
   2-fluoro-5-((5-oxo-1,2,3,4,5,6-hexahydropyrido(2,3-d)pyridazin-8-yl)methyl)benzoic acid;
   N-ethyl-2-fluoro-5-((5-oxo-1,2,3,4,5,6-hexahydropyrido(2,3-d)pyridazin-8-yl)methyl)benzamide;
   N-cyclobutyl-2-fluoro-5-((5-oxo-1,2,3,4,5,6-hexahydropyrido(2,3-d)pyridazin-8-yl)methyl)benzamide;
   2-fluoro-5-((5-oxo-1,2,3,4,5,6-hexahydropyrido(2,3-d)pyridazin-8-yl)methyl)-N-(2-pyrrolidin-1-ylethyl)benzamide;
   8-(4-fluoro-3-((4-(morpholin-4-ylcarbonyl)piperazin-1-yl)carbonyl)benzyl)-2,3,4,6-tetrahydropyrido(2,3-d)pyridazin-5(1H)-one;
   N-(2-fluoro-5-((5-oxo-1,2,3,4,5,6-hexahydropyrido(2,3-d)pyridazin-8-yl)methyl)phenyl)-N'-phenylpentanediamide;
   1-(2-fluoro-5-((5-oxo-1,2,3,4,5,6-hexahydropyrido(2,3-d)pyridazin-8-yl)methyl)phenyl)pynolidine-2,5-dione;
   N-(2-fluoro-5-((5-oxo-1,2,3,4,5,6-hexahydropyrido(2,3-d)pyridazin-8-yl)methyl)phenyl)-3-methoxypropanamide;
   N-(2-fluoro-5-((5-oxo-1,2,3,4,5(6-hexahydropyrido(2,3-d)pyridazin-8-yl)methyl)phenyl)-5-oxohexanamide;
   N-(2-fluoro-5-((5-oxo-1,2,3,4,5,6-hexahydropyrido(2,3-d)pyridazin-8-yl)methyl)phenyl)-3-phenoxypropanamide;
   N-(2-fluoro-5-((5-oxo-1,2,3,4,5,6-hexahydropyrido(2,3-d)pyridazin-8-yl)methyl)phenyl)-4-oxo-4-phenylbutanamide;
   2-(4-(benzyloxy)phenoxy)-N-(2-fluoro-5-((5-oxo-1,2,3,4,5,6-hexahydropyrido(2,3-d)pyridazin-8-yl)methyl)phenyl)acetamide;
   N-(2-fluoro-5-((5-oxo-1,2,3,4,5,6-hexahydropyrido(2,3-d)pyridazin-8-yl)methyl)phenyl)-2-(4-methoxyphenoxy)acetamide;
   N-cyclopropyl-2-fluoro-5-((5-oxo-1,2,3,4,5,6-hexahydropyrido(2,3-d)pyridazin-8-yl)methyl)benzamide;
   8-(3-((4-(2-ethoxyethyl)piperazin-1-yl)carbonyl)-4-fluorobenzyl)-2,3,4,6-tetrahydropyrido(2,3-d)pyridazin-5(1H)-one; or
   2-fluoro-5-((5-oxo-1,2,3,4,5,6-hexahydropyrido(2,3-d)pyridazin-8-yl)methyl)-N-(2-piperidin-1-ylethyl)benzamide;
   or a pharmaceutically acceptable salt thereof.
29. The compound 1-(2-fluoro-5-((4-oxo-3,4,5,6,7,8-hexahydrophthalazin-1-yl)methyl)phenyl)pyrrolidine-2,5-dione; or a pharmaceutically acceptable salt thereof.
30. The compound 4-(3-(1,4-diazepan-1-ylcarbonyl)-4-fluorobenzyl)-5,6,7,8-tetrahydrophthalazin-1(2H)-one; or a pharmaceutically acceptable salt thereof.
31. The compound 4-((2-fluoro-5-((4-oxo-3,4,5,6,7,8-hexahydrophthalazin-1-yl)methyl)phenyl)amino)-4-oxobutanoic acid; or a pharmaceutically acceptable salt thereof.
31. A pharmaceutical composition comprising a compound of item 1 and pharmaceutically acceptable excipient.
32. A method of treating cancer in a mammal comprising administering thereto a therapeutically acceptable amount of a compound of item 1.
33. A method for decreasing tumor volume in a mammal comprising administering thereto a therapeutically acceptable amount of a compound of item 1.
34. A method of treating cancer in a mammal comprising administering thereto a therapeutically acceptable amount of a compound of item 1 in combination with radiotherapy.
35. A method of treating cancer in a mammal comprising administering thereto a therapeutically acceptable amount of a compound of item 1 in combination with a chemotherapeutic agent selected from temozolomide, dacarbazine, cyclophosphamide, carmustine, melphalan, lomustine, carboplatin, cisplatin, 5-FU +/- leucovorin, gemcitabine, methotrexate, bleomycin, irinotecan, camptothecin, or topotecan.

## Claims

1. A compound selected from the group consisting of 1-(2-fluoro-5-((4-oxo-3,4,5,6,7,8-hexahydrophthalazin-1-yl)methyl)phenyl)pyrrolidine-2,5-dione and 4-(3-(1,4-diazepan-1-ylcarbonyl)-4-fluorobenzyl)-5,6,7,8-tetrahydrophthalazin-1(2H)-one, or a pharmaceutically acceptable salt thereof, for use in treating leukemia, colon cancer, glioblastomas, lymphomas, melanomas, carcinomas of the breast or cervical carcinomas in a mammal by administering thereto a therapeutically acceptable amount of said compound.

## Patentansprüche

1. Eine Verbindung gewählt aus der Gruppe bestehend aus 1-(2-Fluoro-5-((4-oxo-3,4,5,6,7,8-hexahydrophthalazin-1-yl)methyl)phenyl)pyrrolidin-2,5-dion und 4-(3-(1,4-Diazepan-1-ylcarbonyl)-4-fluorobenzyl)-5,6,7,8-tetrahydrophthalazin-1(2H)-on, oder ein pharmazeutisch verträgliches Salz davon, für die Verwendung in der Behandlung von Leukämie, Kolonkarzinom, Glioblastomen, Lymphomen, Melanomen, Karzinomen der Brust oder Zervixkarzinomen in einem Säugetier, durch Verabreichung einer therapeutisch verträglichen Menge der Verbindung an dieses.

## Revendications

1. Composé choisi dans le groupe consistant en la 1-(2-fluoro-5-((4-oxo-3,4,5,6,7,8-hexahydrophtalazin-1-yl)méthyl)phényl)pyrrolidine-2,5-dione et la 4-(3-(1,4-diazépan-1-ylcarbonyl)-4-fluorobenzyl)-5,6,7,8-tétrahydraphtalazin-1(2H)-one, ou sel pharmaceutiquement acceptable de celui-ci, pour une utilisation dans le traitement de leucémie, cancer du côlon, glioblastomes, lymphomes, mélanomes, cancers du sein ou carcinomes cervicaux chez un mammifère par administration à celui-ci d'une quantité thérapeutiquement acceptable dudit composé.
